(19) Europäisches Patentamt
European Patent Office
Office européen des brevets

(11) EP 1 487 979 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**19.12.2018 Patentblatt 2018/51**

(45) Hinweis auf die Patenterteilung:
**26.08.2009 Patentblatt 2009/35**

(21) Anmeldenummer: **03712028.4**

(22) Anmeldetag: **17.03.2003**

(51) Int Cl.:
***C12N 15/11*** *(2006.01)* ***C12N 15/82*** *(2006.01)*

(86) Internationale Anmeldenummer:
**PCT/EP2003/002735**

(87) Internationale Veröffentlichungsnummer:
**WO 2003/078629 (25.09.2003 Gazette 2003/39)**

(54) **KONSTRUKTE UND VERFAHREN ZUR REGULATION DER GENEXPRESSION**

CONSTRUCTS AND METHODS FOR THE REGULATION OF GENE EXPRESSION

PRODUIT DE SYNTHESE ET PROCEDE DE REGULATION DE L'EXPRESSION GENIQUE

(84) Benannte Vertragsstaaten:
**AT BE BG CH CY CZ DE DK EE ES FI FR GB GR HU IE IT LI LU MC NL PT RO SE SI SK TR**

(30) Priorität: **20.03.2002 DE 10212892**

(43) Veröffentlichungstag der Anmeldung:
**22.12.2004 Patentblatt 2004/52**

(73) Patentinhaber: **BASF Plant Science GmbH**
**67056 Ludwigshafen (DE)**

(72) Erfinder:
- **KOCK, Michael**
  **67105 Schifferstadt (DE)**
- **BAUER, Jörg**
  **67063 Ludwigshafen (DE)**

(74) Vertreter: **Herzog, Fiesser & Partner**
**Patentanwälte PartG mbB**
**Dudenstrasse 46**
**68167 Mannheim (DE)**

(56) Entgegenhaltungen:
**WO-A-02/00894 WO-A-93/23551**
**WO-A-03/076620**

- **FIRE A ET AL: "Potent and specific genetic interference by double-stranded RNA in Caenorhabditis elegans" NATURE, MACMILLAN JOURNALS LTD. LONDON, GB, Bd. 391, 19. Februar 1998 (1998-02-19), Seiten 806-811, XP002095876 ISSN: 0028-0836 in der Anmeldung erwähnt**
- **FIRE A: "RNA-triggered gene silencing" TRENDS IN GENETICS, ELSEVIER SCIENCE PUBLISHERS B.V. AMSTERDAM, NL, Bd. 15, Nr. 9, 1. September 1999 (1999-09-01), Seiten 358-363, XP004176656 ISSN: 0168-9525**
- **MONTGOMERY ET AL: "RNA as a target of double-stranded RNA-mediated genetic interference in Caenorhabditis elegans" PROCEEDINGS OF THE NATIONAL ACADEMY OF SCIENCES OF USA, NATIONAL ACADEMY OF SCIENCE. WASHINGTON, US, Bd. 95, Dezember 1998 (1998-12), Seiten 15502-15507, XP002138441 ISSN: 0027-8424**
- **WESLEY S VARSHA ET AL: "Construct design for efficient, effective and high-throughput gene silencing in plants" PLANT JOURNAL, BLACKWELL SCIENTIFIC PUBLICATIONS, OXFORD, GB, Bd. 27, Nr. 6, September 2001 (2001-09), Seiten 581-590, XP002187670 ISSN: 0960-7412**

EP 1 487 979 B2

SEQUENZPROTOKOLL

<110> BASF Plant Science GmbH

<120> Konstrukte und Verfahren zur Regulation der Genexpression

<130> PD009300062-AT

<140>
<141>

<160> 126

<170> PatentIn Ver. 2.1

<210> 1
<211> 495
<212> DNA
<213> Arabidopsis thaliana

<220>
<221> CDS
<221> CDS
<222> (1)..(492)
<223> albumine 2S subunit 1

<400> 1

```
atg gca aac aag ttg ttc ctc gtc tgc gca gct ctc gct ctc tgc ttc      48
Met Ala Asn Lys Leu Phe Leu Val Cys Ala Ala Leu Ala Leu Cys Phe
 1               5                  10                  15

ctc ctc acc aac gct tcc atc tac cgc acc gtc gtt gag ttc gaa gaa      96
Leu Leu Thr Asn Ala Ser Ile Tyr Arg Thr Val Val Glu Phe Glu Glu
            20                  25                  30

gat gac gcc act aac ccc ata ggc cca aaa atg agg aaa tgc cgc aag     144
Asp Asp Ala Thr Asn Pro Ile Gly Pro Lys Met Arg Lys Cys Arg Lys
        35                  40                  45

gag ttt cag aaa gaa caa cac cta aga gct tgc cag caa ttg atg ctc     192
Glu Phe Gln Lys Glu Gln His Leu Arg Ala Cys Gln Gln Leu Met Leu
    50                  55                  60

cag caa gca agg caa ggc cgt agc gat gag ttt gat ttc gaa gac gac     240
Gln Gln Ala Arg Gln Gly Arg Ser Asp Glu Phe Asp Phe Glu Asp Asp
65                  70                  75                  80

atg gag aac cca cag gga caa cag cag gaa caa cag cta ttc cag cag     288
Met Glu Asn Pro Gln Gly Gln Gln Gln Glu Gln Gln Leu Phe Gln Gln
                85                  90                  95

tgc tgc aac gag ctt cgc cag gaa gag cca gat tgt gtt tgc ccc acc     336
Cys Cys Asn Glu Leu Arg Gln Glu Glu Pro Asp Cys Val Cys Pro Thr
            100                 105                 110

ttg aaa caa gct gcc aag gcc gtt aga ctc cag gga cag cac caa cca     384
Leu Lys Gln Ala Ala Lys Ala Val Arg Leu Gln Gly Gln His Gln Pro
        115                 120                 125

atg caa gtc agg aaa att tac cag aca gcc aag cac ttg ccc aac gtt     432
Met Gln Val Arg Lys Ile Tyr Gln Thr Ala Lys His Leu Pro Asn Val
    130                 135                 140

tgc gac atc ccg caa gtt gat gtt tgt ccc ttc aac atc cct tca ttc     480
Cys Asp Ile Pro Gln Val Asp Val Cys Pro Phe Asn Ile Pro Ser Phe
145                 150                 155                 160

cct tct ttc tac taa                                                 495
Pro Ser Phe Tyr
```

<210> 2
<211> 164
<212> PRT
<213> Arabidopsis thaliana

<400> 2

```
Met Ala Asn Lys Leu Phe Leu Val Cys Ala Ala Leu Ala Leu Cys Phe
1               5                   10                  15

Leu Leu Thr Asn Ala Ser Ile Tyr Arg Thr Val Val Glu Phe Glu Glu
            20                  25                  30

Asp Asp Ala Thr Asn Pro Ile Gly Pro Lys Met Arg Lys Cys Arg Lys
        35                  40                  45

Glu Phe Gln Lys Glu Gln His Leu Arg Ala Cys Gln Gln Leu Met Leu
    50                  55                  60

Gln Gln Ala Arg Gln Gly Arg Ser Asp Glu Phe Asp Phe Glu Asp Asp
65                  70                  75                  80

Met Glu Asn Pro Gln Gly Gln Gln Gln Glu Gln Gln Leu Phe Gln Gln
                85                  90                  95

Cys Cys Asn Glu Leu Arg Gln Glu Glu Pro Asp Cys Val Cys Pro Thr
            100                 105                 110

Leu Lys Gln Ala Ala Lys Ala Val Arg Leu Gln Gly Gln His Gln Pro
        115                 120                 125

Met Gln Val Arg Lys Ile Tyr Gln Thr Ala Lys His Leu Pro Asn Val
    130                 135                 140

Cys Asp Ile Pro Gln Val Asp Val Cys Pro Phe Asn Ile Pro Ser Phe
145                 150                 155                 160

Pro Ser Phe Tyr
```

<210> 3
<211> 495
<212> DNA
<213> Arabidopsis thaliana

<220>
<221> CDS
<222> (1)..(492)
<223> albumine 2S subunit 3

<400> 3

```
atg gct aac aag ctc ttc ctc gtc tgc gca act ctc gcc ctc tgc ttc      48
Met Ala Asn Lys Leu Phe Leu Val Cys Ala Thr Leu Ala Leu Cys Phe
1               5                   10                  15

ctc ctc acc aac gct tcc atc tac cgc acc gtt gtc gaa ttc gaa gaa      96
Leu Leu Thr Asn Ala Ser Ile Tyr Arg Thr Val Val Glu Phe Glu Glu
            20                  25                  30

gat gac gcc agc aac ccc gta ggt cca aga cag aga tgc cag aag gag      144
Asp Asp Ala Ser Asn Pro Val Gly Pro Arg Gln Arg Cys Gln Lys Glu
        35                  40                  45

ttt cag caa tca caa cac cta aga gct tgc cag aga tgg atg agc aag      192
Phe Gln Gln Ser Gln His Leu Arg Ala Cys Gln Arg Trp Met Ser Lys
    50                  55                  60
```

```
caa atg agg caa gga cgt ggt ggt ggt cct tcc ctc gac gat gag ttc      240
Gln Met Arg Gln Gly Arg Gly Gly Gly Pro Ser Leu Asp Asp Glu Phe
 65                  70                  75                  80

gat ttc gag ggc ccc cag cag gga tac cag cta ctc cag cag tgc tgc      288
Asp Phe Glu Gly Pro Gln Gln Gly Tyr Gln Leu Leu Gln Gln Cys Cys
                 85                  90                  95

aac gag ctt cgc cag gaa gag cca gtt tgc gtt tgc ccc acc ttg aaa      336
Asn Glu Leu Arg Gln Glu Glu Pro Val Cys Val Cys Pro Thr Leu Lys
            100                 105                 110

caa gct gcc agg gca gtt agc ctc cag gga cag cac gga cca ttc caa      384
Gln Ala Ala Arg Ala Val Ser Leu Gln Gly Gln His Gly Pro Phe Gln
        115                 120                 125

tcc agg aaa att tac cag tca gct aag tac ttg cct aac att tgc aag      432
Ser Arg Lys Ile Tyr Gln Ser Ala Lys Tyr Leu Pro Asn Ile Cys Lys
    130                 135                 140

atc cag caa gtt ggt gaa tgt ccc ttc cag acc acc atc cct ttc ttc      480
Ile Gln Gln Val Gly Glu Cys Pro Phe Gln Thr Thr Ile Pro Phe Phe
145                 150                 155                 160

cct cct tac tac tag                                                  495
Pro Pro Tyr Tyr
```

<210> 4
<211> 164
<212> PRT
<213> Arabidopsis thaliana

<400> 4

```
Met Ala Asn Lys Leu Phe Leu Val Cys Ala Thr Leu Ala Leu Cys Phe
1               5                   10                  15

Leu Leu Thr Asn Ala Ser Ile Tyr Arg Thr Val Val Glu Phe Glu Glu
            20                  25                  30

Asp Asp Ala Ser Asn Pro Val Gly Pro Arg Gln Arg Cys Gln Lys Glu
        35                  40                  45

Phe Gln Gln Ser Gln His Leu Arg Ala Cys Gln Arg Trp Met Ser Lys
    50                  55                  60

Gln Met Arg Gln Gly Arg Gly Gly Gly Pro Ser Leu Asp Asp Glu Phe
65                  70                  75                  80

Asp Phe Glu Gly Pro Gln Gln Gly Tyr Gln Leu Leu Gln Gln Cys Cys
                85                  90                  95

Asn Glu Leu Arg Gln Glu Glu Pro Val Cys Val Cys Pro Thr Leu Lys
            100                 105                 110

Gln Ala Ala Arg Ala Val Ser Leu Gln Gly Gln His Gly Pro Phe Gln
        115                 120                 125

Ser Arg Lys Ile Tyr Gln Ser Ala Lys Tyr Leu Pro Asn Ile Cys Lys
    130                 135                 140

Ile Gln Gln Val Gly Glu Cys Pro Phe Gln Thr Thr Ile Pro Phe Phe
145                 150                 155                 160

Pro Pro Tyr Tyr
```

<210> 5
<211> 513
<212> DNA
<213> Arabidopsis thaliana

<220>
<221> CDS
<222> (1) .. (510)
<223> albumine 2S subunit 2

<400> 5

```
atg gca aac aag ctc ttc ctc gtc tgc gca act ttc gcc ctc tgc ttc      48
Met Ala Asn Lys Leu Phe Leu Val Cys Ala Thr Phe Ala Leu Cys Phe
1               5                   10                  15

ctc ctc acc aac gct tcc atc tac cgc act gtt gtc gag ttc gac gaa      96
Leu Leu Thr Asn Ala Ser Ile Tyr Arg Thr Val Val Glu Phe Asp Glu
            20                  25                  30

gat gac gcc agc aac ccc atg ggc cca aga cag aaa tgt cag aag gag     144
Asp Asp Ala Ser Asn Pro Met Gly Pro Arg Gln Lys Cys Gln Lys Glu
        35                  40                  45

ttt cag caa tca cag cac cta aga gct tgc cag aaa ttg atg cgc atg     192
Phe Gln Gln Ser Gln His Leu Arg Ala Cys Gln Lys Leu Met Arg Met
    50                  55                  60

caa atg agg caa ggc cgt ggt ggt ggt ccc tcc ctc gac gat gag ttc     240
Gln Met Arg Gln Gly Arg Gly Gly Gly Pro Ser Leu Asp Asp Glu Phe
65                  70                  75                  80

gat ttg gaa gac gac atc gag aac cca caa ggc ccc cag cag gga cac     288
Asp Leu Glu Asp Asp Ile Glu Asn Pro Gln Gly Pro Gln Gln Gly His
                85                  90                  95

cag atc ctc cag cag tgc tgc agc gag ctt cgc cag gaa gag cca gtt     336
Gln Ile Leu Gln Gln Cys Cys Ser Glu Leu Arg Gln Glu Glu Pro Val
            100                 105                 110

tgt gtt tgc ccc acc ttg aga caa gct gcc agg gcc gtt agc ctc cag     384
Cys Val Cys Pro Thr Leu Arg Gln Ala Ala Arg Ala Val Ser Leu Gln
        115                 120                 125

gga caa cac gga cca ttc caa tcc agg aaa att tac aag aca gct aag     432
Gly Gln His Gly Pro Phe Gln Ser Arg Lys Ile Tyr Lys Thr Ala Lys
    130                 135                 140

tac ttg cct aac att tgc aag atc cag caa gtt ggt gaa tgc ccc ttc     480
Tyr Leu Pro Asn Ile Cys Lys Ile Gln Gln Val Gly Glu Cys Pro Phe
145                 150                 155                 160

cag acc acc atc cct ttc ttc cct cct tac taa                          513
Gln Thr Thr Ile Pro Phe Phe Pro Pro Tyr
                165                 170
```

<210> 6
<211> 170
<212> PRT
<213> Arabidopsis thaliana

<400> 6

Met Ala Asn Lys Leu Phe Leu Val Cys Ala Thr Phe Ala Leu Cys Phe
1 5 10 15

Leu Leu Thr Asn Ala Ser Ile Tyr Arg Thr Val Val Glu Phe Asp Glu
20 25 30

Asp Asp Ala Ser Asn Pro Met Gly Pro Arg Gln Lys Cys Gln Lys Glu
35 40 45

Phe Gln Gln Ser Gln His Leu Arg Ala Cys Gln Lys Leu Met Arg Met
50 55 60

Gln Met Arg Gln Gly Arg Gly Gly Gly Pro Ser Leu Asp Asp Glu Phe
65 70 75 80

Asp Leu Glu Asp Asp Ile Glu Asn Pro Gln Gly Pro Gln Gln Gly His
85 90 95

Gln Ile Leu Gln Gln Cys Cys Ser Glu Leu Arg Gln Glu Glu Pro Val
100 105 110

Cys Val Cys Pro Thr Leu Arg Gln Ala Ala Arg Ala Val Ser Leu Gln
115 120 125

Gly Gln His Gly Pro Phe Gln Ser Arg Lys Ile Tyr Lys Thr Ala Lys
130 135 140

Tyr Leu Pro Asn Ile Cys Lys Ile Gln Gln Val Gly Glu Cys Pro Phe
145 150 155 160

Gln Thr Thr Ile Pro Phe Phe Pro Pro Tyr
165 170

<210> 7
<211> 501
<212> DNA
<213> Arabidopsis thaliana

<220>
<221> CDS
<222> (1) .. (498)
<223> albumine 2S subunit 4

<400> 7

```
atg gcg aac aag ctc ttc ctc gtc tgc gca gct ctc gcc ctg tgt ttc    48
Met Ala Asn Lys Leu Phe Leu Val Cys Ala Ala Leu Ala Leu Cys Phe
 1               5                  10                  15

atc ctc acc aac gct tcc gtc tat cgc acc gtt gtc gag ttc gac gaa    96
Ile Leu Thr Asn Ala Ser Val Tyr Arg Thr Val Val Glu Phe Asp Glu
            20                  25                  30

gat gac gcc agt aac ccc ata ggc cca ata cag aaa tgt cag aag gag   144
Asp Asp Ala Ser Asn Pro Ile Gly Pro Ile Gln Lys Cys Gln Lys Glu
        35                  40                  45

ttt cag caa gac cag cac cta aga gct tgc cag aga tgg atg cgc aag   192
Phe Gln Gln Asp Gln His Leu Arg Ala Cys Gln Arg Trp Met Arg Lys
    50                  55                  60

caa atg tgg caa gga cgt ggt ggt ggt cct tcc ctc gac gat gag ttc   240
Gln Met Trp Gln Gly Arg Gly Gly Gly Pro Ser Leu Asp Asp Glu Phe
65                  70                  75                  80

gat atg gaa gac gac atc gag aac ccg cag aga cga cag cta ctc cag   288
Asp Met Glu Asp Asp Ile Glu Asn Pro Gln Arg Arg Gln Leu Leu Gln
                85                  90                  95

aag tgc tgc agc gag ctt cgc caa gaa gag cca gtt tgc gtt tgc ccc   336
Lys Cys Cys Ser Glu Leu Arg Gln Glu Glu Pro Val Cys Val Cys Pro
            100                 105                 110

acc ttg aga caa gct gcc aag gcc gtt aga ttc cag gga cag caa cac   384
Thr Leu Arg Gln Ala Ala Lys Ala Val Arg Phe Gln Gly Gln Gln His
        115                 120                 125

caa cca gag caa gtc agg aaa att tac cag gca gct aag tac ttg cct   432
Gln Pro Glu Gln Val Arg Lys Ile Tyr Gln Ala Ala Lys Tyr Leu Pro
    130                 135                 140

aac att tgc aaa atc cag caa gtt ggt gtt tgc ccc ttc cag atc cct   480
Asn Ile Cys Lys Ile Gln Gln Val Gly Val Cys Pro Phe Gln Ile Pro
145                 150                 155                 160

tca atc cct tct tac tac taa                                       501
Ser Ile Pro Ser Tyr Tyr
                165
```

<210> 8
<211> 166
<212> PRT
<213> Arabidopsis thaliana

<400> 8

```
Met Ala Asn Lys Leu Phe Leu Val Cys Ala Ala Leu Ala Leu Cys Phe
 1               5                  10                  15

Ile Leu Thr Asn Ala Ser Val Tyr Arg Thr Val Val Glu Phe Asp Glu
            20                  25                  30

Asp Asp Ala Ser Asn Pro Ile Gly Pro Ile Gln Lys Cys Gln Lys Glu
        35                  40                  45

Phe Gln Gln Asp Gln His Leu Arg Ala Cys Gln Arg Trp Met Arg Lys
    50                  55                  60

Gln Met Trp Gln Gly Arg Gly Gly Gly Pro Ser Leu Asp Asp Glu Phe
65                  70                  75                  80

Asp Met Glu Asp Asp Ile Glu Asn Pro Gln Arg Arg Gln Leu Leu Gln
                85                  90                  95

Lys Cys Cys Ser Glu Leu Arg Gln Glu Glu Pro Val Cys Val Cys Pro
            100                 105                 110

Thr Leu Arg Gln Ala Ala Lys Ala Val Arg Phe Gln Gly Gln Gln His
        115                 120                 125

Gln Pro Glu Gln Val Arg Lys Ile Tyr Gln Ala Ala Lys Tyr Leu Pro
    130                 135                 140

Asn Ile Cys Lys Ile Gln Gln Val Gly Val Cys Pro Phe Gln Ile Pro
145                 150                 155                 160

Ser Ile Pro Ser Tyr Tyr
                165
```

<210> 9
<211> 1473
<212> DNA
<213> Brassica napus

<220>
<221> CDS
<222> (1)..(1470)
<223> cruciferin

<400> 9

```
atg gct cgg ctc tca tct ctt ctc tct ttt tcc tta gca ctt ttg atc     48
Met Ala Arg Leu Ser Ser Leu Leu Ser Phe Ser Leu Ala Leu Leu Ile
 1               5                  10                  15
```

ttt ctc cat ggc tct aca gct caa cag ttt cca aac gag tgt cag cta 96
Phe Leu His Gly Ser Thr Ala Gln Gln Phe Pro Asn Glu Cys Gln Leu
20 25 30

gac cag ctc aat gca ctg gag ccg tca cac gta ctt aag gct gag gct 144
Asp Gln Leu Asn Ala Leu Glu Pro Ser His Val Leu Lys Ala Glu Ala
35 40 45

ggt cgc atc gag gtg tgg gac cac cac gct cct cag cta cgt tgc tct 192
Gly Arg Ile Glu Val Trp Asp His His Ala Pro Gln Leu Arg Cys Ser
50 55 60

ggt gtc tcc ttt gta cgt tac atc atc gag tct aag ggt ctc tac ttg 240
Gly Val Ser Phe Val Arg Tyr Ile Ile Glu Ser Lys Gly Leu Tyr Leu
65 70 75 80

ccc tct ttc ttt agc acc gcg aag ctc tcc ttc gtg gct aaa gga gaa 288
Pro Ser Phe Phe Ser Thr Ala Lys Leu Ser Phe Val Ala Lys Gly Glu
85 90 95

ggt ctt atg ggg aga gtg gtc cct gga tgc gcc gag aca ttc cag gac 336
Gly Leu Met Gly Arg Val Val Pro Gly Cys Ala Glu Thr Phe Gln Asp
100 105 110

tca tca gtg ttt caa cca agc ggt ggt agc ccc tcg gga gaa ggt cag 384
Ser Ser Val Phe Gln Pro Ser Gly Gly Ser Pro Ser Gly Glu Gly Gln
115 120 125

ggc caa gga caa caa ggt cag ggc caa ggc cac caa ggt caa ggc caa 432
Gly Gln Gly Gln Gln Gly Gln Gly Gln Gly His Gln Gly Gln Gly Gln
130 135 140

gga caa cag ggc caa caa ggt cag caa gga caa cag agt caa ggc cag 480
Gly Gln Gln Gly Gln Gln Gly Gln Gln Gly Gln Gln Ser Gln Gly Gln
145 150 155 160

ggc ttc cgt gat atg cac cag aaa gtg gag cac ata agg act ggg gac 528
Gly Phe Arg Asp Met His Gln Lys Val Glu His Ile Arg Thr Gly Asp
165 170 175

acc atc gct aca cat ccc ggt gta gcc caa tgg ttc tac aac gac gga 576
Thr Ile Ala Thr His Pro Gly Val Ala Gln Trp Phe Tyr Asn Asp Gly
180 185 190

aac caa cca ctt gtc atc gtt tcc gtc ctc gat tta gcc agc cac cag 624
Asn Gln Pro Leu Val Ile Val Ser Val Leu Asp Leu Ala Ser His Gln
195 200 205

aat cag ctc gac cgc aac cca agg cca ttt tac tta gcc gga aac aac 672
Asn Gln Leu Asp Arg Asn Pro Arg Pro Phe Tyr Leu Ala Gly Asn Asn
210 215 220

cca caa ggc caa gta tgg ata gaa gga cgc gag caa cag cca caa aag 720
Pro Gln Gly Gln Val Trp Ile Glu Gly Arg Glu Gln Gln Pro Gln Lys
225 230 235 240

aac atc ctt aat ggc ttc aca cca gag gtt ctt gct aaa gct ttc aag 768
Asn Ile Leu Asn Gly Phe Thr Pro Glu Val Leu Ala Lys Ala Phe Lys
245 250 255

atc gat gtt agg aca gcg caa caa ctt cag aac cag caa gac aac cgt 816
Ile Asp Val Arg Thr Ala Gln Gln Leu Gln Asn Gln Gln Asp Asn Arg
260 265 270

gga aac att atc cga gtc caa ggc cca ttc agt gtc att agg ccg cct 864
Gly Asn Ile Ile Arg Val Gln Gly Pro Phe Ser Val Ile Arg Pro Pro
275 280 285

```
ttg agg agt cag aga ccg cag gag aca gaa gtt aac ggt tta gaa gag     912
Leu Arg Ser Gln Arg Pro Gln Glu Thr Glu Val Asn Gly Leu Glu Glu
    290                 295                 300

acc ata tgc agc gcg agg tgc acc gat aac ctc gat gac cca tct aat     960
Thr Ile Cys Ser Ala Arg Cys Thr Asp Asn Leu Asp Asp Pro Ser Asn
305                 310                 315                 320

gct gac gta tac aag cca cag ctc ggt tac atc agc act ctg aac agc    1008
Ala Asp Val Tyr Lys Pro Gln Leu Gly Tyr Ile Ser Thr Leu Asn Ser
                325                 330                 335

tat gat ctc ccc atc ctt cgc ttc ctt cgt ctc tca gcc ctc cgt gga    1056
Tyr Asp Leu Pro Ile Leu Arg Phe Leu Arg Leu Ser Ala Leu Arg Gly
            340                 345                 350

tct atc cgt caa aac gcg atg gtg ctt cca cag tgg aac gca aac gca    1104
Ser Ile Arg Gln Asn Ala Met Val Leu Pro Gln Trp Asn Ala Asn Ala
        355                 360                 365

aac gcg gtt ctc tac gtg aca gac ggg gaa gcc cat gtg cag gtg gtt    1152
Asn Ala Val Leu Tyr Val Thr Asp Gly Glu Ala His Val Gln Val Val
    370                 375                 380

aac gac aac ggt gac aga gtg ttc gac gga caa gtc tct caa gga cag    1200
Asn Asp Asn Gly Asp Arg Val Phe Asp Gly Gln Val Ser Gln Gly Gln
385                 390                 395                 400

cta ctt tcc ata cca caa ggt ttc tcc gtg gtg aaa cgc gca aca agc    1248
Leu Leu Ser Ile Pro Gln Gly Phe Ser Val Val Lys Arg Ala Thr Ser
                405                 410                 415

gaa cag ttc cgg tgg atc gag ttc aag aca aac gca aac gca cag atc    1296
Glu Gln Phe Arg Trp Ile Glu Phe Lys Thr Asn Ala Asn Ala Gln Ile
            420                 425                 430

aac aca ctt gct gga cga acc tcg gtc ttg aga ggt tta cca tta gag    1344
Asn Thr Leu Ala Gly Arg Thr Ser Val Leu Arg Gly Leu Pro Leu Glu
        435                 440                 445

gtc ata tcc aat ggg tac caa atc tca ctc gaa gaa gca aga agg gtt    1392
Val Ile Ser Asn Gly Tyr Gln Ile Ser Leu Glu Glu Ala Arg Arg Val
    450                 455                 460

aag ttc aac acg atc gag acc act ttg acg cac agc agt ggc cca gct    1440
Lys Phe Asn Thr Ile Glu Thr Thr Leu Thr His Ser Ser Gly Pro Ala
465                 470                 475                 480

agc tac gga ggg cca agg aag gct gat gct taa                        1473
Ser Tyr Gly Gly Pro Arg Lys Ala Asp Ala
                485                 490
```

<210> 10
<211> 490
<212> PRT
<213> Brassica napus

<400> 10

```
Met Ala Arg Leu Ser Ser Leu Leu Ser Phe Ser Leu Ala Leu Leu Ile
 1               5                  10                  15
Phe Leu His Gly Ser Thr Ala Gln Gln Phe Pro Asn Glu Cys Gln Leu
            20                  25                  30
Asp Gln Leu Asn Ala Leu Glu Pro Ser His Val Leu Lys Ala Glu Ala
        35                  40                  45
```

Gly Arg Ile Glu Val Trp Asp His His Ala Pro Gln Leu Arg Cys Ser
50 55 60

Gly Val Ser Phe Val Arg Tyr Ile Ile Glu Ser Lys Gly Leu Tyr Leu
65 70 75 80

Pro Ser Phe Phe Ser Thr Ala Lys Leu Ser Phe Val Ala Lys Gly Glu
85 90 95

Gly Leu Met Gly Arg Val Val Pro Gly Cys Ala Glu Thr Phe Gln Asp
100 105 110

Ser Ser Val Phe Gln Pro Ser Gly Gly Ser Pro Ser Gly Glu Gly Gln
115 120 125

Gly Gln Gly Gln Gln Gly Gln Gly Gln Gly His Gln Gly Gln Gly Gln
130 135 140

Gly Gln Gln Gly Gln Gln Gly Gln Gln Gly Gln Gln Ser Gln Gly Gln
145 150 155 160

Gly Phe Arg Asp Met His Gln Lys Val Glu His Ile Arg Thr Gly Asp
165 170 175

Thr Ile Ala Thr His Pro Gly Val Ala Gln Trp Phe Tyr Asn Asp Gly
180 185 190

Asn Gln Pro Leu Val Ile Val Ser Val Leu Asp Leu Ala Ser His Gln
195 200 205

Asn Gln Leu Asp Arg Asn Pro Arg Pro Phe Tyr Leu Ala Gly Asn Asn
210 215 220

Pro Gln Gly Gln Val Trp Ile Glu Gly Arg Glu Gln Gln Pro Gln Lys
225 230 235 240

Asn Ile Leu Asn Gly Phe Thr Pro Glu Val Leu Ala Lys Ala Phe Lys
245 250 255

Ile Asp Val Arg Thr Ala Gln Gln Leu Gln Asn Gln Gln Asp Asn Arg
260 265 270

Gly Asn Ile Ile Arg Val Gln Gly Pro Phe Ser Val Ile Arg Pro Pro
275 280 285

Leu Arg Ser Gln Arg Pro Gln Glu Thr Glu Val Asn Gly Leu Glu Glu
290 295 300

Thr Ile Cys Ser Ala Arg Cys Thr Asp Asn Leu Asp Asp Pro Ser Asn
305 310 315 320

Ala Asp Val Tyr Lys Pro Gln Leu Gly Tyr Ile Ser Thr Leu Asn Ser
325 330 335

Tyr Asp Leu Pro Ile Leu Arg Phe Leu Arg Leu Ser Ala Leu Arg Gly
340 345 350

Ser Ile Arg Gln Asn Ala Met Val Leu Pro Gln Trp Asn Ala Asn Ala
355 360 365

Asn Ala Val Leu Tyr Val Thr Asp Gly Glu Ala His Val Gln Val Val
370 375 380

Asn Asp Asn Gly Asp Arg Val Phe Asp Gly Gln Val Ser Gln Gly Gln
385 390 395 400

Leu Leu Ser Ile Pro Gln Gly Phe Ser Val Val Lys Arg Ala Thr Ser
405 410 415

Glu Gln Phe Arg Trp Ile Glu Phe Lys Thr Asn Ala Asn Ala Gln Ile
420 425 430

Asn Thr Leu Ala Gly Arg Thr Ser Val Leu Arg Gly Leu Pro Leu Glu
435 440 445

Val Ile Ser Asn Gly Tyr Gln Ile Ser Leu Glu Glu Ala Arg Arg Val
450 455 460

Lys Phe Asn Thr Ile Glu Thr Thr Leu Thr His Ser Ser Gly Pro Ala
465 470 475 480

Ser Tyr Gly Gly Pro Arg Lys Ala Asp Ala
485 490

<210> 11
<211> 1467
<212> DNA
<213> Brassica napus

<220>
<221> CDS
<222> (1)..(1464)
<223> cruciferin

<400> 11

```
atg gct cgg ctc tca tct ctt ctc tct ttt tcc tta gca ctt ttg act      48
Met Ala Arg Leu Ser Ser Leu Leu Ser Phe Ser Leu Ala Leu Leu Thr
 1               5                  10                  15

ttt ctc cat ggc tct aca gct caa cag ttt cca aac gag tgt cag cta      96
Phe Leu His Gly Ser Thr Ala Gln Gln Phe Pro Asn Glu Cys Gln Leu
            20                  25                  30

gac cag ctc aat gca ctg gag ccg tca cac gta ctt aag gct gag gct     144
Asp Gln Leu Asn Ala Leu Glu Pro Ser His Val Leu Lys Ala Glu Ala
        35                  40                  45

ggt cgc atc gag gtg tgg gac cac cac gct cct cag cta cgt tgc tct     192
Gly Arg Ile Glu Val Trp Asp His His Ala Pro Gln Leu Arg Cys Ser
    50                  55                  60

ggt gtc tcc ttt gta cgt tac atc atc gag tct aag ggt ctc tac ttg     240
Gly Val Ser Phe Val Arg Tyr Ile Ile Glu Ser Lys Gly Leu Tyr Leu
65                  70                  75                  80

ccc tct ttc ttt agc acc gcg agg ctc tcc ttc gtg gct aaa gga gaa     288
Pro Ser Phe Phe Ser Thr Ala Arg Leu Ser Phe Val Ala Lys Gly Glu
                85                  90                  95

ggt ctt atg ggg aga gtg gtc ctg tgc gcc gag aca ttc cag gac tca     336
Gly Leu Met Gly Arg Val Val Leu Cys Ala Glu Thr Phe Gln Asp Ser
            100                 105                 110

tca gtg ttt caa cca agc ggt ggt agc ccc ttc gga gaa ggt cag ggc     384
Ser Val Phe Gln Pro Ser Gly Gly Ser Pro Phe Gly Glu Gly Gln Gly
        115                 120                 125

caa gga caa caa ggt cag ggc caa ggc cac caa ggt caa ggc caa gga     432
Gln Gly Gln Gln Gly Gln Gly Gln Gly His Gln Gly Gln Gly Gln Gly
    130                 135                 140

caa cag ggc caa caa ggt cag caa gga caa cag agt caa ggc cag ggt     480
Gln Gln Gly Gln Gln Gly Gln Gln Gly Gln Gln Ser Gln Gly Gln Gly
145                 150                 155                 160

ttc cgt gat atg cac cag aaa gtg gag cac ata agg act ggg gac acc     528
Phe Arg Asp Met His Gln Lys Val Glu His Ile Arg Thr Gly Asp Thr
                165                 170                 175
```

```
atc gct aca cat ccc ggt gta gcc caa tgg ttc tac aac gac gga aac    576
Ile Ala Thr His Pro Gly Val Ala Gln Trp Phe Tyr Asn Asp Gly Asn
            180                 185                 190

caa cca ctt gtc atc gtt tcc gtc ctc gat tta gcc agc cac cag aat    624
Gln Pro Leu Val Ile Val Ser Val Leu Asp Leu Ala Ser His Gln Asn
        195                 200                 205

cag ctc gac cgc aac cca agg cca ttt tac tta gcc gga aac aac cca    672
Gln Leu Asp Arg Asn Pro Arg Pro Phe Tyr Leu Ala Gly Asn Asn Pro
    210                 215                 220

caa ggc caa gta tgg ata gaa gga cgc gag caa cag cca caa aag aac    720
Gln Gly Gln Val Trp Ile Glu Gly Arg Glu Gln Gln Pro Gln Lys Asn
225                 230                 235                 240

atc ctt aat ggc ttc aca cca gag gtt ctt gct aaa gct ttc aag atc    768
Ile Leu Asn Gly Phe Thr Pro Glu Val Leu Ala Lys Ala Phe Lys Ile
                245                 250                 255

gat gtt agg aca gcg caa caa ctt cag aac cag caa gac aac cgt gga    816
Asp Val Arg Thr Ala Gln Gln Leu Gln Asn Gln Gln Asp Asn Arg Gly
            260                 265                 270

aac att atc cga gtc caa ggc cca ttc agt gtc att agg ccg cct ttg    864
Asn Ile Ile Arg Val Gln Gly Pro Phe Ser Val Ile Arg Pro Pro Leu
        275                 280                 285

agg agt cag aga ccg cag gag gaa gtt aac ggt tta gaa gag acc ata    912
Arg Ser Gln Arg Pro Gln Glu Glu Val Asn Gly Leu Glu Glu Thr Ile
    290                 295                 300

tgc agc gcg agg tgc acc gat aac ctc gat gac cca tct aat gct gac    960
Cys Ser Ala Arg Cys Thr Asp Asn Leu Asp Asp Pro Ser Asn Ala Asp
305                 310                 315                 320

gta tac aag cca cag ctc ggt tac atc agc act ctg aac agc tat gat    1008
Val Tyr Lys Pro Gln Leu Gly Tyr Ile Ser Thr Leu Asn Ser Tyr Asp
                325                 330                 335

ctc ccc atc ctt cgc ttc ctt cgt ctc tca gcc ctc cgt gga tct atc    1056
Leu Pro Ile Leu Arg Phe Leu Arg Leu Ser Ala Leu Arg Gly Ser Ile
            340                 345                 350

cgt caa aac gcg atg gtg ctt cca cag tgg aac gca aac gca aac gcg    1104
Arg Gln Asn Ala Met Val Leu Pro Gln Trp Asn Ala Asn Ala Asn Ala
        355                 360                 365

gtt ctc tac gtg aca gac ggg gaa gcc cat gtg cag gtg gtt aac gac    1152
Val Leu Tyr Val Thr Asp Gly Glu Ala His Val Gln Val Val Asn Asp
    370                 375                 380

aac ggt gac aga gtg ttc gac gga caa gtc tct caa gga cag cta ctt    1200
Asn Gly Asp Arg Val Phe Asp Gly Gln Val Ser Gln Gly Gln Leu Leu
385                 390                 395                 400

tcc ata cca caa ggt ttc tcc gtg gtg aaa cgc gca aca agc gaa cag    1248
Ser Ile Pro Gln Gly Phe Ser Val Val Lys Arg Ala Thr Ser Glu Gln
                405                 410                 415

ttc cgg tgg atc gag ttc aag aca aac gca aac gca cag atc aac aca    1296
Phe Arg Trp Ile Glu Phe Lys Thr Asn Ala Asn Ala Gln Ile Asn Thr
            420                 425                 430

ctt gct gga cga acc tcg gtc ttg aga ggt tta cca tta gag gtc ata    1344
Leu Ala Gly Arg Thr Ser Val Leu Arg Gly Leu Pro Leu Glu Val Ile
        435                 440                 445
```

```
tcc aat ggg tac caa atc tca ctc gaa gaa gca aga agg gtt aag ttc     1392
Ser Asn Gly Tyr Gln Ile Ser Leu Glu Glu Ala Arg Arg Val Lys Phe
    450                 455                 460

aac acg atc gag acc act ttg acg cac agc agt ggc cca gct agc tac     1440
Asn Thr Ile Glu Thr Thr Leu Thr His Ser Ser Gly Pro Ala Ser Tyr
465                 470                 475                 480

gga ggg cca agg aag gct gat gct taa                                 1467
Gly Gly Pro Arg Lys Ala Asp Ala
                485
```

<210> 12
<211> 488
<212> PRT
<213> Brassica napus

<400> 12

```
Met Ala Arg Leu Ser Ser Leu Leu Ser Phe Ser Leu Ala Leu Leu Thr
1               5                   10                  15

Phe Leu His Gly Ser Thr Ala Gln Gln Phe Pro Asn Glu Cys Gln Leu
            20                  25                  30

Asp Gln Leu Asn Ala Leu Glu Pro Ser His Val Leu Lys Ala Glu Ala
        35                  40                  45

Gly Arg Ile Glu Val Trp Asp His His Ala Pro Gln Leu Arg Cys Ser
    50                  55                  60

Gly Val Ser Phe Val Arg Tyr Ile Ile Glu Ser Lys Gly Leu Tyr Leu
65                  70                  75                  80

Pro Ser Phe Phe Ser Thr Ala Arg Leu Ser Phe Val Ala Lys Gly Glu
                85                  90                  95

Gly Leu Met Gly Arg Val Val Leu Cys Ala Glu Thr Phe Gln Asp Ser
            100                 105                 110

Ser Val Phe Gln Pro Ser Gly Gly Ser Pro Phe Gly Glu Gly Gln Gly
        115                 120                 125

Gln Gly Gln Gln Gly Gln Gly Gln Gly His Gln Gly Gln Gly Gln Gly
    130                 135                 140

Gln Gln Gly Gln Gln Gly Gln Gln Gly Gln Gln Ser Gln Gly Gln Gly
145                 150                 155                 160

Phe Arg Asp Met His Gln Lys Val Glu His Ile Arg Thr Gly Asp Thr
                165                 170                 175

Ile Ala Thr His Pro Gly Val Ala Gln Trp Phe Tyr Asn Asp Gly Asn
            180                 185                 190

Gln Pro Leu Val Ile Val Ser Val Leu Asp Leu Ala Ser His Gln Asn
        195                 200                 205

Gln Leu Asp Arg Asn Pro Arg Pro Phe Tyr Leu Ala Gly Asn Asn Pro
    210                 215                 220

Gln Gly Gln Val Trp Ile Glu Gly Arg Glu Gln Gln Pro Gln Lys Asn
225                 230                 235                 240

Ile Leu Asn Gly Phe Thr Pro Glu Val Leu Ala Lys Ala Phe Lys Ile
                245                 250                 255

Asp Val Arg Thr Ala Gln Gln Leu Gln Asn Gln Gln Asp Asn Arg Gly
            260                 265                 270
```

```
Asn Ile Ile Arg Val Gln Gly Pro Phe Ser Val Ile Arg Pro Pro Leu
        275                 280                 285

Arg Ser Gln Arg Pro Gln Glu Glu Val Asn Gly Leu Glu Glu Thr Ile
    290                 295                 300

Cys Ser Ala Arg Cys Thr Asp Asn Leu Asp Asp Pro Ser Asn Ala Asp
305                 310                 315                 320

Val Tyr Lys Pro Gln Leu Gly Tyr Ile Ser Thr Leu Asn Ser Tyr Asp
                325                 330                 335

Leu Pro Ile Leu Arg Phe Leu Arg Leu Ser Ala Leu Arg Gly Ser Ile
            340                 345                 350

Arg Gln Asn Ala Met Val Leu Pro Gln Trp Asn Ala Asn Ala Asn Ala
        355                 360                 365

Val Leu Tyr Val Thr Asp Gly Glu Ala His Val Gln Val Val Asn Asp
    370                 375                 380

Asn Gly Asp Arg Val Phe Asp Gly Gln Val Ser Gln Gly Gln Leu Leu
385                 390                 395                 400

Ser Ile Pro Gln Gly Phe Ser Val Val Lys Arg Ala Thr Ser Glu Gln
                405                 410                 415

Phe Arg Trp Ile Glu Phe Lys Thr Asn Ala Asn Ala Gln Ile Asn Thr
            420                 425                 430

Leu Ala Gly Arg Thr Ser Val Leu Arg Gly Leu Pro Leu Glu Val Ile
        435                 440                 445

Ser Asn Gly Tyr Gln Ile Ser Leu Glu Glu Ala Arg Arg Val Lys Phe
    450                 455                 460

Asn Thr Ile Glu Thr Thr Leu Thr His Ser Ser Gly Pro Ala Ser Tyr
465                 470                 475                 480

Gly Gly Pro Arg Lys Ala Asp Ala
                485
```

<210> 13
<211> 1491
<212> DNA
<213> Brassica napus

<220>
<221> CDS
<222> (1)..(1488)
<223> cruciferin BnC2

<400> 13

```
atg gct cga ctc tcg tct ctt ctc tat ttt tcg ata aca gtt ttg atc    48
Met Ala Arg Leu Ser Ser Leu Leu Tyr Phe Ser Ile Thr Val Leu Ile
 1               5                  10                  15
ttt ctc cat ggc tct aca gct caa cag ttt cca aac gag tgc caa cta    96
Phe Leu His Gly Ser Thr Ala Gln Gln Phe Pro Asn Glu Cys Gln Leu
            20                  25                  30
gac cag ctc aat gcg ctg gag ccg tca cac gta ctt aag gcc gag gct   144
Asp Gln Leu Asn Ala Leu Glu Pro Ser His Val Leu Lys Ala Glu Ala
        35                  40                  45
ggt cgc atc gaa gtg tgg gac cac cac gct cct cag cta cgc tgc tct   192
Gly Arg Ile Glu Val Trp Asp His His Ala Pro Gln Leu Arg Cys Ser
    50                  55                  60
```

```
ggt gtc tcc ttc gta cgt tac ata atc gag tct cag ggt cta tac ttg    240
Gly Val Ser Phe Val Arg Tyr Ile Ile Glu Ser Gln Gly Leu Tyr Leu
 65                 70                  75                  80

ccc tct ttc tta aat acc gcg aac gtc tct ttc gtt gct aaa gga caa    288
Pro Ser Phe Leu Asn Thr Ala Asn Val Ser Phe Val Ala Lys Gly Gln
                 85                  90                  95

ggc ctt atg ggg aga gtg gtc cct gga tgc gct gag act ttc cag gac    336
Gly Leu Met Gly Arg Val Val Pro Gly Cys Ala Glu Thr Phe Gln Asp
            100                 105                 110

tca tca gta ttc caa cca ggc agt ggc agc ccc ttc gga gaa ggt caa    384
Ser Ser Val Phe Gln Pro Gly Ser Gly Ser Pro Phe Gly Glu Gly Gln
        115                 120                 125

ggc caa ggt cag cag ggt cag ggg caa ggt cag ggt cag ggt caa ggc    432
Gly Gln Gly Gln Gln Gly Gln Gly Gln Gly Gln Gly Gln Gly Gln Gly
    130                 135                 140

aag ggc caa cag ggt caa ggc aag ggc caa cag ggt caa tcc cag ggc    480
Lys Gly Gln Gln Gly Gln Gly Lys Gly Gln Gln Gly Gln Ser Gln Gly
145                 150                 155                 160

caa cag ggt caa ggt caa ggt ttc cgt gat atg cac cag aaa gta gag    528
Gln Gln Gly Gln Gly Gln Gly Phe Arg Asp Met His Gln Lys Val Glu
                165                 170                 175

cac ata agg agc ggc gac acc att gct aca cat ccc ggt gta gct caa    576
His Ile Arg Ser Gly Asp Thr Ile Ala Thr His Pro Gly Val Ala Gln
            180                 185                 190

tgg ttc tac aac aat gga aac caa cct ctt gtc atc gtt gcc gtc atg    624
Trp Phe Tyr Asn Asn Gly Asn Gln Pro Leu Val Ile Val Ala Val Met
        195                 200                 205

gat tta gct agc cac cag aac cag ctt gac cgc aac cca agc caa ttt    672
Asp Leu Ala Ser His Gln Asn Gln Leu Asp Arg Asn Pro Ser Gln Phe
    210                 215                 220

tac tta gca gga aaa aac cca caa ggc caa tca tgg cta cac gga cga    720
Tyr Leu Ala Gly Lys Asn Pro Gln Gly Gln Ser Trp Leu His Gly Arg
225                 230                 235                 240

ggg caa cag cca caa aac aac atc ctt aat ggc ttc tct cca gag gtt    768
Gly Gln Gln Pro Gln Asn Asn Ile Leu Asn Gly Phe Ser Pro Glu Val
                245                 250                 255

ctt gct caa gcg ttc aag atc gat gtt agg aca gcg caa caa ctt cag    816
Leu Ala Gln Ala Phe Lys Ile Asp Val Arg Thr Ala Gln Gln Leu Gln
            260                 265                 270

aac cag caa gat aac cgg gga aac att gtc cgt gtc caa ggc ccc ttc    864
Asn Gln Gln Asp Asn Arg Gly Asn Ile Val Arg Val Gln Gly Pro Phe
        275                 280                 285

ggt gtt att agg ccg cca ttg aaa agc cag aga cca cag gag aca gaa    912
Gly Val Ile Arg Pro Pro Leu Lys Ser Gln Arg Pro Gln Glu Thr Glu
    290                 295                 300

gct aac ggt cta gaa gag acc ata tgc agc gca agg tgc acg gat aac    960
Ala Asn Gly Leu Glu Glu Thr Ile Cys Ser Ala Arg Cys Thr Asp Asn
305                 310                 315                 320

ctc gat gac cca tct aac gcg gat gtg tat aag cca cag ctt ggt tac   1008
Leu Asp Asp Pro Ser Asn Ala Asp Val Tyr Lys Pro Gln Leu Gly Tyr
                325                 330                 335
```

```
atc agc att ctt aac agt tat gat cta ccc atc ctt cgc gta ctt cgc    1056
Ile Ser Ile Leu Asn Ser Tyr Asp Leu Pro Ile Leu Arg Val Leu Arg
            340                 345                 350

ctc tca gcc ctc cgt gga tca atc cgt caa aat gca atg gtt ctt cca    1104
Leu Ser Ala Leu Arg Gly Ser Ile Arg Gln Asn Ala Met Val Leu Pro
        355                 360                 365

cag tgg aag tca aag tca aac gcg gtt ctc tac gtg aca gac ggg gaa    1152
Gln Trp Lys Ser Lys Ser Asn Ala Val Leu Tyr Val Thr Asp Gly Glu
    370                 375                 380

gcc caa ata cag gtg gtt aac gac aac ggt gac aga gtg ttc gat gga    1200
Ala Gln Ile Gln Val Val Asn Asp Asn Gly Asp Arg Val Phe Asp Gly
385                 390                 395                 400

caa gtc tct caa ggg cag cta ctt tcc att cca caa gga ttc tcc gtt    1248
Gln Val Ser Gln Gly Gln Leu Leu Ser Ile Pro Gln Gly Phe Ser Val
                405                 410                 415

gtg aaa cgc gca aca agc gat cag ttc agg tgg ata gaa ttc aag aca    1296
Val Lys Arg Ala Thr Ser Asp Gln Phe Arg Trp Ile Glu Phe Lys Thr
            420                 425                 430

aac gca aac gcc cag atc aac act ctt gct gga cgt acc tca gtc atg    1344
Asn Ala Asn Ala Gln Ile Asn Thr Leu Ala Gly Arg Thr Ser Val Met
        435                 440                 445

aga ggt tta cca tta gag gtc ata gcc aat ggg tac caa atc tca ctt    1392
Arg Gly Leu Pro Leu Glu Val Ile Ala Asn Gly Tyr Gln Ile Ser Leu
    450                 455                 460

gaa gaa gca aga agg gtt aag ttc aac aca ata gag acc act ttg acc    1440
Glu Glu Ala Arg Arg Val Lys Phe Asn Thr Ile Glu Thr Thr Leu Thr
465                 470                 475                 480

cac agt agt ggc cca gcg agc tac gga agg cca agg aag gct gat gct    1488
His Ser Ser Gly Pro Ala Ser Tyr Gly Arg Pro Arg Lys Ala Asp Ala
                485                 490                 495

tga                                                                1491
```

<210> 14
<211> 496
<212> PRT
<213> Brassica napus

<400> 14

Met Ala Arg Leu Ser Ser Leu Leu Tyr Phe Ser Ile Thr Val Leu Ile
1               5                   10                  15

Phe Leu His Gly Ser Thr Ala Gln Gln Phe Pro Asn Glu Cys Gln Leu
            20                  25                  30

Asp Gln Leu Asn Ala Leu Glu Pro Ser His Val Leu Lys Ala Glu Ala
        35                  40                  45

Gly Arg Ile Glu Val Trp Asp His His Ala Pro Gln Leu Arg Cys Ser
    50                  55                  60

Gly Val Ser Phe Val Arg Tyr Ile Ile Glu Ser Gln Gly Leu Tyr Leu
65                  70                  75                  80

Pro Ser Phe Leu Asn Thr Ala Asn Val Ser Phe Val Ala Lys Gly Gln
                85                  90                  95

Gly Leu Met Gly Arg Val Val Pro Gly Cys Ala Glu Thr Phe Gln Asp
            100                 105                 110

```
Ser Ser Val Phe Gln Pro Gly Ser Gly Ser Pro Phe Gly Glu Gly Gln
        115                 120                 125

Gly Gln Gly Gln Gln Gly Gln Gly Gln Gly Gln Gly Gln Gly Gln Gly
    130                 135                 140

Lys Gly Gln Gln Gly Gln Gly Lys Gly Gln Gln Gly Gln Ser Gln Gly
145                 150                 155                 160

Gln Gln Gly Gln Gly Gln Gly Phe Arg Asp Met His Gln Lys Val Glu
                165                 170                 175

His Ile Arg Ser Gly Asp Thr Ile Ala Thr His Pro Gly Val Ala Gln
            180                 185                 190

Trp Phe Tyr Asn Asn Gly Asn Gln Pro Leu Val Ile Val Ala Val Met
        195                 200                 205

Asp Leu Ala Ser His Gln Asn Gln Leu Asp Arg Asn Pro Ser Gln Phe
    210                 215                 220

Tyr Leu Ala Gly Lys Asn Pro Gln Gly Gln Ser Trp Leu His Gly Arg
225                 230                 235                 240

Gly Gln Gln Pro Gln Asn Asn Ile Leu Asn Gly Phe Ser Pro Glu Val
                245                 250                 255

Leu Ala Gln Ala Phe Lys Ile Asp Val Arg Thr Ala Gln Gln Leu Gln
            260                 265                 270

Asn Gln Gln Asp Asn Arg Gly Asn Ile Val Arg Val Gln Gly Pro Phe
        275                 280                 285

Gly Val Ile Arg Pro Pro Leu Lys Ser Gln Arg Pro Gln Glu Thr Glu
    290                 295                 300

Ala Asn Gly Leu Glu Glu Thr Ile Cys Ser Ala Arg Cys Thr Asp Asn
305                 310                 315                 320

Leu Asp Asp Pro Ser Asn Ala Asp Val Tyr Lys Pro Gln Leu Gly Tyr
                325                 330                 335

Ile Ser Ile Leu Asn Ser Tyr Asp Leu Pro Ile Leu Arg Val Leu Arg
            340                 345                 350

Leu Ser Ala Leu Arg Gly Ser Ile Arg Gln Asn Ala Met Val Leu Pro
        355                 360                 365

Gln Trp Lys Ser Lys Ser Asn Ala Val Leu Tyr Val Thr Asp Gly Glu
    370                 375                 380

Ala Gln Ile Gln Val Val Asn Asp Asn Gly Asp Arg Val Phe Asp Gly
385                 390                 395                 400

Gln Val Ser Gln Gly Gln Leu Leu Ser Ile Pro Gln Gly Phe Ser Val
                405                 410                 415

Val Lys Arg Ala Thr Ser Asp Gln Phe Arg Trp Ile Glu Phe Lys Thr
            420                 425                 430

Asn Ala Asn Ala Gln Ile Asn Thr Leu Ala Gly Arg Thr Ser Val Met
        435                 440                 445

Arg Gly Leu Pro Leu Glu Val Ile Ala Asn Gly Tyr Gln Ile Ser Leu
    450                 455                 460

Glu Glu Ala Arg Arg Val Lys Phe Asn Thr Ile Glu Thr Thr Leu Thr
465                 470                 475                 480

His Ser Ser Gly Pro Ala Ser Tyr Gly Arg Pro Arg Lys Ala Asp Ala
                485                 490                 495
```

<210> 15
<211> 555
<212> DNA
<213> Brassica napus

<220>
<221> CDS

<222> (1) .. (552)
<223> cruciferin cru4

<400> 15

```
ttg tgc aca atg aga tgc acc gaa aac ctt gat gac ccg tca agt gct      48
Leu Cys Thr Met Arg Cys Thr Glu Asn Leu Asp Asp Pro Ser Ser Ala
1               5                   10                  15

gat gtc tac aag cca tcg ctc gga tac att agc aca ctc aac agc tac      96
Asp Val Tyr Lys Pro Ser Leu Gly Tyr Ile Ser Thr Leu Asn Ser Tyr
            20                  25                  30

aac ctc cct atc ctc aga ttc ctc cgc ctt agc gct ctt cgt ggc tcc     144
Asn Leu Pro Ile Leu Arg Phe Leu Arg Leu Ser Ala Leu Arg Gly Ser
        35                  40                  45

atc cat aac aac gct atg gtg ctg ccg caa tgg aac gtg aac gca aac     192
Ile His Asn Asn Ala Met Val Leu Pro Gln Trp Asn Val Asn Ala Asn
    50                  55                  60

gcg gca ctc tac gtg aca aag ggg aag gct cat ata cag atg gtg aac     240
Ala Ala Leu Tyr Val Thr Lys Gly Lys Ala His Ile Gln Met Val Asn
65                  70                  75                  80

gac aac gga caa aga gtg ttt gac caa gag atc tcc cag gga cag tta     288
Asp Asn Gly Gln Arg Val Phe Asp Gln Glu Ile Ser Gln Gly Gln Leu
                85                  90                  95

ctt gtc gtg cca caa ggc ttc gcg gtc gtg aaa cgt gcc aca agc caa     336
Leu Val Val Pro Gln Gly Phe Ala Val Val Lys Arg Ala Thr Ser Gln
            100                 105                 110

cag ttc cag tgg atc gag ttc aag agc aac gac aac gca cag atc aac     384
Gln Phe Gln Trp Ile Glu Phe Lys Ser Asn Asp Asn Ala Gln Ile Asn
        115                 120                 125

aca ctc gcg gga cgc acc tca gtc atg aga ggt tta cca ctt gag gtt     432
Thr Leu Ala Gly Arg Thr Ser Val Met Arg Gly Leu Pro Leu Glu Val
    130                 135                 140

ata tcc aac ggg tat cag atc tca ccc caa gaa gct aga agt gtt aag     480
Ile Ser Asn Gly Tyr Gln Ile Ser Pro Gln Glu Ala Arg Ser Val Lys
145                 150                 155                 160

ttc agc act ctt gag acc aca ttg act caa agc agt ggt cct atg ggc     528
Phe Ser Thr Leu Glu Thr Thr Leu Thr Gln Ser Ser Gly Pro Met Gly
                165                 170                 175

tac ggt atg cct aga gtc gag gct tga                                 555
Tyr Gly Met Pro Arg Val Glu Ala
            180
```

<210> 16
<211> 184
<212> PRT
<213> Brassica napus

<400> 16

Leu Cys Thr Met Arg Cys Thr Glu Asn Leu Asp Asp Pro Ser Ser Ala
1 5 10 15

Asp Val Tyr Lys Pro Ser Leu Gly Tyr Ile Ser Thr Leu Asn Ser Tyr
20 25 30

Asn Leu Pro Ile Leu Arg Phe Leu Arg Leu Ser Ala Leu Arg Gly Ser
35 40 45

Ile His Asn Asn Ala Met Val Leu Pro Gln Trp Asn Val Asn Ala Asn
50 55 60

Ala Ala Leu Tyr Val Thr Lys Gly Lys Ala His Ile Gln Met Val Asn
65 70 75 80

Asp Asn Gly Gln Arg Val Phe Asp Gln Glu Ile Ser Gln Gly Gln Leu
85 90 95

Leu Val Val Pro Gln Gly Phe Ala Val Val Lys Arg Ala Thr Ser Gln
100 105 110

Gln Phe Gln Trp Ile Glu Phe Lys Ser Asn Asp Asn Ala Gln Ile Asn
115 120 125

Thr Leu Ala Gly Arg Thr Ser Val Met Arg Gly Leu Pro Leu Glu Val
130 135 140

Ile Ser Asn Gly Tyr Gln Ile Ser Pro Gln Glu Ala Arg Ser Val Lys
145 150 155 160

Phe Ser Thr Leu Glu Thr Thr Leu Thr Gln Ser Ser Gly Pro Met Gly
165 170 175

Tyr Gly Met Pro Arg Val Glu Ala
180

<210> 17
<211> 1530
<212> DNA
<213> Brassica napus

<220>
<221> CDS
<222> (1)..(1527)
<223> cruciferin cru4

<400> 17

```
atg gtt aaa gtt cct cat ctc ctc gtc gca acg ttc ggg gtt ctc ctc      48
Met Val Lys Val Pro His Leu Leu Val Ala Thr Phe Gly Val Leu Leu
 1               5                  10                  15

gtc ctc aac ggc tgt ctc gca agg cag tcg cta ggg gtt cct cct cag      96
Val Leu Asn Gly Cys Leu Ala Arg Gln Ser Leu Gly Val Pro Pro Gln
            20                  25                  30

cta ggg aac gcg tgt aac ctc gat aac tta gac gtt ctc cag cct acc     144
Leu Gly Asn Ala Cys Asn Leu Asp Asn Leu Asp Val Leu Gln Pro Thr
        35                  40                  45

gaa act atc aag agc gag gct ggt cgg gtc gag tac tgg gat cac aac     192
Glu Thr Ile Lys Ser Glu Ala Gly Arg Val Glu Tyr Trp Asp His Asn
    50                  55                  60

aat cct cag atc cga tgt gct ggt gtc tct gtc tct cgt gtt ata atc     240
Asn Pro Gln Ile Arg Cys Ala Gly Val Ser Val Ser Arg Val Ile Ile
65                  70                  75                  80

gaa caa ggc ggt ctc tac ctt cct acc ttc ttc agc tcc ccc aaa att     288
Glu Gln Gly Gly Leu Tyr Leu Pro Thr Phe Phe Ser Ser Pro Lys Ile
                85                  90                  95
```

```
tca tac gtt gtt caa gga atg ggt att agc gga aga gtg gtc cct gga      336
Ser Tyr Val Val Gln Gly Met Gly Ile Ser Gly Arg Val Val Pro Gly
                100                 105                 110

tgc gcg gaa acc ttc atg gac tcg cag cct atg caa gga caa caa caa      384
Cys Ala Glu Thr Phe Met Asp Ser Gln Pro Met Gln Gly Gln Gln Gln
            115                 120                 125

ggt caa cca tgg cag gga caa caa gga caa cag ggt cag cag gga caa      432
Gly Gln Pro Trp Gln Gly Gln Gln Gly Gln Gln Gly Gln Gln Gly Gln
        130                 135                 140

caa ggt caa cag ggt cag cag gga caa caa ggt caa cag ggt cag cag      480
Gln Gly Gln Gln Gly Gln Gln Gly Gln Gln Gly Gln Gln Gly Gln Gln
145                 150                 155                 160

ggt caa cag gga cag cag ggt cag cag cag caa ggg ttc cgt gac atg      528
Gly Gln Gln Gly Gln Gln Gly Gln Gln Gln Gln Gly Phe Arg Asp Met
                165                 170                 175

cac cag aag gtc gaa cat gtt cga cat gga gac atc att gcc att act      576
His Gln Lys Val Glu His Val Arg His Gly Asp Ile Ile Ala Ile Thr
            180                 185                 190

gca ggc tct tcc cat tgg atc tac aac acc ggt gac cag cca ctt gtc      624
Ala Gly Ser Ser His Trp Ile Tyr Asn Thr Gly Asp Gln Pro Leu Val
        195                 200                 205

att atc tgc ctt ctc gac att gcc aac tac caa aac caa ctc gac cgc      672
Ile Ile Cys Leu Leu Asp Ile Ala Asn Tyr Gln Asn Gln Leu Asp Arg
    210                 215                 220

aac cca aga acg ttc cgt ctg gcc gga aac aac cca cag ggc ggt tcc      720
Asn Pro Arg Thr Phe Arg Leu Ala Gly Asn Asn Pro Gln Gly Gly Ser
225                 230                 235                 240

cag cag cag cag caa caa caa cag aac atg ttg agc ggg ttc gac cct      768
Gln Gln Gln Gln Gln Gln Gln Gln Asn Met Leu Ser Gly Phe Asp Pro
                245                 250                 255

cag gtc cta gcc cag gca ttg aaa atc gac gtt agg ttg gct cag gag      816
Gln Val Leu Ala Gln Ala Leu Lys Ile Asp Val Arg Leu Ala Gln Glu
            260                 265                 270

ctt cag aac caa caa gac agc aga gga aac atc gtt cgt gtt aag gga      864
Leu Gln Asn Gln Gln Asp Ser Arg Gly Asn Ile Val Arg Val Lys Gly
        275                 280                 285

cct ttc cag gtt gtg agg ccg cct ctt aga cag cca tac gag agt gag      912
Pro Phe Gln Val Val Arg Pro Pro Leu Arg Gln Pro Tyr Glu Ser Glu
    290                 295                 300

cag tgg aga cac ccc cgt ggc cca cca caa agc cca caa gac aac ggc      960
Gln Trp Arg His Pro Arg Gly Pro Pro Gln Ser Pro Gln Asp Asn Gly
305                 310                 315                 320

ttg gag gag act atc tgc agc atg agg acc cac gag aac att gat gac     1008
Leu Glu Glu Thr Ile Cys Ser Met Arg Thr His Glu Asn Ile Asp Asp
                325                 330                 335

cca gcc cgt gct gac gtg tat aag ccc aac ctc ggc cgt gtg act agc     1056
Pro Ala Arg Ala Asp Val Tyr Lys Pro Asn Leu Gly Arg Val Thr Ser
            340                 345                 350

gtc aac agc tac act tta ccc atc ttg cag tat atc aga ctc agc gcc     1104
Val Asn Ser Tyr Thr Leu Pro Ile Leu Gln Tyr Ile Arg Leu Ser Ala
        355                 360                 365
```

```
acc cgt ggc att ctc cag ggt aat gcg atg gtg ctt ccg aaa tac aac      1152
Thr Arg Gly Ile Leu Gln Gly Asn Ala Met Val Leu Pro Lys Tyr Asn
    370                 375                 380

atg aac gcg aac gag atc ttg tac tgc act caa gga caa gca agg att      1200
Met Asn Ala Asn Glu Ile Leu Tyr Cys Thr Gln Gly Gln Ala Arg Ile
385                 390                 395                 400

caa gtg gtg aac gac aac gga cag aac gtg ctg gac cag cag gtg cag      1248
Gln Val Val Asn Asp Asn Gly Gln Asn Val Leu Asp Gln Gln Val Gln
                405                 410                 415

aag gga cag ctc gtg gtc atc cca caa gga ttc gcc tat gtt gtc cag      1296
Lys Gly Gln Leu Val Val Ile Pro Gln Gly Phe Ala Tyr Val Val Gln
            420                 425                 430

tcc cac caa aac aac ttc gaa tgg att tct ttc aag aca aac gct aac      1344
Ser His Gln Asn Asn Phe Glu Trp Ile Ser Phe Lys Thr Asn Ala Asn
        435                 440                 445

gcg atg gtc agc act ttg gcc ggt aga acc tcg gcc ttg agg gca ttg      1392
Ala Met Val Ser Thr Leu Ala Gly Arg Thr Ser Ala Leu Arg Ala Leu
    450                 455                 460

cca cta gag gtc ata acc aac gct ttc caa att tct ctc gag gaa gct      1440
Pro Leu Glu Val Ile Thr Asn Ala Phe Gln Ile Ser Leu Glu Glu Ala
465                 470                 475                 480

aga agg atc aag ttc aac acg ctt gag acc act ttg act cgt gcg cgc      1488
Arg Arg Ile Lys Phe Asn Thr Leu Glu Thr Thr Leu Thr Arg Ala Arg
                485                 490                 495

ggt gga caa ccc cag ttg atc gag gag ata gtc gag gct taa              1530
Gly Gly Gln Pro Gln Leu Ile Glu Glu Ile Val Glu Ala
            500                 505
```

<210> 18
<211> 509
<212> PRT
<213> Brassica napus

<400> 18

```
Met Val Lys Val Pro His Leu Leu Val Ala Thr Phe Gly Val Leu Leu
1               5                   10                  15
Val Leu Asn Gly Cys Leu Ala Arg Gln Ser Leu Gly Val Pro Pro Gln
            20                  25                  30
Leu Gly Asn Ala Cys Asn Leu Asp Asn Leu Asp Val Leu Gln Pro Thr
        35                  40                  45
Glu Thr Ile Lys Ser Glu Ala Gly Arg Val Glu Tyr Trp Asp His Asn
    50                  55                  60
Asn Pro Gln Ile Arg Cys Ala Gly Val Ser Val Ser Arg Val Ile Ile
65                  70                  75                  80
Glu Gln Gly Gly Leu Tyr Leu Pro Thr Phe Phe Ser Ser Pro Lys Ile
                85                  90                  95
Ser Tyr Val Val Gln Gly Met Gly Ile Ser Gly Arg Val Val Pro Gly
            100                 105                 110
Cys Ala Glu Thr Phe Met Asp Ser Gln Pro Met Gln Gly Gln Gln Gln
        115                 120                 125
Gly Gln Pro Trp Gln Gly Gln Gln Gly Gln Gln Gly Gln Gln Gly Gln
    130                 135                 140
```

Gln Gly Gln Gln Gly Gln Gln Gly Gln Gln Gly Gln Gln Gly Gln Gln
145 150 155 160

Gly Gln Gln Gly Gln Gln Gly Gln Gln Gln Gln Gly Phe Arg Asp Met
165 170 175

His Gln Lys Val Glu His Val Arg His Gly Asp Ile Ile Ala Ile Thr
180 185 190

Ala Gly Ser Ser His Trp Ile Tyr Asn Thr Gly Asp Gln Pro Leu Val
195 200 205

Ile Ile Cys Leu Leu Asp Ile Ala Asn Tyr Gln Asn Gln Leu Asp Arg
210 215 220

Asn Pro Arg Thr Phe Arg Leu Ala Gly Asn Asn Pro Gln Gly Gly Ser
225 230 235 240

Gln Gln Gln Gln Gln Gln Gln Gln Asn Met Leu Ser Gly Phe Asp Pro
245 250 255

Gln Val Leu Ala Gln Ala Leu Lys Ile Asp Val Arg Leu Ala Gln Glu
260 265 270

Leu Gln Asn Gln Gln Asp Ser Arg Gly Asn Ile Val Arg Val Lys Gly
275 280 285

Pro Phe Gln Val Val Arg Pro Pro Leu Arg Gln Pro Tyr Glu Ser Glu
290 295 300

Gln Trp Arg His Pro Arg Gly Pro Pro Gln Ser Pro Gln Asp Asn Gly
305 310 315 320

Leu Glu Glu Thr Ile Cys Ser Met Arg Thr His Glu Asn Ile Asp Asp
325 330 335

Pro Ala Arg Ala Asp Val Tyr Lys Pro Asn Leu Gly Arg Val Thr Ser
340 345 350

Val Asn Ser Tyr Thr Leu Pro Ile Leu Gln Tyr Ile Arg Leu Ser Ala
355 360 365

Thr Arg Gly Ile Leu Gln Gly Asn Ala Met Val Leu Pro Lys Tyr Asn
370 375 380

Met Asn Ala Asn Glu Ile Leu Tyr Cys Thr Gln Gly Gln Ala Arg Ile
385 390 395 400

Gln Val Val Asn Asp Asn Gly Gln Asn Val Leu Asp Gln Gln Val Gln
405 410 415

Lys Gly Gln Leu Val Val Ile Pro Gln Gly Phe Ala Tyr Val Val Gln
420 425 430

Ser His Gln Asn Asn Phe Glu Trp Ile Ser Phe Lys Thr Asn Ala Asn
435 440 445

Ala Met Val Ser Thr Leu Ala Gly Arg Thr Ser Ala Leu Arg Ala Leu
450 455 460

Pro Leu Glu Val Ile Thr Asn Ala Phe Gln Ile Ser Leu Glu Glu Ala
465 470 475 480

Arg Arg Ile Lys Phe Asn Thr Leu Glu Thr Thr Leu Thr Arg Ala Arg
485 490 495

Gly Gly Gln Pro Gln Leu Ile Glu Glu Ile Val Glu Ala
500 505

<210> 19
<211> 1488
<212> DNA
<213> Glycine max

<220>

<221> CDS
<222> (1)..(1485)
<223> Gycinin A-1a-B-x subunit

<400> 19

```
atg gcc aag cta gtt ttt tcc ctt tgt ttt ctg ctt ttc agt ggc tgc      48
Met Ala Lys Leu Val Phe Ser Leu Cys Phe Leu Leu Phe Ser Gly Cys
1               5                   10                  15

tgc ttc gct ttc agt tcc aga gag cag cct cag caa aac gag tgc cag      96
Cys Phe Ala Phe Ser Ser Arg Glu Gln Pro Gln Gln Asn Glu Cys Gln
            20                  25                  30

atc caa aaa ctc aat gcc ctc aaa ccg gat aac cgt ata gag tca gaa     144
Ile Gln Lys Leu Asn Ala Leu Lys Pro Asp Asn Arg Ile Glu Ser Glu
        35                  40                  45

gga ggg ctc att gag aca tgg aac cct aac aac aag cca ttc cag tgt     192
Gly Gly Leu Ile Glu Thr Trp Asn Pro Asn Asn Lys Pro Phe Gln Cys
    50                  55                  60

gcc ggt gtt gcc ctc tct cgc tgc acc ctc aac cgc aac gcc ctt cgt     240
Ala Gly Val Ala Leu Ser Arg Cys Thr Leu Asn Arg Asn Ala Leu Arg
65                  70                  75                  80

aga cct tcc tac acc aac ggt ccc cag gaa atc tac atc caa caa ggt     288
Arg Pro Ser Tyr Thr Asn Gly Pro Gln Glu Ile Tyr Ile Gln Gln Gly
                85                  90                  95

aag ggt att ttt ggc atg ata tac ccg ggt tgt cct agc aca ttt gaa     336
Lys Gly Ile Phe Gly Met Ile Tyr Pro Gly Cys Pro Ser Thr Phe Glu
            100                 105                 110

gag cct caa caa cct caa caa aga gga caa agc agc aga cca caa gac     384
Glu Pro Gln Gln Pro Gln Gln Arg Gly Gln Ser Ser Arg Pro Gln Asp
        115                 120                 125

cgt cac cag aag atc tat aac ttc aga gag ggt gat ttg atc gca gtg     432
Arg His Gln Lys Ile Tyr Asn Phe Arg Glu Gly Asp Leu Ile Ala Val
    130                 135                 140

cct act ggt gtt gca tgg tgg atg tac aac aat gaa gac act cct gtt     480
Pro Thr Gly Val Ala Trp Trp Met Tyr Asn Asn Glu Asp Thr Pro Val
145                 150                 155                 160

gtt gcc gtt tct att att gac acc aac agc ttg gag aac cag ctc gac     528
Val Ala Val Ser Ile Ile Asp Thr Asn Ser Leu Glu Asn Gln Leu Asp
                165                 170                 175

cag atg cct agg aga ttc tat ctt gct ggg aac caa gag caa gag ttt     576
Gln Met Pro Arg Arg Phe Tyr Leu Ala Gly Asn Gln Glu Gln Glu Phe
            180                 185                 190

cta aaa tat cag caa gag caa gga ggt cat caa agc cag aaa gga aag     624
Leu Lys Tyr Gln Gln Glu Gln Gly Gly His Gln Ser Gln Lys Gly Lys
        195                 200                 205

cat cag caa gaa gaa gaa aac gaa gga ggc agc ata ttg agt ggc ttc     672
His Gln Gln Glu Glu Glu Asn Glu Gly Gly Ser Ile Leu Ser Gly Phe
    210                 215                 220
```

```
acc ctg gaa ttc ttg gaa cat gca ttc agc gtg gac aag cag ata gcg    720
Thr Leu Glu Phe Leu Glu His Ala Phe Ser Val Asp Lys Gln Ile Ala
225                 230                 235                 240

aaa aac cta caa gga gag aac gaa ggg gaa gac aag gga gcc att gtg    768
Lys Asn Leu Gln Gly Glu Asn Glu Gly Glu Asp Lys Gly Ala Ile Val
                245                 250                 255

aca gtg aaa gga ggt ctg agc gtg ata aaa cca ccc acg gac gag cag    816
Thr Val Lys Gly Gly Leu Ser Val Ile Lys Pro Pro Thr Asp Glu Gln
            260                 265                 270

caa caa aga ccc cag gaa gag gaa gaa gaa gaa gag gat gag aag cca    864
Gln Gln Arg Pro Gln Glu Glu Glu Glu Glu Glu Glu Asp Glu Lys Pro
        275                 280                 285

cag tgc aag ggt aaa gac aaa cac tgc caa cgc ccc cga gga agc caa    912
Gln Cys Lys Gly Lys Asp Lys His Cys Gln Arg Pro Arg Gly Ser Gln
    290                 295                 300

agc aaa agc aga aga aat ggc att gac gag acc ata tgc acc atg aga    960
Ser Lys Ser Arg Arg Asn Gly Ile Asp Glu Thr Ile Cys Thr Met Arg
305                 310                 315                 320

ctt cgc cac aac att ggc cag act tca tca cct gac atc tac aac cct    1008
Leu Arg His Asn Ile Gly Gln Thr Ser Ser Pro Asp Ile Tyr Asn Pro
                325                 330                 335

caa gcc ggt agc gtc aca acc gcc acc agc ctt gac ttc cca gcc ctc    1056
Gln Ala Gly Ser Val Thr Thr Ala Thr Ser Leu Asp Phe Pro Ala Leu
            340                 345                 350

tcg tgg ctc aga ctc agt gct gag ttt gga tct ctc cgc aag aat gca    1104
Ser Trp Leu Arg Leu Ser Ala Glu Phe Gly Ser Leu Arg Lys Asn Ala
        355                 360                 365

atg ttc gtg cca cac tac aac ctg aac gcg aac agc ata ata tac gca    1152
Met Phe Val Pro His Tyr Asn Leu Asn Ala Asn Ser Ile Ile Tyr Ala
    370                 375                 380

ttg aat gga cgg gca ttg ata caa gtg gtg aat tgc aac ggt gag aga    1200
Leu Asn Gly Arg Ala Leu Ile Gln Val Val Asn Cys Asn Gly Glu Arg
385                 390                 395                 400

gtg ttt gat gga gag ctg caa gag gga cgg gtg ctg atc gtg cca caa    1248
Val Phe Asp Gly Glu Leu Gln Glu Gly Arg Val Leu Ile Val Pro Gln
                405                 410                 415

aac ttt gtg gtg gct gca aga tca cag agt gac aac ttc gag tat gtg    1296
Asn Phe Val Val Ala Ala Arg Ser Gln Ser Asp Asn Phe Glu Tyr Val
            420                 425                 430

tca ttc aag acc aat gat aca ccc atg atc ggc act ctt gca ggg gca    1344
Ser Phe Lys Thr Asn Asp Thr Pro Met Ile Gly Thr Leu Ala Gly Ala
        435                 440                 445

aac tca ttg ttg aac gca tta cca gag gaa gtg att cag cac act ttc    1392
Asn Ser Leu Leu Asn Ala Leu Pro Glu Glu Val Ile Gln His Thr Phe
    450                 455                 460

aac cta aaa agc cag cag gcc agg cag ata aag aac aac aac cct ttc    1440
Asn Leu Lys Ser Gln Gln Ala Arg Gln Ile Lys Asn Asn Asn Pro Phe
465                 470                 475                 480

aag ttc ctg gtt cca cct cag gag tct cag aag aga gct gtg gct tag    1488
Lys Phe Leu Val Pro Pro Gln Glu Ser Gln Lys Arg Ala Val Ala
                485                 490                 495
```

<210> 20
<211> 495
<212> PRT
<213> Glycine max

<400> 20

Met Ala Lys Leu Val Phe Ser Leu Cys Phe Leu Leu Phe Ser Gly Cys
1 5 10 15

Cys Phe Ala Phe Ser Ser Arg Glu Gln Pro Gln Gln Asn Glu Cys Gln
20 25 30

Ile Gln Lys Leu Asn Ala Leu Lys Pro Asp Asn Arg Ile Glu Ser Glu
35 40 45

Gly Gly Leu Ile Glu Thr Trp Asn Pro Asn Asn Lys Pro Phe Gln Cys
50 55 60

Ala Gly Val Ala Leu Ser Arg Cys Thr Leu Asn Arg Asn Ala Leu Arg
65 70 75 80

Arg Pro Ser Tyr Thr Asn Gly Pro Gln Glu Ile Tyr Ile Gln Gln Gly
85 90 95

Lys Gly Ile Phe Gly Met Ile Tyr Pro Gly Cys Pro Ser Thr Phe Glu
100 105 110

Glu Pro Gln Gln Pro Gln Gln Arg Gly Gln Ser Ser Arg Pro Gln Asp
115 120 125

Arg His Gln Lys Ile Tyr Asn Phe Arg Glu Gly Asp Leu Ile Ala Val
130 135 140

Pro Thr Gly Val Ala Trp Trp Met Tyr Asn Asn Glu Asp Thr Pro Val
145 150 155 160

Val Ala Val Ser Ile Ile Asp Thr Asn Ser Leu Glu Asn Gln Leu Asp
165 170 175

Gln Met Pro Arg Arg Phe Tyr Leu Ala Gly Asn Gln Glu Gln Glu Phe
180 185 190

Leu Lys Tyr Gln Gln Glu Gln Gly Gly His Gln Ser Gln Lys Gly Lys
195 200 205

His Gln Gln Glu Glu Glu Asn Glu Gly Gly Ser Ile Leu Ser Gly Phe
210 215 220

Thr Leu Glu Phe Leu Glu His Ala Phe Ser Val Asp Lys Gln Ile Ala
225 230 235 240

Lys Asn Leu Gln Gly Glu Asn Glu Gly Glu Asp Lys Gly Ala Ile Val
245 250 255

Thr Val Lys Gly Gly Leu Ser Val Ile Lys Pro Pro Thr Asp Glu Gln
260 265 270

Gln Gln Arg Pro Gln Glu Glu Glu Glu Glu Glu Glu Asp Glu Lys Pro
275 280 285

Gln Cys Lys Gly Lys Asp Lys His Cys Gln Arg Pro Arg Gly Ser Gln
290 295 300

Ser Lys Ser Arg Arg Asn Gly Ile Asp Glu Thr Ile Cys Thr Met Arg
305 310 315 320

Leu Arg His Asn Ile Gly Gln Thr Ser Ser Pro Asp Ile Tyr Asn Pro
325 330 335

Gln Ala Gly Ser Val Thr Thr Ala Thr Ser Leu Asp Phe Pro Ala Leu
340 345 350

```
Ser Trp Leu Arg Leu Ser Ala Glu Phe Gly Ser Leu Arg Lys Asn Ala
        355                 360                 365

Met Phe Val Pro His Tyr Asn Leu Asn Ala Asn Ser Ile Ile Tyr Ala
    370                 375                 380

Leu Asn Gly Arg Ala Leu Ile Gln Val Val Asn Cys Asn Gly Glu Arg
385                 390                 395                 400

Val Phe Asp Gly Glu Leu Gln Glu Gly Arg Val Leu Ile Val Pro Gln
                405                 410                 415

Asn Phe Val Val Ala Ala Arg Ser Gln Ser Asp Asn Phe Glu Tyr Val
            420                 425                 430

Ser Phe Lys Thr Asn Asp Thr Pro Met Ile Gly Thr Leu Ala Gly Ala
        435                 440                 445

Asn Ser Leu Leu Asn Ala Leu Pro Glu Glu Val Ile Gln His Thr Phe
    450                 455                 460

Asn Leu Lys Ser Gln Gln Ala Arg Gln Ile Lys Asn Asn Asn Pro Phe
465                 470                 475                 480

Lys Phe Leu Val Pro Pro Gln Glu Ser Gln Lys Arg Ala Val Ala
                485                 490                 495
```

<210> 21
<211> 1458
<212> DNA
<213> Glycine max

<220>
<221> CDS
<222> (1)..(1455)
<223> glycinin G2 subunit

<400> 21

```
atg gcc aag ctt gtt ctt tcc ctt tgt ttc ctt ctt ttc agt ggc tgc    48
Met Ala Lys Leu Val Leu Ser Leu Cys Phe Leu Leu Phe Ser Gly Cys
 1               5                  10                  15

ttc gct ctg aga gag cag gca cag caa aat gag tgc cag atc caa aag    96
Phe Ala Leu Arg Glu Gln Ala Gln Gln Asn Glu Cys Gln Ile Gln Lys
            20                  25                  30

ctg aat gcc ctc aaa ccg gat aac cgt ata gag tcg gaa ggt ggg ttc   144
Leu Asn Ala Leu Lys Pro Asp Asn Arg Ile Glu Ser Glu Gly Gly Phe
        35                  40                  45

att gag aca tgg aac cct aac aac aag cca ttc cag tgt gcc ggt gtt   192
Ile Glu Thr Trp Asn Pro Asn Asn Lys Pro Phe Gln Cys Ala Gly Val
    50                  55                  60

gcc ctc tct cgc tgc acc ctt aac cgc aat gcc ctt cgt aga cct tcc   240
Ala Leu Ser Arg Cys Thr Leu Asn Arg Asn Ala Leu Arg Arg Pro Ser
65                  70                  75                  80

tac acc aac ggt ccc cag gaa atc tac ata caa caa ggt aat ggt att   288
Tyr Thr Asn Gly Pro Gln Glu Ile Tyr Ile Gln Gln Gly Asn Gly Ile
                85                  90                  95

ttt ggc atg ata ttc ccg ggt tgt cct agc act tat caa gag ccg caa   336
Phe Gly Met Ile Phe Pro Gly Cys Pro Ser Thr Tyr Gln Glu Pro Gln
            100                 105                 110
```

```
gaa tct cag caa cga gga cga agc cag agg ccc caa gac cgt cac caa     384
Glu Ser Gln Gln Arg Gly Arg Ser Gln Arg Pro Gln Asp Arg His Gln
        115                 120                 125

aag gta cat cgc ttc aga gag ggt gat ttg atc gca gtg cct act ggt     432
Lys Val His Arg Phe Arg Glu Gly Asp Leu Ile Ala Val Pro Thr Gly
    130                 135                 140

gtt gca tgg tgg atg tac aac aat gaa gac act cct gtt gtt gcc gtt     480
Val Ala Trp Trp Met Tyr Asn Asn Glu Asp Thr Pro Val Val Ala Val
145                 150                 155                 160

tct att att gac acc aac agc ttg gag aac cag ctc gac cag atg cct     528
Ser Ile Ile Asp Thr Asn Ser Leu Glu Asn Gln Leu Asp Gln Met Pro
                165                 170                 175

agg aga ttc tat ctt gct ggg aac caa gag caa gag ttt cta aaa tat     576
Arg Arg Phe Tyr Leu Ala Gly Asn Gln Glu Gln Glu Phe Leu Lys Tyr
            180                 185                 190

cag cag cag cag caa gga ggt tcc caa agc cag aaa gga aag caa caa     624
Gln Gln Gln Gln Gln Gly Gly Ser Gln Ser Gln Lys Gly Lys Gln Gln
        195                 200                 205

gaa gaa gaa aac gaa gga agc aac ata ttg agt ggc ttc gcc cct gaa     672
Glu Glu Glu Asn Glu Gly Ser Asn Ile Leu Ser Gly Phe Ala Pro Glu
    210                 215                 220

ttc ttg aaa gaa gcg ttc ggc gtg aac atg cag ata gtg aga aac cta     720
Phe Leu Lys Glu Ala Phe Gly Val Asn Met Gln Ile Val Arg Asn Leu
225                 230                 235                 240

caa ggt gag aac gaa gag gag gat agt gga gcc att gtg aca gtg aaa     768
Gln Gly Glu Asn Glu Glu Glu Asp Ser Gly Ala Ile Val Thr Val Lys
                245                 250                 255

gga ggt cta aga gtc aca gct cca gcc atg agg aag cca cag caa gaa     816
Gly Gly Leu Arg Val Thr Ala Pro Ala Met Arg Lys Pro Gln Gln Glu
            260                 265                 270

gaa gat gat gat gat gag gaa gag cag cca cag tgc gtg gag aca gac     864
Glu Asp Asp Asp Asp Glu Glu Glu Gln Pro Gln Cys Val Glu Thr Asp
        275                 280                 285

aaa ggt tgc caa cgc caa agc aaa agg agc aga aat ggc att gat gag     912
Lys Gly Cys Gln Arg Gln Ser Lys Arg Ser Arg Asn Gly Ile Asp Glu
    290                 295                 300

acc att tgc aca atg aga ctt cgc caa aac att ggt cag aat tca tca     960
Thr Ile Cys Thr Met Arg Leu Arg Gln Asn Ile Gly Gln Asn Ser Ser
305                 310                 315                 320

cct gac atc tac aac cct caa gct ggt agc atc aca acc gcc acc agc     1008
Pro Asp Ile Tyr Asn Pro Gln Ala Gly Ser Ile Thr Thr Ala Thr Ser
                325                 330                 335

ctt gac ttc cca gcc ctc tgg ctt ctc aaa ctc agt gcc cag tat gga     1056
Leu Asp Phe Pro Ala Leu Trp Leu Leu Lys Leu Ser Ala Gln Tyr Gly
            340                 345                 350

tca ctc cgc aag aat gct atg ttc gtg cca cac tac acc ctg aac gcg     1104
Ser Leu Arg Lys Asn Ala Met Phe Val Pro His Tyr Thr Leu Asn Ala
        355                 360                 365

aac agc ata ata tac gca ttg aat ggg cgg gca ttg gta caa gtg gtg     1152
Asn Ser Ile Ile Tyr Ala Leu Asn Gly Arg Ala Leu Val Gln Val Val
    370                 375                 380
```

```
aat tgc aat ggt gag aga gtg ttt gat gga gag ctg caa gag gga ggg     1200
Asn Cys Asn Gly Glu Arg Val Phe Asp Gly Glu Leu Gln Glu Gly Gly
385                 390                 395                 400

gtg ctg atc gtt cca caa aac ttt gcg gtg gct gca aaa tcc cag agc     1248
Val Leu Ile Val Pro Gln Asn Phe Ala Val Ala Ala Lys Ser Gln Ser
                405                 410                 415

gat aac ttt gag tat gtg tca ttc aag acc aat gat aga ccc tcg atc     1296
Asp Asn Phe Glu Tyr Val Ser Phe Lys Thr Asn Asp Arg Pro Ser Ile
            420                 425                 430

gga aac ctt gca ggg gca aac tca ttg ttg aac gca ttg cca gag gaa     1344
Gly Asn Leu Ala Gly Ala Asn Ser Leu Leu Asn Ala Leu Pro Glu Glu
        435                 440                 445

gtg att cag cac act ttt aac cta aag agc cag cag gcc agg cag gtg     1392
Val Ile Gln His Thr Phe Asn Leu Lys Ser Gln Gln Ala Arg Gln Val
    450                 455                 460

aag aac aac aac cct ttc agc ttc ctt gtt cca cct cag gag tct cag     1440
Lys Asn Asn Asn Pro Phe Ser Phe Leu Val Pro Pro Gln Glu Ser Gln
465                 470                 475                 480

agg aga gct gtg gct tag                                             1458
Arg Arg Ala Val Ala
                485
```

<210> 22
<211> 485
<212> PRT
<213> Glycine max

<400> 22

```
Met Ala Lys Leu Val Leu Ser Leu Cys Phe Leu Leu Phe Ser Gly Cys
1               5                   10                  15

Phe Ala Leu Arg Glu Gln Ala Gln Gln Asn Glu Cys Gln Ile Gln Lys
            20                  25                  30

Leu Asn Ala Leu Lys Pro Asp Asn Arg Ile Glu Ser Glu Gly Gly Phe
        35                  40                  45

Ile Glu Thr Trp Asn Pro Asn Asn Lys Pro Phe Gln Cys Ala Gly Val
    50                  55                  60

Ala Leu Ser Arg Cys Thr Leu Asn Arg Asn Ala Leu Arg Arg Pro Ser
65                  70                  75                  80

Tyr Thr Asn Gly Pro Gln Glu Ile Tyr Ile Gln Gln Gly Asn Gly Ile
                85                  90                  95

Phe Gly Met Ile Phe Pro Gly Cys Pro Ser Thr Tyr Gln Glu Pro Gln
            100                 105                 110

Glu Ser Gln Gln Arg Gly Arg Ser Gln Arg Pro Gln Asp Arg His Gln
        115                 120                 125

Lys Val His Arg Phe Arg Glu Gly Asp Leu Ile Ala Val Pro Thr Gly
    130                 135                 140

Val Ala Trp Trp Met Tyr Asn Asn Glu Asp Thr Pro Val Val Ala Val
145                 150                 155                 160

Ser Ile Ile Asp Thr Asn Ser Leu Glu Asn Gln Leu Asp Gln Met Pro
                165                 170                 175

Arg Arg Phe Tyr Leu Ala Gly Asn Gln Glu Gln Glu Phe Leu Lys Tyr
            180                 185                 190
```

Gln Gln Gln Gln Gln Gly Gly Ser Gln Ser Gln Lys Gly Lys Gln Gln
195 200 205

Glu Glu Glu Asn Glu Gly Ser Asn Ile Leu Ser Gly Phe Ala Pro Glu
210 215 220

Phe Leu Lys Glu Ala Phe Gly Val Asn Met Gln Ile Val Arg Asn Leu
225 230 235 240

Gln Gly Glu Asn Glu Glu Glu Asp Ser Gly Ala Ile Val Thr Val Lys
245 250 255

Gly Gly Leu Arg Val Thr Ala Pro Ala Met Arg Lys Pro Gln Gln Glu
260 265 270

Glu Asp Asp Asp Asp Glu Glu Glu Gln Pro Gln Cys Val Glu Thr Asp
275 280 285

Lys Gly Cys Gln Arg Gln Ser Lys Arg Ser Arg Asn Gly Ile Asp Glu
290 295 300

Thr Ile Cys Thr Met Arg Leu Arg Gln Asn Ile Gly Gln Asn Ser Ser
305 310 315 320

Pro Asp Ile Tyr Asn Pro Gln Ala Gly Ser Ile Thr Thr Ala Thr Ser
325 330 335

Leu Asp Phe Pro Ala Leu Trp Leu Leu Lys Leu Ser Ala Gln Tyr Gly
340 345 350

Ser Leu Arg Lys Asn Ala Met Phe Val Pro His Tyr Thr Leu Asn Ala
355 360 365

Asn Ser Ile Ile Tyr Ala Leu Asn Gly Arg Ala Leu Val Gln Val Val
370 375 380

Asn Cys Asn Gly Glu Arg Val Phe Asp Gly Glu Leu Gln Glu Gly Gly
385 390 395 400

Val Leu Ile Val Pro Gln Asn Phe Ala Val Ala Ala Lys Ser Gln Ser
405 410 415

Asp Asn Phe Glu Tyr Val Ser Phe Lys Thr Asn Asp Arg Pro Ser Ile
420 425 430

Gly Asn Leu Ala Gly Ala Asn Ser Leu Leu Asn Ala Leu Pro Glu Glu
435 440 445

Val Ile Gln His Thr Phe Asn Leu Lys Ser Gln Gln Ala Arg Gln Val
450 455 460

Lys Asn Asn Asn Pro Phe Ser Phe Leu Val Pro Pro Gln Glu Ser Gln
465 470 475 480

Arg Arg Ala Val Ala
485

<210> 23
<211> 1689
<212> DNA
<213> Glycine max

<220>
<221> CDS
<222> (1)..(1686)
<223> glycinin A5A4B3 subunits

<400> 23

```
atg ggg aag ccc ttc act ctc tct ctt tct tcc ctt tgc ttg cta ctc   48

 Met Gly Lys Pro Phe Thr Leu Ser Leu Ser Ser Leu Cys Leu Leu Leu
  1               5                  10                  15

 ttg tcg agt gca tgc ttt gct att agc tcc agc aag ctc aac gag tgc   96
 Leu Ser Ser Ala Cys Phe Ala Ile Ser Ser Ser Lys Leu Asn Glu Cys
             20                  25                  30

 caa ctc aac aac ctc aac gcg ttg gaa ccc gac cac cgc gtt gag tcc   144
 Gln Leu Asn Asn Leu Asn Ala Leu Glu Pro Asp His Arg Val Glu Ser
         35                  40                  45

 gaa ggt ggt ttg att caa aca tgg aac tct caa cac cct gag ctg aaa   192
 Glu Gly Gly Leu Ile Gln Thr Trp Asn Ser Gln His Pro Glu Leu Lys
     50                  55                  60

 tgc gcc ggt gtc act gtt tcc aaa ctc acc ctc aac cgc aat ggc ctc   240
 Cys Ala Gly Val Thr Val Ser Lys Leu Thr Leu Asn Arg Asn Gly Leu
 65                  70                  75                  80

 cac tcg cca tct tac tca cct tat ccc cgg atg atc atc atc gcc caa   288
 His Ser Pro Ser Tyr Ser Pro Tyr Pro Arg Met Ile Ile Ile Ala Gln
                 85                  90                  95

 ggg aaa gga gca ctt gga gtt gca att cca gga tgt cct gag acg ttt   336
 Gly Lys Gly Ala Leu Gly Val Ala Ile Pro Gly Cys Pro Glu Thr Phe
             100                 105                 110

 gag gag cca caa gaa caa tca aac aga aga ggc tca agg tcg cag aag   384
 Glu Glu Pro Gln Glu Gln Ser Asn Arg Arg Gly Ser Arg Ser Gln Lys
         115                 120                 125

 cag cag cta cag gac agt cac cag aag att cgt cac ttc aat gaa gga   432
 Gln Gln Leu Gln Asp Ser His Gln Lys Ile Arg His Phe Asn Glu Gly
     130                 135                 140

 gac gta ctc gtg att cct cct agt gtt cct tac tgg acc tat aac act   480
 Asp Val Leu Val Ile Pro Pro Ser Val Pro Tyr Trp Thr Tyr Asn Thr
 145                 150                 155                 160

 ggc gat gaa cca gtt gtt gcc atc agt ctt ctt gac acc tct aac ttc   528
 Gly Asp Glu Pro Val Val Ala Ile Ser Leu Leu Asp Thr Ser Asn Phe
                 165                 170                 175

 aat aac cag ctt gat caa acc cct agg gta ttt tac ctt gct ggg aac   576
 Asn Asn Gln Leu Asp Gln Thr Pro Arg Val Phe Tyr Leu Ala Gly Asn
             180                 185                 190

 cca gat ata gag tac cca gag acc atg caa caa caa caa cag cag aaa   624
 Pro Asp Ile Glu Tyr Pro Glu Thr Met Gln Gln Gln Gln Gln Gln Lys
         195                 200                 205

 agt cat ggt gga cgc aag cag ggg caa cac cag cag gag gaa gag gaa   672
 Ser His Gly Gly Arg Lys Gln Gly Gln His Gln Gln Glu Glu Glu Glu
     210                 215                 220

 gaa ggt ggc agc gtg ctc agt ggc ttc agc aaa cac ttc ttg gca caa   720
 Glu Gly Gly Ser Val Leu Ser Gly Phe Ser Lys His Phe Leu Ala Gln
 225                 230                 235                 240

 tcc ttc aac acc aac gag gac ata gct gag aaa ctt gag tct cca gac   768
 Ser Phe Asn Thr Asn Glu Asp Ile Ala Glu Lys Leu Glu Ser Pro Asp
                 245                 250                 255

 gac gaa agg aag cag atc gtg aca gtg gaa gga ggt ctc agc gtt atc   816
 Asp Glu Arg Lys Gln Ile Val Thr Val Glu Gly Gly Leu Ser Val Ile
             260                 265                 270
```

agc ccc aag tgg caa gaa caa caa gat gaa gat gaa gat gaa gac gaa 864
Ser Pro Lys Trp Gln Glu Gln Gln Asp Glu Asp Glu Asp Glu Asp Glu
275 280 285

gat gat gaa gat gaa caa att ccc tct cac cct cct cgc cga cca agc 912
Asp Asp Glu Asp Glu Gln Ile Pro Ser His Pro Pro Arg Arg Pro Ser
290 295 300

cat gga aag cgt gaa caa gac gag gac gag gac gaa gat gaa gat aaa 960
His Gly Lys Arg Glu Gln Asp Glu Asp Glu Asp Glu Asp Glu Asp Lys
305 310 315 320

cct cgt cct agt cga cca agc caa gga aag cgg aac aag aca gga cag 1008
Pro Arg Pro Ser Arg Pro Ser Gln Gly Lys Arg Asn Lys Thr Gly Gln
325 330 335

gac gag gac gaa gat gaa gat gaa gat caa cct cgc aag agc cgc gaa 1056
Asp Glu Asp Glu Asp Glu Asp Glu Asp Gln Pro Arg Lys Ser Arg Glu
340 345 350

tgg aga tcg aaa aag aca caa ccc aga aga cct aga caa gaa gaa cca 1104
Trp Arg Ser Lys Lys Thr Gln Pro Arg Arg Pro Arg Gln Glu Glu Pro
355 360 365

cgt gaa aga gga tgc gag aca aga aac ggg gtt gag gaa aat atc tgc 1152
Arg Glu Arg Gly Cys Glu Thr Arg Asn Gly Val Glu Glu Asn Ile Cys
370 375 380

acc ttg aag ctt cac gag aac att gct cgc cct tca cgc gct gac ttc 1200
Thr Leu Lys Leu His Glu Asn Ile Ala Arg Pro Ser Arg Ala Asp Phe
385 390 395 400

tac aac cct aaa gct ggt cgc att agt acc ctc aac agc ctc acc ctc 1248
Tyr Asn Pro Lys Ala Gly Arg Ile Ser Thr Leu Asn Ser Leu Thr Leu
405 410 415

cca gcc ctc cgc caa ttc caa ctc agt gcc caa tat gtt gtc ctc tac 1296
Pro Ala Leu Arg Gln Phe Gln Leu Ser Ala Gln Tyr Val Val Leu Tyr
420 425 430

aag aat gga att tac tct cca cat tgg aat ctg aat gca aac agt gtg 1344
Lys Asn Gly Ile Tyr Ser Pro His Trp Asn Leu Asn Ala Asn Ser Val
435 440 445

atc tat gtg act cga gga caa gga aag gtt aga gtt gtg aac tgc caa 1392
Ile Tyr Val Thr Arg Gly Gln Gly Lys Val Arg Val Val Asn Cys Gln
450 455 460

ggg aat gca gtg ttc gac ggt gag ctt agg agg gga caa ttg ctg gtg 1440
Gly Asn Ala Val Phe Asp Gly Glu Leu Arg Arg Gly Gln Leu Leu Val
465 470 475 480

gta cca cag aac ttc gtg gtg gcg gag caa gcc gga gaa caa gga ttc 1488
Val Pro Gln Asn Phe Val Val Ala Glu Gln Ala Gly Glu Gln Gly Phe
485 490 495

gaa tac ata gta ttc aag aca cac cac aac gca gtc act agc tac ttg 1536
Glu Tyr Ile Val Phe Lys Thr His His Asn Ala Val Thr Ser Tyr Leu
500 505 510

aag gat gtg ttt agg gca att ccc tca gag gtt ctt gcc cat tct tac 1584
Lys Asp Val Phe Arg Ala Ile Pro Ser Glu Val Leu Ala His Ser Tyr
515 520 525

aac ctt cga cag agt caa gtg tct gag ctt aag tat gaa gga aat tgg 1632
Asn Leu Arg Gln Ser Gln Val Ser Glu Leu Lys Tyr Glu Gly Asn Trp
530 535 540

ggt cct ttg gtc aac cct gag tct caa caa ggc tca ccc cgt gtt aaa 1680
Gly Pro Leu Val Asn Pro Glu Ser Gln Gln Gly Ser Pro Arg Val Lys
545 550 555 560

gtc gca taa 1689
Val Ala

<210> 24
<211> 562
<212> PRT
<213> Glycine max

<400> 24

```
Met Gly Lys Pro Phe Thr Leu Ser Leu Ser Ser Leu Cys Leu Leu Leu
1               5                   10                  15
Leu Ser Ser Ala Cys Phe Ala Ile Ser Ser Ser Lys Leu Asn Glu Cys
            20                  25                  30
Gln Leu Asn Asn Leu Asn Ala Leu Glu Pro Asp His Arg Val Glu Ser
        35                  40                  45
Glu Gly Gly Leu Ile Gln Thr Trp Asn Ser Gln His Pro Glu Leu Lys
    50                  55                  60
Cys Ala Gly Val Thr Val Ser Lys Leu Thr Leu Asn Arg Asn Gly Leu
65                  70                  75                  80
His Ser Pro Ser Tyr Ser Pro Tyr Pro Arg Met Ile Ile Ile Ala Gln
                85                  90                  95
Gly Lys Gly Ala Leu Gly Val Ala Ile Pro Gly Cys Pro Glu Thr Phe
            100                 105                 110
Glu Glu Pro Gln Glu Gln Ser Asn Arg Arg Gly Ser Arg Ser Gln Lys
        115                 120                 125
Gln Gln Leu Gln Asp Ser His Gln Lys Ile Arg His Phe Asn Glu Gly
    130                 135                 140
Asp Val Leu Val Ile Pro Pro Ser Val Pro Tyr Trp Thr Tyr Asn Thr
145                 150                 155                 160
Gly Asp Glu Pro Val Val Ala Ile Ser Leu Leu Asp Thr Ser Asn Phe
                165                 170                 175
Asn Asn Gln Leu Asp Gln Thr Pro Arg Val Phe Tyr Leu Ala Gly Asn
            180                 185                 190
Pro Asp Ile Glu Tyr Pro Glu Thr Met Gln Gln Gln Gln Gln Gln Lys
        195                 200                 205
Ser His Gly Gly Arg Lys Gln Gly Gln His Gln Gln Glu Glu Glu Glu
    210                 215                 220
Glu Gly Gly Ser Val Leu Ser Gly Phe Ser Lys His Phe Leu Ala Gln
225                 230                 235                 240
Ser Phe Asn Thr Asn Glu Asp Ile Ala Glu Lys Leu Glu Ser Pro Asp
                245                 250                 255
Asp Glu Arg Lys Gln Ile Val Thr Val Glu Gly Gly Leu Ser Val Ile
            260                 265                 270
Ser Pro Lys Trp Gln Glu Gln Gln Asp Glu Asp Glu Asp Glu Asp Glu
        275                 280                 285
Asp Asp Glu Asp Glu Gln Ile Pro Ser His Pro Pro Arg Arg Pro Ser
    290                 295                 300
```

His Gly Lys Arg Glu Gln Asp Glu Asp Glu Asp Glu Asp Glu Asp Lys
305 310 315 320

Pro Arg Pro Ser Arg Pro Ser Gln Gly Lys Arg Asn Lys Thr Gly Gln
325 330 335

Asp Glu Asp Glu Asp Glu Asp Glu Asp Gln Pro Arg Lys Ser Arg Glu
340 345 350

Trp Arg Ser Lys Lys Thr Gln Pro Arg Arg Pro Arg Gln Glu Glu Pro
355 360 365

Arg Glu Arg Gly Cys Glu Thr Arg Asn Gly Val Glu Glu Asn Ile Cys
370 375 380

Thr Leu Lys Leu His Glu Asn Ile Ala Arg Pro Ser Arg Ala Asp Phe
385 390 395 400

Tyr Asn Pro Lys Ala Gly Arg Ile Ser Thr Leu Asn Ser Leu Thr Leu
405 410 415

Pro Ala Leu Arg Gln Phe Gln Leu Ser Ala Gln Tyr Val Val Leu Tyr
420 425 430

Lys Asn Gly Ile Tyr Ser Pro His Trp Asn Leu Asn Ala Asn Ser Val
435 440 445

Ile Tyr Val Thr Arg Gly Gln Gly Lys Val Arg Val Val Asn Cys Gln
450 455 460

Gly Asn Ala Val Phe Asp Gly Glu Leu Arg Arg Gly Gln Leu Leu Val
465 470 475 480

Val Pro Gln Asn Phe Val Val Ala Glu Gln Ala Gly Glu Gln Gly Phe
485 490 495

Glu Tyr Ile Val Phe Lys Thr His His Asn Ala Val Thr Ser Tyr Leu
500 505 510

Lys Asp Val Phe Arg Ala Ile Pro Ser Glu Val Leu Ala His Ser Tyr
515 520 525

Asn Leu Arg Gln Ser Gln Val Ser Glu Leu Lys Tyr Glu Gly Asn Trp
530 535 540

Gly Pro Leu Val Asn Pro Glu Ser Gln Gln Gly Ser Pro Arg Val Lys
545 550 555 560

Val Ala

<210> 25
<211> 1551
<212> DNA
<213> Glycine max

<220>
<221> CDS
<222> (1)..(1548)
<223> glycinin A3-B4 subunit

<400> 25

atg ggg aag ccc ttc ttc act ctc tct ctt tct tcc ctt tgc ttg cta 48
Met Gly Lys Pro Phe Phe Thr Leu Ser Leu Ser Ser Leu Cys Leu Leu
1 5 10 15

ctc ttg tcg agt gca tgc ttt gct att acc tcc agc aag ttc aac gag 96
Leu Leu Ser Ser Ala Cys Phe Ala Ile Thr Ser Ser Lys Phe Asn Glu
20 25 30

```
tgc caa ctc aac aac ctc aac gcg ttg gaa ccc gac cac cgc gtt gag     144
Cys Gln Leu Asn Asn Leu Asn Ala Leu Glu Pro Asp His Arg Val Glu
            35                  40                  45

tcc gaa ggt ggt ctt att gaa aca tgg aac tct caa cac cct gag ctg     192
Ser Glu Gly Gly Leu Ile Glu Thr Trp Asn Ser Gln His Pro Glu Leu
        50                  55                  60

caa tgc gcc ggt gtc act gtt tcc aaa cgc acc ctc aac cgc aac ggc     240
Gln Cys Ala Gly Val Thr Val Ser Lys Arg Thr Leu Asn Arg Asn Gly
65                  70                  75                  80

tcc cac ttg cca tct tac tta cct tat ccc caa atg atc att gtc gtt     288
Ser His Leu Pro Ser Tyr Leu Pro Tyr Pro Gln Met Ile Ile Val Val
                85                  90                  95

caa ggg aag gga gca att gga ttt gca ttt ccg gga tgt ccc gag acg     336
Gln Gly Lys Gly Ala Ile Gly Phe Ala Phe Pro Gly Cys Pro Glu Thr
            100                 105                 110

ttt gag aag cca caa caa caa tca agc aga aga ggc tca agg tca cag     384
Phe Glu Lys Pro Gln Gln Gln Ser Ser Arg Arg Gly Ser Arg Ser Gln
        115                 120                 125

cag caa cta caa gac agt cac cag aag att cgt cac ttc aat gaa gga     432
Gln Gln Leu Gln Asp Ser His Gln Lys Ile Arg His Phe Asn Glu Gly
    130                 135                 140

gac gta cta gtg att cct ctt ggt gtt cct tac tgg acc tat aac act     480
Asp Val Leu Val Ile Pro Leu Gly Val Pro Tyr Trp Thr Tyr Asn Thr
145                 150                 155                 160

ggc gat gaa cca gtt gtt gcc atc agt cct ctt gac acc tcc aac ttc     528
Gly Asp Glu Pro Val Val Ala Ile Ser Pro Leu Asp Thr Ser Asn Phe
                165                 170                 175

aac aat cag ctt gat caa aac ccc aga gta ttt tac ctt gct ggg aac     576
Asn Asn Gln Leu Asp Gln Asn Pro Arg Val Phe Tyr Leu Ala Gly Asn
            180                 185                 190

cca gat ata gag cat ccc gag acc atg caa caa cag cag cag cag aag     624
Pro Asp Ile Glu His Pro Glu Thr Met Gln Gln Gln Gln Gln Gln Lys
        195                 200                 205

agt cat ggt gga cgc aag cag ggg caa cac cga cag cag gag gaa gaa     672
Ser His Gly Gly Arg Lys Gln Gly Gln His Arg Gln Gln Glu Glu Glu
    210                 215                 220

ggt ggc agt gtg ctc agt ggc ttc agc aaa cat ttc tta gca caa tcc     720
Gly Gly Ser Val Leu Ser Gly Phe Ser Lys His Phe Leu Ala Gln Ser
225                 230                 235                 240

ttc aac acc aac gag gac aca gct gag aaa ctt cgg tct cca gat gac     768
Phe Asn Thr Asn Glu Asp Thr Ala Glu Lys Leu Arg Ser Pro Asp Asp
                245                 250                 255

gaa agg aag cag atc gtg aca gtg gag gga ggc ctc agc gtt atc agc     816
Glu Arg Lys Gln Ile Val Thr Val Glu Gly Gly Leu Ser Val Ile Ser
            260                 265                 270

ccc aag tgg caa gaa caa gaa gac gaa gac gaa gac gaa gac gaa gaa     864
Pro Lys Trp Gln Glu Gln Glu Asp Glu Asp Glu Asp Glu Asp Glu Glu
        275                 280                 285

tat gga cgg acg ccc tct tat cct cca cga cga cca agc cat gga aag     912
Tyr Gly Arg Thr Pro Ser Tyr Pro Pro Arg Arg Pro Ser His Gly Lys
    290                 295                 300
```

```
cat gaa gat gac gag gac gag gac gaa gaa gaa gat caa cct cgt cct     960
His Glu Asp Asp Glu Asp Glu Asp Glu Glu Glu Asp Gln Pro Arg Pro
305                 310                 315                 320

gat cac cct cca cag cga cca agc agg ccc gaa caa caa gaa cca cgt    1008
Asp His Pro Pro Gln Arg Pro Ser Arg Pro Glu Gln Gln Glu Pro Arg
                325                 330                 335

gga aga gga tgt cag act aga aat ggg gtt gag gaa aat att tgc acc    1056
Gly Arg Gly Cys Gln Thr Arg Asn Gly Val Glu Glu Asn Ile Cys Thr
            340                 345                 350

atg aag ctt cac gag aac att gct cgc cct tca cgt gct gac ttc tac    1104
Met Lys Leu His Glu Asn Ile Ala Arg Pro Ser Arg Ala Asp Phe Tyr
        355                 360                 365

aac cca aaa gct ggt cgc att agc acc ctc aac agt ctc acc ctc cca    1152
Asn Pro Lys Ala Gly Arg Ile Ser Thr Leu Asn Ser Leu Thr Leu Pro
    370                 375                 380

gcc ctc cgc caa ttc gga ctc agt gcc caa tat gtt gtc ctc tac agg    1200
Ala Leu Arg Gln Phe Gly Leu Ser Ala Gln Tyr Val Val Leu Tyr Arg
385                 390                 395                 400

aat gga att tac tct cca gat tgg aac ttg aac gcg aac agt gtg acg    1248
Asn Gly Ile Tyr Ser Pro Asp Trp Asn Leu Asn Ala Asn Ser Val Thr
                405                 410                 415

atg act cga ggg aaa gga aga gtt aga gtg gtg aac tgc caa ggg aat    1296
Met Thr Arg Gly Lys Gly Arg Val Arg Val Val Asn Cys Gln Gly Asn
            420                 425                 430

gca gtg ttc gac ggt gag cta agg agg gga caa ttg cta gtg gtg ccg    1344
Ala Val Phe Asp Gly Glu Leu Arg Arg Gly Gln Leu Leu Val Val Pro
        435                 440                 445

cag aac ccc gcg gtg gct gag caa ggg gga gaa caa gga ttg gaa tat    1392
Gln Asn Pro Ala Val Ala Glu Gln Gly Gly Glu Gln Gly Leu Glu Tyr
    450                 455                 460

gta gtg ttc aag aca cac cac aac gcc gtg agc agc tac att aag gat    1440
Val Val Phe Lys Thr His His Asn Ala Val Ser Ser Tyr Ile Lys Asp
465                 470                 475                 480

gtg ttt agg gta atc cct tcg gag gtt ctt tcc aat tct tac aac ctt    1488
Val Phe Arg Val Ile Pro Ser Glu Val Leu Ser Asn Ser Tyr Asn Leu
                485                 490                 495

ggc cag agt caa gtg cgt cag ctc aag tat caa gga aac tcc ggc cct    1536
Gly Gln Ser Gln Val Arg Gln Leu Lys Tyr Gln Gly Asn Ser Gly Pro
            500                 505                 510

ttg gtc aac cca taa                                                1551
Leu Val Asn Pro
        515
```

<210> 26
<211> 516
<212> PRT
<213> Glycine max

<400> 26

```
Met Gly Lys Pro Phe Phe Thr Leu Ser Leu Ser Ser Leu Cys Leu Leu
 1               5                  10                  15
Leu Leu Ser Ser Ala Cys Phe Ala Ile Thr Ser Ser Lys Phe Asn Glu
            20                  25                  30
```

```
Cys Gln Leu Asn Asn Leu Asn Ala Leu Glu Pro Asp His Arg Val Glu
        35                  40                  45
Ser Glu Gly Gly Leu Ile Glu Thr Trp Asn Ser Gln His Pro Glu Leu
    50                  55                  60
Gln Cys Ala Gly Val Thr Val Ser Lys Arg Thr Leu Asn Arg Asn Gly
65                  70                  75                  80
Ser His Leu Pro Ser Tyr Leu Pro Tyr Pro Gln Met Ile Ile Val Val
                85                  90                  95
Gln Gly Lys Gly Ala Ile Gly Phe Ala Phe Pro Gly Cys Pro Glu Thr
            100                 105                 110
Phe Glu Lys Pro Gln Gln Gln Ser Ser Arg Arg Gly Ser Arg Ser Gln
        115                 120                 125
Gln Gln Leu Gln Asp Ser His Gln Lys Ile Arg His Phe Asn Glu Gly
    130                 135                 140
Asp Val Leu Val Ile Pro Leu Gly Val Pro Tyr Trp Thr Tyr Asn Thr
145                 150                 155                 160
Gly Asp Glu Pro Val Val Ala Ile Ser Pro Leu Asp Thr Ser Asn Phe
                165                 170                 175
Asn Asn Gln Leu Asp Gln Asn Pro Arg Val Phe Tyr Leu Ala Gly Asn
            180                 185                 190
Pro Asp Ile Glu His Pro Glu Thr Met Gln Gln Gln Gln Gln Gln Lys
        195                 200                 205
Ser His Gly Gly Arg Lys Gln Gly Gln His Arg Gln Gln Glu Glu Glu
    210                 215                 220
Gly Gly Ser Val Leu Ser Gly Phe Ser Lys His Phe Leu Ala Gln Ser
225                 230                 235                 240
Phe Asn Thr Asn Glu Asp Thr Ala Glu Lys Leu Arg Ser Pro Asp Asp
                245                 250                 255
Glu Arg Lys Gln Ile Val Thr Val Glu Gly Gly Leu Ser Val Ile Ser
            260                 265                 270
Pro Lys Trp Gln Glu Gln Glu Asp Glu Asp Glu Asp Glu Asp Glu Glu
        275                 280                 285
Tyr Gly Arg Thr Pro Ser Tyr Pro Pro Arg Arg Pro Ser His Gly Lys
    290                 295                 300
His Glu Asp Asp Glu Asp Glu Asp Glu Glu Glu Asp Gln Pro Arg Pro
305                 310                 315                 320
Asp His Pro Pro Gln Arg Pro Ser Arg Pro Glu Gln Gln Glu Pro Arg
                325                 330                 335
Gly Arg Gly Cys Gln Thr Arg Asn Gly Val Glu Glu Asn Ile Cys Thr
            340                 345                 350
Met Lys Leu His Glu Asn Ile Ala Arg Pro Ser Arg Ala Asp Phe Tyr
        355                 360                 365
Asn Pro Lys Ala Gly Arg Ile Ser Thr Leu Asn Ser Leu Thr Leu Pro
    370                 375                 380
Ala Leu Arg Gln Phe Gly Leu Ser Ala Gln Tyr Val Val Leu Tyr Arg
385                 390                 395                 400
Asn Gly Ile Tyr Ser Pro Asp Trp Asn Leu Asn Ala Asn Ser Val Thr
                405                 410                 415
```

Met Thr Arg Gly Lys Gly Arg Val Arg Val Val Asn Cys Gln Gly Asn
420 425 430

Ala Val Phe Asp Gly Glu Leu Arg Arg Gly Gln Leu Leu Val Val Pro
435 440 445

Gln Asn Pro Ala Val Ala Glu Gln Gly Gly Glu Gln Gly Leu Glu Tyr
450 455 460

Val Val Phe Lys Thr His His Asn Ala Val Ser Ser Tyr Ile Lys Asp
465 470 475 480

Val Phe Arg Val Ile Pro Ser Glu Val Leu Ser Asn Ser Tyr Asn Leu
485 490 495

Gly Gln Ser Gln Val Arg Gln Leu Lys Tyr Gln Gly Asn Ser Gly Pro
500 505 510

Leu Val Asn Pro
515

<210> 27
<211> 1446
<212> DNA
<213> Glycine max

<220>
<221> CDS
<222> (1)..(1443)
<223> glycinin G3 subunit

<400> 27

```
atg gct aag ctt gtt ctt tcc ctt tgt ttt ctg ctt ttc agt ggc tgc    48
Met Ala Lys Leu Val Leu Ser Leu Cys Phe Leu Leu Phe Ser Gly Cys
 1               5                  10                  15

tgc ttc gct ttc agt ttc aga gag cag cca cag caa aac gag tgc cag    96
Cys Phe Ala Phe Ser Phe Arg Glu Gln Pro Gln Gln Asn Glu Cys Gln
            20                  25                  30

atc caa cgc ctc aat gcc cta aaa ccg gat aac cgt ata gag tca gaa   144
Ile Gln Arg Leu Asn Ala Leu Lys Pro Asp Asn Arg Ile Glu Ser Glu
        35                  40                  45

ggt ggc ttc att gag aca tgg aac cct aac aac aag cca ttc cag tgt   192
Gly Gly Phe Ile Glu Thr Trp Asn Pro Asn Asn Lys Pro Phe Gln Cys
    50                  55                  60

gcc ggt gtt gcc ctc tct cgc tgc acc ctc aac cgc aac gcc ctt cgc   240
Ala Gly Val Ala Leu Ser Arg Cys Thr Leu Asn Arg Asn Ala Leu Arg
65                  70                  75                  80

aga cct tcc tac acc aac gct ccc cag gag atc tac atc caa caa ggt   288
Arg Pro Ser Tyr Thr Asn Ala Pro Gln Glu Ile Tyr Ile Gln Gln Gly
                85                  90                  95

agt ggt att ttt ggc atg ata ttc ccg ggt tgt cct agc aca ttt gaa   336
Ser Gly Ile Phe Gly Met Ile Phe Pro Gly Cys Pro Ser Thr Phe Glu
            100                 105                 110

gag cct caa caa aaa gga caa agc agc agg ccc caa gac cgt cac cag   384
Glu Pro Gln Gln Lys Gly Gln Ser Ser Arg Pro Gln Asp Arg His Gln
        115                 120                 125

aag atc tat cac ttc aga gag ggt gat ttg att gca gtg cca acc ggt   432
Lys Ile Tyr His Phe Arg Glu Gly Asp Leu Ile Ala Val Pro Thr Gly
    130                 135                 140
```

```
ttt gca tac tgg atg tac aac aat gaa gac act cct gtt gtt gcc gtt    480
Phe Ala Tyr Trp Met Tyr Asn Asn Glu Asp Thr Pro Val Val Ala Val
145                 150                 155                 160

tct ctt att gac acc aac agc ttc cag aac cag ctc gac cag atg cct    528
Ser Leu Ile Asp Thr Asn Ser Phe Gln Asn Gln Leu Asp Gln Met Pro
                165                 170                 175

agg aga ttc tat ctt gct ggg aac caa gag caa gag ttt cta cag tat    576
Arg Arg Phe Tyr Leu Ala Gly Asn Gln Glu Gln Glu Phe Leu Gln Tyr
            180                 185                 190

cag cca cag aag cag caa gga ggt act caa agc cag aaa gga aag cgt    624
Gln Pro Gln Lys Gln Gln Gly Gly Thr Gln Ser Gln Lys Gly Lys Arg
        195                 200                 205

cag caa gaa gaa gaa aac gaa gga ggc agc ata ttg agt ggc ttc gcc    672
Gln Gln Glu Glu Glu Asn Glu Gly Gly Ser Ile Leu Ser Gly Phe Ala
    210                 215                 220

ccg gaa ttc ttg gaa cat gcg ttc gtc gtg gac agg cag ata gtg aga    720
Pro Glu Phe Leu Glu His Ala Phe Val Val Asp Arg Gln Ile Val Arg
225                 230                 235                 240

aag cta caa ggt gag aac gaa gag gaa gag aag ggt gcc att gtg aca    768
Lys Leu Gln Gly Glu Asn Glu Glu Glu Glu Lys Gly Ala Ile Val Thr
                245                 250                 255

gtg aaa gga ggt ctc agc gtg ata agc cca ccc acg gaa gag cag caa    816
Val Lys Gly Gly Leu Ser Val Ile Ser Pro Pro Thr Glu Glu Gln Gln
            260                 265                 270

caa aga ccc gag gaa gag gag aag cca gat tgt gac gag aaa gac aaa    864
Gln Arg Pro Glu Glu Glu Glu Lys Pro Asp Cys Asp Glu Lys Asp Lys
        275                 280                 285

cat tgc caa agc caa agc aga aat ggc att gac gag acc att tgc aca    912
His Cys Gln Ser Gln Ser Arg Asn Gly Ile Asp Glu Thr Ile Cys Thr
    290                 295                 300

atg aga ctt cgc cac aac att ggc cag act tca tca cct gac atc ttc    960
Met Arg Leu Arg His Asn Ile Gly Gln Thr Ser Ser Pro Asp Ile Phe
305                 310                 315                 320

aac cct caa gct ggt agc atc aca acc gct acc agc ctc gac ttc cca    1008
Asn Pro Gln Ala Gly Ser Ile Thr Thr Ala Thr Ser Leu Asp Phe Pro
                325                 330                 335

gcc ctc tcg tgg ctc aaa ctc agt gcc cag ttt gga tca ctc cgc aag    1056
Ala Leu Ser Trp Leu Lys Leu Ser Ala Gln Phe Gly Ser Leu Arg Lys
            340                 345                 350

aat gct atg ttc gtg cca cac tac aac ctg aac gca aac agc ata ata    1104
Asn Ala Met Phe Val Pro His Tyr Asn Leu Asn Ala Asn Ser Ile Ile
        355                 360                 365

tac gca ttg aat gga cgg gca ttg gta caa gtg gtg aat tgc aat ggt    1152
Tyr Ala Leu Asn Gly Arg Ala Leu Val Gln Val Val Asn Cys Asn Gly
    370                 375                 380

gag aga gtg ttt gat gga gag ctg caa gag gga cag gtg tta att gtg    1200
Glu Arg Val Phe Asp Gly Glu Leu Gln Glu Gly Gln Val Leu Ile Val
385                 390                 395                 400

cca caa aac ttt gcg gtg gct gca aga tca cag agc gac aac ttc gag    1248
Pro Gln Asn Phe Ala Val Ala Ala Arg Ser Gln Ser Asp Asn Phe Glu
                405                 410                 415
```

```
tat gtt tca ttc aag acc aat gat aga ccc tcg atc ggc aac ctt gca    1296
Tyr Val Ser Phe Lys Thr Asn Asp Arg Pro Ser Ile Gly Asn Leu Ala
            420                 425                 430

ggt gca aac tca ttg ttg aac gca ttg ccg gag gaa gtg att cag caa    1344
Gly Ala Asn Ser Leu Leu Asn Ala Leu Pro Glu Glu Val Ile Gln Gln
        435                 440                 445

act ttt aac cta agg agg cag cag gcc agg cag gtc aag aac aac aac    1392
Thr Phe Asn Leu Arg Arg Gln Gln Ala Arg Gln Val Lys Asn Asn Asn
    450                 455                 460

cct ttc agc ttc ctg gtt cca cct aag gag tct cag agg aga gtt gtg    1440
Pro Phe Ser Phe Leu Val Pro Pro Lys Glu Ser Gln Arg Arg Val Val
465                 470                 475                 480

gct tag                                                            1446
Ala
```

<210> 28
<211> 481
<212> PRT
<213> Glycine max

<400> 28

```
Met Ala Lys Leu Val Leu Ser Leu Cys Phe Leu Leu Phe Ser Gly Cys
1               5                   10                  15

Cys Phe Ala Phe Ser Phe Arg Glu Gln Pro Gln Gln Asn Glu Cys Gln
            20                  25                  30

Ile Gln Arg Leu Asn Ala Leu Lys Pro Asp Asn Arg Ile Glu Ser Glu
        35                  40                  45

Gly Gly Phe Ile Glu Thr Trp Asn Pro Asn Asn Lys Pro Phe Gln Cys
    50                  55                  60

Ala Gly Val Ala Leu Ser Arg Cys Thr Leu Asn Arg Asn Ala Leu Arg
65                  70                  75                  80

Arg Pro Ser Tyr Thr Asn Ala Pro Gln Glu Ile Tyr Ile Gln Gln Gly
                85                  90                  95

Ser Gly Ile Phe Gly Met Ile Phe Pro Gly Cys Pro Ser Thr Phe Glu
            100                 105                 110

Glu Pro Gln Gln Lys Gly Gln Ser Ser Arg Pro Gln Asp Arg His Gln
        115                 120                 125

Lys Ile Tyr His Phe Arg Glu Gly Asp Leu Ile Ala Val Pro Thr Gly
    130                 135                 140

Phe Ala Tyr Trp Met Tyr Asn Asn Glu Asp Thr Pro Val Val Ala Val
145                 150                 155                 160

Ser Leu Ile Asp Thr Asn Ser Phe Gln Asn Gln Leu Asp Gln Met Pro
                165                 170                 175

Arg Arg Phe Tyr Leu Ala Gly Asn Gln Glu Gln Glu Phe Leu Gln Tyr
            180                 185                 190

Gln Pro Gln Lys Gln Gln Gly Gly Thr Gln Ser Gln Lys Gly Lys Arg
        195                 200                 205

Gln Gln Glu Glu Glu Asn Glu Gly Gly Ser Ile Leu Ser Gly Phe Ala
    210                 215                 220

Pro Glu Phe Leu Glu His Ala Phe Val Val Asp Arg Gln Ile Val Arg
225                 230                 235                 240
```

```
Lys Leu Gln Gly Glu Asn Glu Glu Glu Glu Lys Gly Ala Ile Val Thr
                245                 250                 255

Val Lys Gly Gly Leu Ser Val Ile Ser Pro Pro Thr Glu Glu Gln Gln
            260                 265                 270

Gln Arg Pro Glu Glu Glu Glu Lys Pro Asp Cys Asp Glu Lys Asp Lys
        275                 280                 285

His Cys Gln Ser Gln Ser Arg Asn Gly Ile Asp Glu Thr Ile Cys Thr
    290                 295                 300

Met Arg Leu Arg His Asn Ile Gly Gln Thr Ser Ser Pro Asp Ile Phe
305                 310                 315                 320

Asn Pro Gln Ala Gly Ser Ile Thr Thr Ala Thr Ser Leu Asp Phe Pro
                325                 330                 335

Ala Leu Ser Trp Leu Lys Leu Ser Ala Gln Phe Gly Ser Leu Arg Lys
            340                 345                 350

Asn Ala Met Phe Val Pro His Tyr Asn Leu Asn Ala Asn Ser Ile Ile
        355                 360                 365

Tyr Ala Leu Asn Gly Arg Ala Leu Val Gln Val Val Asn Cys Asn Gly
    370                 375                 380

Glu Arg Val Phe Asp Gly Glu Leu Gln Glu Gly Gln Val Leu Ile Val
385                 390                 395                 400

Pro Gln Asn Phe Ala Val Ala Ala Arg Ser Gln Ser Asp Asn Phe Glu
                405                 410                 415

Tyr Val Ser Phe Lys Thr Asn Asp Arg Pro Ser Ile Gly Asn Leu Ala
            420                 425                 430

Gly Ala Asn Ser Leu Leu Asn Ala Leu Pro Glu Glu Val Ile Gln Gln
        435                 440                 445

Thr Phe Asn Leu Arg Arg Gln Gln Ala Arg Gln Val Lys Asn Asn Asn
    450                 455                 460

Pro Phe Ser Phe Leu Val Pro Pro Lys Glu Ser Gln Arg Arg Val Val
465                 470                 475                 480

Ala
```

<210> 29
<211> 1482
<212> DNA
<213> Helianthus annuus

<220>
<221> CDS
<222> (1)..(1479)
<223> 11S storage protein G3-D1

<400> 29

```
atg gca tcc aaa gca act ttg ctc tta gct ttt acc ctt ctc ttt gcc      48
Met Ala Ser Lys Ala Thr Leu Leu Leu Ala Phe Thr Leu Leu Phe Ala
1               5                   10                  15

act tgc att gcc cgc cac cag caa cgg caa cag caa cag aac cag tgc      96
Thr Cys Ile Ala Arg His Gln Gln Arg Gln Gln Gln Gln Asn Gln Cys
            20                  25                  30
```

```
cag ctt caa aac atc gag gcg ctc gag ccc atc gaa gtt atc caa gct    144
Gln Leu Gln Asn Ile Glu Ala Leu Glu Pro Ile Glu Val Ile Gln Ala
        35                  40                  45

gaa gcc ggt gtg acc gaa att tgg gac gcc tat gac caa cag ttc cag    192
Glu Ala Gly Val Thr Glu Ile Trp Asp Ala Tyr Asp Gln Gln Phe Gln
    50                  55                  60

tgt gcg tgg tcg att tta ttc gac acc gga ttc aac ctg gtg gcc ttc    240
Cys Ala Trp Ser Ile Leu Phe Asp Thr Gly Phe Asn Leu Val Ala Phe
65                  70                  75                  80

tct tgc ctt cct acg tca aca ccc cta ttt tgg cct tcg tcg aga gag    288
Ser Cys Leu Pro Thr Ser Thr Pro Leu Phe Trp Pro Ser Ser Arg Glu
                85                  90                  95

ggg gtt ata ttg ccg gga tgc cgc aga acc tat gaa tat tcg cag gag    336
Gly Val Ile Leu Pro Gly Cys Arg Arg Thr Tyr Glu Tyr Ser Gln Glu
            100                 105                 110

caa cag ttt tcc ggt gag ggt ggc cgc aga gga gga gga gag ggc aca    384
Gln Gln Phe Ser Gly Glu Gly Gly Arg Arg Gly Gly Gly Glu Gly Thr
        115                 120                 125

ttc agg acc gtc atc aga aag tta gag aac tta aag gag ggt gac gtg    432
Phe Arg Thr Val Ile Arg Lys Leu Glu Asn Leu Lys Glu Gly Asp Val
    130                 135                 140

gtt gcc atc ccc acc gga aca gct cac tgg ctt cac aac gac ggc aac    480
Val Ala Ile Pro Thr Gly Thr Ala His Trp Leu His Asn Asp Gly Asn
145                 150                 155                 160

aca gaa ctt gtg gtc gtc ttc ttg gat act cag aac cat gag aac cag    528
Thr Glu Leu Val Val Val Phe Leu Asp Thr Gln Asn His Glu Asn Gln
                165                 170                 175

ctt gac gaa aac caa agg aga ttc ttc tta gcc gga aac cct caa gct    576
Leu Asp Glu Asn Gln Arg Arg Phe Phe Leu Ala Gly Asn Pro Gln Ala
            180                 185                 190

caa gct caa agc cag cag caa caa caa aga caa cca cgc caa caa tct    624
Gln Ala Gln Ser Gln Gln Gln Gln Gln Arg Gln Pro Arg Gln Gln Ser
        195                 200                 205

cct caa agg caa agg caa agg caa agg caa ggg caa ggt cag aac gcc    672
Pro Gln Arg Gln Arg Gln Arg Gln Arg Gln Gly Gln Gly Gln Asn Ala
    210                 215                 220

ggc aac atc ttc aac ggt ttc acc ccc gag ctc att gca caa tca ttc    720
Gly Asn Ile Phe Asn Gly Phe Thr Pro Glu Leu Ile Ala Gln Ser Phe
225                 230                 235                 240

aac gtc gac caa gag acc gcc cag aag cta caa gga caa aac gac cag    768
Asn Val Asp Gln Glu Thr Ala Gln Lys Leu Gln Gly Gln Asn Asp Gln
                245                 250                 255

aga ggc cac att gtt aat gtc gga caa gac ctt caa ata gtc cgc cca    816
Arg Gly His Ile Val Asn Val Gly Gln Asp Leu Gln Ile Val Arg Pro
            260                 265                 270

cca caa gac aga cgc tct cct cgc caa caa caa gag caa gcg acg tct    864
Pro Gln Asp Arg Arg Ser Pro Arg Gln Gln Gln Glu Gln Ala Thr Ser
        275                 280                 285

cct cgc caa caa caa gag cag cag caa ggc aga cgt ggc gga tgg agc    912
Pro Arg Gln Gln Gln Glu Gln Gln Gln Gly Arg Arg Gly Gly Trp Ser
    290                 295                 300
```

```
aac ggt gtg gaa gaa acc atc tgc agc atg aag ttc aaa gtg aac att   960
Asn Gly Val Glu Glu Thr Ile Cys Ser Met Lys Phe Lys Val Asn Ile
305                 310                 315                 320

gac aac cct tcc cag gct gac ttt gta aac ccg caa gcc ggc agc att   1008
Asp Asn Pro Ser Gln Ala Asp Phe Val Asn Pro Gln Ala Gly Ser Ile
                325                 330                 335

gca aac ctc aac agc ttc aaa ttc ccc att ctc gag cac ctc cgg ctc   1056
Ala Asn Leu Asn Ser Phe Lys Phe Pro Ile Leu Glu His Leu Arg Leu
            340                 345                 350

agc gtg gaa aga ggc gaa ctc cgt ccg aat gcc atc caa tcc cca cac   1104
Ser Val Glu Arg Gly Glu Leu Arg Pro Asn Ala Ile Gln Ser Pro His
        355                 360                 365

tgg aca atc aac gcc cac aat ctt ctc tac gta acc gag gga gcc ttg   1152
Trp Thr Ile Asn Ala His Asn Leu Leu Tyr Val Thr Glu Gly Ala Leu
    370                 375                 380

agg gta caa atc gtc gac aac caa gga aac tca gtt ttc gac aac gag   1200
Arg Val Gln Ile Val Asp Asn Gln Gly Asn Ser Val Phe Asp Asn Glu
385                 390                 395                 400

ctc cgt gag gga cag gtg gtg gtg atc ccg cag aac ttt gcg gtg atc   1248
Leu Arg Glu Gly Gln Val Val Val Ile Pro Gln Asn Phe Ala Val Ile
                405                 410                 415

aag aga gcc aat gaa caa gga agc agg tgg gtg tct ttc aag act aat   1296
Lys Arg Ala Asn Glu Gln Gly Ser Arg Trp Val Ser Phe Lys Thr Asn
            420                 425                 430

gat aat gcc atg ata gca aac ctt gca ggg cgt gtg tcc gca tca gca   1344
Asp Asn Ala Met Ile Ala Asn Leu Ala Gly Arg Val Ser Ala Ser Ala
        435                 440                 445

gca tcg ccg ttg acg ttg tgg gcg aat cgg tat cag cta tct cga gag   1392
Ala Ser Pro Leu Thr Leu Trp Ala Asn Arg Tyr Gln Leu Ser Arg Glu
    450                 455                 460

gaa gct cag cag ctc aag ttt agc cag agg gag acg gtt ttg ttt gca   1440
Glu Ala Gln Gln Leu Lys Phe Ser Gln Arg Glu Thr Val Leu Phe Ala
465                 470                 475                 480

cca agt ttt tcc agg ggc caa ggg atc agg gct tca cgt taa           1482
Pro Ser Phe Ser Arg Gly Gln Gly Ile Arg Ala Ser Arg
                485                 490
```

<210> 30
<211> 493
<212> PRT
<213> Helianthus annuus

<400> 30

```
Met Ala Ser Lys Ala Thr Leu Leu Leu Ala Phe Thr Leu Leu Phe Ala
 1               5                  10                  15
Thr Cys Ile Ala Arg His Gln Gln Arg Gln Gln Gln Gln Asn Gln Cys
            20                  25                  30
Gln Leu Gln Asn Ile Glu Ala Leu Glu Pro Ile Glu Val Ile Gln Ala
        35                  40                  45
Glu Ala Gly Val Thr Glu Ile Trp Asp Ala Tyr Asp Gln Gln Phe Gln
    50                  55                  60
Cys Ala Trp Ser Ile Leu Phe Asp Thr Gly Phe Asn Leu Val Ala Phe
65                  70                  75                  80
```

Ser Cys Leu Pro Thr Ser Thr Pro Leu Phe Trp Pro Ser Ser Arg Glu
85 90 95

Gly Val Ile Leu Pro Gly Cys Arg Arg Thr Tyr Glu Tyr Ser Gln Glu
100 105 110

Gln Gln Phe Ser Gly Glu Gly Gly Arg Arg Gly Gly Gly Glu Gly Thr
115 120 125

Phe Arg Thr Val Ile Arg Lys Leu Glu Asn Leu Lys Glu Gly Asp Val
130 135 140

Val Ala Ile Pro Thr Gly Thr Ala His Trp Leu His Asn Asp Gly Asn
145 150 155 160

Thr Glu Leu Val Val Val Phe Leu Asp Thr Gln Asn His Glu Asn Gln
165 170 175

Leu Asp Glu Asn Gln Arg Arg Phe Phe Leu Ala Gly Asn Pro Gln Ala
180 185 190

Gln Ala Gln Ser Gln Gln Gln Gln Gln Arg Gln Pro Arg Gln Gln Ser
195 200 205

Pro Gln Arg Gln Arg Gln Arg Gln Arg Gln Gly Gln Gly Gln Asn Ala
210 215 220

Gly Asn Ile Phe Asn Gly Phe Thr Pro Glu Leu Ile Ala Gln Ser Phe
225 230 235 240

Asn Val Asp Gln Glu Thr Ala Gln Lys Leu Gln Gly Gln Asn Asp Gln
245 250 255

Arg Gly His Ile Val Asn Val Gly Gln Asp Leu Gln Ile Val Arg Pro
260 265 270

Pro Gln Asp Arg Arg Ser Pro Arg Gln Gln Gln Glu Gln Ala Thr Ser
275 280 285

Pro Arg Gln Gln Gln Glu Gln Gln Gln Gly Arg Arg Gly Gly Trp Ser
290 295 300

Asn Gly Val Glu Glu Thr Ile Cys Ser Met Lys Phe Lys Val Asn Ile
305 310 315 320

Asp Asn Pro Ser Gln Ala Asp Phe Val Asn Pro Gln Ala Gly Ser Ile
325 330 335

Ala Asn Leu Asn Ser Phe Lys Phe Pro Ile Leu Glu His Leu Arg Leu
340 345 350

Ser Val Glu Arg Gly Glu Leu Arg Pro Asn Ala Ile Gln Ser Pro His
355 360 365

Trp Thr Ile Asn Ala His Asn Leu Leu Tyr Val Thr Glu Gly Ala Leu
370 375 380

Arg Val Gln Ile Val Asp Asn Gln Gly Asn Ser Val Phe Asp Asn Glu
385 390 395 400

Leu Arg Glu Gly Gln Val Val Val Ile Pro Gln Asn Phe Ala Val Ile
405 410 415

Lys Arg Ala Asn Glu Gln Gly Ser Arg Trp Val Ser Phe Lys Thr Asn
420 425 430

Asp Asn Ala Met Ile Ala Asn Leu Ala Gly Arg Val Ser Ala Ser Ala
435 440 445

Ala Ser Pro Leu Thr Leu Trp Ala Asn Arg Tyr Gln Leu Ser Arg Glu
450 455 460

Glu Ala Gln Gln Leu Lys Phe Ser Gln Arg Glu Thr Val Leu Phe Ala
465 470 475 480

Pro Ser Phe Ser Arg Gly Gln Gly Ile Arg Ala Ser Arg
485 490

<210> 31
<211> 537
<212> DNA
<213> Brassica napus

<220>
<221> CDS
<222> (1) .. (534)
<223> NAPIN

<400> 31

```
atg gcg aac aag ctc ttc ctc gtc tcg gca act ctc gcc ttc ttc ttc      48
Met Ala Asn Lys Leu Phe Leu Val Ser Ala Thr Leu Ala Phe Phe Phe
1               5                   10                  15

ctt ctc acc aat gcc tcc atc tac cgg acg gtc gtc gag ttc gac gaa      96
Leu Leu Thr Asn Ala Ser Ile Tyr Arg Thr Val Val Glu Phe Asp Glu
            20                  25                  30

gat gat gcc aca gac tca gcc ggc cca ttt agg att cca aaa tgt agg      144
Asp Asp Ala Thr Asp Ser Ala Gly Pro Phe Arg Ile Pro Lys Cys Arg
        35                  40                  45

aag gag ttt cag caa gca caa cac cta aga gct tgc cag cag tgg ctc      192
Lys Glu Phe Gln Gln Ala Gln His Leu Arg Ala Cys Gln Gln Trp Leu
    50                  55                  60

cac aag caa gca atg cag tct ggc ggt ggt cct agc tgg acc ctc gac      240
His Lys Gln Ala Met Gln Ser Gly Gly Gly Pro Ser Trp Thr Leu Asp
65                  70                  75                  80

ggt gag ttt gac ttt gaa gac gac atg gag aac ccg cag ggt cca cag      288
Gly Glu Phe Asp Phe Glu Asp Asp Met Glu Asn Pro Gln Gly Pro Gln
                85                  90                  95

cag aga ccg cct cta ctc cag cag tgc tgt aac gag ctc cac cag gaa      336
Gln Arg Pro Pro Leu Leu Gln Gln Cys Cys Asn Glu Leu His Gln Glu
            100                 105                 110

gag ccc ctt tgc gtt tgc cca acc ttg aaa gga gca tcc aaa gcg gtt      384
Glu Pro Leu Cys Val Cys Pro Thr Leu Lys Gly Ala Ser Lys Ala Val
        115                 120                 125

aaa caa caa att caa caa cag gga caa cag caa gga aag cag caa atg      432
Lys Gln Gln Ile Gln Gln Gln Gly Gln Gln Gln Gly Lys Gln Gln Met
    130                 135                 140

gtg agc cgt atc tac cag acc gct acg cac tta cct aaa gtt tgc aac      480
Val Ser Arg Ile Tyr Gln Thr Ala Thr His Leu Pro Lys Val Cys Asn
145                 150                 155                 160

atc ccg caa gtt agc gtt tgt ccc ttc cag aag acc atg cct ggg ccc      528
Ile Pro Gln Val Ser Val Cys Pro Phe Gln Lys Thr Met Pro Gly Pro
                165                 170                 175

tcc tac tag                                                          537
Ser Tyr
```

<210> 32
<211> 178
<212> PRT
<213> Brassica napus

<400> 32

```
Met Ala Asn Lys Leu Phe Leu Val Ser Ala Thr Leu Ala Phe Phe Phe
1               5                   10                  15

Leu Leu Thr Asn Ala Ser Ile Tyr Arg Thr Val Val Glu Phe Asp Glu
            20                  25                  30

Asp Asp Ala Thr Asp Ser Ala Gly Pro Phe Arg Ile Pro Lys Cys Arg
        35                  40                  45

Lys Glu Phe Gln Gln Ala Gln His Leu Arg Ala Cys Gln Gln Trp Leu
    50                  55                  60

His Lys Gln Ala Met Gln Ser Gly Gly Gly Pro Ser Trp Thr Leu Asp
65                  70                  75                  80

Gly Glu Phe Asp Phe Glu Asp Asp Met Glu Asn Pro Gln Gly Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Ile Gln Gln Gln Gly Gln Gln Gln Gly Lys Gln Gln Met
    130                 135                 140

Val Ser Arg Ile Tyr Gln Thr Ala Thr His Leu Pro Lys Val Cys Asn
145                 150                 155                 160

Ile Pro Gln Val Ser Val Cys Pro Phe Gln Lys Thr Met Pro Gly Pro
                165                 170                 175

Ser Tyr
```

<210> 33
<211> 537
<212> DNA
<213> Brassica juncea

<220>
<221> CDS
<222> (1)..(534)
<223> 2S storage protein

<400> 33

```
atg gcg aac aag ctc ttc ctc gtc tcg gca act ctc gcc ttc ttc ttc      48
Met Ala Asn Lys Leu Phe Leu Val Ser Ala Thr Leu Ala Phe Phe Phe
1               5                   10                  15

ctt ctc acc aat gcc tcc atc tac cgg acg gtc gtc gag ttc gac gaa      96
Leu Leu Thr Asn Ala Ser Ile Tyr Arg Thr Val Val Glu Phe Asp Glu
            20                  25                  30

gat gat gcc aca gac tca gcc ggc cca ttt agg att cca aaa tgt agg      144
Asp Asp Ala Thr Asp Ser Ala Gly Pro Phe Arg Ile Pro Lys Cys Arg
        35                  40                  45

aag gag ttt cag caa gca caa cac cta aga gtc tgc cag cag tgg ctc      192
Lys Glu Phe Gln Gln Ala Gln His Leu Arg Val Cys Gln Gln Trp Leu
    50                  55                  60
```

```
cac aag caa gca atg cag tct ggc ggt ggt ctc agc tgg acc ctc gac     240
His Lys Gln Ala Met Gln Ser Gly Gly Gly Leu Ser Trp Thr Leu Asp
 65                 70                  75                  80

ggt gag ttt gac ttt gaa gac gac atg gag aac tcg cag ggt cca cag     288
Gly Glu Phe Asp Phe Glu Asp Asp Met Glu Asn Ser Gln Gly Pro Gln
                85                  90                  95

cag aga ccg cct cta ctc cag cag tgc tgt aac gag ctc cac cag gaa     336
Gln Arg Pro Pro Leu Leu Gln Gln Cys Cys Asn Glu Leu His Gln Glu
            100                 105                 110

gag ccc ctt tgc gtt tgc cca acc ttg aaa gga gca tcc aaa gcg gtt     384
Glu Pro Leu Cys Val Cys Pro Thr Leu Lys Gly Ala Ser Lys Ala Val
        115                 120                 125

aaa caa caa att caa caa cag gga caa cag caa gga aag cag caa atg     432
Lys Gln Gln Ile Gln Gln Gln Gly Gln Gln Gln Gly Lys Gln Gln Met
    130                 135                 140

gtg agc cgt atc tac cag acc gct acg cac tta cct aaa gtt tgc aac     480
Val Ser Arg Ile Tyr Gln Thr Ala Thr His Leu Pro Lys Val Cys Asn
145                 150                 155                 160

atc ccg caa gtt agc gtt tgt ccc ttc cag aag acc atg cct ggg ccc     528
Ile Pro Gln Val Ser Val Cys Pro Phe Gln Lys Thr Met Pro Gly Pro
                165                 170                 175

tcc tac tag                                                         537
Ser Tyr
```

<210> 34
<211> 178
<212> PRT
<213> Brassica juncea

<400> 34

Met Ala Asn Lys Leu Phe Leu Val Ser Ala Thr Leu Ala Phe Phe Phe
1 5 10 15

Leu Leu Thr Asn Ala Ser Ile Tyr Arg Thr Val Val Glu Phe Asp Glu
20 25 30

Asp Asp Ala Thr Asp Ser Ala Gly Pro Phe Arg Ile Pro Lys Cys Arg
35 40 45

Lys Glu Phe Gln Gln Ala Gln His Leu Arg Val Cys Gln Gln Trp Leu
50 55 60

His Lys Gln Ala Met Gln Ser Gly Gly Gly Leu Ser Trp Thr Leu Asp
65 70 75 80

Gly Glu Phe Asp Phe Glu Asp Asp Met Glu Asn Ser Gln Gly Pro Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Ile Gln Gln Gln Gly Gln Gln Gln Gly Lys Gln Gln Met
130 135 140

Val Ser Arg Ile Tyr Gln Thr Ala Thr His Leu Pro Lys Val Cys Asn
145 150 155 160

Ile Pro Gln Val Ser Val Cys Pro Phe Gln Lys Thr Met Pro Gly Pro
165 170 175

Ser Tyr

<210> 35
<211> 537
<212> DNA
<213> Brassica oleracea

<220>
<221> CDS
<222> (1)..(534)
<223> 2S storage protein

<400> 35

```
atg gcg aac aag ctc ttc ctc gtc tcg gca act ctc gcc ttc ttc ttc     48
Met Ala Asn Lys Leu Phe Leu Val Ser Ala Thr Leu Ala Phe Phe Phe
1               5                   10                  15

ctt ctc acc aat gcc tcc atc tac cgg acg gtg gtc gag ttc gac gaa     96
Leu Leu Thr Asn Ala Ser Ile Tyr Arg Thr Val Val Glu Phe Asp Glu
            20                  25                  30

gat gat gcc aca aac cca gcc ggc cca ttt agg atc cca aaa tgt agg    144
Asp Asp Ala Thr Asn Pro Ala Gly Pro Phe Arg Ile Pro Lys Cys Arg
        35                  40                  45

aag gag ttt cag caa gca caa cac cta aga gct tgc cag cag tgg ctc    192
Lys Glu Phe Gln Gln Ala Gln His Leu Arg Ala Cys Gln Gln Trp Leu
    50                  55                  60

cac aag caa gca atg cag tct ggc ggt ggt cct agc tgg acc ctc gac    240
His Lys Gln Ala Met Gln Ser Gly Gly Gly Pro Ser Trp Thr Leu Asp
65                  70                  75                  80

agt gag ttt gac ttt gaa gac gac atg gag aac ccg cag ggt cca cag    288
Ser Glu Phe Asp Phe Glu Asp Asp Met Glu Asn Pro Gln Gly Pro Gln
                85                  90                  95

cag aga ccg cct cta ctc ctg caa tgc tgt aac gag ctg gac cag gaa    336
Gln Arg Pro Pro Leu Leu Leu Gln Cys Cys Asn Glu Leu Asp Gln Glu
            100                 105                 110

gag ccc ctt tgc gtt tgc cca acc ttg aaa gga gca tcc aaa gcg gtt    384
Glu Pro Leu Cys Val Cys Pro Thr Leu Lys Gly Ala Ser Lys Ala Val
        115                 120                 125

aaa caa caa att caa caa cag gga caa cag caa gga aag cag caa atg    432
Lys Gln Gln Ile Gln Gln Gln Gly Gln Gln Gln Gly Lys Gln Gln Met
    130                 135                 140

gtg agc cgt atc tac cag acc gct acg cac tta cct aaa gtt tgc aac    480
Val Ser Arg Ile Tyr Gln Thr Ala Thr His Leu Pro Lys Val Cys Asn
145                 150                 155                 160

atc ccg caa gtt agc gtt tgt ccc ttc cag aag acc atg cct ggg ccc    528
Ile Pro Gln Val Ser Val Cys Pro Phe Gln Lys Thr Met Pro Gly Pro
                165                 170                 175

tcc tac tag                                                        537
Ser Tyr
```

<210> 36
<211> 178
<212> PRT
<213> Brassica oleracea

<400> 36

```
Met Ala Asn Lys Leu Phe Leu Val Ser Ala Thr Leu Ala Phe Phe Phe
1               5                   10                  15

Leu Leu Thr Asn Ala Ser Ile Tyr Arg Thr Val Val Glu Phe Asp Glu
            20                  25                  30

Asp Asp Ala Thr Asn Pro Ala Gly Pro Phe Arg Ile Pro Lys Cys Arg
        35                  40                  45

Lys Glu Phe Gln Gln Ala Gln His Leu Arg Ala Cys Gln Gln Trp Leu
    50                  55                  60

His Lys Gln Ala Met Gln Ser Gly Gly Gly Pro Ser Trp Thr Leu Asp
65                  70                  75                  80

Ser Glu Phe Asp Phe Glu Asp Asp Met Glu Asn Pro Gln Gly Pro Gln
                85                  90                  95

Gln Arg Pro Pro Leu Leu Leu Gln Cys Cys Asn Glu Leu Asp Gln Glu
            100                 105                 110

Glu Pro Leu Cys Val Cys Pro Thr Leu Lys Gly Ala Ser Lys Ala Val
        115                 120                 125

Lys Gln Gln Ile Gln Gln Gln Gly Gln Gln Gln Gly Lys Gln Gln Met
    130                 135                 140

Val Ser Arg Ile Tyr Gln Thr Ala Thr His Leu Pro Lys Val Cys Asn
145                 150                 155                 160

Ile Pro Gln Val Ser Val Cys Pro Phe Gln Lys Thr Met Pro Gly Pro
                165                 170                 175

Ser Tyr
```

<210> 37
<211> 543
<212> DNA
<213> Brassica napus cv. Topas

<220>
<221> CDS
<222> (1)..(540)
<223> Napin

<400> 37

```
atg gcg aac aag ctc ttc ctc gtc tcg gca act ctt gcc ttc ttc ttc    48
Met Ala Asn Lys Leu Phe Leu Val Ser Ala Thr Leu Ala Phe Phe Phe
 1               5                  10                  15

ctt ctc acc aac gcc tcc atc tac cgc acc atc gtg gaa gtc gac gaa    96
Leu Leu Thr Asn Ala Ser Ile Tyr Arg Thr Ile Val Glu Val Asp Glu
            20                  25                  30

gat gat gcc aca aac cca gcc ggc cca ttt agg att cca aaa tgt agg   144
Asp Asp Ala Thr Asn Pro Ala Gly Pro Phe Arg Ile Pro Lys Cys Arg
        35                  40                  45

aag gag ttt cag caa gca caa cac ctg aaa gct tgc caa caa tgg ctc   192
Lys Glu Phe Gln Gln Ala Gln His Leu Lys Ala Cys Gln Gln Trp Leu
    50                  55                  60

cac aag cag gca atg cag tcc ggt agt ggc cca agc tgg acc ctc gac   240
His Lys Gln Ala Met Gln Ser Gly Ser Gly Pro Ser Trp Thr Leu Asp
65                  70                  75                  80

ggt gag ttt gat ttt gaa gat gac atg gag aac ccc cag ggc cca caa   288
Gly Glu Phe Asp Phe Glu Asp Asp Met Glu Asn Pro Gln Gly Pro Gln
                85                  90                  95

cag agg ccg cca cta ctc cag cag tgc tgc aac gag ctc cac cag gaa   336
Gln Arg Pro Pro Leu Leu Gln Gln Cys Cys Asn Glu Leu His Gln Glu
            100                 105                 110

gag cca ctt tgc gtt tgc cca acc ttg aaa gga gca tcc aaa gcc gtt   384
Glu Pro Leu Cys Val Cys Pro Thr Leu Lys Gly Ala Ser Lys Ala Val
        115                 120                 125

aaa caa cag gtt cga caa cag caa gga cag cag gga cag cag ctg cag   432
Lys Gln Gln Val Arg Gln Gln Gln Gly Gln Gln Gly Gln Gln Leu Gln
    130                 135                 140

caa gta att agc cgt atc tac cag act gct acg cac tta cct aaa gtt   480
Gln Val Ile Ser Arg Ile Tyr Gln Thr Ala Thr His Leu Pro Lys Val
145                 150                 155                 160

tgc aac atc ccg caa gtt agc gtt tgt ccc ttc cag aag acc atg cct   528
Cys Asn Ile Pro Gln Val Ser Val Cys Pro Phe Gln Lys Thr Met Pro
                165                 170                 175

gga ccc tcc tac tag                                               543
Gly Pro Ser Tyr
            180
```

<210> 38
<211> 180
<212> PRT
<213> Brassica napus cv. Topas

<400> 38

```
Met Ala Asn Lys Leu Phe Leu Val Ser Ala Thr Leu Ala Phe Phe Phe
1               5                   10                  15
Leu Leu Thr Asn Ala Ser Ile Tyr Arg Thr Ile Val Glu Val Asp Glu
            20                  25                  30
Asp Asp Ala Thr Asn Pro Ala Gly Pro Phe Arg Ile Pro Lys Cys Arg
        35                  40                  45
Lys Glu Phe Gln Gln Ala Gln His Leu Lys Ala Cys Gln Gln Trp Leu
    50                  55                  60
His Lys Gln Ala Met Gln Ser Gly Ser Gly Pro Ser Trp Thr Leu Asp
65                  70                  75                  80
Gly Glu Phe Asp Phe Glu Asp Asp Met Glu Asn Pro Gln Gly Pro Gln
                85                  90                  95
Gln Arg Pro Pro Leu Leu Gln Gln Cys Cys Asn Glu Leu His Gln Glu
            100                 105                 110
Glu Pro Leu Cys Val Cys Pro Thr Leu Lys Gly Ala Ser Lys Ala Val
        115                 120                 125
Lys Gln Gln Val Arg Gln Gln Gln Gly Gln Gln Gly Gln Gln Leu Gln
    130                 135                 140
Gln Val Ile Ser Arg Ile Tyr Gln Thr Ala Thr His Leu Pro Lys Val
145                 150                 155                 160
Cys Asn Ile Pro Gln Val Ser Val Cys Pro Phe Gln Lys Thr Met Pro
                165                 170                 175
Gly Pro Ser Tyr
            180
```

<210> 39
<211> 435
<212> DNA
<213> Sinapis alba

<220>
<221> CDS
<222> (1) .. (432)
<223> coding for partial sin1 storage protein

<400> 39

```
cca gcc ggc cca ttt ggg att cca aaa tgt agg aag gag ttt caa caa    48
Pro Ala Gly Pro Phe Gly Ile Pro Lys Cys Arg Lys Glu Phe Gln Gln
1               5                   10                  15

gca caa cac cta aga gct tgc cag caa tgg ctc cac aag cag gca atg    96
Ala Gln His Leu Arg Ala Cys Gln Gln Trp Leu His Lys Gln Ala Met
            20                  25                  30

cag tct ggt agt ggt cca agc tgg acc ctc gac gat gag ttt gat ttt    144
Gln Ser Gly Ser Gly Pro Ser Trp Thr Leu Asp Asp Glu Phe Asp Phe
        35                  40                  45

gaa gat gac atg gag aac cca cag gga cca cag cag agg cca cca cta    192
Glu Asp Asp Met Glu Asn Pro Gln Gly Pro Gln Gln Arg Pro Pro Leu
    50                  55                  60

ctc cag cag tgc tgc aac gag ctc cac cag gaa gag cca ctt tgc gtt    240
Leu Gln Gln Cys Cys Asn Glu Leu His Gln Glu Glu Pro Leu Cys Val
65                  70                  75                  80

tgc cca acc ttg aaa gga gca tcc aaa gcc gtt aaa cag cag gtt aga    288
Cys Pro Thr Leu Lys Gly Ala Ser Lys Ala Val Lys Gln Gln Val Arg
                85                  90                  95

caa cag ctg gag cag cag gga cag cag gga ccg cac ctg cag cat gta    336
Gln Gln Leu Glu Gln Gln Gly Gln Gln Gly Pro His Leu Gln His Val
            100                 105                 110

att agc cgt atc tac cag acc gct acg cac tta cct aga gtt tgc aac    384
Ile Ser Arg Ile Tyr Gln Thr Ala Thr His Leu Pro Arg Val Cys Asn
        115                 120                 125

att agg caa gtt agc gtt tgt ccc ttc cag aag acc atg cct gga ccc    432
Ile Arg Gln Val Ser Val Cys Pro Phe Gln Lys Thr Met Pro Gly Pro
    130                 135                 140

tcc                                                                435
```

<210> 40
<211> 144
<212> PRT
<213> Sinapis alba

<400> 40

```
Pro Ala Gly Pro Phe Gly Ile Pro Lys Cys Arg Lys Glu Phe Gln Gln
1               5                   10                  15

Ala Gln His Leu Arg Ala Cys Gln Gln Trp Leu His Lys Gln Ala Met
            20                  25                  30

Gln Ser Gly Ser Gly Pro Ser Trp Thr Leu Asp Asp Glu Phe Asp Phe
        35                  40                  45

Glu Asp Asp Met Glu Asn Pro Gln Gly Pro Gln Gln Arg Pro Pro Leu
    50                  55                  60
```

```
Leu Gln Gln Cys Cys Asn Glu Leu His Gln Glu Glu Pro Leu Cys Val
65                  70                  75                  80

Cys Pro Thr Leu Lys Gly Ala Ser Lys Ala Val Lys Gln Gln Val Arg
                85                  90                  95

Gln Gln Leu Glu Gln Gln Gly Gln Gln Gly Pro His Leu Gln His Val
            100                 105                 110

Ile Ser Arg Ile Tyr Gln Thr Ala Thr His Leu Pro Arg Val Cys Asn
        115                 120                 125

Ile Arg Gln Val Ser Val Cys Pro Phe Gln Lys Thr Met Pro Gly Pro
    130                 135                 140
```

<210> 41
<211> 468
<212> DNA
<213> Glycine max

<220>
<221> CDS
<222> (1)..(465)
<223> 2S albumine 1

<400> 41

```
atg acc aag ctt aca att ctc ctc atc gct ctt ctc ttc atc gcc cac     48
Met Thr Lys Leu Thr Ile Leu Leu Ile Ala Leu Leu Phe Ile Ala His
1               5                   10                  15

acc tgc tgc gcc tcc aaa tgg caa cag cac cag caa gag agc tgc cgc     96
Thr Cys Cys Ala Ser Lys Trp Gln Gln His Gln Gln Glu Ser Cys Arg
            20                  25                  30

gag cag ctc aag ggg atc aac ctc aac ccc tgt gag cac atc atg gag     144
Glu Gln Leu Lys Gly Ile Asn Leu Asn Pro Cys Glu His Ile Met Glu
        35                  40                  45

aag atc caa gct ggc cgc cgc ggc gag gac ggc agc gac gaa gat cac     192
Lys Ile Gln Ala Gly Arg Arg Gly Glu Asp Gly Ser Asp Glu Asp His
    50                  55                  60

att ctc atc agg acc atg ccg gga aga atc aac tac atc agg aag aag     240
Ile Leu Ile Arg Thr Met Pro Gly Arg Ile Asn Tyr Ile Arg Lys Lys
65                  70                  75                  80

gaa gga aaa gaa gaa gaa gaa gaa gga cac atg cag aag tgc tgc agc     288
Glu Gly Lys Glu Glu Glu Glu Glu Gly His Met Gln Lys Cys Cys Ser
                85                  90                  95

gaa atg agc gag ctg aaa agc ccc ata tgc cag tgc aaa gcg cta cag     336
Glu Met Ser Glu Leu Lys Ser Pro Ile Cys Gln Cys Lys Ala Leu Gln
            100                 105                 110

aag ata atg gat aac cag agc gag caa ctg gag ggg aag gag aag aag     384
Lys Ile Met Asp Asn Gln Ser Glu Gln Leu Glu Gly Lys Glu Lys Lys
        115                 120                 125

cag atg gag aga gag ctc atg aac ttg gct att agg tgc agg ttg gga     432
Gln Met Glu Arg Glu Leu Met Asn Leu Ala Ile Arg Cys Arg Leu Gly
    130                 135                 140

ccc atg ata ggg tgc gac ttg tcc tcc gat gac tga                     468
Pro Met Ile Gly Cys Asp Leu Ser Ser Asp Asp
145                 150                 155
```

<210> 42
<211> 155
<212> PRT
<213> Glycine max

<400> 42

```
Met Thr Lys Leu Thr Ile Leu Leu Ile Ala Leu Leu Phe Ile Ala His
1               5                   10                  15

Thr Cys Cys Ala Ser Lys Trp Gln Gln His Gln Gln Glu Ser Cys Arg
            20                  25                  30

Glu Gln Leu Lys Gly Ile Asn Leu Asn Pro Cys Glu His Ile Met Glu
        35                  40                  45

Lys Ile Gln Ala Gly Arg Arg Gly Glu Asp Gly Ser Asp Glu Asp His
    50                  55                  60

Ile Leu Ile Arg Thr Met Pro Gly Arg Ile Asn Tyr Ile Arg Lys Lys
65                  70                  75                  80

Glu Gly Lys Glu Glu Glu Glu Glu Gly His Met Gln Lys Cys Cys Ser
                85                  90                  95

Glu Met Ser Glu Leu Lys Ser Pro Ile Cys Gln Cys Lys Ala Leu Gln
            100                 105                 110

Lys Ile Met Asp Asn Gln Ser Glu Gln Leu Glu Gly Lys Glu Lys Lys
        115                 120                 125

Gln Met Glu Arg Glu Leu Met Asn Leu Ala Ile Arg Cys Arg Leu Gly
    130                 135                 140

Pro Met Ile Gly Cys Asp Leu Ser Ser Asp Asp
145                 150                 155
```

<210> 43
<211> 477
<212> DNA
<213> Glycine max

<220>
<221> CDS
<222> (1)..(474)
<223> 2S albumine

<400> 43

```
atg acc aag ttc aca atc ctc ctc atc tct ctt ctc ttc tgc atc gcc      48
Met Thr Lys Phe Thr Ile Leu Leu Ile Ser Leu Leu Phe Cys Ile Ala
1               5                   10                  15

cac act tgc agc gcc tcc aaa tgg cag cac cag caa gat agc tgc cgc      96
His Thr Cys Ser Ala Ser Lys Trp Gln His Gln Gln Asp Ser Cys Arg
            20                  25                  30

aag cag ctc cag ggg gtg aac ctc acg ccc tgc gag aag cac atc atg     144
Lys Gln Leu Gln Gly Val Asn Leu Thr Pro Cys Glu Lys His Ile Met
        35                  40                  45

gag aag atc caa ggc cgc ggc gat gac gat gat gat gat gac gac gac     192
Glu Lys Ile Gln Gly Arg Gly Asp Asp Asp Asp Asp Asp Asp Asp Asp
    50                  55                  60

aat cac att ctc agg acc atg cgg gga aga atc aac tac ata agg agg     240
Asn His Ile Leu Arg Thr Met Arg Gly Arg Ile Asn Tyr Ile Arg Arg
65                  70                  75                  80
```

```
aac gaa gga aaa gac gaa gac gaa gaa gaa gaa gga cac atg cag aag     288
Asn Glu Gly Lys Asp Glu Asp Glu Glu Glu Glu Gly His Met Gln Lys
                    85                  90                  95

tgc tgc aca gaa atg agc gag ctg aga agc ccc aaa tgc cag tgc aaa     336
Cys Cys Thr Glu Met Ser Glu Leu Arg Ser Pro Lys Cys Gln Cys Lys
            100                 105                 110

gcg ctg cag aag ata atg gag aac cag agc gag gaa ctg gag gag aag     384
Ala Leu Gln Lys Ile Met Glu Asn Gln Ser Glu Glu Leu Glu Glu Lys
        115                 120                 125

cag aag aag aaa atg gag aag gag ctc att aac ttg gct act atg tgc     432
Gln Lys Lys Lys Met Glu Lys Glu Leu Ile Asn Leu Ala Thr Met Cys
    130                 135                 140

agg ttt gga ccc atg atc cag tgc gac ttg tcc tcc gat gac taa         477
Arg Phe Gly Pro Met Ile Gln Cys Asp Leu Ser Ser Asp Asp
145                 150                 155
```

<210> 44
<211> 158
<212> PRT
<213> Glycine max

<400> 44

```
Met Thr Lys Phe Thr Ile Leu Leu Ile Ser Leu Leu Phe Cys Ile Ala
1               5                   10                  15

His Thr Cys Ser Ala Ser Lys Trp Gln His Gln Gln Asp Ser Cys Arg
            20                  25                  30

Lys Gln Leu Gln Gly Val Asn Leu Thr Pro Cys Glu Lys His Ile Met
        35                  40                  45

Glu Lys Ile Gln Gly Arg Gly Asp Asp Asp Asp Asp Asp Asp Asp Asp
    50                  55                  60

Asn His Ile Leu Arg Thr Met Arg Gly Arg Ile Asn Tyr Ile Arg Arg
65                  70                  75                  80

Asn Glu Gly Lys Asp Glu Asp Glu Glu Glu Glu Gly His Met Gln Lys
                85                  90                  95

Cys Cys Thr Glu Met Ser Glu Leu Arg Ser Pro Lys Cys Gln Cys Lys
            100                 105                 110

Ala Leu Gln Lys Ile Met Glu Asn Gln Ser Glu Glu Leu Glu Glu Lys
        115                 120                 125

Gln Lys Lys Lys Met Glu Lys Glu Leu Ile Asn Leu Ala Thr Met Cys
    130                 135                 140

Arg Phe Gly Pro Met Ile Gln Cys Asp Leu Ser Ser Asp Asp
145                 150                 155
```

<210> 45
<211> 537
<212> DNA
<213> Brassica nigra

<220>
<221> CDS
<222> (1)..(534)
<223> 2S storage protein

<400> 45

```
atg gcg aac aag ctc ttc ctc gtc tcg gca act ctc gcc ttc ttc ttc      48
Met Ala Asn Lys Leu Phe Leu Val Ser Ala Thr Leu Ala Phe Phe Phe
1               5                   10                  15

ctg ctc acc aat gcc tcc atc tac cgg acg gtc gtc gag ttc gac gaa      96
Leu Leu Thr Asn Ala Ser Ile Tyr Arg Thr Val Val Glu Phe Asp Glu
            20                  25                  30

gat gat gac aca aac caa gcc gga cca ttt agg att cca aga tgt cga      144
Asp Asp Asp Thr Asn Gln Ala Gly Pro Phe Arg Ile Pro Arg Cys Arg
        35                  40                  45

aag gag ttt cgg caa gca caa cat cta aga gct tgc cag caa tgg ctc      192
Lys Glu Phe Arg Gln Ala Gln His Leu Arg Ala Cys Gln Gln Trp Leu
    50                  55                  60

cac agg cag gca atg cag tcc ggt agt ggt cca agc tgg acc ctg gac      240
His Arg Gln Ala Met Gln Ser Gly Ser Gly Pro Ser Trp Thr Leu Asp
65                  70                  75                  80

ggt gag ttt gac ttt gaa gac gac atg gag aac caa cag ggc cca cag      288
Gly Glu Phe Asp Phe Glu Asp Asp Met Glu Asn Gln Gln Gly Pro Gln
                85                  90                  95

cag agg cca cct cta ctc cag caa tgc tgc aac gag ctc cac cag gaa      336
Gln Arg Pro Pro Leu Leu Gln Gln Cys Cys Asn Glu Leu His Gln Glu
            100                 105                 110

gag gca ctt tgt gtt tgc cca acc ttg aaa gga gca tcc aaa gcg gtt      384
Glu Ala Leu Cys Val Cys Pro Thr Leu Lys Gly Ala Ser Lys Ala Val
        115                 120                 125

aga caa cag gtt cga caa cag gga cac cag cag cag atg cag cat gta      432
Arg Gln Gln Val Arg Gln Gln Gly His Gln Gln Gln Met Gln His Val
    130                 135                 140

att agc cgt atc tac cag acc gct acg cac tta cct aga gtt tgc aac      480
Ile Ser Arg Ile Tyr Gln Thr Ala Thr His Leu Pro Arg Val Cys Asn
145                 150                 155                 160

atc ccg caa gtt agc gtt tgt ccc ttc cag aag acc atg cct ggg ccc      528
Ile Pro Gln Val Ser Val Cys Pro Phe Gln Lys Thr Met Pro Gly Pro
                165                 170                 175

tcc tac tag                                                          537
Ser Tyr
```

<210> 46
<211> 178
<212> PRT
<213> Brassica nigra

<400> 46

```
Met Ala Asn Lys Leu Phe Leu Val Ser Ala Thr Leu Ala Phe Phe Phe
1               5                   10                  15

Leu Leu Thr Asn Ala Ser Ile Tyr Arg Thr Val Val Glu Phe Asp Glu
            20                  25                  30

Asp Asp Asp Thr Asn Gln Ala Gly Pro Phe Arg Ile Pro Arg Cys Arg
        35                  40                  45

Lys Glu Phe Arg Gln Ala Gln His Leu Arg Ala Cys Gln Gln Trp Leu
    50                  55                  60

His Arg Gln Ala Met Gln Ser Gly Ser Gly Pro Ser Trp Thr Leu Asp
65                  70                  75                  80

Gly Glu Phe Asp Phe Glu Asp Asp Met Glu Asn Gln Gln Gly Pro Gln
                85                  90                  95

Gln Arg Pro Pro Leu Leu Gln Gln Cys Cys Asn Glu Leu His Gln Glu
            100                 105                 110

Glu Ala Leu Cys Val Cys Pro Thr Leu Lys Gly Ala Ser Lys Ala Val
        115                 120                 125

Arg Gln Gln Val Arg Gln Gln Gly His Gln Gln Gln Met Gln His Val
    130                 135                 140

Ile Ser Arg Ile Tyr Gln Thr Ala Thr His Leu Pro Arg Val Cys Asn
145                 150                 155                 160

Ile Pro Gln Val Ser Val Cys Pro Phe Gln Lys Thr Met Pro Gly Pro
                165                 170                 175

Ser Tyr
```

<210> 47
<211> 435
<212> DNA
<213> Sinapis alba

<220>
<221> CDS
<222> (1)..(432)
<223> sin5 storage protein

<400> 47

```
cca gcc ggc cca ttt ggg att cca aaa tgt agg aag gag ttt caa caa      48
Pro Ala Gly Pro Phe Gly Ile Pro Lys Cys Arg Lys Glu Phe Gln Gln
 1               5                  10                  15

gca caa cac cta aga gct tgc cag caa tgg ctc cac aag cag gca atg      96
Ala Gln His Leu Arg Ala Cys Gln Gln Trp Leu His Lys Gln Ala Met
            20                  25                  30

cag tct ggt agt ggt cca agc tgg acc ctc gac gat gag ttt gat ttt     144
Gln Ser Gly Ser Gly Pro Ser Trp Thr Leu Asp Asp Glu Phe Asp Phe
        35                  40                  45

gaa gac gac atg gag aac ccc cag gga cca cag cag aag ccg cca cta     192
Glu Asp Asp Met Glu Asn Pro Gln Gly Pro Gln Gln Lys Pro Pro Leu
    50                  55                  60

ctc cag caa tgc tgc aac gag ctt cac cag gag gag cca ctt tgc gtt     240
Leu Gln Gln Cys Cys Asn Glu Leu His Gln Glu Glu Pro Leu Cys Val
65                  70                  75                  80

tgc cca act ttg aaa gga gct tcc aaa gcc gtt aaa caa cag gtt cga     288
Cys Pro Thr Leu Lys Gly Ala Ser Lys Ala Val Lys Gln Gln Val Arg
                85                  90                  95

caa cag ttg ggg cag cag gga cag cag gga ccg cag gtg cag cat gta     336
Gln Gln Leu Gly Gln Gln Gly Gln Gln Gly Pro Gln Val Gln His Val
            100                 105                 110

att agc cgt atc tac cag acc gct acg cac tta cct aaa gtt tgc aac     384
Ile Ser Arg Ile Tyr Gln Thr Ala Thr His Leu Pro Lys Val Cys Asn
        115                 120                 125

atc ccc caa gta agc gtt tgt ccc ttc aag aag acc atg cct gga ccc     432
Ile Pro Gln Val Ser Val Cys Pro Phe Lys Lys Thr Met Pro Gly Pro
    130                 135                 140

tcc                                                                 435
```

<210> 48
<211> 144
<212> PRT
<213> Sinapis alba

<400> 48

```
Pro Ala Gly Pro Phe Gly Ile Pro Lys Cys Arg Lys Glu Phe Gln Gln
1               5                   10                  15

Ala Gln His Leu Arg Ala Cys Gln Gln Trp Leu His Lys Gln Ala Met
            20                  25                  30

Gln Ser Gly Ser Gly Pro Ser Trp Thr Leu Asp Asp Glu Phe Asp Phe
        35                  40                  45

Glu Asp Asp Met Glu Asn Pro Gln Gly Pro Gln Gln Lys Pro Pro Leu
    50                  55                  60

Leu Gln Gln Cys Cys Asn Glu Leu His Gln Glu Glu Pro Leu Cys Val
65                  70                  75                  80

Cys Pro Thr Leu Lys Gly Ala Ser Lys Ala Val Lys Gln Gln Val Arg
                85                  90                  95

Gln Gln Leu Gly Gln Gln Gly Gln Gln Gly Pro Gln Val Gln His Val
            100                 105                 110

Ile Ser Arg Ile Tyr Gln Thr Ala Thr His Leu Pro Lys Val Cys Asn
        115                 120                 125

Ile Pro Gln Val Ser Val Cys Pro Phe Lys Lys Thr Met Pro Gly Pro
    130                 135                 140
```

<210> 49
<211> 888
<212> DNA
<213> Helianthus annuus

<220>
<221> CDS
<222> (1)..(885)
<223> HaG5 2S albumine

<400> 49

```
atg gca aag caa ata gtt ctc gca ctc gct ttc gcc gcc ctt gta gcc      48
Met Ala Lys Gln Ile Val Leu Ala Leu Ala Phe Ala Ala Leu Val Ala
1               5                   10                  15

ttt gct acc gcc cac aca acc ata atc acc acc acc atc gaa gac gag      96
Phe Ala Thr Ala His Thr Thr Ile Ile Thr Thr Thr Ile Glu Asp Glu
            20                  25                  30

aac ccg atc tcc gga caa agg caa gtg agc caa cgg ata cag gga caa     144
Asn Pro Ile Ser Gly Gln Arg Gln Val Ser Gln Arg Ile Gln Gly Gln
        35                  40                  45

agg ctg aac cag tgt cgc atg ttc ctc cag cag ggt cag aac att cct     192
Arg Leu Asn Gln Cys Arg Met Phe Leu Gln Gln Gly Gln Asn Ile Pro
    50                  55                  60

cgc gaa ttc gat aac cct cag atg ggg cgg cag cag gag cag cag ctc     240
Arg Glu Phe Asp Asn Pro Gln Met Gly Arg Gln Gln Glu Gln Gln Leu
65                  70                  75                  80
```

```
cag cag tgt tgt caa gag ctc caa aac atc gaa ggg cag tgc caa tgt      288
Gln Gln Cys Cys Gln Glu Leu Gln Asn Ile Glu Gly Gln Cys Gln Cys
                    85                  90                  95

gag gcg gtg aag cag gtg ttc cga gaa gcc cag cag caa gta caa cag      336
Glu Ala Val Lys Gln Val Phe Arg Glu Ala Gln Gln Gln Val Gln Gln
            100                 105                 110

caa cag gga cgg cag ctt gta ccc ttc cgc ggt tcg cag cag acc caa      384
Gln Gln Gly Arg Gln Leu Val Pro Phe Arg Gly Ser Gln Gln Thr Gln
        115                 120                 125

cag ttg aag cag aag gct cag att ctc cct aac gta tgc aac ctt caa      432
Gln Leu Lys Gln Lys Ala Gln Ile Leu Pro Asn Val Cys Asn Leu Gln
    130                 135                 140

tca aga cga tgt gaa atc gga acc atc acc acc acc gtc acc gag agc      480
Ser Arg Arg Cys Glu Ile Gly Thr Ile Thr Thr Thr Val Thr Glu Ser
145                 150                 155                 160

aat atc gat atc ccc ttc cgt gac agg ccc ttt ggc act gga tca caa      528
Asn Ile Asp Ile Pro Phe Arg Asp Arg Pro Phe Gly Thr Gly Ser Gln
                165                 170                 175

cag tgc aga gaa act gaa atc caa cga ccc gtt ggt gaa tgc caa agg      576
Gln Cys Arg Glu Thr Glu Ile Gln Arg Pro Val Gly Glu Cys Gln Arg
            180                 185                 190

ttc gtg gag cag caa atg cag cag tct ccg agg tcc act aga cca tac      624
Phe Val Glu Gln Gln Met Gln Gln Ser Pro Arg Ser Thr Arg Pro Tyr
        195                 200                 205

caa cag cgg cca gga caa cag cag cag cag cag aga ggg ctc caa caa      672
Gln Gln Arg Pro Gly Gln Gln Gln Gln Gln Gln Arg Gly Leu Gln Gln
    210                 215                 220

caa tgc tgc aac gag cta caa aac gtg aag agg gag tgt cat tgc gag      720
Gln Cys Cys Asn Glu Leu Gln Asn Val Lys Arg Glu Cys His Cys Glu
225                 230                 235                 240

gca att caa gaa gtg gct agg aga gtg atg agg cag cca cag cag cag      768
Ala Ile Gln Glu Val Ala Arg Arg Val Met Arg Gln Pro Gln Gln Gln
                245                 250                 255

cag cag caa cgt cgt ggg cag ttc ggt ggg cag gag atg gaa acc gcg      816
Gln Gln Gln Arg Arg Gly Gln Phe Gly Gly Gln Glu Met Glu Thr Ala
            260                 265                 270

agg agg gtg att cag aat ctg ccc aac cag tgc gac ttg gaa gtc cag      864
Arg Arg Val Ile Gln Asn Leu Pro Asn Gln Cys Asp Leu Glu Val Gln
        275                 280                 285

caa tgc aca acc tgt acg gga tga                                      888
Gln Cys Thr Thr Cys Thr Gly
    290                 295
```

<210> 50
<211> 295
<212> PRT
<213> Helianthus annuus

<400> 50

```
Met Ala Lys Gln Ile Val Leu Ala Leu Ala Phe Ala Ala Leu Val Ala
1               5                   10                  15
Phe Ala Thr Ala His Thr Thr Ile Ile Thr Thr Thr Ile Glu Asp Glu
            20                  25                  30

Asn Pro Ile Ser Gly Gln Arg Gln Val Ser Gln Arg Ile Gln Gly Gln
        35                  40                  45
Arg Leu Asn Gln Cys Arg Met Phe Leu Gln Gln Gly Gln Asn Ile Pro
    50                  55                  60
Arg Glu Phe Asp Asn Pro Gln Met Gly Arg Gln Gln Glu Gln Gln Leu
65                  70                  75                  80
Gln Gln Cys Cys Gln Glu Leu Gln Asn Ile Glu Gly Gln Cys Gln Cys
                85                  90                  95
Glu Ala Val Lys Gln Val Phe Arg Glu Ala Gln Gln Gln Val Gln Gln
            100                 105                 110
Gln Gln Gly Arg Gln Leu Val Pro Phe Arg Gly Ser Gln Gln Thr Gln
        115                 120                 125
Gln Leu Lys Gln Lys Ala Gln Ile Leu Pro Asn Val Cys Asn Leu Gln
    130                 135                 140
Ser Arg Arg Cys Glu Ile Gly Thr Ile Thr Thr Thr Val Thr Glu Ser
145                 150                 155                 160
Asn Ile Asp Ile Pro Phe Arg Asp Arg Pro Phe Gly Thr Gly Ser Gln
                165                 170                 175
Gln Cys Arg Glu Thr Glu Ile Gln Arg Pro Val Gly Glu Cys Gln Arg
            180                 185                 190
Phe Val Glu Gln Gln Met Gln Gln Ser Pro Arg Ser Thr Arg Pro Tyr
        195                 200                 205
Gln Gln Arg Pro Gly Gln Gln Gln Gln Gln Gln Arg Gly Leu Gln Gln
    210                 215                 220
Gln Cys Cys Asn Glu Leu Gln Asn Val Lys Arg Glu Cys His Cys Glu
225                 230                 235                 240
Ala Ile Gln Glu Val Ala Arg Arg Val Met Arg Gln Pro Gln Gln Gln
                245                 250                 255
Gln Gln Gln Arg Arg Gly Gln Phe Gly Gly Gln Glu Met Glu Thr Ala
            260                 265                 270
Arg Arg Val Ile Gln Asn Leu Pro Asn Gln Cys Asp Leu Glu Val Gln
        275                 280                 285
Gln Cys Thr Thr Cys Thr Gly
    290                 295
```

<210> 51
<211> 973
<212> DNA
<213> Helianthus annuus

<220>
<221> CDS
<222> (2)..(970)
<223> coding for partial 2S albumine

<400> 51

```
g gca aag ata aca ctt ctc ttg ctc gcc tta gct gct ctt gta gcc ttg 49
  Ala Lys Ile Thr Leu Leu Leu Leu Ala Leu Ala Ala Leu Val Ala Leu
    1               5                  10                  15

gct aca gcc cac aca acc atc atc acc acc acc atc gac gac gag aac   97
Ala Thr Ala His Thr Thr Ile Ile Thr Thr Thr Ile Asp Asp Glu Asn
            20                  25                  30
```

```
ccg atc tcc gaa caa agg caa tgt tgg caa cag gta cag gga caa agg     145
Pro Ile Ser Glu Gln Arg Gln Cys Trp Gln Gln Val Gln Gly Gln Arg
            35                  40                  45

ttg aac cag tgt cgc atg ttc ctc cag caa ggt cag agg ggg cag caa     193
Leu Asn Gln Cys Arg Met Phe Leu Gln Gln Gly Gln Arg Gly Gln Gln
        50                  55                  60

cac caa cag caa cag cat cag cag cag gag cag cag ctc ctc cag cag     241
His Gln Gln Gln Gln His Gln Gln Gln Glu Gln Gln Leu Leu Gln Gln
65                  70                  75                  80

tgt tgt caa gag ctt caa aac atc gaa gga cag tgc caa tgt gag gcg     289
Cys Cys Gln Glu Leu Gln Asn Ile Glu Gly Gln Cys Gln Cys Glu Ala
                85                  90                  95

gtg aag cag gtg gtc cga gat gct cag cga cac gag caa cag cga ccg     337
Val Lys Gln Val Val Arg Asp Ala Gln Arg His Glu Gln Gln Arg Pro
            100                 105                 110

cga gtg ccc ttc cag ggt tct cag cag tct caa cag ttg aag cag agg     385
Arg Val Pro Phe Gln Gly Ser Gln Gln Ser Gln Gln Leu Lys Gln Arg
        115                 120                 125

gct cag att ctc cct aac gta tgc aac ctt caa tca aga cga tgc gaa     433
Ala Gln Ile Leu Pro Asn Val Cys Asn Leu Gln Ser Arg Arg Cys Glu
    130                 135                 140

atc gaa agc gtc agg agt gtt gct gag agc aat ttt gaa atc cca ttt     481
Ile Glu Ser Val Arg Ser Val Ala Glu Ser Asn Phe Glu Ile Pro Phe
145                 150                 155                 160

gat atg ccg ttt gat atc cct tgg ccc ttt cgc cca agc tca gag tca     529
Asp Met Pro Phe Asp Ile Pro Trp Pro Phe Arg Pro Ser Ser Glu Ser
                165                 170                 175

cag caa tgc aga cag agt gaa atc caa agg cct gtg agt cag tgc caa     577
Gln Gln Cys Arg Gln Ser Glu Ile Gln Arg Pro Val Ser Gln Cys Gln
            180                 185                 190

agg tat gtg gag cag caa att cag tcc tcc agg cca tac caa cag agc     625
Arg Tyr Val Glu Gln Gln Ile Gln Ser Ser Arg Pro Tyr Gln Gln Ser
        195                 200                 205

ccg tac gac cgg agg caa cag agc cca tac gac cgg agg caa cag agc     673
Pro Tyr Asp Arg Arg Gln Gln Ser Pro Tyr Asp Arg Arg Gln Gln Ser
    210                 215                 220

cca tat gaa cag agg caa gga cca tac gaa cag agg cca tac gaa cag     721
Pro Tyr Glu Gln Arg Gln Gly Pro Tyr Glu Gln Arg Pro Tyr Glu Gln
225                 230                 235                 240

agg cca tac caa cag cga gga gga cga cag cag gag cag caa ggg ctc     769
Arg Pro Tyr Gln Gln Arg Gly Gly Arg Gln Gln Glu Gln Gln Gly Leu
                245                 250                 255

cag caa tgc tgc aac gag ctc caa aac gtg agg agg gag tgt cag tgc     817
Gln Gln Cys Cys Asn Glu Leu Gln Asn Val Arg Arg Glu Cys Gln Cys
            260                 265                 270

gag gcg att aag gaa gtg ggc caa aga atg agg cag cag caa caa caa     865
Glu Ala Ile Lys Glu Val Gly Gln Arg Met Arg Gln Gln Gln Gln Gln
        275                 280                 285

caa cgt agg cag tat ggt ggg cag cag aca caa act gtg gag aga att     913
Gln Arg Arg Gln Tyr Gly Gly Gln Gln Thr Gln Thr Val Glu Arg Ile
    290                 295                 300

ctt gag aat ctg cct aac caa tgc gac cta gat gtc cag caa tgc aac     961
Leu Glu Asn Leu Pro Asn Gln Cys Asp Leu Asp Val Gln Gln Cys Asn
305                 310                 315                 320

atc ccc tac tga                                                     973
Ile Pro Tyr
```

<210> 52
<211> 323
<212> PRT
<213> Helianthus annuus

<400> 52

Ala Lys Ile Thr Leu Leu Leu Leu Ala Leu Ala Ala Leu Val Ala Leu
1               5                   10                  15

Ala Thr Ala His Thr Thr Ile Ile Thr Thr Thr Ile Asp Asp Glu Asn
            20                  25                  30

Pro Ile Ser Glu Gln Arg Gln Cys Trp Gln Gln Val Gln Gly Gln Arg
        35                  40                  45

Leu Asn Gln Cys Arg Met Phe Leu Gln Gln Gly Gln Arg Gly Gln Gln
    50                  55                  60

His Gln Gln Gln Gln His Gln Gln Gln Glu Gln Gln Leu Leu Gln Gln
65                  70                  75                  80

Cys Cys Gln Glu Leu Gln Asn Ile Glu Gly Gln Cys Gln Cys Glu Ala
                85                  90                  95

Val Lys Gln Val Val Arg Asp Ala Gln Arg His Glu Gln Gln Arg Pro
            100                 105                 110

Arg Val Pro Phe Gln Gly Ser Gln Gln Ser Gln Gln Leu Lys Gln Arg
        115                 120                 125

Ala Gln Ile Leu Pro Asn Val Cys Asn Leu Gln Ser Arg Arg Cys Glu
    130                 135                 140

Ile Glu Ser Val Arg Ser Val Ala Glu Ser Asn Phe Glu Ile Pro Phe
145                 150                 155                 160

Asp Met Pro Phe Asp Ile Pro Trp Pro Phe Arg Pro Ser Ser Glu Ser
                165                 170                 175

Gln Gln Cys Arg Gln Ser Glu Ile Gln Arg Pro Val Ser Gln Cys Gln
            180                 185                 190

Arg Tyr Val Glu Gln Gln Ile Gln Ser Ser Arg Pro Tyr Gln Gln Ser
        195                 200                 205

Pro Tyr Asp Arg Arg Gln Gln Ser Pro Tyr Asp Arg Arg Gln Gln Ser
    210                 215                 220

Pro Tyr Glu Gln Arg Gln Gly Pro Tyr Glu Gln Arg Pro Tyr Glu Gln
225                 230                 235                 240

Arg Pro Tyr Gln Gln Arg Gly Gly Arg Gln Gln Glu Gln Gln Gly Leu
                245                 250                 255

Gln Gln Cys Cys Asn Glu Leu Gln Asn Val Arg Arg Glu Cys Gln Cys
            260                 265                 270

Glu Ala Ile Lys Glu Val Gly Gln Arg Met Arg Gln Gln Gln Gln Gln
        275                 280                 285

Gln Arg Arg Gln Tyr Gly Gly Gln Gln Thr Gln Thr Val Glu Arg Ile
    290                 295                 300

Leu Glu Asn Leu Pro Asn Gln Cys Asp Leu Asp Val Gln Gln Cys Asn
305                 310                 315                 320

Ile Pro Tyr

<210> 53
<211> 1114
<212> DNA
<213> Künstliche Sequenz

<220>
<223> Beschreibung der künstlichen Sequenz: DNA construct coding for dsRNA

<400> 53

```
ggccgcgtgt tccatttggc cggaaacaac cagcagggag gctttggcgg ttcacagcaa 60
caacaagaac agaaaaactt gtggagcggg ttcgacgcac aggtcatagc tcaagcattg 120
aaaattgacg ttcagttggc tcagcagctt cagaaccaac aagacagcag aggaaacatc 180
gttcgtgtta agggaccttt ccaggtcgtg aggccacctc taagacagcc ctacgagagc 240
gaggagtgga gacacccacg tagcccacag ggcaacggcc ttgaggagac tatctgcagc 300
atgaggtccc acgagaacat tgacgaccct gctcgtgctg acgtgtacaa gcccagccta 360
ggtcgcgtga ccagcgtcaa cagctatacc ttgcccatct tggagtatgt caggctcagt 420
gccactcgtg gcgttctcca gggtggatcc ttctgtaaca tttgacaaaa catgtgaaca 480
cgtcatccgt catatagaac ttccaatttt aatatgtttt gctaaagaaa aaaaaaagga 540
ataaatatct atcaaattca tttttaaaac atttgtatac gttcttaaat aatttaggat 600
atgactaatt tttctttttg gtaaaaatgt taatatctat atttaattta ttaagaaaaa 660
tgtacttaca ccctggagaa cgccacgagt ggcactgagc ctgacatact ccaagatggg 720
caaggtatag ctgttgacgc tggtcacgcg acctaggctg ggcttgtaca cgtcagcacg 780
agcagggtcg tcaatgttct cgtgggacct catgctgcag atagtctcct caaggccgtt 840
gccctgtggg ctacgtgggt gtctccactc ctcgctctcg tagggctgtc ttagaggtgg 900
cctcacgacc tggaaaggtc ccttaacacg aacgatgttt cctctgctgt cttgttggtt 960
ctgaagctgc tgagccaact gaacgtcaat tttcaatgct tgagctatga cctgtgcgtc 1020
gaacccgctc cacaagtttt tctgttcttg ttgttgctgt gaaccgccaa agcctccctg 1080
ctggttgttt ccggccaaat ggaacacgcg gccg                             1114
```

<210> 54
<211> 1114
<212> RNA
<213> Künstliche Sequenz

<220>
<223> Beschreibung der künstlichen Sequenz: RNA sequence for forming dsRNA

<400> 54

```
ggccgcgugu uccauuuggc cggaaacaac cagcagggag gcuuuggcgg uucacagcaa 60
caacaagaac agaaaaacuu guggagcggg uucgacgcac aggucauagc ucaagcauug 120
aaaauugacg uucaguuggc ucagcagcuu cagaaccaac aagacagcag aggaaacauc 180
guucguguua agggaccuuu ccaggucgug aggccaccuc uaagacagcc cuacgagagc 240
gaggagugga gacacccacg uagcccacag ggcaacggcc uugaggagac uaucugcagc 300
augaggaccc acgagaacau ugacgacccu gcucgugcug acguguacaa gcccagccua 360
ggucgcguga ccagcgucaa cagcuauacc uugcccaucu uggaguaugu caggcucagu 420
gccacucgug gcguucucca ggguggaucc uucuguaaca uuugacaaaa caugugaaca 480
cgucauccgu cauauagaac uuccaauuuu aauauguuuu gcuaaagaaa aaaaaaagga 540
auaaauaucu aucaaauuca uuuuuaaaac auuuguauac guucuuaaau aauuuaggau 600
augacuaauu uuucuuuuug guaaaaaugu uaauaucuau auuuaauuua uuaagaaaaa 660
uguacuuaca cccuggagaa cgccacgagu ggcacugagc cugacauacu ccaagauggg 720
caagguauag cuguugacgc uggucacgcg accuaggcug ggcuuguaca cgucagcacg 780
agcagggucg ucaauguucu cgugggaccu caugcugcag auagucuccu caaggccguu 840
```

gcccuguggg cuacgugggu gucuccacuc cucgcucucg uagggcuguc uuagaggugg 900
ccucacgacc uggaaagguc ccuuaacacg aacgauguuu ccucugcugu cuuguugguu 960
cugaagcugc ugagccaacu gaacgucaau uuucaaugcu ugagcuauga ccugugcguc 1020
gaacccgcuc cacaaguuuu ucuguucuug uuguugcugu gaaccgccaa agccucccug 1080
cugguuguuu ccggccaaau ggaacacgcg gccg 1114

<210> 55
<211> 1789
<212> DNA
<213> Künstliche Sequenz

<220>
<223> Beschreibung der künstlichen Sequenz: DNA construct coding for dsRNA

<400> 55

gcggccgcgg atcctcaggg tcttttcttg cccactttct tgaacgccgg caaactcacg 60
tttgttgttc acggaagggg tctaatggga agagttattc cgggatgcgc cgagacgttc 120
atggagtcac cggtatttgg agaaggtcaa ggtcagggtc agagtcaagg gttccgtgac 180
atgcaccaga aagtagagca cctacggtgc ggtgacacca ttgcaacacc atctggtgta 240
gctcaatggt tctacaacaa tggaaatgag cctctcattc ttgttgcagc cgcggatctc 300
gccagcaacc agaaccagct tgaccgcaac cttagaccat ttttgatagc cggaaacaac 360
ccacaagggc aggaatggct acaaggccga aagcaacaga agcaaaacaa catcttcaat 420
ggcttcgcac ctgagatctt ggctcaagcc ttcaagatca atgtcgagac ggctcagcag 480
ctccagaacc agcaagataa ccgtggcaac atcgtcaagg tcaacggacc tttcggcgtc 540
attaggccac ccttgagacg cggcgaaggc ggccaacaac cacatgaaat agctaatggt 600
ttagaggaga ctttgtgcac catgcgatgc actgaaaacc tcgatgaccc gtcggatgct 660
gacgtgtaca agccatcact cggatacatt agcacactta acagctacaa tcttcctatc 720
ctcagacttc tccgccttag cgctcttcgt ggctccatcc gtaaaactcg aggtaagctc 780
aacaaatctt tagaaaatta attttatgtg acatatgcaa taatttgatt tggcaagata 840
aactaataga ttttgcgatt tggagtttta aactctaaat aatctaaatc gttttcaatt 900
ggtttaaata tatatcttgc atttttaatc gtttttaatt aaaaaatata tatatatata 960
tatatcttgc atttttaatc gttttcaatt taaaaaatat cttgcacgca gaacgctgtc 1020
gagttttacg gatggagcca cgaagagcgc taaggcggag aagtctgagg ataggaagat 1080
tgtagctgtt aagtgtgcta atgtatccga gtgatggctt gtacacgtca gcatccgacg 1140
ggtcatcgag gttttcagtg catcgcatgg tgcacaaagt ctcctctaaa ccattagcta 1200
tttcatgtgg ttgttggccg ccttcgccgc gtctcaaggg tggcctaatg acgccgaaag 1260
gtccgttgac cttgacgatg ttgccacggt tatcttgctg gttctggagc tgctgagccg 1320
tctcgacatt gatcttgaag gcttgagcca agatctcagg tgcgaagcca ttgaagatgt 1380
tgttttgctt ctgttgcttt cggccttgta gccattcctg cccttgtggg ttgtttccgg 1440
ctatcaaaaa tggtctaagg ttgcggtcaa gctggttctg gttgctggcg agatccgcgg 1500
ctgcaacaag aatgagaggc tcatttccat tgttgtagaa ccattgagct acaccagatg 1560
gtgttgcaat ggtgtcaccg caccgtaggt gctctacttt ctggtgcatg tcacggaacc 1620
cttgactctg accctgacct tgaccttctc caaataccgg tgactccatg aacgtctcgg 1680
cgcatcccgg aataactctt cccattagac cccttccgtg aacaacaaac gtgagtttgc 1740
cggcgttcaa gaaagtgggc aagaaaagac cctgaggatc cgcggccgc 1789

<210> 56
<211> 1789
<212> RNA
<213> Künstliche Sequenz

<220>
<223> Beschreibung der künstlichen Sequenz: : RNA sequence for forming dsRNA

<400> 56

gcggccgcgg auccucaggg ucuuuucuug cccacuuucu ugaacgccgg caaacucacg 60
uuuguuguuc acggaagggg ucuaauggga agaguuauuc cgggaugcgc cgagacguuc 120
auggagucac cgguauuugg agaaggucaa ggucagggguc agagucaagg guuccgugac 180
augcaccaga aaguagagca ccuacggugc ggugacacca uugcaacacc aucuggugua 240

gcucaauggu ucuacaacaa uggaaaugag ccucucauuc uuguugcagc cgcggaucuc 300
gccagcaacc agaaccagcu ugaccgcaac cuuagaccau uuuugauagc cggaaacaac 360
ccacaagggc aggaauggcu acaaggccga aagcaacaga agcaaaacaa caucuucaau 420
ggcuucgcac cugagaucuu ggcucaagcc uucaagauca augucgagac ggcucagcag 480
cuccagaacc agcaagauaa ccguggcaac aucgucaagg ucaacggacc uuucggcguc 540
auuaggccac ccuugagacg cggcgaaggc ggccaacaac cacaugaaau agcuaauggu 600
uuagaggaga cuuugugcac caugcgaugc acugaaaacc ucgaugaccc gucggaugcu 660
gacguguaca agccaucacu cggauacauu agcacacuua acagcuacaa ucuuccuauc 720
cucagacuuc ucccgccuuag cgcucuucgu ggcuccaucc guaaaacucg agguaagcuc 780
aacaaaucuu uagaaaauua auuuuaugug acauaugcaa uaauuugauu uggcaagaua 840
aacuaauaga uuuugcgauu uggaguuuua aacucuaaau aaucuaaauc guuuucaauu 900
gguuuaaaua uauaucuugc auuuuuaauc guuuuuaauu aaaaaauaua uauauauaua 960
uauaucuugc auuuuuaauc guuuucaauu uaaaaaauau cuugcacgca gaacgcuguc 1020
gaguuuuacg gauggagcca cgaagagcgc uaaggcggag aagucugagg auaggaagau 1080
uguagcuguu aagugugcua auguauccga gugauggcuu guacacguca gcauccgacg 1140
ggucaucgag guuuucagug caucgcaugg ugcacaaagu cuccucuaaa ccauuagcua 1200
uuucaugugg uuguuggccg ccuucgccgc gucucaaggg uggccuaaug acgccgaaag 1260
guccguugac cuugacgaug uugccacggu uaucuugcug guucuggagc ugcugagccg 1320
ucucgacauu gaucuugaag gcuugagcca agaucucagg ugcgaagcca uugaagaugu 1380
uguuuugcuu cuguugcuuu cggccuugua gccauuccug cccuuguggg uuguuuccgg 1440
cuaucaaaaa uggucuaagg uugcggucaa gcugguucug guugcuggcg agauccgcgg 1500
cugcaacaag aaugagaggc ucauuuccau uguuguagaa ccauugagcu acaccagaug 1560
guguugcaau ggugucaccg caccguaggu gcucuacuuu cuggugcaug ucacggaacc 1620
cuugacucug acccugaccu ugaccuucuc caaauaccgg ugacuccaug aacgucucgg 1680
cgcaucccgg aauaacucuu cccauuagac ccuuuccgug aacaacaaac gugaguuugc 1740
cggcguucaa gaaagugggc aagaaaagac ccugaggauc cgcggccgc 1789

<210> 57
<211> 1273
<212> DNA
<213> Künstliche Sequenz

<220>
<223> Beschreibung der künstlichen Sequenz: DNA construct coding for dsRNA

<400> 57

gcggccgcgg atccatggct aacaagctct tcctcgtctg cgcaactctc gccctctgct 60
tcctcctcac caacgcttcc atctaccgca ccgttgtcga attcgaagaa gatgacgcca 120
gcaaccccgt aggtccaaga cagagatgcc agaaggagtt tcagcaatca caacacctaa 180
gagcttgcca gagatggatg agcaagcaaa tgaggcaagg acgtggtggt ggtccttccc 240
tcgacgatga gttcgatttc gagggccccc agcagggata ccagctactc cagcagtgct 300
gcaacgagct tcgccaggaa gagccagttt gcgtttgccc caccttgaaa caagctgcca 360
gggcagttag cctccaggga cagcacggac cattccaatc caggaaaatt taccagtcag 420
ctaagtactt gcctaacatt tgcaagatcc agcaagttgg tgaatgtccc ttccagacca 480
ccatcccttt cttccctcct tactactagg gtactcgagg taagctcaac aaatctttag 540
aaaattaatt ttatgtgaca tatgcaataa tttgatttgg caagataaac taatagattt 600
tgcgatttgg agttttaaac tctaaataat ctaaatcgtt ttcaattggt ttaaatatat 660
atcttgcatt tttaatcgtt tttaattaaa aaatatatat atatatatat atcttgcatt 720
tttaatcgtt ttcaatttaa aaaatatctt gcacgcagaa cgctgtcgac taccctagta 780
gtaaggaggg aagaaaggga tggtggtctg gaagggacat tcaccaactt gctggatctt 840
gcaaatgtta ggcaagtact tagctgactg gtaaattttc ctggattgga atggtccgtg 900
ctgtccctgg aggctaactg ccctggcagc ttgtttcaag gtggggcaaa cgcaaactgg 960
ctcttcctgg cgaagctcgt tgcagcactg ctggagtagc tggtatccct gctgggggcc 1020
ctcgaaatcg aactcatcgt cgagggaagg accaccacca cgtccttgcc tcatttgctt 1080
gctcatccat ctctggcaag ctcttaggtg ttgtgattgc tgaaactcct tctggcatct 1140
ctgtcttgga cctacggggt tgctggcgtc atcttcttcg aattcgacaa cggtgcggta 1200
gatggaagcg ttggtgagga ggaagcagag ggcgagagtt gcgcagacga ggaagagctt 1260
gttagccatg gat 1273

<210> 58
<211> 1273
<212> RNA
<213> Künstliche Sequenz

<220>
<223> Beschreibung der künstlichen Sequenz: : RNA sequence for forming dsRNA

<400> 58

gcggccgcgg auccauggcu aacaagcucu uccucgucug cgcaacucuc gcccucugcu 60
uccuccucac caacgcuucc aucuaccgca ccguugucga auucgaagaa gaugacgcca 120
gcaaccccgu agguccaaga cagagaugcc agaaggaguu ucagcaauca caacaccuaa 180
gagcuugcca gagauggaug agcaagcaaa ugaggcaagg acgugguggu gguccuuccc 240
ucgacgauga guucgauuuc gagggccccc agcagggaua ccagcuacuc cagcagugcu 300
gcaacgagcu ucgccaggaa gagccaguuu gcguuugccc caccuugaaa caagcugcca 360
gggcaguuag ccuccaggga cagcacggac cauuccaauc caggaaaauu uaccagucag 420
cuaaguacuu gccuaacauu ugcaagaucc agcaaguugg ugaauguccc uuccagacca 480
ccaucccuuu cuucccuccu uacuacuagg guacucgagg uaagcucaac aaaucuuuag 540
aaaauuaauu uuaugugaca uaugcaauaa uuugauuugg caagauaaac uaauagauuu 600
ugcgauuugg aguuuuaaac ucuaaauaau cuaaaucguu uucaauuggu uuaaauauau 660
aucuugcauu uuuaaucguu uuuaauuaaa aaauauauau auauauauau aucuugcauu 720
uuuaaucguu uucaauuuaa aaaauaucuu gcacgcagaa cgcugucgac uacccuagua 780
guaaggaggg aagaaaggga ugguggucug gaagggacau ucaccaacuu gcuggaucuu 840
gcaaauguua ggcaaguacu uagcugacug guaaauuuuc cuggauugga augguccgug 900
cugucccugg aggcuaacug cccuggcagc uuguuucaag guggggcaaa cgcaaacugg 960
cucuuccugg cgaagcucgu ugcagcacug cuggaguagc ugguaucccu gcugggggcc 1020
cucgaaaucg aacucaucgu cgagggaagg accaccacca cguccuugcc ucauuugcuu 1080
gcucauccau cucuggcaag cucuuaggug uugugauugc ugaaacuccu ucuggcaucu 1140
cugucuugga ccuacggggu ugcuggcguc aucuucuucg aauucgacaa cggugcggua 1200
gauggaagcg uuggugagga ggaagcagag ggcgagaguu gcgcagacga ggaagagcuu 1260
guuagccaug gau 1273

<210> 59
<211> 1575
<212> DNA

<213> Arabidopsis thaliana

<220>
<221> CDS
<222> (1)..(1572)
<223> 12S cruciferin

<400> 59

```
atg gtt aag ctc agc aat ctc ctc gtt gca acc ttc ggg gtt ctc ctc    48
Met Val Lys Leu Ser Asn Leu Leu Val Ala Thr Phe Gly Val Leu Leu
 1               5                  10                  15

gtc ctt aac ggc tgc ctt gcg agg cag tca ctt ggg gtt cct cct cag    96
Val Leu Asn Gly Cys Leu Ala Arg Gln Ser Leu Gly Val Pro Pro Gln
            20                  25                  30

cta cag aac gag tgt aac ctc gac aac cta gat gtt ctc caa gcc acc   144
Leu Gln Asn Glu Cys Asn Leu Asp Asn Leu Asp Val Leu Gln Ala Thr
        35                  40                  45

gaa act atc aag agt gaa gcc ggt cag atc gag tac tgg gac cac aac   192
Glu Thr Ile Lys Ser Glu Ala Gly Gln Ile Glu Tyr Trp Asp His Asn
    50                  55                  60

cac cct cag ctc cga tgt gtt ggt gtt tcc gtt gct cgt tat gta att   240
His Pro Gln Leu Arg Cys Val Gly Val Ser Val Ala Arg Tyr Val Ile
65                  70                  75                  80
```

```
gaa caa ggc ggt ctt tac ttg ccc acc ttc ttc act tcc cca aaa att     288
Glu Gln Gly Gly Leu Tyr Leu Pro Thr Phe Phe Thr Ser Pro Lys Ile
                85                  90                  95

tcc tac gtc gtt caa gga acg ggt atc agc gga aga gtg gtc cct gga     336
Ser Tyr Val Val Gln Gly Thr Gly Ile Ser Gly Arg Val Val Pro Gly
            100                 105                 110

tgt gcc gag acc ttc atg gac tcg cag ccg atg caa gga caa caa caa     384
Cys Ala Glu Thr Phe Met Asp Ser Gln Pro Met Gln Gly Gln Gln Gln
        115                 120                 125

ggc caa cca tgg caa gga cga cag gga caa caa ggc caa cca tgg gaa     432
Gly Gln Pro Trp Gln Gly Arg Gln Gly Gln Gln Gly Gln Pro Trp Glu
    130                 135                 140

gga cag gga caa cag gga caa caa gga aga caa ggc caa cca tgg gaa     480
Gly Gln Gly Gln Gln Gly Gln Gln Gly Arg Gln Gly Gln Pro Trp Glu
145                 150                 155                 160

gga cag gga caa cag gga caa caa gga cga cag gga caa caa ggc caa     528
Gly Gln Gly Gln Gln Gly Gln Gln Gly Arg Gln Gly Gln Gln Gly Gln
                165                 170                 175

cca tgg gaa gga cag gga cag cag gga caa caa ggg ttc cgt gac atg     576
Pro Trp Glu Gly Gln Gly Gln Gln Gly Gln Gln Gly Phe Arg Asp Met
            180                 185                 190

cac cag aag gtg gaa cat gtg aga cgc gga gac gtc ttt gcc aac act     624
His Gln Lys Val Glu His Val Arg Arg Gly Asp Val Phe Ala Asn Thr
        195                 200                 205

cca ggc tct gcc cac tgg atc tac aac tca gga gaa cag cca ctt gtc     672
Pro Gly Ser Ala His Trp Ile Tyr Asn Ser Gly Glu Gln Pro Leu Val
    210                 215                 220

atc atc gct ctt ctc gac atc gcc aac tac caa aac caa ctc gac cgc     720
Ile Ile Ala Leu Leu Asp Ile Ala Asn Tyr Gln Asn Gln Leu Asp Arg
225                 230                 235                 240

aac cct aga gtg ttc cat ttg gcc gga aac aac cag cag gga ggc ttt     768
Asn Pro Arg Val Phe His Leu Ala Gly Asn Asn Gln Gln Gly Gly Phe
                245                 250                 255

ggc ggt tca cag caa caa caa gaa cag aaa aac ttg tgg agc ggg ttc     816
Gly Gly Ser Gln Gln Gln Gln Glu Gln Lys Asn Leu Trp Ser Gly Phe
            260                 265                 270

gac gca cag gtc ata gct caa gca ttg aaa att gac gtt cag ttg gct     864
Asp Ala Gln Val Ile Ala Gln Ala Leu Lys Ile Asp Val Gln Leu Ala
        275                 280                 285

cag cag ctt cag aac caa caa gac agc aga gga aac atc gtt cgt gtt     912
Gln Gln Leu Gln Asn Gln Gln Asp Ser Arg Gly Asn Ile Val Arg Val
    290                 295                 300

aag gga cct ttc cag gtc gtg agg cca cct cta aga cag ccc tac gag     960
Lys Gly Pro Phe Gln Val Val Arg Pro Pro Leu Arg Gln Pro Tyr Glu
305                 310                 315                 320

agc gag gag tgg aga cac cca cgt agc cca cag ggc aac ggc ctt gag     1008
Ser Glu Glu Trp Arg His Pro Arg Ser Pro Gln Gly Asn Gly Leu Glu
                325                 330                 335

gag act atc tgc agc atg agg tcc cac gag aac att gac gac cct gct     1056
Glu Thr Ile Cys Ser Met Arg Ser His Glu Asn Ile Asp Asp Pro Ala
            340                 345                 350
```

```
cgt gct gac gtg tac aag ccc agc cta ggt cgc gtg acc agc gtc aac      1104
Arg Ala Asp Val Tyr Lys Pro Ser Leu Gly Arg Val Thr Ser Val Asn
            355                 360                 365

agc tat acc ttg ccc atc ttg gag tat gtc agg ctc agt gcc act cgt      1152
Ser Tyr Thr Leu Pro Ile Leu Glu Tyr Val Arg Leu Ser Ala Thr Arg
    370                 375                 380

ggc gtt ctc cag ggt aat gcg atg gtg ctt cct aaa tac aac atg aac      1200
Gly Val Leu Gln Gly Asn Ala Met Val Leu Pro Lys Tyr Asn Met Asn
385                 390                 395                 400

gct aac gag atc ttg tac tgc act gga gga caa gga agg atc caa gtg      1248
Ala Asn Glu Ile Leu Tyr Cys Thr Gly Gly Gln Gly Arg Ile Gln Val
                405                 410                 415

gtc aac gac aac gga cag aac gtg ttg gac caa cag gtg cag aag gga      1296
Val Asn Asp Asn Gly Gln Asn Val Leu Asp Gln Gln Val Gln Lys Gly
            420                 425                 430

cag ctc gtg gtc atc cca caa ggg ttc gca tac gtt gtc cag tcc cac      1344
Gln Leu Val Val Ile Pro Gln Gly Phe Ala Tyr Val Val Gln Ser His
        435                 440                 445

gga aac aag ttc gag tgg atc tct ttc aaa act aat gaa aac gca atg      1392
Gly Asn Lys Phe Glu Trp Ile Ser Phe Lys Thr Asn Glu Asn Ala Met
    450                 455                 460

atc agc act ttg gcg ggt aga acc tcg ctc ttg agg gca ttg cca ttg      1440
Ile Ser Thr Leu Ala Gly Arg Thr Ser Leu Leu Arg Ala Leu Pro Leu
465                 470                 475                 480

gag gtc ata tca aat ggt ttc cag atc tct ccc gag gaa gct agg aag      1488
Glu Val Ile Ser Asn Gly Phe Gln Ile Ser Pro Glu Glu Ala Arg Lys
                485                 490                 495

atc aag ttc aac aca ctt gag acc act ttg acc cgc gct gcc ggt agg      1536
Ile Lys Phe Asn Thr Leu Glu Thr Thr Leu Thr Arg Ala Ala Gly Arg
            500                 505                 510

caa caa caa cag ttg atc gag gag att gtc gag gct taa                  1575
Gln Gln Gln Gln Leu Ile Glu Glu Ile Val Glu Ala
        515                 520
```

<210> 60
<211> 524
<212> PRT
<213> Arabidopsis thaliana

<400> 60

```
Met Val Lys Leu Ser Asn Leu Leu Val Ala Thr Phe Gly Val Leu Leu
1               5                   10                  15

Val Leu Asn Gly Cys Leu Ala Arg Gln Ser Leu Gly Val Pro Pro Gln
            20                  25                  30

Leu Gln Asn Glu Cys Asn Leu Asp Asn Leu Asp Val Leu Gln Ala Thr
        35                  40                  45

Glu Thr Ile Lys Ser Glu Ala Gly Gln Ile Glu Tyr Trp Asp His Asn
    50                  55                  60

His Pro Gln Leu Arg Cys Val Gly Val Ser Val Ala Arg Tyr Val Ile
65                  70                  75                  80

Glu Gln Gly Gly Leu Tyr Leu Pro Thr Phe Phe Thr Ser Pro Lys Ile
                85                  90                  95
```

Ser Tyr Val Val Gln Gly Thr Gly Ile Ser Gly Arg Val Val Pro Gly
100 105 110

Cys Ala Glu Thr Phe Met Asp Ser Gln Pro Met Gln Gly Gln Gln Gln
115 120 125

Gly Gln Pro Trp Gln Gly Arg Gln Gly Gln Gln Gly Gln Pro Trp Glu
130 135 140

Gly Gln Gly Gln Gln Gly Gln Gln Gly Arg Gln Gly Gln Pro Trp Glu
145 150 155 160

Gly Gln Gly Gln Gln Gly Gln Gln Gly Arg Gln Gly Gln Gln Gly Gln
165 170 175

Pro Trp Glu Gly Gln Gly Gln Gln Gly Gln Gln Gly Phe Arg Asp Met
180 185 190

His Gln Lys Val Glu His Val Arg Arg Gly Asp Val Phe Ala Asn Thr
195 200 205

Pro Gly Ser Ala His Trp Ile Tyr Asn Ser Gly Glu Gln Pro Leu Val
210 215 220

Ile Ile Ala Leu Leu Asp Ile Ala Asn Tyr Gln Asn Gln Leu Asp Arg
225 230 235 240

Asn Pro Arg Val Phe His Leu Ala Gly Asn Asn Gln Gln Gly Gly Phe
245 250 255

Gly Gly Ser Gln Gln Gln Gln Glu Gln Lys Asn Leu Trp Ser Gly Phe
260 265 270

Asp Ala Gln Val Ile Ala Gln Ala Leu Lys Ile Asp Val Gln Leu Ala
275 280 285

Gln Gln Leu Gln Asn Gln Gln Asp Ser Arg Gly Asn Ile Val Arg Val
290 295 300

Lys Gly Pro Phe Gln Val Val Arg Pro Pro Leu Arg Gln Pro Tyr Glu
305 310 315 320

Ser Glu Glu Trp Arg His Pro Arg Ser Pro Gln Gly Asn Gly Leu Glu
325 330 335

Glu Thr Ile Cys Ser Met Arg Ser His Glu Asn Ile Asp Asp Pro Ala
340 345 350

Arg Ala Asp Val Tyr Lys Pro Ser Leu Gly Arg Val Thr Ser Val Asn
355 360 365

Ser Tyr Thr Leu Pro Ile Leu Glu Tyr Val Arg Leu Ser Ala Thr Arg
370 375 380

Gly Val Leu Gln Gly Asn Ala Met Val Leu Pro Lys Tyr Asn Met Asn
385 390 395 400

Ala Asn Glu Ile Leu Tyr Cys Thr Gly Gly Gln Gly Arg Ile Gln Val
405 410 415

Val Asn Asp Asn Gly Gln Asn Val Leu Asp Gln Gln Val Gln Lys Gly
420 425 430

Gln Leu Val Val Ile Pro Gln Gly Phe Ala Tyr Val Val Gln Ser His
435 440 445

Gly Asn Lys Phe Glu Trp Ile Ser Phe Lys Thr Asn Glu Asn Ala Met
450 455 460

Ile Ser Thr Leu Ala Gly Arg Thr Ser Leu Leu Arg Ala Leu Pro Leu
465 470 475 480

Glu Val Ile Ser Asn Gly Phe Gln Ile Ser Pro Glu Glu Ala Arg Lys
485 490 495

Ile Lys Phe Asn Thr Leu Glu Thr Thr Leu Thr Arg Ala Ala Gly Arg
500 505 510

Gln Gln Gln Gln Leu Ile Glu Glu Ile Val Glu Ala
515 520

<210> 61
<211> 1419
<212> DNA
<213> Arabidopsis thaliana

<220>
<221> CDS
<222> (1)..(1416)
<223> 12S Cra1 storage protein

<400> 61

```
atg gct cga gtc tct tct ctt ctt tct ttc tgc tta aca ctt ttg atc     48
Met Ala Arg Val Ser Ser Leu Leu Ser Phe Cys Leu Thr Leu Leu Ile
1               5                   10                  15

ctt ttc cat ggc tac gcg gct caa cag ggt cag cag ggt cag cag ttt     96
Leu Phe His Gly Tyr Ala Ala Gln Gln Gly Gln Gln Gly Gln Gln Phe
            20                  25                  30

ccg aac gag tgc cag ctc gac cag ctc aat gcg ctc gag ccg tca cac     144
Pro Asn Glu Cys Gln Leu Asp Gln Leu Asn Ala Leu Glu Pro Ser His
        35                  40                  45

gta ctg aag agc gag gct ggt cgc atc gag gtg tgg gac cac cac gct     192
Val Leu Lys Ser Glu Ala Gly Arg Ile Glu Val Trp Asp His His Ala
    50                  55                  60

cct cag ctc cgt tgc tca ggt gtc tcc ttt gca cgt tac atc atc gag     240
Pro Gln Leu Arg Cys Ser Gly Val Ser Phe Ala Arg Tyr Ile Ile Glu
65                  70                  75                  80

tct aag ggt ctc tac ttg ccc tct ttc ttt aac acc gcg aag ctc tct     288
Ser Lys Gly Leu Tyr Leu Pro Ser Phe Phe Asn Thr Ala Lys Leu Ser
                85                  90                  95

ttc gtg gct aag gga cga ggt ctt atg gga aaa gtg atc cct gga tgc     336
Phe Val Ala Lys Gly Arg Gly Leu Met Gly Lys Val Ile Pro Gly Cys
            100                 105                 110

gcc gaa aca ttc caa gac tca tca gag ttc caa cca cgc ttc gaa ggt     384
Ala Glu Thr Phe Gln Asp Ser Ser Glu Phe Gln Pro Arg Phe Glu Gly
        115                 120                 125

caa ggt caa agc cag agg ttc cgt gac atg cac cag aaa gtg gag cac     432
Gln Gly Gln Ser Gln Arg Phe Arg Asp Met His Gln Lys Val Glu His
    130                 135                 140

att agg agc ggt gat acc att gcc aca aca ccc ggt gta gca cag tgg     480
Ile Arg Ser Gly Asp Thr Ile Ala Thr Thr Pro Gly Val Ala Gln Trp
145                 150                 155                 160

ttc tac aac gac gga cag cag cca ctt gtc atc gtc agc gtc ttc gat     528
Phe Tyr Asn Asp Gly Gln Gln Pro Leu Val Ile Val Ser Val Phe Asp
                165                 170                 175

cta gcc agt cac cag aac cag ctt gac cgc aac cca agg cca ttt tac     576
Leu Ala Ser His Gln Asn Gln Leu Asp Arg Asn Pro Arg Pro Phe Tyr
            180                 185                 190
```

```
tta gcc gga aac aac cca caa ggt caa gta tgg cta caa gga cga gag      624
Leu Ala Gly Asn Asn Pro Gln Gly Gln Val Trp Leu Gln Gly Arg Glu
        195                 200                 205

caa cag cca cag aag aac att ttc aat gga ttt gga ccc gag gtt att      672
Gln Gln Pro Gln Lys Asn Ile Phe Asn Gly Phe Gly Pro Glu Val Ile
    210                 215                 220

gct caa gct ttg aag atc gat ctt cag aca gca cag caa ctt cag aac      720
Ala Gln Ala Leu Lys Ile Asp Leu Gln Thr Ala Gln Gln Leu Gln Asn
225                 230                 235                 240

caa gat gac aac cgt gga aac att gtc cga gtc caa gga ccg ttc ggt      768
Gln Asp Asp Asn Arg Gly Asn Ile Val Arg Val Gln Gly Pro Phe Gly
                245                 250                 255

gtc att agg ccg cct ttg agg ggc cag aga cct cag gag gag gaa gaa      816
Val Ile Arg Pro Pro Leu Arg Gly Gln Arg Pro Gln Glu Glu Glu Glu
            260                 265                 270

gaa gaa gga cga cat gga cga cac ggt aat ggc tta gag gag acc atc      864
Glu Glu Gly Arg His Gly Arg His Gly Asn Gly Leu Glu Glu Thr Ile
        275                 280                 285

tgc agc gcc agg tgc acc gat aac ctc gat gac ccg tct cgt gct gac      912
Cys Ser Ala Arg Cys Thr Asp Asn Leu Asp Asp Pro Ser Arg Ala Asp
    290                 295                 300

gtg tac aag cca cag ctc ggt tac atc agc act ctc aac agt tac gat      960
Val Tyr Lys Pro Gln Leu Gly Tyr Ile Ser Thr Leu Asn Ser Tyr Asp
305                 310                 315                 320

ctc ccc att ctt cgc ttc atc cgt ctc tca gcc ctc cgt gga tct atc      1008
Leu Pro Ile Leu Arg Phe Ile Arg Leu Ser Ala Leu Arg Gly Ser Ile
                325                 330                 335

cgt caa aac gca atg gtg ctt cca cag tgg aac gca aac gcg aac gct      1056
Arg Gln Asn Ala Met Val Leu Pro Gln Trp Asn Ala Asn Ala Asn Ala
            340                 345                 350

att ctt tac gag aca gac ggg gaa gcc caa atc cag atc gta aac gac      1104
Ile Leu Tyr Glu Thr Asp Gly Glu Ala Gln Ile Gln Ile Val Asn Asp
        355                 360                 365

aat ggt aac aga gtg ttt gac gga caa gtc tct caa gga cag ctc ata      1152
Asn Gly Asn Arg Val Phe Asp Gly Gln Val Ser Gln Gly Gln Leu Ile
    370                 375                 380

gcc gta cca caa ggt ttc tcg gtg gtg aaa cgc gca aca agc aac cga      1200
Ala Val Pro Gln Gly Phe Ser Val Val Lys Arg Ala Thr Ser Asn Arg
385                 390                 395                 400

ttc cag tgg gtt gag ttc aaa aca aac gct aac gcg caa atc aac act      1248
Phe Gln Trp Val Glu Phe Lys Thr Asn Ala Asn Ala Gln Ile Asn Thr
                405                 410                 415

ctg gcg gga cga acc tca gtc ttg aga ggt tta cca ctt gaa gtc ata      1296
Leu Ala Gly Arg Thr Ser Val Leu Arg Gly Leu Pro Leu Glu Val Ile
            420                 425                 430

acc aat ggg ttc caa atc tca ccc gaa gaa gca agg agg gtc aag ttc      1344
Thr Asn Gly Phe Gln Ile Ser Pro Glu Glu Ala Arg Arg Val Lys Phe
        435                 440                 445

aac acg ctc gag acc act ttg act cac agc agt ggc cca gct agc tac      1392
Asn Thr Leu Glu Thr Thr Leu Thr His Ser Ser Gly Pro Ala Ser Tyr
    450                 455                 460

gga agg cca aga gtg gct gca gct taa                                  1419
Gly Arg Pro Arg Val Ala Ala Ala
465                 470
```

<210> 62
<211> 472
<212> PRT
<213> Arabidopsis thaliana

<400> 62

```
Met Ala Arg Val Ser Ser Leu Leu Ser Phe Cys Leu Thr Leu Leu Ile
1               5                   10                  15

Leu Phe His Gly Tyr Ala Ala Gln Gln Gly Gln Gln Gly Gln Gln Phe
            20                  25                  30

Pro Asn Glu Cys Gln Leu Asp Gln Leu Asn Ala Leu Glu Pro Ser His
        35                  40                  45

Val Leu Lys Ser Glu Ala Gly Arg Ile Glu Val Trp Asp His His Ala
    50                  55                  60

Pro Gln Leu Arg Cys Ser Gly Val Ser Phe Ala Arg Tyr Ile Ile Glu
65                  70                  75                  80

Ser Lys Gly Leu Tyr Leu Pro Ser Phe Phe Asn Thr Ala Lys Leu Ser
                85                  90                  95

Phe Val Ala Lys Gly Arg Gly Leu Met Gly Lys Val Ile Pro Gly Cys
            100                 105                 110

Ala Glu Thr Phe Gln Asp Ser Ser Glu Phe Gln Pro Arg Phe Glu Gly
        115                 120                 125

Gln Gly Gln Ser Gln Arg Phe Arg Asp Met His Gln Lys Val Glu His
    130                 135                 140

Ile Arg Ser Gly Asp Thr Ile Ala Thr Thr Pro Gly Val Ala Gln Trp
145                 150                 155                 160

Phe Tyr Asn Asp Gly Gln Gln Pro Leu Val Ile Val Ser Val Phe Asp
                165                 170                 175

Leu Ala Ser His Gln Asn Gln Leu Asp Arg Asn Pro Arg Pro Phe Tyr
            180                 185                 190

Leu Ala Gly Asn Asn Pro Gln Gly Gln Val Trp Leu Gln Gly Arg Glu
        195                 200                 205

Gln Gln Pro Gln Lys Asn Ile Phe Asn Gly Phe Gly Pro Glu Val Ile
    210                 215                 220

Ala Gln Ala Leu Lys Ile Asp Leu Gln Thr Ala Gln Gln Leu Gln Asn
225                 230                 235                 240

Gln Asp Asp Asn Arg Gly Asn Ile Val Arg Val Gln Gly Pro Phe Gly
                245                 250                 255

Val Ile Arg Pro Pro Leu Arg Gly Gln Arg Pro Gln Glu Glu Glu Glu
            260                 265                 270

Glu Glu Gly Arg His Gly Arg His Gly Asn Gly Leu Glu Glu Thr Ile
        275                 280                 285

Cys Ser Ala Arg Cys Thr Asp Asn Leu Asp Asp Pro Ser Arg Ala Asp
    290                 295                 300

Val Tyr Lys Pro Gln Leu Gly Tyr Ile Ser Thr Leu Asn Ser Tyr Asp
305                 310                 315                 320
```

Leu Pro Ile Leu Arg Phe Ile Arg Leu Ser Ala Leu Arg Gly Ser Ile
325 330 335

Arg Gln Asn Ala Met Val Leu Pro Gln Trp Asn Ala Asn Ala Asn Ala
340 345 350

Ile Leu Tyr Glu Thr Asp Gly Glu Ala Gln Ile Gln Ile Val Asn Asp
355 360 365

Asn Gly Asn Arg Val Phe Asp Gly Gln Val Ser Gln Gly Gln Leu Ile
370 375 380

Ala Val Pro Gln Gly Phe Ser Val Val Lys Arg Ala Thr Ser Asn Arg
385 390 395 400

Phe Gln Trp Val Glu Phe Lys Thr Asn Ala Asn Ala Gln Ile Asn Thr
405 410 415

Leu Ala Gly Arg Thr Ser Val Leu Arg Gly Leu Pro Leu Glu Val Ile
420 425 430

Thr Asn Gly Phe Gln Ile Ser Pro Glu Glu Ala Arg Arg Val Lys Phe
435 440 445

Asn Thr Leu Glu Thr Thr Leu Thr His Ser Ser Gly Pro Ala Ser Tyr
450 455 460

Gly Arg Pro Arg Val Ala Ala Ala
465 470

<210> 63
<211> 1419
<212> DNA
<213> Arabidopsis thaliana

<220>
<221> CDS
<222> (1)..(1416)
<223> At5g442120/MLN1_4 storage protein

<400> 63

atg gct cga gtc tct tct ctt ctt tct ttc tgc tta aca ctt ttg atc 48
Met Ala Arg Val Ser Ser Leu Leu Ser Phe Cys Leu Thr Leu Leu Ile
1 5 10 15

ctt ttc cat ggc tac gcg gct caa cag ggt cag cag ggt cag cag ttt 96
Leu Phe His Gly Tyr Ala Ala Gln Gln Gly Gln Gln Gly Gln Gln Phe
20 25 30

ccg aac gag tgc cag ctc gac cag ctc aat gcg ctc gag ccg tca cac 144
Pro Asn Glu Cys Gln Leu Asp Gln Leu Asn Ala Leu Glu Pro Ser His
35 40 45

gta ctg aag agc gag gct ggt cgc atc gag gtg tgg gac cac cac gct 192
Val Leu Lys Ser Glu Ala Gly Arg Ile Glu Val Trp Asp His His Ala
50 55 60

cct cag ctc cgt tgc tca ggt gtc tcc ttt gca cgt tac atc atc gag 240
Pro Gln Leu Arg Cys Ser Gly Val Ser Phe Ala Arg Tyr Ile Ile Glu
65 70 75 80

tct aag ggt ctc tac ttg ccc tct ttc ttt aac acc gcg aag ctc tct 288
Ser Lys Gly Leu Tyr Leu Pro Ser Phe Phe Asn Thr Ala Lys Leu Ser
85 90 95

ttc gtg gct aag gga cga ggt ctt atg gga aaa gtg atc cct gga tgc 336
Phe Val Ala Lys Gly Arg Gly Leu Met Gly Lys Val Ile Pro Gly Cys
100 105 110

```
gcc gaa aca ttc caa gac tca tca gag ttc caa cca cgc ttc gaa ggt    384
Ala Glu Thr Phe Gln Asp Ser Ser Glu Phe Gln Pro Arg Phe Glu Gly
        115                 120                 125

caa ggt caa agc cag agg ttc cgt gac atg cac cag aaa gtg gag cac    432
Gln Gly Gln Ser Gln Arg Phe Arg Asp Met His Gln Lys Val Glu His
    130                 135                 140

att agg agc ggt gat acc att gcc aca aca ccc ggt gta gca cag tgg    480
Ile Arg Ser Gly Asp Thr Ile Ala Thr Thr Pro Gly Val Ala Gln Trp
145                 150                 155                 160

ttc tac aac gac gga cag gaa cca ctt gtc atc gtc agc gtc ttc gat    528
Phe Tyr Asn Asp Gly Gln Glu Pro Leu Val Ile Val Ser Val Phe Asp
                165                 170                 175

cta gcc agt cac cag aac cag ctt gac cgc aac cca agg cca ttt tac    576
Leu Ala Ser His Gln Asn Gln Leu Asp Arg Asn Pro Arg Pro Phe Tyr
            180                 185                 190

tta gcc gga aac aac cca caa ggt caa gta tgg cta caa gga cga gag    624
Leu Ala Gly Asn Asn Pro Gln Gly Gln Val Trp Leu Gln Gly Arg Glu
        195                 200                 205

caa cag cca cag aag aac att ttc aat gga ttt gga ccc gag gtt att    672
Gln Gln Pro Gln Lys Asn Ile Phe Asn Gly Phe Gly Pro Glu Val Ile
    210                 215                 220

gct caa gct ttg aag atc gat ctt cag aca gca cag caa ctt cag aac    720
Ala Gln Ala Leu Lys Ile Asp Leu Gln Thr Ala Gln Gln Leu Gln Asn
225                 230                 235                 240

caa gat gac aac cgt gga aac att gtc cga gtc caa gga ccg ttc ggt    768
Gln Asp Asp Asn Arg Gly Asn Ile Val Arg Val Gln Gly Pro Phe Gly
                245                 250                 255

gtc att agg ccg cct ttg agg ggc cag aga cct cag gag gag gaa gaa    816
Val Ile Arg Pro Pro Leu Arg Gly Gln Arg Pro Gln Glu Glu Glu Glu
            260                 265                 270

gaa gaa gga cga cat gga cga cac ggt aat ggc tta gag gag acc atc    864
Glu Glu Gly Arg His Gly Arg His Gly Asn Gly Leu Glu Glu Thr Ile
        275                 280                 285

tgc agc gcc agg tgc acc gat aac ctc gat gac ccg tct cgt gct gac    912
Cys Ser Ala Arg Cys Thr Asp Asn Leu Asp Asp Pro Ser Arg Ala Asp
    290                 295                 300

gtg tac aag cca cag ctc ggt tac atc agc act ctc aac agt tac gat    960
Val Tyr Lys Pro Gln Leu Gly Tyr Ile Ser Thr Leu Asn Ser Tyr Asp
305                 310                 315                 320

ctc ccc atc ctt cgc ttc atc cgt ctc tca gcc ctc cgt gga tct atc   1008
Leu Pro Ile Leu Arg Phe Ile Arg Leu Ser Ala Leu Arg Gly Ser Ile
                325                 330                 335

cgt caa aac gca atg gtg ctt cca cag tgg aac gca aac gcg aac gct   1056
Arg Gln Asn Ala Met Val Leu Pro Gln Trp Asn Ala Asn Ala Asn Ala
            340                 345                 350

att ctt tac gtg aca gac ggg gaa gcc caa atc cag atc gta aac gac   1104
Ile Leu Tyr Val Thr Asp Gly Glu Ala Gln Ile Gln Ile Val Asn Asp
        355                 360                 365

aat ggt aac aga gtg ttt gac gga caa gtc tct caa gga cag ctc ata   1152
Asn Gly Asn Arg Val Phe Asp Gly Gln Val Ser Gln Gly Gln Leu Ile
    370                 375                 380
```

```
gcc gta cca caa ggt ttc tcg gtg gtg aaa cgc gca aca agc aac cga     1200
Ala Val Pro Gln Gly Phe Ser Val Val Lys Arg Ala Thr Ser Asn Arg
385                 390                 395                 400

ttc cag tgg gtt gag ttc aaa aca aac gct aac gcg caa atc aac act     1248
Phe Gln Trp Val Glu Phe Lys Thr Asn Ala Asn Ala Gln Ile Asn Thr
                405                 410                 415

ctg gcg gga cga acc tca gtc ttg aga ggt tta cca ctt gaa gtc ata     1296
Leu Ala Gly Arg Thr Ser Val Leu Arg Gly Leu Pro Leu Glu Val Ile
            420                 425                 430

acc aat ggg ttc caa atc tca ccc gaa gaa gca agg agg gtc aag ttc     1344
Thr Asn Gly Phe Gln Ile Ser Pro Glu Glu Ala Arg Arg Val Lys Phe
        435                 440                 445

aac acg ctc gag acc act ttg act cac agc agt ggc cca gct agc tac     1392
Asn Thr Leu Glu Thr Thr Leu Thr His Ser Ser Gly Pro Ala Ser Tyr
    450                 455                 460

gga agg cca agg gtg gct gca gct taa                                 1419
Gly Arg Pro Arg Val Ala Ala Ala
465                 470
```

<210> 64
<211> 472
<212> PRT
<213> Arabidopsis thaliana

<400> 64

```
Met Ala Arg Val Ser Ser Leu Leu Ser Phe Cys Leu Thr Leu Leu Ile
1               5                   10                  15

Leu Phe His Gly Tyr Ala Ala Gln Gln Gly Gln Gln Gly Gln Gln Phe
            20                  25                  30

Pro Asn Glu Cys Gln Leu Asp Gln Leu Asn Ala Leu Glu Pro Ser His
        35                  40                  45

Val Leu Lys Ser Glu Ala Gly Arg Ile Glu Val Trp Asp His His Ala
    50                  55                  60

Pro Gln Leu Arg Cys Ser Gly Val Ser Phe Ala Arg Tyr Ile Ile Glu
65                  70                  75                  80

Ser Lys Gly Leu Tyr Leu Pro Ser Phe Phe Asn Thr Ala Lys Leu Ser
                85                  90                  95

Phe Val Ala Lys Gly Arg Gly Leu Met Gly Lys Val Ile Pro Gly Cys
            100                 105                 110

Ala Glu Thr Phe Gln Asp Ser Ser Glu Phe Gln Pro Arg Phe Glu Gly
        115                 120                 125

Gln Gly Gln Ser Gln Arg Phe Arg Asp Met His Gln Lys Val Glu His
    130                 135                 140

Ile Arg Ser Gly Asp Thr Ile Ala Thr Thr Pro Gly Val Ala Gln Trp
145                 150                 155                 160

Phe Tyr Asn Asp Gly Gln Glu Pro Leu Val Ile Val Ser Val Phe Asp
                165                 170                 175

Leu Ala Ser His Gln Asn Gln Leu Asp Arg Asn Pro Arg Pro Phe Tyr
            180                 185                 190

Leu Ala Gly Asn Asn Pro Gln Gly Gln Val Trp Leu Gln Gly Arg Glu
        195                 200                 205
```

Gln Gln Pro Gln Lys Asn Ile Phe Asn Gly Phe Gly Pro Glu Val Ile
210 215 220

Ala Gln Ala Leu Lys Ile Asp Leu Gln Thr Ala Gln Gln Leu Gln Asn
225 230 235 240

Gln Asp Asp Asn Arg Gly Asn Ile Val Arg Val Gln Gly Pro Phe Gly
245 250 255

Val Ile Arg Pro Pro Leu Arg Gly Gln Arg Pro Gln Glu Glu Glu Glu
260 265 270

Glu Glu Gly Arg His Gly Arg His Gly Asn Gly Leu Glu Glu Thr Ile
275 280 285

Cys Ser Ala Arg Cys Thr Asp Asn Leu Asp Asp Pro Ser Arg Ala Asp
290 295 300

Val Tyr Lys Pro Gln Leu Gly Tyr Ile Ser Thr Leu Asn Ser Tyr Asp
305 310 315 320

Leu Pro Ile Leu Arg Phe Ile Arg Leu Ser Ala Leu Arg Gly Ser Ile
325 330 335

Arg Gln Asn Ala Met Val Leu Pro Gln Trp Asn Ala Asn Ala Asn Ala
340 345 350

Ile Leu Tyr Val Thr Asp Gly Glu Ala Gln Ile Gln Ile Val Asn Asp
355 360 365

Asn Gly Asn Arg Val Phe Asp Gly Gln Val Ser Gln Gly Gln Leu Ile
370 375 380

Ala Val Pro Gln Gly Phe Ser Val Val Lys Arg Ala Thr Ser Asn Arg
385 390 395 400

Phe Gln Trp Val Glu Phe Lys Thr Asn Ala Asn Ala Gln Ile Asn Thr
405 410 415

Leu Ala Gly Arg Thr Ser Val Leu Arg Gly Leu Pro Leu Glu Val Ile
420 425 430

Thr Asn Gly Phe Gln Ile Ser Pro Glu Glu Ala Arg Arg Val Lys Phe
435 440 445

Asn Thr Leu Glu Thr Thr Leu Thr His Ser Ser Gly Pro Ala Ser Tyr
450 455 460

Gly Arg Pro Arg Val Ala Ala Ala
465 470

<210> 65
<211> 1368
<212> DNA
<213> Arabidopsis thaliana

<220>
<221> CDS
<222> (1)..(1365)
<223> 12S Crb storage protein

<400> 65

atg ggt cga gtc tca tct att atc tct ttc tct ttg aca ctc ttg atc 48
Met Gly Arg Val Ser Ser Ile Ile Ser Phe Ser Leu Thr Leu Leu Ile
1 5 10 15

ctc ttc aat ggc tac act gcc caa cag tgg ccc aac gag tgc cag ctc 96
Leu Phe Asn Gly Tyr Thr Ala Gln Gln Trp Pro Asn Glu Cys Gln Leu
20 25 30

```
gat caa ctc aat gcg ctc gaa cca tcc caa atc atc aag agc gag ggt    144
Asp Gln Leu Asn Ala Leu Glu Pro Ser Gln Ile Ile Lys Ser Glu Gly
        35                  40                  45

ggt cgc atc gag gtc tgg gac cac cat gca ccc cag ctc cgt tgc tcc    192
Gly Arg Ile Glu Val Trp Asp His His Ala Pro Gln Leu Arg Cys Ser
    50                  55                  60

ggc ttt gcc ttt gag cgt ttc gtc att gag cct cag ggt ctt ttc ttg    240
Gly Phe Ala Phe Glu Arg Phe Val Ile Glu Pro Gln Gly Leu Phe Leu
65                  70                  75                  80

ccc act ttc ttg aac gcc ggc aaa ctc acg ttt gtt gtt cac gga agg    288
Pro Thr Phe Leu Asn Ala Gly Lys Leu Thr Phe Val Val His Gly Arg
                85                  90                  95

ggt cta atg gga aga gtt att ccg gga tgc gcc gag acg ttc atg gag    336
Gly Leu Met Gly Arg Val Ile Pro Gly Cys Ala Glu Thr Phe Met Glu
            100                 105                 110

tca ccg gta ttt gga gaa ggt caa ggt cag ggt cag agt caa ggg ttc    384
Ser Pro Val Phe Gly Glu Gly Gln Gly Gln Gly Gln Ser Gln Gly Phe
        115                 120                 125

cgt gac atg cac cag aaa gta gag cac cta cgg tgc ggt gac acc att    432
Arg Asp Met His Gln Lys Val Glu His Leu Arg Cys Gly Asp Thr Ile
    130                 135                 140

gca aca cca tct ggt gta gct caa tgg ttc tac aac aat gga aat gag    480
Ala Thr Pro Ser Gly Val Ala Gln Trp Phe Tyr Asn Asn Gly Asn Glu
145                 150                 155                 160

cct ctc att ctt gtt gca gcc gcg gat ctc gcc agc aac cag aac cag    528
Pro Leu Ile Leu Val Ala Ala Ala Asp Leu Ala Ser Asn Gln Asn Gln
                165                 170                 175

ctt gac cgc aac ctt aga cca ttt ttg ata gcc gga aac aac cca caa    576
Leu Asp Arg Asn Leu Arg Pro Phe Leu Ile Ala Gly Asn Asn Pro Gln
            180                 185                 190

ggg cag gaa tgg cta caa ggc cga aag caa cag aag caa aac aac atc    624
Gly Gln Glu Trp Leu Gln Gly Arg Lys Gln Gln Lys Gln Asn Asn Ile
        195                 200                 205

ttc aat ggc ttc gca cct gag atc ttg gct caa gcc ttc aag atc aat    672
Phe Asn Gly Phe Ala Pro Glu Ile Leu Ala Gln Ala Phe Lys Ile Asn
    210                 215                 220

gtc gag acg gct cag cag ctc cag aac cag caa gat aac cgt ggc aac    720
Val Glu Thr Ala Gln Gln Leu Gln Asn Gln Gln Asp Asn Arg Gly Asn
225                 230                 235                 240

atc gtc aag gtc aac gga cct ttc ggc gtc att agg cca ccc ttg aga    768
Ile Val Lys Val Asn Gly Pro Phe Gly Val Ile Arg Pro Pro Leu Arg
                245                 250                 255

cgc ggc gaa ggc ggc caa caa cca cat gaa ata gct aat ggt tta gag    816
Arg Gly Glu Gly Gly Gln Gln Pro His Glu Ile Ala Asn Gly Leu Glu
            260                 265                 270

gag act ttg tgc acc atg cga tgc act gaa aac ctc gat gac ccg tcg    864
Glu Thr Leu Cys Thr Met Arg Cys Thr Glu Asn Leu Asp Asp Pro Ser
        275                 280                 285

gat gct gac gtg tac aag cca tca ctc gga tac att agc aca ctt aac    912
Asp Ala Asp Val Tyr Lys Pro Ser Leu Gly Tyr Ile Ser Thr Leu Asn
    290                 295                 300
```

```
agc tac aat ctt cct atc ctc aga ctt ctc cgc ctt agc gct ctt cgt     960
Ser Tyr Asn Leu Pro Ile Leu Arg Leu Leu Arg Leu Ser Ala Leu Arg
305                 310                 315                 320

ggc tcc atc cgt aaa aac gct atg gtg cta ccg caa tgg aac gta aac    1008
Gly Ser Ile Arg Lys Asn Ala Met Val Leu Pro Gln Trp Asn Val Asn
                325                 330                 335

gca aac gcg gca ctc tac gtg aca aac gga aag gct cat ata caa atg    1056
Ala Asn Ala Ala Leu Tyr Val Thr Asn Gly Lys Ala His Ile Gln Met
            340                 345                 350

gtg aac gac aac gga gaa aga gtg ttc gac caa gag atc tcc agc gga    1104
Val Asn Asp Asn Gly Glu Arg Val Phe Asp Gln Glu Ile Ser Ser Gly
        355                 360                 365

cag tta cta gtc gtg cca caa ggc ttt tcg gtc atg aaa cat cgc ata    1152
Gln Leu Leu Val Val Pro Gln Gly Phe Ser Val Met Lys His Arg Ile
    370                 375                 380

ggc gaa cag ttc gag tgg atc gaa ttc aag aca aac gaa aac gca cag    1200
Gly Glu Gln Phe Glu Trp Ile Glu Phe Lys Thr Asn Glu Asn Ala Gln
385                 390                 395                 400

gtc aac aca ctc gcg ggc cgt acc tca gtc atg aga ggt ttg ccg ctt    1248
Val Asn Thr Leu Ala Gly Arg Thr Ser Val Met Arg Gly Leu Pro Leu
                405                 410                 415

gag gtt ata acc aat ggg tac cag atc tct ccc gaa gaa gct aaa cga    1296
Glu Val Ile Thr Asn Gly Tyr Gln Ile Ser Pro Glu Glu Ala Lys Arg
            420                 425                 430

gta aag ttt agc acg att gag acc aca ctg acc cat agc agt cca atg    1344
Val Lys Phe Ser Thr Ile Glu Thr Thr Leu Thr His Ser Ser Pro Met
        435                 440                 445

agc tac gga agg cct agg gct tga                                    1368
Ser Tyr Gly Arg Pro Arg Ala
    450                 455
```

<210> 66
<211> 455
<212> PRT
<213> Arabidopsis thaliana

<400> 66

```
Met Gly Arg Val Ser Ser Ile Ile Ser Phe Ser Leu Thr Leu Leu Ile
1               5                   10                  15

Leu Phe Asn Gly Tyr Thr Ala Gln Gln Trp Pro Asn Glu Cys Gln Leu
            20                  25                  30

Asp Gln Leu Asn Ala Leu Glu Pro Ser Gln Ile Ile Lys Ser Glu Gly
        35                  40                  45

Gly Arg Ile Glu Val Trp Asp His His Ala Pro Gln Leu Arg Cys Ser
    50                  55                  60

Gly Phe Ala Phe Glu Arg Phe Val Ile Glu Pro Gln Gly Leu Phe Leu
65                  70                  75                  80

Pro Thr Phe Leu Asn Ala Gly Lys Leu Thr Phe Val Val His Gly Arg
                85                  90                  95

Gly Leu Met Gly Arg Val Ile Pro Gly Cys Ala Glu Thr Phe Met Glu
            100                 105                 110

Ser Pro Val Phe Gly Glu Gly Gln Gly Gln Gly Gln Ser Gln Gly Phe
        115                 120                 125
```

Arg Asp Met His Gln Lys Val Glu His Leu Arg Cys Gly Asp Thr Ile
130 135 140

Ala Thr Pro Ser Gly Val Ala Gln Trp Phe Tyr Asn Asn Gly Asn Glu
145 150 155 160

Pro Leu Ile Leu Val Ala Ala Ala Asp Leu Ala Ser Asn Gln Asn Gln
165 170 175

Leu Asp Arg Asn Leu Arg Pro Phe Leu Ile Ala Gly Asn Asn Pro Gln
180 185 190

Gly Gln Glu Trp Leu Gln Gly Arg Lys Gln Gln Lys Gln Asn Asn Ile
195 200 205

Phe Asn Gly Phe Ala Pro Glu Ile Leu Ala Gln Ala Phe Lys Ile Asn
210 215 220

Val Glu Thr Ala Gln Gln Leu Gln Asn Gln Gln Asp Asn Arg Gly Asn
225 230 235 240

Ile Val Lys Val Asn Gly Pro Phe Gly Val Ile Arg Pro Pro Leu Arg
245 250 255

Arg Gly Glu Gly Gly Gln Gln Pro His Glu Ile Ala Asn Gly Leu Glu
260 265 270

Glu Thr Leu Cys Thr Met Arg Cys Thr Glu Asn Leu Asp Asp Pro Ser
275 280 285

Asp Ala Asp Val Tyr Lys Pro Ser Leu Gly Tyr Ile Ser Thr Leu Asn
290 295 300

Ser Tyr Asn Leu Pro Ile Leu Arg Leu Leu Arg Leu Ser Ala Leu Arg
305 310 315 320

Gly Ser Ile Arg Lys Asn Ala Met Val Leu Pro Gln Trp Asn Val Asn
325 330 335

Ala Asn Ala Ala Leu Tyr Val Thr Asn Gly Lys Ala His Ile Gln Met
340 345 350

Val Asn Asp Asn Gly Glu Arg Val Phe Asp Gln Glu Ile Ser Ser Gly
355 360 365

Gln Leu Leu Val Val Pro Gln Gly Phe Ser Val Met Lys His Arg Ile
370 375 380

Gly Glu Gln Phe Glu Trp Ile Glu Phe Lys Thr Asn Glu Asn Ala Gln
385 390 395 400

Val Asn Thr Leu Ala Gly Arg Thr Ser Val Met Arg Gly Leu Pro Leu
405 410 415

Glu Val Ile Thr Asn Gly Tyr Gln Ile Ser Pro Glu Glu Ala Lys Arg
420 425 430

Val Lys Phe Ser Thr Ile Glu Thr Thr Leu Thr His Ser Ser Pro Met
435 440 445

Ser Tyr Gly Arg Pro Arg Ala
450 455

<210> 67
<211> 1356
<212> DNA
<213> Arabidopsis thaliana

<220>

<221> CDS
<222> (1)..(1353)
<223> putative 12S storage protein

<400> 67

```
atg cat aag ctt ttg ttt tct ctt ctc tcc gtc gtc tca ctc tca ttt      48
Met His Lys Leu Leu Phe Ser Leu Leu Ser Val Val Ser Leu Ser Phe
1               5                   10                  15

ctc ctc ttc ttc cat ggc gcc gag gca cgc cag cga gag gcg ccg ttt      96
Leu Leu Phe Phe His Gly Ala Glu Ala Arg Gln Arg Glu Ala Pro Phe
            20                  25                  30

cca aac gcc tgc cat ttc agc caa atc aac agc ctc gcg ccc gct cag      144
Pro Asn Ala Cys His Phe Ser Gln Ile Asn Ser Leu Ala Pro Ala Gln
        35                  40                  45

gcg acg aag ttc gaa gcc ggt cag atg gaa gta tgg gac cac atg agc      192
Ala Thr Lys Phe Glu Ala Gly Gln Met Glu Val Trp Asp His Met Ser
    50                  55                  60

cct gag ctc cga tgc gcc ggt gta acg gtg gct cgc atc acc ctt cag      240
Pro Glu Leu Arg Cys Ala Gly Val Thr Val Ala Arg Ile Thr Leu Gln
65                  70                  75                  80

ccc aat tcc att ttc ttg ccc gct ttc ttt agc cca cct gcc ctt gct      288
Pro Asn Ser Ile Phe Leu Pro Ala Phe Phe Ser Pro Pro Ala Leu Ala
                85                  90                  95

tac gtt gtc caa gga gaa gga gtt atg ggg acg att gct tct ggt tgt      336
Tyr Val Val Gln Gly Glu Gly Val Met Gly Thr Ile Ala Ser Gly Cys
            100                 105                 110

cct gag act ttt gca gaa gtt gaa gga tca tca gga aga gga gga gga      384
Pro Glu Thr Phe Ala Glu Val Glu Gly Ser Ser Gly Arg Gly Gly Gly
        115                 120                 125

gga gac ccg ggt cga cgt ttt gag gac atg cac cag aag ttg gag aat      432
Gly Asp Pro Gly Arg Arg Phe Glu Asp Met His Gln Lys Leu Glu Asn
    130                 135                 140

ttc cgg cga ggg gat gtg ttt gct tcg ctt gcc gga gtt tca cag tgg      480
Phe Arg Arg Gly Asp Val Phe Ala Ser Leu Ala Gly Val Ser Gln Trp
145                 150                 155                 160

tgg tac aac cgc ggt gat tcc gat gcc gtc att gtc att gtt ctt gat      528
Trp Tyr Asn Arg Gly Asp Ser Asp Ala Val Ile Val Ile Val Leu Asp
                165                 170                 175

gtc acc aac aga gaa aac cag ctt gac caa gtc cct agg atg ttc caa      576
Val Thr Asn Arg Glu Asn Gln Leu Asp Gln Val Pro Arg Met Phe Gln
            180                 185                 190

cta gcc ggg agc aga acg caa gaa gaa gaa caa cca tta acg tgg cca      624
Leu Ala Gly Ser Arg Thr Gln Glu Glu Glu Gln Pro Leu Thr Trp Pro
        195                 200                 205

tca ggc aac aac gct ttc agc ggt ttc gac cca aac ata atc gcg gaa      672
Ser Gly Asn Asn Ala Phe Ser Gly Phe Asp Pro Asn Ile Ile Ala Glu
    210                 215                 220

gca ttc aaa atc aac atc gag aca gct aag caa cta caa aac cag aag      720
Ala Phe Lys Ile Asn Ile Glu Thr Ala Lys Gln Leu Gln Asn Gln Lys
225                 230                 235                 240

gac aac aga gga aac ata atc cga gca aat ggt cct ctc cat ttc gtc      768
Asp Asn Arg Gly Asn Ile Ile Arg Ala Asn Gly Pro Leu His Phe Val
                245                 250                 255
```

```
atc cca ccg cct cgt gaa tgg cag caa gat ggc att gct aat ggc atc     816
Ile Pro Pro Pro Arg Glu Trp Gln Gln Asp Gly Ile Ala Asn Gly Ile
            260                 265                 270

gaa gag act tat tgc acg gct aag att cat gag aat atc gat gat cca     864
Glu Glu Thr Tyr Cys Thr Ala Lys Ile His Glu Asn Ile Asp Asp Pro
        275                 280                 285

gaa cgg tct gac cat ttt agc aca cga gcc gga aga atc agc act ctt     912
Glu Arg Ser Asp His Phe Ser Thr Arg Ala Gly Arg Ile Ser Thr Leu
    290                 295                 300

aac agc ctt aat ctc cct gtt cta cgt cta gtc aga ctt aac gcc ctt     960
Asn Ser Leu Asn Leu Pro Val Leu Arg Leu Val Arg Leu Asn Ala Leu
305                 310                 315                 320

aga ggt tat ctc tac agc gga gga atg gtg ttg cca caa tgg acg gca     1008
Arg Gly Tyr Leu Tyr Ser Gly Gly Met Val Leu Pro Gln Trp Thr Ala
                325                 330                 335

aac gcg cac acg gtg cta tac gtc aca gga ggt caa gcc aag ata caa     1056
Asn Ala His Thr Val Leu Tyr Val Thr Gly Gly Gln Ala Lys Ile Gln
            340                 345                 350

gtg gtg gac gac aat ggt cag tcg gtg ttc aat gag caa gtg gga caa     1104
Val Val Asp Asp Asn Gly Gln Ser Val Phe Asn Glu Gln Val Gly Gln
        355                 360                 365

ggc caa atc att gtg att cca caa ggc ttt gca gtt tca aaa acg gct     1152
Gly Gln Ile Ile Val Ile Pro Gln Gly Phe Ala Val Ser Lys Thr Ala
    370                 375                 380

ggt gaa acg ggt ttc gag tgg ata tca ttc aag aca aac gat aac gct     1200
Gly Glu Thr Gly Phe Glu Trp Ile Ser Phe Lys Thr Asn Asp Asn Ala
385                 390                 395                 400

tac att aac aca ctg agc ggc caa aca tcg tac ttg aga gca gtt cca     1248
Tyr Ile Asn Thr Leu Ser Gly Gln Thr Ser Tyr Leu Arg Ala Val Pro
                405                 410                 415

gtg gat gtg atc aaa gcg tca tat gga gtg aac gag gaa gaa gcc aag     1296
Val Asp Val Ile Lys Ala Ser Tyr Gly Val Asn Glu Glu Glu Ala Lys
            420                 425                 430

agg atc aag ttt agt cag caa gag acc atg ttg tct atg aca cca agc     1344
Arg Ile Lys Phe Ser Gln Gln Glu Thr Met Leu Ser Met Thr Pro Ser
        435                 440                 445

tct tct tct taa                                                     1356
Ser Ser Ser
    450
```

<210> 68
<211> 451
<212> PRT
<213> Arabidopsis thaliana

<400> 68

```
Met His Lys Leu Leu Phe Ser Leu Leu Ser Val Val Ser Leu Ser Phe
1               5                   10                  15

Leu Leu Phe Phe His Gly Ala Glu Ala Arg Gln Arg Glu Ala Pro Phe
            20                  25                  30

Pro Asn Ala Cys His Phe Ser Gln Ile Asn Ser Leu Ala Pro Ala Gln
        35                  40                  45
```

```
Ala Thr Lys Phe Glu Ala Gly Gln Met Glu Val Trp Asp His Met Ser
        50                  55                  60
Pro Glu Leu Arg Cys Ala Gly Val Thr Val Ala Arg Ile Thr Leu Gln
65                  70                  75                  80
Pro Asn Ser Ile Phe Leu Pro Ala Phe Phe Ser Pro Pro Ala Leu Ala
                85                  90                  95
Tyr Val Val Gln Gly Glu Gly Val Met Gly Thr Ile Ala Ser Gly Cys
            100                 105                 110
Pro Glu Thr Phe Ala Glu Val Glu Gly Ser Ser Gly Arg Gly Gly Gly
        115                 120                 125
Gly Asp Pro Gly Arg Arg Phe Glu Asp Met His Gln Lys Leu Glu Asn
    130                 135                 140
Phe Arg Arg Gly Asp Val Phe Ala Ser Leu Ala Gly Val Ser Gln Trp
145                 150                 155                 160
Trp Tyr Asn Arg Gly Asp Ser Asp Ala Val Ile Val Ile Val Leu Asp
                165                 170                 175
Val Thr Asn Arg Glu Asn Gln Leu Asp Gln Val Pro Arg Met Phe Gln
            180                 185                 190
Leu Ala Gly Ser Arg Thr Gln Glu Glu Glu Gln Pro Leu Thr Trp Pro
        195                 200                 205
Ser Gly Asn Asn Ala Phe Ser Gly Phe Asp Pro Asn Ile Ile Ala Glu
    210                 215                 220
Ala Phe Lys Ile Asn Ile Glu Thr Ala Lys Gln Leu Gln Asn Gln Lys
225                 230                 235                 240
Asp Asn Arg Gly Asn Ile Ile Arg Ala Asn Gly Pro Leu His Phe Val
                245                 250                 255
Ile Pro Pro Pro Arg Glu Trp Gln Gln Asp Gly Ile Ala Asn Gly Ile
            260                 265                 270
Glu Glu Thr Tyr Cys Thr Ala Lys Ile His Glu Asn Ile Asp Asp Pro
        275                 280                 285
Glu Arg Ser Asp His Phe Ser Thr Arg Ala Gly Arg Ile Ser Thr Leu
    290                 295                 300
Asn Ser Leu Asn Leu Pro Val Leu Arg Leu Val Arg Leu Asn Ala Leu
305                 310                 315                 320
Arg Gly Tyr Leu Tyr Ser Gly Gly Met Val Leu Pro Gln Trp Thr Ala
                325                 330                 335
Asn Ala His Thr Val Leu Tyr Val Thr Gly Gly Gln Ala Lys Ile Gln
            340                 345                 350
Val Val Asp Asp Asn Gly Gln Ser Val Phe Asn Glu Gln Val Gly Gln
        355                 360                 365
Gly Gln Ile Ile Val Ile Pro Gln Gly Phe Ala Val Ser Lys Thr Ala
    370                 375                 380
Gly Glu Thr Gly Phe Glu Trp Ile Ser Phe Lys Thr Asn Asp Asn Ala
385                 390                 395                 400
Tyr Ile Asn Thr Leu Ser Gly Gln Thr Ser Tyr Leu Arg Ala Val Pro
                405                 410                 415
Val Asp Val Ile Lys Ala Ser Tyr Gly Val Asn Glu Glu Glu Ala Lys
            420                 425                 430

Arg Ile Lys Phe Ser Gln Gln Glu Thr Met Leu Ser Met Thr Pro Ser
        435                 440                 445
Ser Ser Ser
    450
```

<210> 69
<211> 1356
<212> DNA
<213> Arabidopsis thaliana

<220>
<221> CDS
<222> (1)..(1353)
<223> 12S storage protein At1g03890

<400> 69

```
atg cat aag ctt ttg ttt tct ctt ctc tcc gtc gtc tca ctc tca ttt      48
Met His Lys Leu Leu Phe Ser Leu Leu Ser Val Val Ser Leu Ser Phe
1               5                   10                  15

ctc ctc ttc ttc cat ggc gcc gag gca cgc cag cga gag gcg ccg ttt      96
Leu Leu Phe Phe His Gly Ala Glu Ala Arg Gln Arg Glu Ala Pro Phe
            20                  25                  30

cca aac gcc tgc cat ttc agc caa atc aac agc ctc gcg ccc gct cag     144
Pro Asn Ala Cys His Phe Ser Gln Ile Asn Ser Leu Ala Pro Ala Gln
        35                  40                  45

gcg acg aag ttc gaa gcc ggt cag atg gaa gta tgg gac cac atg agc     192
Ala Thr Lys Phe Glu Ala Gly Gln Met Glu Val Trp Asp His Met Ser
    50                  55                  60

cct gag ctc cga tgc gcc ggt gta acg gtg gct cgc atc acc ctt cag     240
Pro Glu Leu Arg Cys Ala Gly Val Thr Val Ala Arg Ile Thr Leu Gln
65                  70                  75                  80

ccc aat tcc att ttc ttg ccc gct ttc ttt agc cca cct gcc ctt gct     288
Pro Asn Ser Ile Phe Leu Pro Ala Phe Phe Ser Pro Pro Ala Leu Ala
                85                  90                  95

tac gtt gtc caa gga gaa gga gtt atg ggg acg att gct tct ggt tgt     336
Tyr Val Val Gln Gly Glu Gly Val Met Gly Thr Ile Ala Ser Gly Cys
            100                 105                 110

cct gag act ttt gca gaa gtt gaa gga tca tca gga aga gga gga gga     384
Pro Glu Thr Phe Ala Glu Val Glu Gly Ser Ser Gly Arg Gly Gly Gly
        115                 120                 125

gga gac ccg ggt cga cgt ttt gag gac atg cac cag aag ttg gag aat     432
Gly Asp Pro Gly Arg Arg Phe Glu Asp Met His Gln Lys Leu Glu Asn
    130                 135                 140

ttc cgg cga ggg gat gtg ttt gct tcg ctt gcc gga gtt tca cag tgg     480
Phe Arg Arg Gly Asp Val Phe Ala Ser Leu Ala Gly Val Ser Gln Trp
145                 150                 155                 160

tgg tac aac cgc ggt gat tcc gat gcc gtc att gtc att gtt ctt gat     528
Trp Tyr Asn Arg Gly Asp Ser Asp Ala Val Ile Val Ile Val Leu Asp
                165                 170                 175

gtc acc aac aga gaa aac cag ctt gac caa gtc cct agg atg ttc caa     576
Val Thr Asn Arg Glu Asn Gln Leu Asp Gln Val Pro Arg Met Phe Gln
            180                 185                 190
```

```
cta gcc ggg agc aga acg caa gaa gaa gaa caa cca tta acg tgg cca      624
Leu Ala Gly Ser Arg Thr Gln Glu Glu Glu Gln Pro Leu Thr Trp Pro
        195                 200                 205

tca ggc aac aac gct ttc agc ggt ttc gac cca aac ata atc gcg gaa      672
Ser Gly Asn Asn Ala Phe Ser Gly Phe Asp Pro Asn Ile Ile Ala Glu
    210                 215                 220

gca ttc aaa atc aac atc gag aca gct aag caa cta caa aac cag aag      720
Ala Phe Lys Ile Asn Ile Glu Thr Ala Lys Gln Leu Gln Asn Gln Lys
225                 230                 235                 240

gac aac aga gga aac ata atc cga gca aat ggt cct ctc cat ttc gtc      768
Asp Asn Arg Gly Asn Ile Ile Arg Ala Asn Gly Pro Leu His Phe Val
                245                 250                 255

atc cca ccg cct cgt gaa tgg cag caa gat ggc att gct aat ggc atc      816
Ile Pro Pro Pro Arg Glu Trp Gln Gln Asp Gly Ile Ala Asn Gly Ile
            260                 265                 270

gaa gag act tat tgc acg gct aag att cat gag aat atc gat gat cca      864
Glu Glu Thr Tyr Cys Thr Ala Lys Ile His Glu Asn Ile Asp Asp Pro
        275                 280                 285

gaa cgg tct gac cat ttt agc aca cga gcc gga aga atc agc act ctt      912
Glu Arg Ser Asp His Phe Ser Thr Arg Ala Gly Arg Ile Ser Thr Leu
    290                 295                 300

aac agc ctt aat ctc cct gtt cta cgt cta gtc aga ctt aac gcc ctt      960
Asn Ser Leu Asn Leu Pro Val Leu Arg Leu Val Arg Leu Asn Ala Leu
305                 310                 315                 320

aga ggt tat ctc tac agc gga gga atg gtg ttg cca caa tgg acg gca     1008
Arg Gly Tyr Leu Tyr Ser Gly Gly Met Val Leu Pro Gln Trp Thr Ala
                325                 330                 335

aac gcg cac acg gtg cta tac gtc aca gga ggt caa gcc aag ata caa     1056
Asn Ala His Thr Val Leu Tyr Val Thr Gly Gly Gln Ala Lys Ile Gln
            340                 345                 350

gtg gtg gac gac aat ggt cag tcg gtg ttc aat gag caa gtg gga caa     1104
Val Val Asp Asp Asn Gly Gln Ser Val Phe Asn Glu Gln Val Gly Gln
        355                 360                 365

ggc caa atc att gtg att cca caa ggc ttt gca gtt tca aaa acg gct     1152
Gly Gln Ile Ile Val Ile Pro Gln Gly Phe Ala Val Ser Lys Thr Ala
    370                 375                 380

ggt gaa acg ggt ttc gag tgg ata tca ttc aag aca aac gat aac gct     1200
Gly Glu Thr Gly Phe Glu Trp Ile Ser Phe Lys Thr Asn Asp Asn Ala
385                 390                 395                 400

tac att aac aca ctg agc ggc caa aca tcg tac ttg aga gca gtt cca     1248
Tyr Ile Asn Thr Leu Ser Gly Gln Thr Ser Tyr Leu Arg Ala Val Pro
                405                 410                 415

gtg gat gtg atc aaa gcg tca tat gga gtg aac gag gaa gaa gcc aag     1296
Val Asp Val Ile Lys Ala Ser Tyr Gly Val Asn Glu Glu Glu Ala Lys
            420                 425                 430

agg atc aag ttt agt cag caa gag acc atg ttg tct atg aca cca agc     1344
Arg Ile Lys Phe Ser Gln Gln Glu Thr Met Leu Ser Met Thr Pro Ser
        435                 440                 445

tct tct tct taa                                                      1356
Ser Ser Ser
    450
```

<210> 70
<211> 451
<212> PRT
<213> Arabidopsis thaliana

<400> 70

Met His Lys Leu Leu Phe Ser Leu Leu Ser Val Val Ser Leu Ser Phe
1 5 10 15

Leu Leu Phe Phe His Gly Ala Glu Ala Arg Gln Arg Glu Ala Pro Phe
20 25 30

Pro Asn Ala Cys His Phe Ser Gln Ile Asn Ser Leu Ala Pro Ala Gln
35 40 45

Ala Thr Lys Phe Glu Ala Gly Gln Met Glu Val Trp Asp His Met Ser
50 55 60

Pro Glu Leu Arg Cys Ala Gly Val Thr Val Ala Arg Ile Thr Leu Gln
65 70 75 80

Pro Asn Ser Ile Phe Leu Pro Ala Phe Phe Ser Pro Pro Ala Leu Ala
85 90 95

Tyr Val Val Gln Gly Glu Gly Val Met Gly Thr Ile Ala Ser Gly Cys
100 105 110

Pro Glu Thr Phe Ala Glu Val Glu Gly Ser Ser Gly Arg Gly Gly Gly
115 120 125

Gly Asp Pro Gly Arg Arg Phe Glu Asp Met His Gln Lys Leu Glu Asn
130 135 140

Phe Arg Arg Gly Asp Val Phe Ala Ser Leu Ala Gly Val Ser Gln Trp
145 150 155 160

Trp Tyr Asn Arg Gly Asp Ser Asp Ala Val Ile Val Ile Val Leu Asp
165 170 175

Val Thr Asn Arg Glu Asn Gln Leu Asp Gln Val Pro Arg Met Phe Gln
180 185 190

Leu Ala Gly Ser Arg Thr Gln Glu Glu Glu Gln Pro Leu Thr Trp Pro
195 200 205

Ser Gly Asn Asn Ala Phe Ser Gly Phe Asp Pro Asn Ile Ile Ala Glu
210 215 220

Ala Phe Lys Ile Asn Ile Glu Thr Ala Lys Gln Leu Gln Asn Gln Lys
225 230 235 240

Asp Asn Arg Gly Asn Ile Ile Arg Ala Asn Gly Pro Leu His Phe Val
245 250 255

Ile Pro Pro Pro Arg Glu Trp Gln Gln Asp Gly Ile Ala Asn Gly Ile
260 265 270

Glu Glu Thr Tyr Cys Thr Ala Lys Ile His Glu Asn Ile Asp Asp Pro
275 280 285

Glu Arg Ser Asp His Phe Ser Thr Arg Ala Gly Arg Ile Ser Thr Leu
290 295 300

Asn Ser Leu Asn Leu Pro Val Leu Arg Leu Val Arg Leu Asn Ala Leu
305 310 315 320

Arg Gly Tyr Leu Tyr Ser Gly Gly Met Val Leu Pro Gln Trp Thr Ala
325 330 335

Asn Ala His Thr Val Leu Tyr Val Thr Gly Gly Gln Ala Lys Ile Gln
340 345 350

Val Val Asp Asp Asn Gly Gln Ser Val Phe Asn Glu Gln Val Gly Gln
355 360 365

Gly Gln Ile Ile Val Ile Pro Gln Gly Phe Ala Val Ser Lys Thr Ala
370 375 380

Gly Glu Thr Gly Phe Glu Trp Ile Ser Phe Lys Thr Asn Asp Asn Ala
385 390 395 400

Tyr Ile Asn Thr Leu Ser Gly Gln Thr Ser Tyr Leu Arg Ala Val Pro
405 410 415

Val Asp Val Ile Lys Ala Ser Tyr Gly Val Asn Glu Glu Glu Ala Lys
420 425 430

Arg Ile Lys Phe Ser Gln Gln Glu Thr Met Leu Ser Met Thr Pro Ser
435 440 445

Ser Ser Ser
450

<210> 71
<211> 867
<212> DNA
<213> Arabidopsis thaliana

<220>
<221> CDS
<222> (1)..(864)
<223> prohibitin 1

<400> 71

atg aac aac gtc aaa gtt cca aag ata cca ggt ggt ggt gcc att tcg 48
Met Asn Asn Val Lys Val Pro Lys Ile Pro Gly Gly Gly Ala Ile Ser
1 5 10 15

acg ttg ctt aag gtt ggg att att ggt ggg ctt ggc ctc tat ggt gct 96
Thr Leu Leu Lys Val Gly Ile Ile Gly Gly Leu Gly Leu Tyr Gly Ala
20 25 30

acg cac agt ctc tac aat gtt gaa gga gga cat cga gcc atc atg ttc 144
Thr His Ser Leu Tyr Asn Val Glu Gly Gly His Arg Ala Ile Met Phe
35 40 45

aat cgt tta gtc ggt att aaa gat aag gtt tac cct gag ggt aca cac 192
Asn Arg Leu Val Gly Ile Lys Asp Lys Val Tyr Pro Glu Gly Thr His
50 55 60

ctt atg att cct tgg ttt gaa agg ccg gtc atc tat gac gtt cgt gct 240
Leu Met Ile Pro Trp Phe Glu Arg Pro Val Ile Tyr Asp Val Arg Ala
65 70 75 80

cga cct tac ctt gtt gag agt aca tcc gga agc cgt gat ctt cag atg 288
Arg Pro Tyr Leu Val Glu Ser Thr Ser Gly Ser Arg Asp Leu Gln Met
85 90 95

gtg aaa att ggg ctt agg gtt ctc aca cgt ccc atg gca gac cag tta 336
Val Lys Ile Gly Leu Arg Val Leu Thr Arg Pro Met Ala Asp Gln Leu
100 105 110

cct gaa atc tac aga agc ctt ggt gag aac tac agc gag aga gtt cta 384
Pro Glu Ile Tyr Arg Ser Leu Gly Glu Asn Tyr Ser Glu Arg Val Leu
115 120 125

cct tct ata atc aac gag act ttg aaa gct gtg gtt gct cag tac aat 432
Pro Ser Ile Ile Asn Glu Thr Leu Lys Ala Val Val Ala Gln Tyr Asn
130 135 140

```
gca agc cag ctt att act cag aga gag gcg gtc agt agg gag atc agg     480
Ala Ser Gln Leu Ile Thr Gln Arg Glu Ala Val Ser Arg Glu Ile Arg
145                 150                 155                 160

aag att ctg act gaa cga gca gca aac ttc aat gtt gcg ctt gac gat     528
Lys Ile Leu Thr Glu Arg Ala Ala Asn Phe Asn Val Ala Leu Asp Asp
                165                 170                 175

gtg tcc atc aca aac ctg aca ttc ggg aag gag ttc aca gct gcc att     576
Val Ser Ile Thr Asn Leu Thr Phe Gly Lys Glu Phe Thr Ala Ala Ile
            180                 185                 190

gaa gca aag cag gtg gcg gct caa gag gct gag cgg gct aag ttc att     624
Glu Ala Lys Gln Val Ala Ala Gln Glu Ala Glu Arg Ala Lys Phe Ile
        195                 200                 205

gtt gag aag gcc gaa caa gac aag aga agt gct gtt atc cgc gcc cag     672
Val Glu Lys Ala Glu Gln Asp Lys Arg Ser Ala Val Ile Arg Ala Gln
    210                 215                 220

gga gaa gcc aag agt gct cag ctc att ggt caa gca att gca aac aac     720
Gly Glu Ala Lys Ser Ala Gln Leu Ile Gly Gln Ala Ile Ala Asn Asn
225                 230                 235                 240

caa gcg ttt atc acg ctc agg aag atc gag gct gca aga gag att gca     768
Gln Ala Phe Ile Thr Leu Arg Lys Ile Glu Ala Ala Arg Glu Ile Ala
                245                 250                 255

cag acc ata gca aac tcg gcg aac aag gtt tac ttg agc tca gac gat     816
Gln Thr Ile Ala Asn Ser Ala Asn Lys Val Tyr Leu Ser Ser Asp Asp
            260                 265                 270

ctt ttg ctt aac cta caa ggg atg aat ttg gat gtt gat gca aag aac     864
Leu Leu Leu Asn Leu Gln Gly Met Asn Leu Asp Val Asp Ala Lys Asn
        275                 280                 285

tag                                                                 867
```

<210> 72
<211> 288
<212> PRT
<213> Arabidopsis thaliana

<400> 72

```
Met Asn Asn Val Lys Val Pro Lys Ile Pro Gly Gly Gly Ala Ile Ser
1               5                   10                  15

Thr Leu Leu Lys Val Gly Ile Ile Gly Gly Leu Gly Leu Tyr Gly Ala
            20                  25                  30

Thr His Ser Leu Tyr Asn Val Glu Gly Gly His Arg Ala Ile Met Phe
        35                  40                  45

Asn Arg Leu Val Gly Ile Lys Asp Lys Val Tyr Pro Glu Gly Thr His
    50                  55                  60

Leu Met Ile Pro Trp Phe Glu Arg Pro Val Ile Tyr Asp Val Arg Ala
65                  70                  75                  80

Arg Pro Tyr Leu Val Glu Ser Thr Ser Gly Ser Arg Asp Leu Gln Met
                85                  90                  95

Val Lys Ile Gly Leu Arg Val Leu Thr Arg Pro Met Ala Asp Gln Leu
            100                 105                 110

Pro Glu Ile Tyr Arg Ser Leu Gly Glu Asn Tyr Ser Glu Arg Val Leu
        115                 120                 125

Pro Ser Ile Ile Asn Glu Thr Leu Lys Ala Val Val Ala Gln Tyr Asn
    130                 135                 140

Ala Ser Gln Leu Ile Thr Gln Arg Glu Ala Val Ser Arg Glu Ile Arg
145                 150                 155                 160

Lys Ile Leu Thr Glu Arg Ala Ala Asn Phe Asn Val Ala Leu Asp Asp
                165                 170                 175

Val Ser Ile Thr Asn Leu Thr Phe Gly Lys Glu Phe Thr Ala Ala Ile
            180                 185                 190

Glu Ala Lys Gln Val Ala Ala Gln Glu Ala Glu Arg Ala Lys Phe Ile
        195                 200                 205

Val Glu Lys Ala Glu Gln Asp Lys Arg Ser Ala Val Ile Arg Ala Gln
    210                 215                 220

Gly Glu Ala Lys Ser Ala Gln Leu Ile Gly Gln Ala Ile Ala Asn Asn
225                 230                 235                 240

Gln Ala Phe Ile Thr Leu Arg Lys Ile Glu Ala Ala Arg Glu Ile Ala
                245                 250                 255

Gln Thr Ile Ala Asn Ser Ala Asn Lys Val Tyr Leu Ser Ser Asp Asp
            260                 265                 270

Leu Leu Leu Asn Leu Gln Gly Met Asn Leu Asp Val Asp Ala Lys Asn
        275                 280                 285
```

<210> 73
<211> 40
<212> DNA
<213> Künstliche Sequenz

<220>
<223> Beschreibung der künstlichen Sequenz: oligonucleotid primer

<400> 73
ataagaatgc ggccgcgtgt tccatttggc cggaaacaac 40

<210> 74
<211> 33

<212> DNA
<213> Künstliche Sequenz

<220>
<223> Beschreibung der künstlichen Sequenz: oligonucleotid primer

<400> 74
cccggatcct tctgtaacat ttgacaaaac atg 33

<210> 75
<211> 40
<212> DNA
<213> Künstliche Sequenz

<220>
<223> Beschreibung der künstlichen Sequenz: oligonucleotid primer

<400> 75
ataagaatgc ggccgcgtgt tccatttggc cggaaacaac 40

<210> 76
<211> 44
<212> DNA
<213> Künstliche Sequenz

<220>
<223> Beschreibung der künstlichen Sequenz: oligonucleotid primer

<400> 76
ataagaatgc ggccgcggat ccaccctgga gaacgccacg agtg 44

<210> 77
<211> 45
<212> DNA
<213> Künstliche Sequenz

<220>
<223> Beschreibung der künstlichen Sequenz: oligonucleotid primer

<400> 77
ataagaatgc ggccgcggat ccctcagggt cttttcttgc ccact 45

<210> 78
<211> 30
<212> DNA
<213> Künstliche Sequenz

<220>
<223> Beschreibung der künstlichen Sequenz: oligonucleotid primer

<400> 78
ccgctcgagt ttacggatgg agccacgaag 30

<210> 79
<211> 30
<212> DNA
<213> Künstliche Sequenz

<220>
<223> Beschreibung der künstlichen Sequenz: oligonucleotid primer

<400> 79
ccgctcgagg taagctcaac aaatctttag 30

<210> 80
<211> 31
<212> DNA
<213> Künstliche Sequenz

<220>
<223> Beschreibung der künstlichen Sequenz: oligonucleotid primer

<400> 80
acgcgtcgac gcgttctgcg tgcaagatat t 31

<210> 81
<211> 46
<212> DNA
<213> Künstliche Sequenz

<220>
<223> Beschreibung der künstlichen Sequenz: oligonucleotid primer

<400> 81
ataagaatgc ggccgcggat ccatggctaa caagctcttc ctcgtc 46

<210> 82
<211> 45
<212> DNA
<213> Künstliche Sequenz

<220>
<223> Beschreibung der künstlichen Sequenz: oligonucleotid primer

<400> 82
ataagaatgc ggccgcggat ccctagtagt aaggagggaa gaaag 45

<210> 83
<211> 4954
<212> DNA
<213> Künstliche Sequenz

<220>
<223> Beschreibung der künstlichen Sequenz: DNA construct coding for dsRNA for suppression of multiple storage proteins

<400> 83

```
agcttggtac cgagctcgga tccactagta acggccgcca gtgtgctgga attcgccctt 60
gcggccgcgt gttccatttg gccggaaaca accagcaggg aggctttggc ggttcacagc 120
aacaacaaga acagaaaaac ttgtggagcg ggttcgacgc acaggtcata gctcaagcat 180
tgaaaattga cgttcagttg gctcagcagc ttcagaacca acaagacagc agaggaaaca 240
tcgttcgtgt taagggacct ttccaggtcg tgaggccacc tctaagacag ccctacgaga 300
gcgaggagtg gagacaccca cgtagcccac agggcaacgg ccttgaggag actatctgca 360
gcatgaggtc ccacgagaac attgacgacc ctgctcgtgc tgacgtgtac aagcccagcc 420
taggtcgcgt gaccagcgtc aacagctata ccttgcccat cttggagtat gtcaggctca 480
gtgccactcg tggcgttctc cagggtggat ccttctgtaa catttgacaa aacatgtgaa 540
cacgtcatcc gtcatataga acttccaatt ttaatatgtt ttgctaaaga aaaaaaaaag 600
gaataaatat ctatcaaatt cattttaaa acatttgtat acgttcttaa ataatttagg 660
atatgactaa ttttctttt tggtaaaaat gttaatatct atatttaatt tattaagaaa 720
aatgtactta caccctggag aacgccacga gtggcactga gcctgacata ctccaagatg 780
ggcaaggtat agctgttgac gctggtcacg cgacctaggc tgggcttgta cacgtcagca 840
cgagcagggt cgtcaatgtt ctcgtgggac ctcatgctgc agatagtctc ctcaaggccg 900
ttgccctgtg ggctacgtgg gtgtctccac tcctcgctct cgtagggctg tcttagaggt 960
ggcctcacga cctggaaagg tcccttaaca cgaacgatgt ttcctctgct gtcttgttgg 1020
ttctgaagct gctgagccaa ctgaacgtca attttcaatg cttgagctat gacctgtgcg 1080
tcgaacccgc tccacaagtt tttctgttct tgttgttgct gtgaaccgcc aaagcctccc 1140
tgctggttgt ttccggccaa atggaacacg cggccgcaag ggcgaattct gcagatatcc 1200
atcacactgg cggccgctcg acgtaagctc aacaaatctt tagaaaatta attttatgtg 1260
acatatgcaa taatttgatt tggcaagata aactaataga ttttgcgatt tggagtttta 1320
aactctaaat aatctaaatc gttttcaatt ggtttaaata tatatcttgc atttttaatc 1380
gtttttaatt aaaaaatata tatatatata tatatcttgc atttttaatc gttttcaatt 1440
taaaaaatat cttgcacgca gaacgctctc gagcggccgc ggatcctcag ggtcttttct 1500
tgcccacttt cttgaacgcc ggcaaactca cgtttgttgt tcacggaagg ggtctaatgg 1560
gaagagttat tccgggatgc gccgagacgt tcatggagtc accggtattt ggagaaggtc 1620
aaggtcaggg tcagagtcaa gggttccgtg acatgcacca gaaagtagag cacctacggt 1680
gcggtgacac cattgcaaca ccatctggtg tagctcaatg gttctacaac aatggaaatg 1740
agcctctcat tcttgttgca gccgcggatc tcgccagcaa ccagaaccag cttgaccgca 1800
accttagacc atttttgata gccggaaaca acccacaagg gcaggaatgg ctacaaggcc 1860
gaaagcaaca gaagcaaaac aacatcttca atggcttcgc acctgagatc ttggctcaag 1920
ccttcaagat caatgtcgag acggctcagc agctccagaa ccagcaagat aaccgtggca 1980
acatcgtcaa ggtcaacgga cctttcggcg tcattaggcc accccttgaga cgcggcgaag 2040
gcggccaaca accacatgaa atagctaatg gtttagagga gactttgtgc accatgcgat 2100
```

```
gcactgaaaa cctcgatgac ccgtcggatg ctgacgtgta caagccatca ctcggataca 2160
ttagcacact taacagctac aatcttccta tcctcagact tctccgcctt agcgctcttc 2220
gtggctccat ccgtaaaact cgaggtaagc tcaacaaatc tttagaaaat taattttatg 2280
tgacatatgc aataatttga tttggcaaga taaactaata gattttgcga tttggagttt 2340
taaactctaa ataatctaaa tcgttttcaa ttggtttaaa tatatatctt gcatttttaa 2400
tcgtttttaa ttaaaaaata tatatatata tatatatctt gcatttttaa tcgttttcaa 2460
tttaaaaaat atcttgcacg cagaacgctg tcgagtttta cggatggagc cacgaagagc 2520
gctaaggcgg agaagtctga ggataggaag attgtagctg ttaagtgtgc taatgtatcc 2580
gagtgatggc ttgtacacgt cagcatccga cgggtcatcg aggttttcag tgcatcgcat 2640
ggtgcacaaa gtctcctcta aaccattagc tatttcatgt ggttgttggc cgccttcgcc 2700
gcgtctcaag ggtggcctaa tgacgccgaa aggtccgttg accttgacga tgttgccacg 2760
gttatcttgc tggttctgga gctgctgagc cgtctcgaca ttgatcttga aggcttgagc 2820
caagatctca ggtgcgaagc cattgaagat gttgttttgc ttctgttgct ttcggccttg 2880
tagccattcc tgcccttgtg ggttgtttcc ggctatcaaa aatggtctaa ggttgcggtc 2940
aagctggttc tggttgctgg cgagatccgc ggctgcaaca agaatgagag gctcatttcc 3000
attgttgtag aaccattgag ctacaccaga tggtgttgca atggtgtcac cgcaccgtag 3060
gtgctctact ttctggtgca tgtcacggaa cccttgactc tgaccctgac cttgaccttc 3120
tccaaatacc ggtgactcca tgaacgtctc ggcgcatccc ggaataactc ttcccattag 3180
acccccttccg tgaacaacaa acgtgagttt gccggcgttc aagaaagtgg gcaagaaaag 3240
accctgagga tccgcggccg cgcatgcatc tagctcgagg taagctcaac aaatctttag 3300
aaaattaatt ttatgtgaca tatgcaataa tttgatttgg caagataaac taatagattt 3360
tgcgatttgg agttttaaac tctaaataat ctaaatcgtt ttcaattggt ttaaatatat 3420
atcttgcatt tttaatcgtt tttaattaaa aaatatatat atatatatat atcttgcatt 3480
tttaatcgtt ttcaatttaa aaaatatctt gcacgcagaa cgctagggcc gcggccgcgg 3540
atccatggct aacaagctct tcctcgtctg cgcaactctc gccctctgct tcctcctcac 3600
caacgcttcc atctaccgca ccgttgtcga attcgaagaa gatgacgcca gcaaccccgt 3660
aggtccaaga cagagatgcc agaaggagtt tcagcaatca caacacctaa gagcttgcca 3720
gagatggatg agcaagcaaa tgaggcaagg acgtggtggt ggtccttccc tcgacgatga 3780
gttcgatttc gagggccccc agcagggata ccagctactc cagcagtgct gcaacgagct 3840
tcgccaggaa gagccagttt gcgtttgccc caccttgaaa caagctgcca gggcagttag 3900
cctccaggga cagcacggac cattccaatc caggaaaatt taccagtcag ctaagtactt 3960
gcctaacatt tgcaagatcc agcaagttgg tgaatgtccc ttccagacca ccatcccttt 4020
cttccctcct tactactagg gtactcgagg taagctcaac aaatctttag aaaattaatt 4080
ttatgtgaca tatgcaataa tttgatttgg caagataaac taatagattt tgcgatttgg 4140
agttttaaac tctaaataat ctaaatcgtt ttcaattggt ttaaatatat atcttgcatt 4200
tttaatcgtt tttaattaaa aaatatatat atatatatat atcttgcatt tttaatcgtt 4260
ttcaatttaa aaaatatctt gcacgcagaa cgctgtcgac taccctagta gtaaggaggg 4320
aagaaaggga tggtggtctg gaagggacat tcaccaactt gctggatctt gcaaatgtta 4380
ggcaagtact tagctgactg gtaaattttc ctggattgga atggtccgtg ctgtccctgg 4440
aggctaactg ccctggcagc ttgtttcaag gtggggcaaa cgcaaactgg ctcttcctgg 4500
cgaagctcgt tgcagcactg ctggagtagc tggtatccct gctgggggcc ctcgaaatcg 4560
aactcatcgt cgagggaagg accaccacca cgtccttgcc tcatttgctt gctcatccat 4620
ctctggcaag ctcttaggtg ttgtgattgc tgaaactcct tctggcatct ctgtcttgga 4680
cctacggggt tgctggcgtc atcttcttcg aattcgacaa cggtgcggta gatggaagcg 4740
ttggtgagga ggaagcagag ggcgagagtt gcgcagacga ggaagagctt gttagccatg 4800
gatcaattcg ccctatagtg agtcgtatta caattcactg gccgtcgttt tacaacgtcg 4860
tgactgggaa aaccctggcg ttacccaact taatcgcctt gcagcacatc ccccttttcgc 4920
cagctggcgt aatagcgaag aggcccgcac cgat                             4954
```

<210> 84
<211> 4954
<212> RNA
<213> Künstliche Sequenz

<220>
<223> Beschreibung der künstlichen Sequenz: RNA coding for dsRNA for suppression of multiple storage proteins

<400> 84

agcuugguac cgagcucgga uccacuagua acggccgcca gugugcugga auucgcccuu 60
gcggccgcgu guuccauuug gccggaaaca accagcaggg aggcuuuggc gguucacagc 120
aacaacaaga acagaaaaac uuguggagcg gguucgacgc acaggucaua gcucaagcau 180
ugaaaauuga cguucaguug gcucagcagc uucagaacca acaagacagc agaggaaaca 240
ucguucgugu uaagggaccu uuccaggucg ugaggccacc ucuaagacag cccuacgaga 300
gcgaggagug gagacaccca cguagcccac agggcaacgg ccuugaggag acuaucugca 360
gcaugagguc ccacgagaac auugacgacc cugcucgugc ugacguguac aagcccagcc 420
uaggucgcgu gaccagcguc aacagcuaua ccuugcccau cuuggaguau gucaggcuca 480
gugccacucg uggcguucuc caggguggau ccuucuguaa cauuugacaa aacaugugaa 540
cacgucaucc gucauauaga acuuccaauu uuaauauguu uugcuaaaga aaaaaaaaag 600
gaauaaauau cuaucaaauu cauuuuuaaa acauuuguau acguucuuaa auaauuuagg 660
auaugacuaa uuuuucuuuu ugguaaaaau guuaauaucu auauuuaauu uauuaagaaa 720
aauguacuua cacccuggag aacgccacga guggcacuga gccugacaua cuccaagaug 780
ggcaagguau agcuguugac gcuggucacg cgaccuaggc ugggcuugua cacgucagca 840
cgagcagggu cgucaauguu cucgugggac cucaugcugc agauagucuc cucaaggccg 900
uugcccugug ggcuacgugg gugucuccac uccucgcucu cguagggcug ucuuagaggu 960
ggccucacga ccuggaaagg ucccuuaaca cgaacgaugu uuccucugcu gucuuguugg 1020
uucugaagcu gcugagccaa cugaacguca auuucaaug cuugagcuau gaccugugcg 1080
ucgaacccgc uccacaaguu uuucuguucu uguuguugcu gugaaccgcc aaagccuccc 1140
ugcugguugu uuccggccaa auggaacacg cggccgcaag ggcgaauucu gcagauaucc 1200
aucacacugg cggccgcucg acguaagcuc aacaaaucuu uagaaaauua auuuuaugug 1260
acauaugcaa uaauuugauu uggcaagaua aacuaauaga uuuugcgauu uggaguuuua 1320
aacucuaaau aaucuaaauc guuuucaauu gguuuaaaua uauaucuugc auuuuuaauc 1380
guuuuuaauu aaaaaauaua uauauauaua uauaucuugc auuuuuaauc guuuucaauu 1440
uaaaaaauau cuugcacgca gaacgcucuc gagcggccgc ggauccucag ggucuuuucu 1500
ugcccacuuu cuugaacgcc ggcaaacuca cguuuguugu ucacggaagg ggucuaaugg 1560
gaagaguuau uccgggaugc gccgagacgu ucauggaguc accgguauuu ggagaagguc 1620
aaggucaggg ucagagucaa ggguuccgug acaugcacca gaaaguagag caccuacggu 1680
gcggugacac cauugcaaca ccaucuggug uagcucaaug guucuacaac aauggaaaug 1740
agccucucau ucuuguugca gccgcggauc ucgccagcaa ccagaaccag cuugaccgca 1800
accuuagacc auuuuugaua gccggaaaca acccacaagg gcaggaaugg cuacaaggcc 1860
gaaagcaaca gaagcaaaac aacaucuuca auggcuucgc accugagauc uuggcucaag 1920
ccuucaagau caaugucgag acggcucagc agcuccagaa ccagcaagau aaccguggca 1980
acaucgucaa ggucaacgga ccuuucggcg ucauuaggcc acccuugaga cgcggcgaag 2040
gcggccaaca accacaugaa auagcuaaug guuuagagga gacuuugugc accaugcgau 2100
gcacugaaaa ccucgaugac ccgucggaug cugacgugua caagccauca cucggauaca 2160
uuagcacacu uaacagcuac aaucuuccua ucucagacu ucuccgccuu agcgcucuuc 2220
guggcuccau ccguaaaacu cgagguaagc ucaacaaauc uuuagaaaau uaauuuuaug 2280
ugacauaugc aauaauuuga uuuggcaaga uaaacuaaua gauuuugcga uuuggaguuu 2340
uaaacucuaa auaaucuaaa ucguuuucaa uugguuuaaa uauauaucuu gcauuuuuaa 2400
ucguuuuuaa uuaaaaaaua uauauauaua uauauaucuu gcauuuuuaa ucguuuucaa 2460
uuuaaaaaau aucuugcacg cagaacgcug ucgaguuuua cggauggagc cacgaagagc 2520
gcuaaggcgg agaagucuga ggauaggaag auuguagcug uuaagugugc uaauguaucc 2580
gagugauggc uuguacacgu cagcauccga cgggucaucg agguuuucag ugcaucgcau 2640
ggugcacaaa gucuccucua aaccauuagc uauuucaugu gguuguuggc cgccuucgcc 2700
gcgucucaag gguggccuaa ugacgccgaa agguccguug accuugacga uguugccacg 2760
guuaucuugc ugguucugga gcugcugagc cgucucgaca uugaucuuga aggcuugagc 2820
caagaucuca ggugcgaagc cauugaagau guuguuuugc uucuguugcu uucggccuug 2880
uagccauucc ugcccuugug gguuguuucc ggcuaucaaa aauggucuaa gguugcgguc 2940
aagcugguuc ugguugcugg cgagauccgc ggcugcaaca agaaugagag gcucauuucc 3000
auuguuguag aaccauugag cuacaccaga ugguguugca auggugucac cgcaccguag 3060
gugcucuacu uucuggugca ugucacggaa cccuugacuc ugacccugac cuugaccuuc 3120
uccaaauacc ggugacucca ugaacgucuc ggcgcauccc ggaauaacuc uucccauuag 3180
accccuuccg ugaacaacaa acgugaguuu gccggcguuc aagaaagugg gcaagaaaag 3240
acccugagga uccgcggccg cgcaugcauc uagcucgagg uaagcucaac aaaucuuuag 3300
aaaauuaauu uuaugugaca uaugcaauaa uuugauuugg caagauaaac uaauagauuu 3360

```
ugcgauuugg aguuuuaaac ucuaaauaau cuaaaucguu uucaauuggu uuaaauauau 3420
aucuugcauu uuuaaucguu uuuaauuaaa aaauauauau auauauauau aucuugcauu 3480
uuuaaucguu uucaauuuaa aaaauaucuu gcacgcagaa cgcuagggcc gcggccgcgg 3540
auccauggcu aacaagcucu uccucgucug cgcaacucuc gcccucugcu uccuccucac 3600
caacgcuucc aucuaccgca ccguugucga auucgaagaa gaugacgcca gcaaccccgu 3660
agguccaaga cagagaugcc agaaggaguu ucagcaauca caacaccuaa gagcuugcca 3720
gagauggaug agcaagcaaa ugaggcaagg acgugguggu gguccuuccc ucgacgauga 3780
guucgauuuc gagggccccc agcagggaua ccagcuacuc cagcagugcu gcaacgagcu 3840
ucgccaggaa gagccaguuu gcguuugccc caccuugaaa caagcugcca gggcaguuag 3900
ccuccaggga cagcacggac cauuccaauc caggaaaauu uaccagucag cuaaguacuu 3960
gccuaacauu ugcaagaucc agcaaguugg ugaauguccc uuccagacca ccaucccuuu 4020
cuucccuccu uacuacuagg guacucgagg uaagcucaac aaaucuuuag aaaauuaauu 4080
uuaugugaca uaugcaauaa uuugauuugg caagauaaac uaauagauuu ugcgauuugg 4140
aguuuuaaac ucuaaauaau cuaaaucguu uucaauuggu uuaaauauau aucuugcauu 4200
uuuaaucguu uuuaauuaaa aaauauauau auauauauau aucuugcauu uuuaaucguu 4260
uucaauuuaa aaaauaucuu gcacgcagaa cgcugucgac uacccuagua guaaggaggg 4320
aagaaaggga ugguggucug gaagggacau ucaccaacuu gcuggaucuu gcaaauguua 4380
ggcaaguacu uagcugacug guaaauuuuc cuggauugga augguccgug cugucccugg 4440
aggcuaacug cccuggcagc uuguuucaag gugggggcaaa cgcaaacugg cucuuccugg 4500
cgaagcucgu ugcagcacug cuggaguagc ugguaucccu gcugggggcc cucgaaaucg 4560
aacucaucgu cgagggaagg accaccacca cguccuugcc ucauuugcuu gcucauccau 4620
cucuggcaag cucuuaggug uugugauugc ugaaacuccu ucuggcaucu cugucuugga 4680
ccuacggggu ugcuggcguc aucuucuucg aauucgacaa cggugcggua gauggaagcg 4740
uuggugagga ggaagcagag ggcgagaguu gcgcagacga ggaagagcuu guuagccaug 4800
gaucaauucg cccuauagug agucguauua caauucacug gccgucguuu uacaacgucg 4860
ugacugggaa aacccuggcg uuacccaacu uaaucgccuu gcagcacauc cccuuucgc 4920
cagcuggcgu aauagcgaag aggcccgcac cgau 4954
```

<210> 85
<211> 4456
<212> DNA
<213> Künstliche Sequenz

<220>
<223> Beschreibung der künstlichen Sequenz: DNA construct coding for dsRNA for suppression of multiple storage proteins

<400> 85

```
agcttggtac cgagctcgga tccactagta acggccgcca gtgtgctgga attcgccctt 60
gcggccgcgg atcctcaggg tcttttcttg cccactttct tgaacgccgg caaactcacg 120
tttgttgttc acggaagggg tctaatggga agagttattc cgggatgcgc cgagacgttc 180
atggagtcac cggtatttgg agaaggtcaa ggtcagggtc agagtcaagg gttccgtgac 240
atgcaccaga aagtagagca cctacggtgc ggtgacacca ttgcaacacc atctggtgta 300
gctcaatggt tctacaacaa tggaaatgag cctctcattc ttgttgcagc cgcggatctc 360
gccagcaacc agaaccagct tgaccgcaac cttagaccat ttttgatagc cggaaacaac 420
ccacaagggc aggaatggct acaaggccga aagcaacaga agcaaaacaa catcttcaat 480
ggcttcgcac ctgagatctt ggctcaagcc ttcaagatca atgtcgagac ggctcagcag 540
ctccagaacc agcaagataa ccgtggcaac atcgtcaagg tcaacggacc tttcggcgtc 600
attaggccac ccttgagacg cggcgaaggc ggccaacaac cacatgaaat agctaatggt 660
ttagaggaga ctttgtgcac catgcgatgc actgaaaacc tcgatgaccc gtcggatgct 720
gacgtgtaca agccatcact cggatacatt agcacactta acagctacaa tcttcctatc 780
ctcagacttc tccgccttag cgctcttcgt ggctccatcc gtaaaaggat ccgcggccgc 840
aagggcgaat tctgcagatc cttcagggtc ttttcttgcc cactttcttg aacgccggca 900
aactcacgtt tgttgttcac ggaaggggtc taatgggaag agttattccg ggatgcgccg 960
agacgttcat ggagtcaccg gtatttggag aaggtcaagg tcagggtcag agtcaagggt 1020
tccgtgacat gcaccagaaa gtagagcacc tacggtgcgg tgacaccatt gcaacaccat 1080
ctggtgtagc tcaatggttc tacaacaatg gaaatgagcc tctcattctt gttgcagccg 1140
cggatctcgc cagcaaccag aaccagcttg accgcaacct tagaccattt ttgatagccg 1200
```

gaaacaaccc acaagggcag gaatggctac aaggccgaaa gcaacagaag caaaacaaca 1260
tcttcaatgg cttcgcacct gagatcttgg ctcaagcctt caagatcaat gtcgagacgg 1320
ctcagcagct ccagaaccag caagataacc gtggcaacat cgtcaaggtc aacggacctt 1380
tcggcgtcat taggccaccc ttgagacgcg gcgaaggcgg ccaacaacca catgaaatag 1440
ctaatggttt agaggagact ttgtgcacca tgcgatgcac tgaaaacctc gatgacccgt 1500
cggatgctga cgtgtacaag ccatcactcg gatacattag cacacttaac agctacaatc 1560
ttcctatcct cagacttctc cgccttagcg ctcttcgtgg ctccatccgt aaaagatcct 1620
atggctaaca agctcttcct cgtctgcgca actctcgccc tctgcttcct cctcaccaac 1680
gcttccatct accgcaccgt tgtcgaattc gaagaagatg acgccagcaa ccccgtaggt 1740
ccaagacaga gatgccagaa ggagtttcag caatcacaac acctaagagc ttgccagaga 1800
tggatgagca agcaaatgag gcaaggacgt ggtggtggtc cttccctcga cgatgagttc 1860
gatttcgagg gcccccagca gggataccag ctactccagc agtgctgcaa cgagcttcgc 1920
caggaagagc cagtttgcgt ttgccccacc ttgaaacaag ctgccagggc agttagcctc 1980
cagggacagc acggaccatt ccaatccagg aaaatttacc agtcagctaa gtacttgcct 2040
aacatttgca agatccagca agttggtgaa tgtcccttcc agaccaccat ccctttcttc 2100
cctccttact actagggtag atatccatca cactggcggc cgctcgacgt aagctcaaca 2160
aatctttaga aaattaattt tatgtgacat atgcaataat ttgatttggc aagataaact 2220
aatagatttt gcgatttgga gttttaaact ctaaataatc taaatcgttt tcaattggtt 2280
taaatatata tcttgcattt ttaatcgttt ttaattaaaa aatatatata tatatatata 2340
tcttgcattt ttaatcgttt tcaatttaaa aaatatcttg cacgcagaac gcctcgacta 2400
ccctagtagt aaggagggaa gaaagggatg gtggtctgga agggacattc accaacttgc 2460
tggatcttgc aaatgttagg caagtactta gctgactggt aaatttcct ggattggaat 2520
ggtccgtgct gtccctggag gctaactgcc ctggcagctt gtttcaaggt ggggcaaacg 2580
caaactggct cttcctggcg aagctcgttg cagcactgct ggagtagctg gtatccctgc 2640
tggggggccct cgaaatcgaa ctcatcgtcg agggaaggac caccaccacg tccttgcctc 2700
atttgcttgc tcatccatct ctggcaagct cttaggtgtt gtgattgctg aaactccttc 2760
tggcatctct gtcttggacc tacggggttg ctggcgtcat cttcttcgaa ttcgacaacg 2820
gtgcggtaga tggaagcgtt ggtgaggagg aagcagaggg cgagagttgc gcagacgagg 2880
aagagcttgt tagccatagg atcttttacg gatggagcca cgaagagcgc taaggcggag 2940
aagtctgagg ataggaagat tgtagctgtt aagtgtgcta atgtatccga gtgatggctt 3000
gtacacgtca gcatccgacg ggtcatcgag gttttcagtg catcgcatgg tgcacaaagt 3060
ctcctctaaa ccattagcta tttcatgtgg ttgttggccg ccttcgccgc gtctcaaggg 3120
tggcctaatg acgccgaaag gtccgttgac cttgacgatg ttgccacggt tatcttgctg 3180
gttctggagc tgctgagccg tctcgacatt gatcttgaag gcttgagcca agatctcagg 3240
tgcgaagcca ttgaagatgt tgttttgctt ctgttgcttt cggccttgta gccattcctg 3300
cccttgtggg ttgtttccgg ctatcaaaaa tggtctaagg ttgcggtcaa gctggttctg 3360
gttgctggcg agatccgcgg ctgcaacaag aatgagaggc tcatttccat tgttgtagaa 3420
ccattgagct acaccagatg gtgttgcaat ggtgtcaccg caccgtaggt gctctacttt 3480
ctggtgcatg tcacggaacc cttgactctg accctgacct tgaccttctc caaataccgg 3540
tgactccatg aacgtctcgg cgcatcccgg aataactctt cccattagac cccttccgtg 3600
aacaacaaac gtgagtttgc cggcgttcaa gaaagtgggc aagaaaagac cctgaaggat 3660
ctgcagaatt cgcccttgcg gccgcggatc cttttacgga tggagccacg aagagcgcta 3720
aggcggagaa gtctgaggat aggaagattg tagctgttaa gtgtgctaat gtatccgagt 3780
gatggcttgt acacgtcagc atccgacggg tcatcgaggt tttcagtgca tcgcatggtg 3840
cacaaagtct cctctaaacc attagctatt tcatgtggtt gttggccgcc ttcgccgcgt 3900
ctcaagggtg gcctaatgac gccgaaaggt ccgttgacct tgacgatgtt gccacggtta 3960
tcttgctggt tctggagctg ctgagccgtc tcgacattga tcttgaaggc ttgagccaag 4020
atctcaggtg cgaagccatt gaagatgttg ttttgcttct gttgctttcg gccttgtagc 4080
cattcctgcc cttgtgggtt gtttccggct atcaaaaatg gtctaaggtt gcggtcaagc 4140
tggttctggt tgctggcgag atccgcggct gcaacaagaa tgagaggctc atttccattg 4200
ttgtagaacc attgagctac accagatggt gttgcaatgg tgtcaccgca ccgtaggtgc 4260
tctactttct ggtgcatgtc acggaaccct tgactctgac cctgaccttg accttctcca 4320
aataccggtg actccatgaa cgtctcggcg catcccggaa taactcttcc cattagaccc 4380
cttccgtgaa caacaaacgt gagtttgccg gcgttcaaga aagtgggcaa gaaaagaccc 4440
tgactcgagc atgcat 4456

<210> 86
<211> 4456
<212> RNA
<213> Künstliche Sequenz

<220>

<223> Beschreibung der künstlichen Sequenz: RNA coding for dsRNA for suppression of multiple storage proteins

<400> 86

```
agcuugguac cgagcucgga uccacuagua acggccgcca gugugcugga auucgcccuu 60
gcggccgcgg auccucaggg ucuuuucuug cccacuuucu ugaacgccgg caaacucacg 120
uuuguuguuc acggaagggg ucuaauggga agaguuauuc cgggaugcgc cgagacguuc 180
auggagucac cgguauuugg agaaggucaa ggucaggguc agagucaagg guuccgugac 240
augcaccaga aaguagagca ccuacggugc ggugacacca uugcaacacc aucuggugua 300
gcucaauggu ucuacaacaa uggaaaugag ccucucauuc uuguugcagc cgcggaucuc 360
gccagcaacc agaaccagcu ugaccgcaac cuuagaccau uuuugauagc cggaaacaac 420
ccacaagggc aggaauggcu acaaggccga aagcaacaga agcaaaacaa caucuucaau 480
ggcuucgcac cugagaucuu ggcucaagcc uucaagauca augucgagac ggcucagcag 540
cuccagaacc agcaagauaa ccguggcaac aucgucaagg ucaacggacc uuucggcguc 600
auuaggccac ccuugagacg cggcgaaggc ggccaacaac cacaugaaau agcuaauggu 660
uuagaggaga cuuugugcac caugcgaugc acugaaaacc ucgaugaccc gucggaugcu 720
gacguguaca agccaucacu cggauacauu agcacacuua acagcuacaa ucuuccuauc 780
cucagacuuc uccgccuuag cgcucuucgu ggcuccaucc guaaaaggau ccgcggccgc 840
aagggcgaau ucugcagauc cuucaggguc uuuucuugcc cacuuucuug aacgccggca 900
aacucacguu uguuguucac ggaagggguc uaaugggaag aguuauuccg ggaugcgccg 960
agacguucau ggagucaccg guauuuggag aaggucaagg ucagggucag agucaagggu 1020
uccgugacau gcaccagaaa guagagcacc uacggugcgg ugacaccauu gcaacaccau 1080
cugguguagc ucaaugguuc uacaacaaug gaaaugagcc ucucauucuu guugcagccg 1140
cggaucucgc cagcaaccag aaccagcuug accgcaaccu uagaccauuu uugauagccg 1200
gaaacaaccc acaagggcag gaauggcuac aaggccgaaa gcaacagaag caaaacaaca 1260
ucuucaaugg cuucgcaccu gagaucuugg cucaagccuu caagaucaau gucgagacgg 1320
cucagcagcu ccagaaccag caagauaacc guggcaacau cgucaagguc aacggaccuu 1380
ucggcgucau uaggccaccc uugagacgcg gcgaaggcgg ccaacaacca caugaaauag 1440
cuaaugguuu agaggagacu uugugcacca ugcgaugcac ugaaaaccuc gaugacccgu 1500
cggaugcuga cguguacaag ccaucacucg gauacauuag cacacuuaac agcuacaauc 1560
uuccuauccu cagacuucuc cgccuuagcg cucuucgugg cuccauccgu aaaagauccu 1620
auggcuaaca agcucuuccu cgucugcgca acucucgccc ucugcuuccu ccucaccaac 1680
gcuuccaucu accgcaccgu ugucgaauuc gaagaagaug acgccagcaa ccccguaggu 1740
ccaagacaga gaugccagaa ggaguuucag caaucacaac accuaagagc uugccagaga 1800
uggaugagca agcaaaugag gcaaggacgu gguggugguc cuucccucga cgaugaguuc 1860
gauuucgagg gcccccagca gggauaccag cuacuccagc agugcugcaa cgagcuucgc 1920
caggaagagc caguuugcgu uugccccacc uugaaacaag cugccagggc aguuagccuc 1980
cagggacagc acggaccauu ccaauccagg aaaauuuacc agucagcuaa guacuugccu 2040
aacauuugca agauccagca aguuggugaa ugucccuucc agaccaccau cccuuucuuc 2100
ccuccuuacu acuaggguag auauccauca cacuggcggc cgcucgacgu aagcucaaca 2160
aaucuuuaga aaauuaauuu uaugugacau augcaauaau uugauuuggc aagauaaacu 2220
aauagauuuu gcgauuugga guuuuaaacu cuaaauaauc uaaaucguuu ucaauugguu 2280
uaaauauaua ucuugcauuu uuaaucguuu uuaauuaaaa aauauauaua uauauauaua 2340
ucuugcauuu uuaaucguuu ucaauuuaaa aaauaucuug cacgcagaac gccucgacua 2400
cccuaguagu aaggagggaa gaaagggaug guggucugga agggacauuc accaacuugc 2460
uggaucuugc aaauguuagg caaguacuua gcugacuggu aaauuuuccu ggauuggaau 2520
gguccgugcu gucccuggag gcuaacugcc cuggcagcuu guuucaaggu ggggcaaacg 2580
caaacuggcu cuuccuggcg aagcucguug cagcacugcu ggaguagcug guaucccugc 2640
uggggggcccu cgaaaucgaa cucaucgucg agggaaggac caccaccacg uccuugccuc 2700
auuugcuugc ucauccaucu cuggcaagcu cuuaggugu u gugauugcug aaacuccuuc 2760
uggcaucucu gucuuggacc uacgggguug cuggcgucau cuucuucgaa uucgacaacg 2820
gugcgguaga uggaagcguu ggugaggagg aagcagaggg cgagaguugc gcagacgagg 2880
aagagcuugu uagccauagg aucuuuuacg gauggagcca cgaagagcgc uaaggcggag 2940
```

aagucugagg auaggaagau uguagcuguu aagugugcua auguauccga gugauggcuu 3000
guacacguca gcauccgacg ggucaucgag guuuucagug caucgcaugg ugcacaaagu 3060
cuccucuaaa ccauuagcua uuucaugugg uuguuggccg ccuucgccgc gucucaaggg 3120
uggccuaaug acgccgaaag guccguugac cuugacgaug uugccacggu uaucuugcug 3180
guucuggagc ugcugagccg ucucgacauu gaucuugaag gcuugagcca agaucucagg 3240
ugcgaagcca uugaagaugu uguuuugcuu cuguugcuuu cggccuugua gccauuccug 3300
cccuuguggg uuguuuccgg cuaucaaaaa uggucuaagg uugcggucaa gcugguucug 3360
guugcuggcg agauccgcgg cugcaacaag aaugagaggc ucauuuccau uguuguagaa 3420
ccauugagcu acaccagaug guguugcaau ggugucaccg caccguaggu gcucuacuuu 3480
cuggugcaug ucacggaacc cuugacucug acccugaccu ugaccuucuc caaauaccgg 3540
ugacuccaug aacgucucgg cgcaucccgg aauaacucuu cccauuagac cccuuccgug 3600
aacaacaaac gugaguuugc cggcguucaa gaaagugggc aagaaaagac ccugaaggau 3660
cugcagaauu cgcccuugcg gccgcggauc cuuuuacgga uggagccacg aagagcgcua 3720
aggcggagaa gucugaggau aggaagauug uagcuguuaa gugugcuaau guauccgagu 3780
gauggcuugu acacgucagc auccgacggg ucaucgaggu uuucagugca ucgcauggug 3840
cacaaagucu ccucuaaacc auuagcuauu ucaugugguu guuggccgcc uucgccgcgu 3900
cucaagggug gccuaaugac gccgaaaggu ccguugaccu ugacgauguu gccacgguua 3960
ucuugcuggu ucuggagcug cugagccguc ucgacauuga ucuugaaggc uugagccaag 4020
aucucaggug cgaagccauu gaagauguug uuuugcuucu guugcuuucg gccuuguagc 4080
cauuccugcc cuugugggu u guuuccggcu aucaaaaaug gucuaagguu gcggucaagc 4140
ugguucuggu ugcuggcgag auccgcggcu gcaacaagaa ugagaggcuc auuuccauug 4200
uuguagaacc auugagcuac accagauggu guugcaaugg ugucaccgca ccguaggugc 4260
ucuacuuucu ggugcauguc acggaacccu ugacucugac ccugaccuug accuucucca 4320
aauaccggug acuccaugaa cgucucggcg caucccggaa uaacucuucc cauuagaccc 4380
cuuccgugaa caacaaacgu gaguuugccg gcguucaaga aagugggcaa gaaaagaccc 4440
ugacucgagc augcau 4456

<210> 87
<211> 33
<212> DNA
<213> Künstliche Sequenz

<220>
<223> Beschreibung der künstlichen Sequenz: oligonucleotide primer

<400> 87
aaaaggcctg tgttccattt ggccggaaac aac 33

<210> 88
<211> 31
<212> DNA
<213> Künstliche Sequenz

<220>
<223> Beschreibung der künstlichen Sequenz: oligonucleotide primer

<400> 88
aaagatatca ccctggagaa cgccacgagt g 31

<210> 89
<211> 33
<212> DNA
<213> Künstliche Sequenz

<220>
<223> Beschreibung der künstlichen Sequenz: oligonucleotide primer

<400> 89
aaaaggccta tggctaacaa gctcttcctc gtc 33

<210> 90
<211> 32
<212> DNA
<213> Künstliche Sequenz

<220>
<223> Beschreibung der künstlichen Sequenz: oligonucleotide primer

<400> 90
aaagatatcc tagtagtaag gagggaagaa ag 32

<210> 91
<211> 32
<212> DNA
<213> Künstliche Sequenz

<220>
<223> Beschreibung der künstlichen Sequenz: oligonucleotide primer

<400> 91
ccgctcgagc tcagggtctt ttcttgccca ct 32

<210> 92
<211> 32
<212> DNA
<213> Künstliche Sequenz

<220>
<223> Beschreibung der künstlichen Sequenz: oligonucleotide primer

<400> 92
ccggtcgacc tagtagtaag gagggaagaa ag 32

<210> 93
<211> 1500
<212> DNA
<213> Arabidopsis thaliana

<220>
<221> CDS
<222> (1)..(1497)
<223> globuline-like protein

<400> 93

```
atg act aga ttt gcg gta ttg cca ctc tct gtt ctt ctt ctc gtt ctc      48
Met Thr Arg Phe Ala Val Leu Pro Leu Ser Val Leu Leu Leu Val Leu
 1               5                  10                  15

ttg ttc ctc tgc act gag tcg ttg gct aag tcg gag gag tct gaa gaa      96
Leu Phe Leu Cys Thr Glu Ser Leu Ala Lys Ser Glu Glu Ser Glu Glu
            20                  25                  30

tac gac gtc gct gtg cct tca tgt tgc ggg ttt tcg tct cct ctt ctg     144
Tyr Asp Val Ala Val Pro Ser Cys Cys Gly Phe Ser Ser Pro Leu Leu
        35                  40                  45

att aag aaa gat caa tgg aaa cca atc ttc gag acg aag ttt gga cag     192
Ile Lys Lys Asp Gln Trp Lys Pro Ile Phe Glu Thr Lys Phe Gly Gln
    50                  55                  60

atc tca acc gtt caa atc ggc aat gga tgc ggt gga atg gga cct tac     240
Ile Ser Thr Val Gln Ile Gly Asn Gly Cys Gly Gly Met Gly Pro Tyr
65                  70                  75                  80
```

```
aag ata cat tcc ata acg ttg gag cca aac aca att ttg ctc cct ctt      288
Lys Ile His Ser Ile Thr Leu Glu Pro Asn Thr Ile Leu Leu Pro Leu
                85                  90                  95

ctt ctt cat tcc gac atg gtc ttc ttc gtt gac tct gga agt ggg att      336
Leu Leu His Ser Asp Met Val Phe Phe Val Asp Ser Gly Ser Gly Ile
            100                 105                 110

ctg aat tgg gtt gac gag gaa gcg aag agt acc gag atc aga cta gga      384
Leu Asn Trp Val Asp Glu Glu Ala Lys Ser Thr Glu Ile Arg Leu Gly
        115                 120                 125

gac gtt tac agg cta cgt ccc ggt tcg gtt ttc tac tta cag agc aaa      432
Asp Val Tyr Arg Leu Arg Pro Gly Ser Val Phe Tyr Leu Gln Ser Lys
    130                 135                 140

ccg gat cct tgc ttt ggt gcc tat tcg agt atc aca gat cta atg ttt      480
Pro Asp Pro Cys Phe Gly Ala Tyr Ser Ser Ile Thr Asp Leu Met Phe
145                 150                 155                 160

ggt ttt gat gag aca att ctc cag tca gct ttt ggg gtt cct gag ggg      528
Gly Phe Asp Glu Thr Ile Leu Gln Ser Ala Phe Gly Val Pro Glu Gly
                165                 170                 175

att ata gag ctg atg agg aac cgt acg aag cca cca ttg atc gtg agt      576
Ile Ile Glu Leu Met Arg Asn Arg Thr Lys Pro Pro Leu Ile Val Ser
            180                 185                 190

gag acg ctt tgc aca cca ggt gtg gcc aac act tgg cag ctc caa ccg      624
Glu Thr Leu Cys Thr Pro Gly Val Ala Asn Thr Trp Gln Leu Gln Pro
        195                 200                 205

cga tta cta aag ctc ttt gct gga agt gca gac ttg gtg gat aac aag      672
Arg Leu Leu Lys Leu Phe Ala Gly Ser Ala Asp Leu Val Asp Asn Lys
    210                 215                 220

aag aag aaa gag aag aaa gag aag aag gag aag gtg aag aaa gcg aag      720
Lys Lys Lys Glu Lys Lys Glu Lys Lys Glu Lys Val Lys Lys Ala Lys
225                 230                 235                 240

aca ttc aat gtt ttc gaa tct gaa ccg gat ttc gag agc ccc tac ggt      768
Thr Phe Asn Val Phe Glu Ser Glu Pro Asp Phe Glu Ser Pro Tyr Gly
                245                 250                 255

cgt act ata acg att aac agg aag gat ctg aaa gtt tta aaa ggc tca      816
Arg Thr Ile Thr Ile Asn Arg Lys Asp Leu Lys Val Leu Lys Gly Ser
            260                 265                 270

atg gtt gga gtt tcc atg gtg aat ctc act caa gga tcg atg atg gga      864
Met Val Gly Val Ser Met Val Asn Leu Thr Gln Gly Ser Met Met Gly
        275                 280                 285

ccg cac tgg aat cca tgg gct tgc gag att tcg att gta ttg aaa gga      912
Pro His Trp Asn Pro Trp Ala Cys Glu Ile Ser Ile Val Leu Lys Gly
    290                 295                 300

gca gga atg gtt agg gtt ctt agg tct tcg ata tca tca aac aca tca      960
Ala Gly Met Val Arg Val Leu Arg Ser Ser Ile Ser Ser Asn Thr Ser
305                 310                 315                 320

tca gag tgt aag aac gtg agg ttt aaa gta gag gaa gga gat att ttc     1008
Ser Glu Cys Lys Asn Val Arg Phe Lys Val Glu Glu Gly Asp Ile Phe
                325                 330                 335

gca gtt cca cgg tta cat cca atg gct caa atg tct ttc aac aat gac     1056
Ala Val Pro Arg Leu His Pro Met Ala Gln Met Ser Phe Asn Asn Asp
            340                 345                 350
```

```
tcg tta gtg ttc gtt ggg ttt act act tca gct aag aat aac gag ccg    1104
Ser Leu Val Phe Val Gly Phe Thr Thr Ser Ala Lys Asn Asn Glu Pro
        355                 360                 365

cag ttc tta gcc ggg gag gac tcg gct ttg cgg atg ctt gac cgg caa    1152
Gln Phe Leu Ala Gly Glu Asp Ser Ala Leu Arg Met Leu Asp Arg Gln
    370                 375                 380

gta ttg gct gca tcg ctt aat gtg agt agt gtg acg att gat gga ttg    1200
Val Leu Ala Ala Ser Leu Asn Val Ser Ser Val Thr Ile Asp Gly Leu
385                 390                 395                 400

ttg gga gct cag aag gaa gct gtt atc ttg gaa tgt cat tct tgt gcg    1248
Leu Gly Ala Gln Lys Glu Ala Val Ile Leu Glu Cys His Ser Cys Ala
                405                 410                 415

gaa gga gag ata gag aag ctt aag gtg gag ata gag agg aag aaa ata    1296
Glu Gly Glu Ile Glu Lys Leu Lys Val Glu Ile Glu Arg Lys Lys Ile
            420                 425                 430

gat gat gag agg aag agg aga cat gat gaa agg aag aaa gaa gaa gaa    1344
Asp Asp Glu Arg Lys Arg Arg His Asp Glu Arg Lys Lys Glu Glu Glu
        435                 440                 445

gag gcg aag aga gaa gag gaa gag agg agg aaa cgt gaa gaa gaa gaa    1392
Glu Ala Lys Arg Glu Glu Glu Glu Arg Arg Lys Arg Glu Glu Glu Glu
    450                 455                 460

gag aag aag cgg tgg cca cct caa caa cca cca caa gaa gaa gaa ctt    1440
Glu Lys Lys Arg Trp Pro Pro Gln Gln Pro Pro Gln Glu Glu Glu Leu
465                 470                 475                 480

agg gaa cgg caa cta ccg atg gag aaa gaa tgg gaa atg gaa ggt gaa    1488
Arg Glu Arg Gln Leu Pro Met Glu Lys Glu Trp Glu Met Glu Gly Glu
                485                 490                 495

gag gag agt taa                                                    1500
Glu Glu Ser
```

<210> 94
<211> 499
<212> PRT
<213> Arabidopsis thaliana

<400> 94

```
Met Thr Arg Phe Ala Val Leu Pro Leu Ser Val Leu Leu Leu Val Leu
1               5                   10                  15

Leu Phe Leu Cys Thr Glu Ser Leu Ala Lys Ser Glu Glu Ser Glu Glu
            20                  25                  30

Tyr Asp Val Ala Val Pro Ser Cys Cys Gly Phe Ser Ser Pro Leu Leu
        35                  40                  45

Ile Lys Lys Asp Gln Trp Lys Pro Ile Phe Glu Thr Lys Phe Gly Gln
    50                  55                  60

Ile Ser Thr Val Gln Ile Gly Asn Gly Cys Gly Gly Met Gly Pro Tyr
65                  70                  75                  80

Lys Ile His Ser Ile Thr Leu Glu Pro Asn Thr Ile Leu Leu Pro Leu
                85                  90                  95

Leu Leu His Ser Asp Met Val Phe Phe Val Asp Ser Gly Ser Gly Ile
            100                 105                 110

Leu Asn Trp Val Asp Glu Glu Ala Lys Ser Thr Glu Ile Arg Leu Gly
        115                 120                 125
```

Asp Val Tyr Arg Leu Arg Pro Gly Ser Val Phe Tyr Leu Gln Ser Lys
130 135 140

Pro Asp Pro Cys Phe Gly Ala Tyr Ser Ser Ile Thr Asp Leu Met Phe
145 150 155 160

Gly Phe Asp Glu Thr Ile Leu Gln Ser Ala Phe Gly Val Pro Glu Gly
165 170 175

Ile Ile Glu Leu Met Arg Asn Arg Thr Lys Pro Pro Leu Ile Val Ser
180 185 190

Glu Thr Leu Cys Thr Pro Gly Val Ala Asn Thr Trp Gln Leu Gln Pro
195 200 205

Arg Leu Leu Lys Leu Phe Ala Gly Ser Ala Asp Leu Val Asp Asn Lys
210 215 220

Lys Lys Lys Glu Lys Lys Glu Lys Lys Glu Lys Val Lys Lys Ala Lys
225 230 235 240

Thr Phe Asn Val Phe Glu Ser Glu Pro Asp Phe Glu Ser Pro Tyr Gly
245 250 255

Arg Thr Ile Thr Ile Asn Arg Lys Asp Leu Lys Val Leu Lys Gly Ser
260 265 270

Met Val Gly Val Ser Met Val Asn Leu Thr Gln Gly Ser Met Met Gly
275 280 285

Pro His Trp Asn Pro Trp Ala Cys Glu Ile Ser Ile Val Leu Lys Gly
290 295 300

Ala Gly Met Val Arg Val Leu Arg Ser Ser Ile Ser Ser Asn Thr Ser
305 310 315 320

Ser Glu Cys Lys Asn Val Arg Phe Lys Val Glu Glu Gly Asp Ile Phe
325 330 335

Ala Val Pro Arg Leu His Pro Met Ala Gln Met Ser Phe Asn Asn Asp
340 345 350

Ser Leu Val Phe Val Gly Phe Thr Thr Ser Ala Lys Asn Asn Glu Pro
355 360 365

Gln Phe Leu Ala Gly Glu Asp Ser Ala Leu Arg Met Leu Asp Arg Gln
370 375 380

Val Leu Ala Ala Ser Leu Asn Val Ser Ser Val Thr Ile Asp Gly Leu
385 390 395 400

Leu Gly Ala Gln Lys Glu Ala Val Ile Leu Glu Cys His Ser Cys Ala
405 410 415

Glu Gly Glu Ile Glu Lys Leu Lys Val Glu Ile Glu Arg Lys Lys Ile
420 425 430

Asp Asp Glu Arg Lys Arg Arg His Asp Glu Arg Lys Lys Glu Glu Glu
435 440 445

Glu Ala Lys Arg Glu Glu Glu Glu Arg Arg Lys Arg Glu Glu Glu Glu
450 455 460

Glu Lys Lys Arg Trp Pro Pro Gln Gln Pro Pro Gln Glu Glu Glu Leu
465 470 475 480

Arg Glu Arg Gln Leu Pro Met Glu Lys Glu Trp Glu Met Glu Gly Glu
485 490 495

Glu Glu Ser

<210> 95
<211> 1284
<212> DNA
<213> Glycine max

<220>
<221> CDS

<222> (1)..(1281)
<223> 7S seed globuline

<400> 95

```
atg gct tcc atc ctc cac tac ttt tta gcc ctc tct ctt tct tgc tct      48
Met Ala Ser Ile Leu His Tyr Phe Leu Ala Leu Ser Leu Ser Cys Ser
1               5                   10                  15

ttt ctt ttc ttc tta tcc gac tca gtc acc cct aca aaa cca ata aac      96
Phe Leu Phe Phe Leu Ser Asp Ser Val Thr Pro Thr Lys Pro Ile Asn
            20                  25                  30

ctt gtt gtt cta ccc gtt caa aat gat ggt tcc aca ggg ctc cat tcg      144
Leu Val Val Leu Pro Val Gln Asn Asp Gly Ser Thr Gly Leu His Ser
        35                  40                  45

gcc aac ctc caa aaa aga acc cct cta atg caa gta cca gtc ctg gtg      192
Ala Asn Leu Gln Lys Arg Thr Pro Leu Met Gln Val Pro Val Leu Val
    50                  55                  60

gac ctc aat gga aat cac ttg tgg gtt aac tgt gag cag cag tac tca      240
Asp Leu Asn Gly Asn His Leu Trp Val Asn Cys Glu Gln Gln Tyr Ser
65                  70                  75                  80

tcc aaa acg tac caa gca ccc ttc tgc cac tcc acc caa tgc tct aga      288
Ser Lys Thr Tyr Gln Ala Pro Phe Cys His Ser Thr Gln Cys Ser Arg
                85                  90                  95

gcc aac acc cac caa tgc ctc agt tgc ccc gcg gca tca agg cca ggg      336
Ala Asn Thr His Gln Cys Leu Ser Cys Pro Ala Ala Ser Arg Pro Gly
            100                 105                 110

tgc cac aaa aac acg tgt ggc ctc atg tcc act aat ccc atc acc caa      384
Cys His Lys Asn Thr Cys Gly Leu Met Ser Thr Asn Pro Ile Thr Gln
        115                 120                 125

caa acc ggt tta ggt gaa cta gga gaa gac gtt ctt gca atc cac gcc      432
Gln Thr Gly Leu Gly Glu Leu Gly Glu Asp Val Leu Ala Ile His Ala
    130                 135                 140

aca caa ggg tcg acc caa caa ctt ggc cca ttg gtc aca gtc cca caa      480
Thr Gln Gly Ser Thr Gln Gln Leu Gly Pro Leu Val Thr Val Pro Gln
145                 150                 155                 160

ttc ctc ttt tct tgt gca cct tcc ttc ctt gtt caa aag ggt ctt cct      528
Phe Leu Phe Ser Cys Ala Pro Ser Phe Leu Val Gln Lys Gly Leu Pro
                165                 170                 175

aga aac act caa ggt gtg gct ggg tta ggc cat gca cca att tct ctt      576
Arg Asn Thr Gln Gly Val Ala Gly Leu Gly His Ala Pro Ile Ser Leu
            180                 185                 190

cca aac caa ctc gct tcc cac ttt ggc cta caa cgc caa ttc acc act      624
Pro Asn Gln Leu Ala Ser His Phe Gly Leu Gln Arg Gln Phe Thr Thr
        195                 200                 205

tgc ctt tct cgc tac cct act tca aag ggt gct ata ata ttc ggg gat      672
Cys Leu Ser Arg Tyr Pro Thr Ser Lys Gly Ala Ile Ile Phe Gly Asp
    210                 215                 220
```

```
gca cct aac aac atg cga cag ttt caa aac caa gat att ttc cac gat     720
Ala Pro Asn Asn Met Arg Gln Phe Gln Asn Gln Asp Ile Phe His Asp
225                 230                 235                 240

ttg gcc ttc acc cca tta acc atc acc ctg cag gga gag tac aac gtg     768
Leu Ala Phe Thr Pro Leu Thr Ile Thr Leu Gln Gly Glu Tyr Asn Val
                245                 250                 255

aga gtc aac tca ata aga atc aac cag cac agt gtg ttc cca ctg aac     816
Arg Val Asn Ser Ile Arg Ile Asn Gln His Ser Val Phe Pro Leu Asn
            260                 265                 270

aag ata tca tcc acc atc gta ggg tcg acc tct gga gga acc atg att     864
Lys Ile Ser Ser Thr Ile Val Gly Ser Thr Ser Gly Gly Thr Met Ile
        275                 280                 285

agc acc tca act cct cac atg gtt ctc cag caa tcc gtg tac cag gct     912
Ser Thr Ser Thr Pro His Met Val Leu Gln Gln Ser Val Tyr Gln Ala
    290                 295                 300

ttc act cag gtg ttt gct cag cag cta cca aag caa gca cag gtg aaa     960
Phe Thr Gln Val Phe Ala Gln Gln Leu Pro Lys Gln Ala Gln Val Lys
305                 310                 315                 320

tct gtg gca cca ttt ggg tta tgc ttc aac tcc aac aag atc aat gca     1008
Ser Val Ala Pro Phe Gly Leu Cys Phe Asn Ser Asn Lys Ile Asn Ala
                325                 330                 335

tat cct agc gtg gac ctt gtg atg gac aag ccc aat ggt cct gtt tgg     1056
Tyr Pro Ser Val Asp Leu Val Met Asp Lys Pro Asn Gly Pro Val Trp
            340                 345                 350

aga atc tct ggt gag gac ttg atg gtg cag gca caa cct ggg gtc acg     1104
Arg Ile Ser Gly Glu Asp Leu Met Val Gln Ala Gln Pro Gly Val Thr
        355                 360                 365

tgt ttg ggt gtt atg aat gga gga atg caa cct aga gct gaa att acc     1152
Cys Leu Gly Val Met Asn Gly Gly Met Gln Pro Arg Ala Glu Ile Thr
    370                 375                 380

tta ggg gca cgt cag ttg gaa gag aac ctg gtg gtg ttc gat ctt gca     1200
Leu Gly Ala Arg Gln Leu Glu Glu Asn Leu Val Val Phe Asp Leu Ala
385                 390                 395                 400

agg tca agg gtt ggg ttt agc acc tca tca ctg cac tcg cat gga gtc     1248
Arg Ser Arg Val Gly Phe Ser Thr Ser Ser Leu His Ser His Gly Val
                405                 410                 415

aaa tgt gct gac ctc ttc aac ttt gcc aat gca tga                     1284
Lys Cys Ala Asp Leu Phe Asn Phe Ala Asn Ala
            420                 425
```

<210> 96
<211> 427
<212> PRT
<213> Glycine max

<400> 96

```
Met Ala Ser Ile Leu His Tyr Phe Leu Ala Leu Ser Leu Ser Cys Ser
1               5                   10                  15

Phe Leu Phe Phe Leu Ser Asp Ser Val Thr Pro Thr Lys Pro Ile Asn
            20                  25                  30

Leu Val Val Leu Pro Val Gln Asn Asp Gly Ser Thr Gly Leu His Ser
        35                  40                  45
```

```
Ala Asn Leu Gln Lys Arg Thr Pro Leu Met Gln Val Pro Val Leu Val
    50                  55                  60

Asp Leu Asn Gly Asn His Leu Trp Val Asn Cys Glu Gln Gln Tyr Ser
65                  70                  75                  80

Ser Lys Thr Tyr Gln Ala Pro Phe Cys His Ser Thr Gln Cys Ser Arg
                85                  90                  95

Ala Asn Thr His Gln Cys Leu Ser Cys Pro Ala Ala Ser Arg Pro Gly
            100                 105                 110

Cys His Lys Asn Thr Cys Gly Leu Met Ser Thr Asn Pro Ile Thr Gln
        115                 120                 125

Gln Thr Gly Leu Gly Glu Leu Gly Glu Asp Val Leu Ala Ile His Ala
    130                 135                 140

Thr Gln Gly Ser Thr Gln Gln Leu Gly Pro Leu Val Thr Val Pro Gln
145                 150                 155                 160

Phe Leu Phe Ser Cys Ala Pro Ser Phe Leu Val Gln Lys Gly Leu Pro
                165                 170                 175

Arg Asn Thr Gln Gly Val Ala Gly Leu Gly His Ala Pro Ile Ser Leu
            180                 185                 190

Pro Asn Gln Leu Ala Ser His Phe Gly Leu Gln Arg Gln Phe Thr Thr
        195                 200                 205

Cys Leu Ser Arg Tyr Pro Thr Ser Lys Gly Ala Ile Ile Phe Gly Asp
    210                 215                 220

Ala Pro Asn Asn Met Arg Gln Phe Gln Asn Gln Asp Ile Phe His Asp
225                 230                 235                 240

Leu Ala Phe Thr Pro Leu Thr Ile Thr Leu Gln Gly Glu Tyr Asn Val
                245                 250                 255

Arg Val Asn Ser Ile Arg Ile Asn Gln His Ser Val Phe Pro Leu Asn
            260                 265                 270

Lys Ile Ser Ser Thr Ile Val Gly Ser Thr Ser Gly Gly Thr Met Ile
        275                 280                 285

Ser Thr Ser Thr Pro His Met Val Leu Gln Gln Ser Val Tyr Gln Ala
    290                 295                 300

Phe Thr Gln Val Phe Ala Gln Gln Leu Pro Lys Gln Ala Gln Val Lys
305                 310                 315                 320

Ser Val Ala Pro Phe Gly Leu Cys Phe Asn Ser Asn Lys Ile Asn Ala
                325                 330                 335

Tyr Pro Ser Val Asp Leu Val Met Asp Lys Pro Asn Gly Pro Val Trp
            340                 345                 350

Arg Ile Ser Gly Glu Asp Leu Met Val Gln Ala Gln Pro Gly Val Thr
        355                 360                 365

Cys Leu Gly Val Met Asn Gly Gly Met Gln Pro Arg Ala Glu Ile Thr
    370                 375                 380

Leu Gly Ala Arg Gln Leu Glu Glu Asn Leu Val Val Phe Asp Leu Ala
385                 390                 395                 400

Arg Ser Arg Val Gly Phe Ser Thr Ser Ser Leu His Ser His Gly Val
                405                 410                 415

Lys Cys Ala Asp Leu Phe Asn Phe Ala Asn Ala
            420                 425
```

<210> 97
<211> 814
<212> DNA
<213> Zea mays

<220>

<221> CDS
<222> (1)..(720)
<223> 19kd zein

<400> 97

```
atg gca gcc aag att ttt gcc ctc ctt gcc ctc ctt gct ctt tca gca    48
Met Ala Ala Lys Ile Phe Ala Leu Leu Ala Leu Leu Ala Leu Ser Ala
1               5                   10                  15

aac gtt gct acc gcg act att att cca caa tgc tca caa caa tac ctc    96
Asn Val Ala Thr Ala Thr Ile Ile Pro Gln Cys Ser Gln Gln Tyr Leu
            20                  25                  30

tct ccg gtg aca gcc gcg aga ttt gaa tac cca act ata caa tcc tac    144
Ser Pro Val Thr Ala Ala Arg Phe Glu Tyr Pro Thr Ile Gln Ser Tyr
        35                  40                  45

agg cta caa cag gcc atc gca gca agc atc tta cgg tcg tta gca ttg    192
Arg Leu Gln Gln Ala Ile Ala Ala Ser Ile Leu Arg Ser Leu Ala Leu
    50                  55                  60

act gtc caa caa cca tat gcc cta ttg caa caa cca tcc tta gtg aat    240
Thr Val Gln Gln Pro Tyr Ala Leu Leu Gln Gln Pro Ser Leu Val Asn
65                  70                  75                  80

cta tat ctc caa aga atc gta gca caa caa cta caa caa caa ttg ctt    288
Leu Tyr Leu Gln Arg Ile Val Ala Gln Gln Leu Gln Gln Gln Leu Leu
                85                  90                  95

cca aca atc aat gaa gta gtt gca gcg aac ctt gat gct tac ctc cag    336
Pro Thr Ile Asn Glu Val Val Ala Ala Asn Leu Asp Ala Tyr Leu Gln
            100                 105                 110

caa caa caa ttt ctt cca ttc aat caa cta gct ggg gtg aac cct gct    384
Gln Gln Gln Phe Leu Pro Phe Asn Gln Leu Ala Gly Val Asn Pro Ala
        115                 120                 125

gct tac ttg cag gca caa cag cta cta cca ttc aac caa ctt gtc agg    432
Ala Tyr Leu Gln Ala Gln Gln Leu Leu Pro Phe Asn Gln Leu Val Arg
    130                 135                 140

agc cct gct gcc ttc tta ctg cag caa cag ttg ttg cca ttc cat cta    480
Ser Pro Ala Ala Phe Leu Leu Gln Gln Gln Leu Leu Pro Phe His Leu
145                 150                 155                 160

caa gtt gtg gca aac att gct gct ttc ttg caa caa caa caa ttg ctg    528
Gln Val Val Ala Asn Ile Ala Ala Phe Leu Gln Gln Gln Gln Leu Leu
                165                 170                 175

cca ttt tac cca cag gtt gtg gga aac att aac gcc ttc ttg caa cag    576
Pro Phe Tyr Pro Gln Val Val Gly Asn Ile Asn Ala Phe Leu Gln Gln
            180                 185                 190

caa cag ttg ctg cca ttc tac cca cag gat gtg gca aac aat gtc gcc    624
Gln Gln Leu Leu Pro Phe Tyr Pro Gln Asp Val Ala Asn Asn Val Ala
        195                 200                 205

ttc tta caa caa caa caa ttg ctg cca ttt agc caa ctt gct ttg acg    672
Phe Leu Gln Gln Gln Gln Leu Leu Pro Phe Ser Gln Leu Ala Leu Thr
    210                 215                 220

aat cct acc acc tta ttg cag cag ccc acc att ggt ggt gcc atc ttc    720
Asn Pro Thr Thr Leu Leu Gln Gln Pro Thr Ile Gly Gly Ala Ile Phe
225                 230                 235                 240

tagatttttt atgctttata ctgtaataat aaagttctca tactgatatg tgcaacttct  780

cagtaataaa agattagaga tctatatttt atta                               814
```

<210> 98
<211> 240
<212> PRT
<213> Zea mays

<400> 98

```
Met Ala Ala Lys Ile Phe Ala Leu Leu Ala Leu Leu Ala Leu Ser Ala
1               5                   10                  15
Asn Val Ala Thr Ala Thr Ile Ile Pro Gln Cys Ser Gln Gln Tyr Leu
            20                  25                  30
Ser Pro Val Thr Ala Ala Arg Phe Glu Tyr Pro Thr Ile Gln Ser Tyr
        35                  40                  45
Arg Leu Gln Gln Ala Ile Ala Ala Ser Ile Leu Arg Ser Leu Ala Leu
    50                  55                  60
Thr Val Gln Gln Pro Tyr Ala Leu Leu Gln Gln Pro Ser Leu Val Asn
65                  70                  75                  80
Leu Tyr Leu Gln Arg Ile Val Ala Gln Gln Leu Gln Gln Gln Leu Leu
                85                  90                  95
Pro Thr Ile Asn Glu Val Val Ala Ala Asn Leu Asp Ala Tyr Leu Gln
            100                 105                 110
Gln Gln Gln Phe Leu Pro Phe Asn Gln Leu Ala Gly Val Asn Pro Ala
        115                 120                 125
Ala Tyr Leu Gln Ala Gln Gln Leu Leu Pro Phe Asn Gln Leu Val Arg
    130                 135                 140
Ser Pro Ala Ala Phe Leu Leu Gln Gln Gln Leu Leu Pro Phe His Leu
145                 150                 155                 160
Gln Val Val Ala Asn Ile Ala Ala Phe Leu Gln Gln Gln Gln Leu Leu
                165                 170                 175
Pro Phe Tyr Pro Gln Val Val Gly Asn Ile Asn Ala Phe Leu Gln Gln
            180                 185                 190
Gln Gln Leu Leu Pro Phe Tyr Pro Gln Asp Val Ala Asn Asn Val Ala
        195                 200                 205
Phe Leu Gln Gln Gln Gln Leu Leu Pro Phe Ser Gln Leu Ala Leu Thr
    210                 215                 220
Asn Pro Thr Thr Leu Leu Gln Gln Pro Thr Ile Gly Gly Ala Ile Phe
225                 230                 235                 240
```

<210> 99
<211> 705
<212> DNA
<213> Zea mays

<220>
<221> CDS
<222> (1)..(702)
<223> 19 kd zein B1

<400> 99

```
atg gca gcc aaa ata ttt tgc ctc ctt atg ctc ctt ggt ctt tct gca      48
Met Ala Ala Lys Ile Phe Cys Leu Leu Met Leu Leu Gly Leu Ser Ala
  1               5                  10                  15

agt gct gct acg gcg acc att ttc cca caa tgc tca caa gct cct ata      96
Ser Ala Ala Thr Ala Thr Ile Phe Pro Gln Cys Ser Gln Ala Pro Ile
             20                  25                  30

gct tcc ctt ctt ccc ccg tac ctc tca cca gcg gtg tct tcg gta tgt     144
Ala Ser Leu Leu Pro Pro Tyr Leu Ser Pro Ala Val Ser Ser Val Cys
         35                  40                  45

gaa aac cca att ctt caa ccc tat agg atc caa cag gca atc gca gct     192
Glu Asn Pro Ile Leu Gln Pro Tyr Arg Ile Gln Gln Ala Ile Ala Ala
     50                  55                  60

ggc atc tta cct tta tca ccc ttg ttc ctc caa caa tca tca gcc cta     240
Gly Ile Leu Pro Leu Ser Pro Leu Phe Leu Gln Gln Ser Ser Ala Leu
 65                  70                  75                  80

tta cag cag tta cct ttg gtg cat tta ttg gca caa aac atc agg gca     288
Leu Gln Gln Leu Pro Leu Val His Leu Leu Ala Gln Asn Ile Arg Ala
                 85                  90                  95

caa caa cta caa caa ctt gtg cta gca aac ctt gct gcc tac tct cag     336
Gln Gln Leu Gln Gln Leu Val Leu Ala Asn Leu Ala Ala Tyr Ser Gln
            100                 105                 110

caa caa cag ttt ctt cca ttc aac caa cta gct gca ttg aac tct gct     384
Gln Gln Gln Phe Leu Pro Phe Asn Gln Leu Ala Ala Leu Asn Ser Ala
        115                 120                 125

tct tat ttg caa caa caa caa cta cca ttc agc cag cta tct gct gcc     432
Ser Tyr Leu Gln Gln Gln Gln Leu Pro Phe Ser Gln Leu Ser Ala Ala
    130                 135                 140

tac ccc cag caa ttt ctt cca ttc aac caa ctg aca gct ttg aac tct     480
Tyr Pro Gln Gln Phe Leu Pro Phe Asn Gln Leu Thr Ala Leu Asn Ser
145                 150                 155                 160

cct gct tat tta cag cag caa caa cta cta cca ttc agc cag cta gct     528
Pro Ala Tyr Leu Gln Gln Gln Gln Leu Leu Pro Phe Ser Gln Leu Ala
                165                 170                 175

ggt gtg agc cct gct acc ttc ttg aca caa cca caa ttg ttg ccg ttc     576
Gly Val Ser Pro Ala Thr Phe Leu Thr Gln Pro Gln Leu Leu Pro Phe
            180                 185                 190

tac cag cac gct gcg cct aac gct ggc acc ctc tta caa ctg caa caa     624
Tyr Gln His Ala Ala Pro Asn Ala Gly Thr Leu Leu Gln Leu Gln Gln
        195                 200                 205

ttg ctg cca ttc aac caa ctt gct ttg aca aac cca aca gca ttc tac     672
Leu Leu Pro Phe Asn Gln Leu Ala Leu Thr Asn Pro Thr Ala Phe Tyr
    210                 215                 220

caa caa ccc atc att ggt ggt gcc ctc ttt tag                         705
Gln Gln Pro Ile Ile Gly Gly Ala Leu Phe
225                 230
```

<210> 100
<211> 234
<212> PRT
<213> Zea mays

<400> 100

Met Ala Ala Lys Ile Phe Cys Leu Leu Met Leu Leu Gly Leu Ser Ala
1 5 10 15

Ser Ala Ala Thr Ala Thr Ile Phe Pro Gln Cys Ser Gln Ala Pro Ile
20 25 30

Ala Ser Leu Leu Pro Pro Tyr Leu Ser Pro Ala Val Ser Ser Val Cys
35 40 45

Glu Asn Pro Ile Leu Gln Pro Tyr Arg Ile Gln Gln Ala Ile Ala Ala
50 55 60

Gly Ile Leu Pro Leu Ser Pro Leu Phe Leu Gln Gln Ser Ser Ala Leu
65 70 75 80

Leu Gln Gln Leu Pro Leu Val His Leu Leu Ala Gln Asn Ile Arg Ala
85 90 95

Gln Gln Leu Gln Gln Leu Val Leu Ala Asn Leu Ala Ala Tyr Ser Gln
100 105 110

Gln Gln Gln Phe Leu Pro Phe Asn Gln Leu Ala Ala Leu Asn Ser Ala
115 120 125

Ser Tyr Leu Gln Gln Gln Gln Leu Pro Phe Ser Gln Leu Ser Ala Ala
130 135 140

Tyr Pro Gln Gln Phe Leu Pro Phe Asn Gln Leu Thr Ala Leu Asn Ser
145 150 155 160

Pro Ala Tyr Leu Gln Gln Gln Gln Leu Leu Pro Phe Ser Gln Leu Ala
165 170 175

Gly Val Ser Pro Ala Thr Phe Leu Thr Gln Pro Gln Leu Leu Pro Phe
180 185 190

Tyr Gln His Ala Ala Pro Asn Ala Gly Thr Leu Leu Gln Leu Gln Gln
195 200 205

Leu Leu Pro Phe Asn Gln Leu Ala Leu Thr Asn Pro Thr Ala Phe Tyr
210 215 220

Gln Gln Pro Ile Ile Gly Gly Ala Leu Phe
225 230

<210> 101
<211> 804
<212> DNA
<213> Zea mays

<220>
<221> CDS
<222> (1)..(801)
<223> 19 kd zein B2

<400> 101

```
atg gca gcc aaa ata ttt tgc ctc att atg ctc ctt ggt ctt tct gca    48
Met Ala Ala Lys Ile Phe Cys Leu Ile Met Leu Leu Gly Leu Ser Ala
 1               5                  10                  15

agt gct gct acg gcg agc att ttc ccg caa tgc tca caa gct cct ata    96
Ser Ala Ala Thr Ala Ser Ile Phe Pro Gln Cys Ser Gln Ala Pro Ile
            20                  25                  30

gct tcc ctt ctt ccc cca tac ctc tca cca gcg atg tct tca gta tgt   144
Ala Ser Leu Leu Pro Pro Tyr Leu Ser Pro Ala Met Ser Ser Val Cys
        35                  40                  45

gaa aat cca att ctt cta ccc tac agg atc caa cag gca atc gca gca   192
Glu Asn Pro Ile Leu Leu Pro Tyr Arg Ile Gln Gln Ala Ile Ala Ala
    50                  55                  60

ggc atc tta cct tta tca ccc ttg ttc ctc caa caa tca tca gcc cta   240
Gly Ile Leu Pro Leu Ser Pro Leu Phe Leu Gln Gln Ser Ser Ala Leu
65                  70                  75                  80

tta cag cag tta cct ttg gtg cat tta ttg gca caa aac atc agg gca   288
Leu Gln Gln Leu Pro Leu Val His Leu Leu Ala Gln Asn Ile Arg Ala
                85                  90                  95

caa caa cta caa caa ctc gtg cta gca aac ctt gct gcc tac tct cag   336
Gln Gln Leu Gln Gln Leu Val Leu Ala Asn Leu Ala Ala Tyr Ser Gln
            100                 105                 110

caa cag cag tta cct ttg gtg cat ttg ttg gca caa aac atc agg gca   384
Gln Gln Gln Leu Pro Leu Val His Leu Leu Ala Gln Asn Ile Arg Ala
        115                 120                 125

caa caa cta caa caa ctc gtg cta gca aac ctt gct gcc tac tct cag   432
Gln Gln Leu Gln Gln Leu Val Leu Ala Asn Leu Ala Ala Tyr Ser Gln
    130                 135                 140

caa caa cag ttt ctg cca ttc aac caa cta gct gca ttg aac tct gct   480
Gln Gln Gln Phe Leu Pro Phe Asn Gln Leu Ala Ala Leu Asn Ser Ala
145                 150                 155                 160

gct tat ttg cag caa caa caa cta cta cca ttc agc cag cta gct gct   528
Ala Tyr Leu Gln Gln Gln Gln Leu Leu Pro Phe Ser Gln Leu Ala Ala
                165                 170                 175

gcc tac ccc cgg caa ttt ctt cca ttc aac caa ctg gca gca ttg aac   576
Ala Tyr Pro Arg Gln Phe Leu Pro Phe Asn Gln Leu Ala Ala Leu Asn
            180                 185                 190

tct cat gct tat gta caa caa caa caa cta cta cca ttc agc cag cta   624
Ser His Ala Tyr Val Gln Gln Gln Gln Leu Leu Pro Phe Ser Gln Leu
        195                 200                 205

gct gct gtg agc cct gct gcc ttc ttg aca cag caa cat ttg ttg ccg   672
Ala Ala Val Ser Pro Ala Ala Phe Leu Thr Gln Gln His Leu Leu Pro
    210                 215                 220

ttc tac ctg cac act gcg cct aac gtt ggc acc ctc tta caa ctg caa   720
Phe Tyr Leu His Thr Ala Pro Asn Val Gly Thr Leu Leu Gln Leu Gln
225                 230                 235                 240

caa ttg ctg cca ttc gac caa ctt gct ttg aca aac cca gca gtg ttc   768
Gln Leu Leu Pro Phe Asp Gln Leu Ala Leu Thr Asn Pro Ala Val Phe
                245                 250                 255

tac caa caa ccc atc att ggt ggt gcc ctc ttt tag                   804
Tyr Gln Gln Pro Ile Ile Gly Gly Ala Leu Phe
            260                 265
```

<210> 102
<211> 267
<212> PRT
<213> Zea mays

<400> 102

```
Met Ala Ala Lys Ile Phe Cys Leu Ile Met Leu Leu Gly Leu Ser Ala
1               5                   10                  15

Ser Ala Ala Thr Ala Ser Ile Phe Pro Gln Cys Ser Gln Ala Pro Ile
            20                  25                  30

Ala Ser Leu Leu Pro Pro Tyr Leu Ser Pro Ala Met Ser Ser Val Cys
        35                  40                  45

Glu Asn Pro Ile Leu Leu Pro Tyr Arg Ile Gln Gln Ala Ile Ala Ala
    50                  55                  60

Gly Ile Leu Pro Leu Ser Pro Leu Phe Leu Gln Gln Ser Ser Ala Leu
65                  70                  75                  80

Leu Gln Gln Leu Pro Leu Val His Leu Leu Ala Gln Asn Ile Arg Ala
                85                  90                  95

Gln Gln Leu Gln Gln Leu Val Leu Ala Asn Leu Ala Ala Tyr Ser Gln
            100                 105                 110

Gln Gln Gln Leu Pro Leu Val His Leu Leu Ala Gln Asn Ile Arg Ala
        115                 120                 125

Gln Gln Leu Gln Gln Leu Val Leu Ala Asn Leu Ala Ala Tyr Ser Gln
    130                 135                 140

Gln Gln Gln Phe Leu Pro Phe Asn Gln Leu Ala Ala Leu Asn Ser Ala
145                 150                 155                 160

Ala Tyr Leu Gln Gln Gln Gln Leu Leu Pro Phe Ser Gln Leu Ala Ala
                165                 170                 175

Ala Tyr Pro Arg Gln Phe Leu Pro Phe Asn Gln Leu Ala Ala Leu Asn
            180                 185                 190

Ser His Ala Tyr Val Gln Gln Gln Gln Leu Leu Pro Phe Ser Gln Leu
        195                 200                 205

Ala Ala Val Ser Pro Ala Ala Phe Leu Thr Gln Gln His Leu Leu Pro
    210                 215                 220

Phe Tyr Leu His Thr Ala Pro Asn Val Gly Thr Leu Leu Gln Leu Gln
225                 230                 235                 240

Gln Leu Leu Pro Phe Asp Gln Leu Ala Leu Thr Asn Pro Ala Val Phe
                245                 250                 255

Tyr Gln Gln Pro Ile Ile Gly Gly Ala Leu Phe
            260                 265
```

<210> 103
<211> 801
<212> DNA
<213> Zea mays

<220>
<221> CDS
<222> (1)..(798)
<223> 22kd alpha-zein

<400> 103

```
atg gct acc aag ata tta gcc ctc ctt gcg ctc ctt tcc ctt tca gtg     48
Met Ala Thr Lys Ile Leu Ala Leu Leu Ala Leu Leu Ser Leu Ser Val
1               5                   10                  15

agc gca aca act gca ttc att att cca caa tgc tca ctt gct cct aat     96
Ser Ala Thr Thr Ala Phe Ile Ile Pro Gln Cys Ser Leu Ala Pro Asn
            20                  25                  30

gcc att att cca cag ttc ctc cca tca gtt aca tca atg ggc atc gaa     144
Ala Ile Ile Pro Gln Phe Leu Pro Ser Val Thr Ser Met Gly Ile Glu
        35                  40                  45

cac cct att gtg caa gcc tat agg cta caa caa gcg ctt gcg gcg agc     192
His Pro Ile Val Gln Ala Tyr Arg Leu Gln Gln Ala Leu Ala Ala Ser
    50                  55                  60

gtc tta caa caa ccg ttt gcc caa tta caa caa caa tcc ttg gca cat     240
Val Leu Gln Gln Pro Phe Ala Gln Leu Gln Gln Gln Ser Leu Ala His
65                  70                  75                  80

cta acc ata caa acc atc gca aca caa cta gag caa cag ttt gtg ccc     288
Leu Thr Ile Gln Thr Ile Ala Thr Gln Leu Glu Gln Gln Phe Val Pro
                85                  90                  95

gca ttg agc caa cta gcc gcg gtg aac cct gtc tcc tac ttg caa cag     336
Ala Leu Ser Gln Leu Ala Ala Val Asn Pro Val Ser Tyr Leu Gln Gln
            100                 105                 110

caa atg ctt gca tcc aac cca ctt gct ctg gcg aac aca gcc gca tac     384
Gln Met Leu Ala Ser Asn Pro Leu Ala Leu Ala Asn Thr Ala Ala Tyr
        115                 120                 125

cag caa caa cta cag ttg caa cag ttt cta cca gct ctt agt caa cta     432
Gln Gln Gln Leu Gln Leu Gln Gln Phe Leu Pro Ala Leu Ser Gln Leu
    130                 135                 140

gcc agg gtg aac cct gcc aca tac ctg caa cag caa caa ctg ctt tca     480
Ala Arg Val Asn Pro Ala Thr Tyr Leu Gln Gln Gln Gln Leu Leu Ser
145                 150                 155                 160

tct agc cca ctc gct gtg ggc aat gcg gct aca tac ctg caa cag cag     528
Ser Ser Pro Leu Ala Val Gly Asn Ala Ala Thr Tyr Leu Gln Gln Gln
                165                 170                 175

ctg cta caa cag atc gta ccg gct ctt agt cag cta gtt gtg gcg aac     576
Leu Leu Gln Gln Ile Val Pro Ala Leu Ser Gln Leu Val Val Ala Asn
            180                 185                 190

cct act gcc tac tta caa cag ctt ctt cca ttc aac caa cta gat gtg     624
Pro Thr Ala Tyr Leu Gln Gln Leu Leu Pro Phe Asn Gln Leu Asp Val
        195                 200                 205

gca aac tct gct gcg tac cta caa cag cgg cag caa cta ctt aat cca     672
Ala Asn Ser Ala Ala Tyr Leu Gln Gln Arg Gln Gln Leu Leu Asn Pro
    210                 215                 220

ctt gca gcg gct aac cca ttg gtc gcc gcc ttc ctg caa cag caa caa     720
Leu Ala Ala Ala Asn Pro Leu Val Ala Ala Phe Leu Gln Gln Gln Gln
225                 230                 235                 240

ttt ctg cca tac aac caa atc tct ttg atg aac ctt gcc ttg tca agg     768
Phe Leu Pro Tyr Asn Gln Ile Ser Leu Met Asn Leu Ala Leu Ser Arg
                245                 250                 255

cag caa ccg atc gtt gga ggt gcc atc ttt tag                         801
Gln Gln Pro Ile Val Gly Gly Ala Ile Phe
            260                 265
```

<210> 104
<211> 266
<212> PRT
<213> Zea mays

<400> 104

```
Met Ala Thr Lys Ile Leu Ala Leu Leu Ala Leu Leu Ser Leu Ser Val
1               5                   10                  15

Ser Ala Thr Thr Ala Phe Ile Ile Pro Gln Cys Ser Leu Ala Pro Asn
            20                  25                  30

Ala Ile Ile Pro Gln Phe Leu Pro Ser Val Thr Ser Met Gly Ile Glu
        35                  40                  45

His Pro Ile Val Gln Ala Tyr Arg Leu Gln Gln Ala Leu Ala Ala Ser
    50                  55                  60

Val Leu Gln Gln Pro Phe Ala Gln Leu Gln Gln Gln Ser Leu Ala His
65                  70                  75                  80

Leu Thr Ile Gln Thr Ile Ala Thr Gln Leu Glu Gln Gln Phe Val Pro
                85                  90                  95

Ala Leu Ser Gln Leu Ala Ala Val Asn Pro Val Ser Tyr Leu Gln Gln
            100                 105                 110

Gln Met Leu Ala Ser Asn Pro Leu Ala Leu Ala Asn Thr Ala Ala Tyr
        115                 120                 125

Gln Gln Gln Leu Gln Leu Gln Gln Phe Leu Pro Ala Leu Ser Gln Leu
    130                 135                 140

Ala Arg Val Asn Pro Ala Thr Tyr Leu Gln Gln Gln Gln Leu Leu Ser
145                 150                 155                 160

Ser Ser Pro Leu Ala Val Gly Asn Ala Ala Thr Tyr Leu Gln Gln Gln
                165                 170                 175

Leu Leu Gln Gln Ile Val Pro Ala Leu Ser Gln Leu Val Val Ala Asn
            180                 185                 190

Pro Thr Ala Tyr Leu Gln Gln Leu Leu Pro Phe Asn Gln Leu Asp Val
        195                 200                 205

Ala Asn Ser Ala Ala Tyr Leu Gln Gln Arg Gln Gln Leu Leu Asn Pro
    210                 215                 220

Leu Ala Ala Ala Asn Pro Leu Val Ala Ala Phe Leu Gln Gln Gln Gln
225                 230                 235                 240

Phe Leu Pro Tyr Asn Gln Ile Ser Leu Met Asn Leu Ala Leu Ser Arg
                245                 250                 255

Gln Gln Pro Ile Val Gly Gly Ala Ile Phe
            260                 265
```

<210> 105
<211> 471
<212> DNA
<213> Oryza sativa

<220>
<221> CDS
<222> (1)..(468)
<223> prolamin

<400> 105

```
atg aag atc att ttc gta ttt gct ctc ctt gct att gtt gca tgc aac     48
Met Lys Ile Ile Phe Val Phe Ala Leu Leu Ala Ile Val Ala Cys Asn
 1               5                  10                  15

gct tct gca cgg ttt gat gct ctt agt caa agt tat aga caa tat caa     96
Ala Ser Ala Arg Phe Asp Ala Leu Ser Gln Ser Tyr Arg Gln Tyr Gln
            20                  25                  30

cta caa tcg cat ctc ctg cta cag caa caa gtg ctc agc cca tgc agt    144
Leu Gln Ser His Leu Leu Leu Gln Gln Gln Val Leu Ser Pro Cys Ser
        35                  40                  45

gag ttc gta agg caa cag cat agc ata gtg gca acc ccc ttc tgg caa    192
Glu Phe Val Arg Gln Gln His Ser Ile Val Ala Thr Pro Phe Trp Gln
    50                  55                  60

cca gct acg ttt caa ttg ata aac aac caa gtc atg cag caa cag tgt    240
Pro Ala Thr Phe Gln Leu Ile Asn Asn Gln Val Met Gln Gln Gln Cys
65                  70                  75                  80

tgc caa cag ctc agg ctg gta gcg caa caa tct cac tac cag gcc att    288
Cys Gln Gln Leu Arg Leu Val Ala Gln Gln Ser His Tyr Gln Ala Ile
                85                  90                  95

agt agc gtt cag gcg att gtg cag caa cta cag ctg cag cag gtc ggt    336
Ser Ser Val Gln Ala Ile Val Gln Gln Leu Gln Leu Gln Gln Val Gly
            100                 105                 110

gtt gtc tac ttt gat cag act caa gct caa gct caa gct ttg ctg gcc    384
Val Val Tyr Phe Asp Gln Thr Gln Ala Gln Ala Gln Ala Leu Leu Ala
        115                 120                 125

tta aac ttg cca tcc ata tgt ggt atc tat cct aac tac tac att gct    432
Leu Asn Leu Pro Ser Ile Cys Gly Ile Tyr Pro Asn Tyr Tyr Ile Ala
    130                 135                 140

ccg agg agc att ccc acc gtt ggt ggt gtc tgg tac tga                471
Pro Arg Ser Ile Pro Thr Val Gly Gly Val Trp Tyr
145                 150                 155
```

<210> 106
<211> 156
<212> PRT
<213> Oryza sativa

<400> 106

```
Met Lys Ile Ile Phe Val Phe Ala Leu Leu Ala Ile Val Ala Cys Asn
1               5                   10                  15

Ala Ser Ala Arg Phe Asp Ala Leu Ser Gln Ser Tyr Arg Gln Tyr Gln
            20                  25                  30

Leu Gln Ser His Leu Leu Leu Gln Gln Gln Val Leu Ser Pro Cys Ser
        35                  40                  45

Glu Phe Val Arg Gln Gln His Ser Ile Val Ala Thr Pro Phe Trp Gln
    50                  55                  60

Pro Ala Thr Phe Gln Leu Ile Asn Asn Gln Val Met Gln Gln Gln Cys
65                  70                  75                  80

Cys Gln Gln Leu Arg Leu Val Ala Gln Gln Ser His Tyr Gln Ala Ile
                85                  90                  95

Ser Ser Val Gln Ala Ile Val Gln Gln Leu Gln Leu Gln Gln Val Gly
            100                 105                 110

Val Val Tyr Phe Asp Gln Thr Gln Ala Gln Ala Gln Ala Leu Leu Ala
        115                 120                 125

Leu Asn Leu Pro Ser Ile Cys Gly Ile Tyr Pro Asn Tyr Tyr Ile Ala
    130                 135                 140

Pro Arg Ser Ile Pro Thr Val Gly Gly Val Trp Tyr
145                 150                 155
```

<210> 107
<211> 645
<212> DNA
<213> Avena sativa

<220>
<221> CDS
<222> (1)..(642)
<223> avenin

<400> 107

atg aag atc ttc ttc ttc tta gct ctc ctt gct ctg gta gtg agc gcc 48
Met Lys Ile Phe Phe Phe Leu Ala Leu Leu Ala Leu Val Val Ser Ala
1 5 10 15

acc ttt gca caa tat gca gaa tct gac ggt agt tat gag gaa gtg gag 96
Thr Phe Ala Gln Tyr Ala Glu Ser Asp Gly Ser Tyr Glu Glu Val Glu
20 25 30

ggt tct cat gat cga tgc caa caa cat cag atg aag ctg gac tct tgc 144
Gly Ser His Asp Arg Cys Gln Gln His Gln Met Lys Leu Asp Ser Cys
35 40 45

aga gag tac gtg gcg gag cgg tgc aca acg atg aga gat ttt ccg atc 192
Arg Glu Tyr Val Ala Glu Arg Cys Thr Thr Met Arg Asp Phe Pro Ile
50 55 60

acc tgg cca tgg aaa tgg tgg aag ggt ggt tgc gag gag ctc cgc aat 240
Thr Trp Pro Trp Lys Trp Trp Lys Gly Gly Cys Glu Glu Leu Arg Asn
65 70 75 80

gag tgc tgc caa ctg ttg ggc cag atg cca tcg gag tgt cgc tgt gat 288
Glu Cys Cys Gln Leu Leu Gly Gln Met Pro Ser Glu Cys Arg Cys Asp
85 90 95

gcg att tgg aga tca atc cag cgc gag ctt ggt ggc ttc ttt gga act 336
Ala Ile Trp Arg Ser Ile Gln Arg Glu Leu Gly Gly Phe Phe Gly Thr
100 105 110

caa caa ggt ctg ata ggg aaa agg ttg aag ata gcc aag agt ttg ccc 384
Gln Gln Gly Leu Ile Gly Lys Arg Leu Lys Ile Ala Lys Ser Leu Pro
115 120 125

acg cag tca aca tgg gcc ctg agt gca ata tcc cca aac tcc atg gtt 432
Thr Gln Ser Thr Trp Ala Leu Ser Ala Ile Ser Pro Asn Ser Met Val
130 135 140

agc cac att gct gga aag agc tcc att ctt cgt gcc ttg ccc gtg gat 480
Ser His Ile Ala Gly Lys Ser Ser Ile Leu Arg Ala Leu Pro Val Asp
145 150 155 160

gtc ctc gcc aat gca tac cgc att tcc agg caa gaa gcc cga aac ctc 528
Val Leu Ala Asn Ala Tyr Arg Ile Ser Arg Gln Glu Ala Arg Asn Leu
165 170 175

aaa aac aac agg gga caa gag tct ggt gta ttc act cca aaa ttt acc 576
Lys Asn Asn Arg Gly Gln Glu Ser Gly Val Phe Thr Pro Lys Phe Thr
180 185 190

caa acg agc ttc caa cct tat cca gag ggc gag gat gag tca tct ttg 624
Gln Thr Ser Phe Gln Pro Tyr Pro Glu Gly Glu Asp Glu Ser Ser Leu
195 200 205

att aat aag gca tca gag taa 645
Ile Asn Lys Ala Ser Glu
210

<210> 108
<211> 214
<212> PRT
<213> Avena sativa

<400> 108

Met Lys Ile Phe Phe Phe Leu Ala Leu Leu Ala Leu Val Val Ser Ala
1               5                   10                  15

Thr Phe Ala Gln Tyr Ala Glu Ser Asp Gly Ser Tyr Glu Glu Val Glu
            20                  25                  30

Gly Ser His Asp Arg Cys Gln Gln His Gln Met Lys Leu Asp Ser Cys
        35                  40                  45

Arg Glu Tyr Val Ala Glu Arg Cys Thr Thr Met Arg Asp Phe Pro Ile
    50                  55                  60

Thr Trp Pro Trp Lys Trp Trp Lys Gly Gly Cys Glu Glu Leu Arg Asn
65                  70                  75                  80

Glu Cys Cys Gln Leu Leu Gly Gln Met Pro Ser Glu Cys Arg Cys Asp
                85                  90                  95

Ala Ile Trp Arg Ser Ile Gln Arg Glu Leu Gly Gly Phe Phe Gly Thr
            100                 105                 110

Gln Gln Gly Leu Ile Gly Lys Arg Leu Lys Ile Ala Lys Ser Leu Pro
        115                 120                 125

Thr Gln Ser Thr Trp Ala Leu Ser Ala Ile Ser Pro Asn Ser Met Val
    130                 135                 140

Ser His Ile Ala Gly Lys Ser Ser Ile Leu Arg Ala Leu Pro Val Asp
145                 150                 155                 160

Val Leu Ala Asn Ala Tyr Arg Ile Ser Arg Gln Glu Ala Arg Asn Leu
                165                 170                 175

Lys Asn Asn Arg Gly Gln Glu Ser Gly Val Phe Thr Pro Lys Phe Thr
            180                 185                 190

Gln Thr Ser Phe Gln Pro Tyr Pro Glu Gly Glu Asp Glu Ser Ser Leu
        195                 200                 205

Ile Asn Lys Ala Ser Glu
    210

<210> 109
<211> 1044
<212> DNA
<213> Hordeum vulgare

<220>
<221> CDS
<222> (1)..(1041)
<223> c-hordein

<220>
<221> misc_feature
<222> (481)..(482)
<223> /transl_except=(pos:481..483,aa:OTHER)

<400> 109

```
atg aag acg ttc ctc acc ttt gtc ctc ctt gcc atg gcg atg agc atc    48
Met Lys Thr Phe Leu Thr Phe Val Leu Leu Ala Met Ala Met Ser Ile
 1               5                  10                  15
gtc act acc gct agg cag cta aac cct agc cac caa gag ttg caa tca    96
Val Thr Thr Ala Arg Gln Leu Asn Pro Ser His Gln Glu Leu Gln Ser
            20                  25                  30
cca caa caa cca ttt ctg aaa caa caa tca tat ctg caa caa cca tat   144
Pro Gln Gln Pro Phe Leu Lys Gln Gln Ser Tyr Leu Gln Gln Pro Tyr
        35                  40                  45
```

```
cca caa caa cca tat cta ccg cag caa cca ttc ccc aca ccc caa caa    192
Pro Gln Gln Pro Tyr Leu Pro Gln Gln Pro Phe Pro Thr Pro Gln Gln
     50                  55                  60

ttt ttc ccc tat cta cca cag caa aca ttt ccc cca tcc caa caa cca    240
Phe Phe Pro Tyr Leu Pro Gln Gln Thr Phe Pro Pro Ser Gln Gln Pro
65                  70                  75                  80

aac ccc cta caa cca caa caa cca ttc ccc ctg caa ccc caa cca cca    288
Asn Pro Leu Gln Pro Gln Gln Pro Phe Pro Leu Gln Pro Gln Pro Pro
                85                  90                  95

caa caa cct ttt cct cag ccc caa caa cca aat ccc cag caa cca caa    336
Gln Gln Pro Phe Pro Gln Pro Gln Gln Pro Asn Pro Gln Gln Pro Gln
            100                 105                 110

caa cct ttc ccc cgg caa cca caa caa ata gta ccc cag caa cca caa    384
Gln Pro Phe Pro Arg Gln Pro Gln Gln Ile Val Pro Gln Gln Pro Gln
        115                 120                 125

caa cca ttc cct cag caa cca caa caa cct ttt cct cag ccc caa caa    432
Gln Pro Phe Pro Gln Gln Pro Gln Gln Pro Phe Pro Gln Pro Gln Gln
    130                 135                 140

cca ttc tct tgg caa cca caa caa cca ttt ctc cag ccc cta caa cta    480
Pro Phe Ser Trp Gln Pro Gln Gln Pro Phe Leu Gln Pro Leu Gln Leu
145                 150                 155                 160

tag ccc ctg caa gca caa caa cca ttc ccc ttg caa cct caa cta cca    528
    Pro Leu Gln Ala Gln Gln Pro Phe Pro Leu Gln Pro Gln Leu Pro
                165                 170                 175

ttt ccg caa ccc caa caa cca att gga cag caa cca aaa caa cca ctc    576
Phe Pro Gln Pro Gln Gln Pro Ile Gly Gln Gln Pro Lys Gln Pro Leu
            180                 185                 190

ctg cag caa cca caa caa aca att ccc cag caa cca caa caa cca ttc    624
Leu Gln Gln Pro Gln Gln Thr Ile Pro Gln Gln Pro Gln Gln Pro Phe
        195                 200                 205

ccc ctg cag ccg caa caa cca ttc ccc caa caa cca caa caa cca ctt    672
Pro Leu Gln Pro Gln Gln Pro Phe Pro Gln Gln Pro Gln Gln Pro Leu
    210                 215                 220

ccc caa caa ccc caa caa ata att tcc cag caa ccc caa caa cca ttc    720
Pro Gln Gln Pro Gln Gln Ile Ile Ser Gln Gln Pro Gln Gln Pro Phe
225                 230                 235                 240

cct cta caa cct caa caa cca ttc ccc caa ccc caa cca ttc ccc cag    768
Pro Leu Gln Pro Gln Gln Pro Phe Pro Gln Pro Gln Pro Phe Pro Gln
                245                 250                 255

gag caa ccc caa caa gca ttc ccc cta caa ccg caa caa cca ttc ccc    816
Glu Gln Pro Gln Gln Ala Phe Pro Leu Gln Pro Gln Gln Pro Phe Pro
            260                 265                 270

gag gaa tca gaa caa ata att acc caa caa cca ttc cct cta caa cca    864
Glu Glu Ser Glu Gln Ile Ile Thr Gln Gln Pro Phe Pro Leu Gln Pro
        275                 280                 285

caa caa ctg ttc ccc cag caa cca caa caa cca ctt ccc cag ccc caa    912
Gln Gln Leu Phe Pro Gln Gln Pro Gln Gln Pro Leu Pro Gln Pro Gln
    290                 295                 300

caa cca ttc cgc caa cta cca aaa tat ata att ccc cag caa cct caa    960
Gln Pro Phe Arg Gln Leu Pro Lys Tyr Ile Ile Pro Gln Gln Pro Gln
305                 310                 315                 320
```

```
caa cca ttc ctt ctg caa cca cac caa cct cag caa cct tat gca caa    1008
Gln Pro Phe Leu Leu Gln Pro His Gln Pro Gln Gln Pro Tyr Ala Gln
                325                 330                 335

caa gac atc tgg agt gat ata gcc ctc ttg ggc taa                    1044
Gln Asp Ile Trp Ser Asp Ile Ala Leu Leu Gly
            340                 345
```

<210> 110
<211> 160
<212> PRT
<213> Hordeum vulgare

<400> 110

```
Met Lys Thr Phe Leu Thr Phe Val Leu Leu Ala Met Ala Met Ser Ile
1               5                   10                  15
Val Thr Thr Ala Arg Gln Leu Asn Pro Ser His Gln Glu Leu Gln Ser
            20                  25                  30
Pro Gln Gln Pro Phe Leu Lys Gln Gln Ser Tyr Leu Gln Gln Pro Tyr
        35                  40                  45
Pro Gln Gln Pro Tyr Leu Pro Gln Gln Pro Phe Pro Thr Pro Gln Gln
    50                  55                  60
Phe Phe Pro Tyr Leu Pro Gln Gln Thr Phe Pro Pro Ser Gln Gln Pro
65                  70                  75                  80
Asn Pro Leu Gln Pro Gln Gln Pro Phe Pro Leu Gln Pro Gln Pro Pro
                85                  90                  95
Gln Gln Pro Phe Pro Gln Pro Gln Gln Pro Asn Pro Gln Gln Pro Gln
            100                 105                 110
Gln Pro Phe Pro Arg Gln Pro Gln Gln Ile Val Pro Gln Gln Pro Gln
        115                 120                 125
Gln Pro Phe Pro Gln Gln Pro Gln Gln Pro Phe Pro Gln Pro Gln Gln
    130                 135                 140
Pro Phe Ser Trp Gln Pro Gln Gln Pro Phe Leu Gln Pro Leu Gln Leu
145                 150                 155                 160
```

<210> 111
<211> 186
<212> PRT
<213> Hordeum vulgare

<400> 111

```
Pro Leu Gln Ala Gln Gln Pro Phe Pro Leu Gln Pro Gln Leu Pro Phe
1               5                   10                  15
Pro Gln Pro Gln Gln Pro Ile Gly Gln Gln Pro Lys Gln Pro Leu Leu
            20                  25                  30
Gln Gln Pro Gln Gln Thr Ile Pro Gln Gln Pro Gln Gln Pro Phe Pro
        35                  40                  45
Leu Gln Pro Gln Gln Pro Phe Pro Gln Gln Pro Gln Gln Pro Leu Pro
    50                  55                  60
Gln Gln Pro Gln Gln Ile Ile Ser Gln Gln Pro Gln Gln Pro Phe Pro
65                  70                  75                  80
Leu Gln Pro Gln Gln Pro Phe Pro Gln Pro Gln Pro Phe Pro Gln Glu
                85                  90                  95
Gln Pro Gln Gln Ala Phe Pro Leu Gln Pro Gln Gln Pro Phe Pro Glu
            100                 105                 110
Glu Ser Glu Gln Ile Ile Thr Gln Gln Pro Phe Pro Leu Gln Pro Gln
        115                 120                 125
Gln Leu Phe Pro Gln Gln Pro Gln Gln Pro Leu Pro Gln Pro Gln Gln
    130                 135                 140
Pro Phe Arg Gln Leu Pro Lys Tyr Ile Ile Pro Gln Gln Pro Gln Gln
145                 150                 155                 160

Pro Phe Leu Leu Gln Pro His Gln Pro Gln Gln Pro Tyr Ala Gln Gln
                165                 170                 175
Asp Ile Trp Ser Asp Ile Ala Leu Leu Gly
            180                 185
```

<210> 112
<211> 924
<212> DNA
<213> Triticum aestivum

<220>
<221> CDS
<222> (1)..(921)
<223> glutenin-1D1

<400> 112

```
atg aag acc ttc ctc gtc ttt gcc ctc ctc gcc gtt gcg gcg aca agt    48
Met Lys Thr Phe Leu Val Phe Ala Leu Leu Ala Val Ala Ala Thr Ser
 1               5                  10                  15

gca att gcg cag atg gag act aga tgc atc cct ggt ttg gag aga cca    96
Ala Ile Ala Gln Met Glu Thr Arg Cys Ile Pro Gly Leu Glu Arg Pro
            20                  25                  30

tgg cag cag caa cca tta cca cca caa cag aca ttt cca caa caa cca   144
Trp Gln Gln Gln Pro Leu Pro Pro Gln Gln Thr Phe Pro Gln Gln Pro
        35                  40                  45

cta ttt tca caa caa caa caa caa caa cta ttt cct caa caa cca tca   192
Leu Phe Ser Gln Gln Gln Gln Gln Gln Leu Phe Pro Gln Gln Pro Ser
    50                  55                  60

ttt tcg cag caa caa cca cca ttt tgg cag caa caa cca cca ttt tct   240
Phe Ser Gln Gln Gln Pro Pro Phe Trp Gln Gln Gln Pro Pro Phe Ser
65                  70                  75                  80

cag caa caa cca att cta cca cag caa cca cca ttt tcg cag caa caa   288
Gln Gln Gln Pro Ile Leu Pro Gln Gln Pro Pro Phe Ser Gln Gln Gln
                85                  90                  95

caa cta gtt cta ccg caa caa cca cca ttt tca cag caa caa caa cca   336
Gln Leu Val Leu Pro Gln Gln Pro Pro Phe Ser Gln Gln Gln Gln Pro
            100                 105                 110

gtt tta cct cca caa caa tca cct ttt cca caa caa caa caa caa cac   384
Val Leu Pro Pro Gln Gln Ser Pro Phe Pro Gln Gln Gln Gln Gln His
        115                 120                 125

caa cag ctg gtg caa caa caa atc cct gtt gtt cag cca tcc att ttg   432
Gln Gln Leu Val Gln Gln Gln Ile Pro Val Val Gln Pro Ser Ile Leu
    130                 135                 140

cag cag cta aac cca tgc aag gta ttc ctc cag cag cag tgc agc cct   480
Gln Gln Leu Asn Pro Cys Lys Val Phe Leu Gln Gln Gln Cys Ser Pro
145                 150                 155                 160

gtg gca atg cca caa cgt ctt gct agg tcg caa atg ttg cag cag agc   528
Val Ala Met Pro Gln Arg Leu Ala Arg Ser Gln Met Leu Gln Gln Ser
                165                 170                 175

agt tgc cat gtg atg caa caa caa tgt tgc cag cag ttg ccg caa atc   576
Ser Cys His Val Met Gln Gln Gln Cys Cys Gln Gln Leu Pro Gln Ile
            180                 185                 190

ccc cag caa tcc cgc tat gag gca atc cgt gct atc atc tac tcc atc   624
Pro Gln Gln Ser Arg Tyr Glu Ala Ile Arg Ala Ile Ile Tyr Ser Ile
        195                 200                 205
```

```
atc ctg caa gaa caa caa cag gtt cag ggt tcc atc caa tct cag cag     672
Ile Leu Gln Glu Gln Gln Gln Val Gln Gly Ser Ile Gln Ser Gln Gln
    210                 215                 220

cag caa ccc caa cag ttg ggc caa tgt gtt tcc caa ccc caa cag cag     720
Gln Gln Pro Gln Gln Leu Gly Gln Cys Val Ser Gln Pro Gln Gln Gln
225                 230                 235                 240

tcg cag cag caa ctc ggg caa caa cct caa caa caa caa ttg gca cag     768
Ser Gln Gln Gln Leu Gly Gln Gln Pro Gln Gln Gln Gln Leu Ala Gln
                245                 250                 255

ggt acc ttt ttg cag cca cac cag ata gct cag ctt gag gtg atg act     816
Gly Thr Phe Leu Gln Pro His Gln Ile Ala Gln Leu Glu Val Met Thr
            260                 265                 270

tcc att gcg ctc cgt atc ctg cca acg atg tgc agt gtt aat gtg ccg     864
Ser Ile Ala Leu Arg Ile Leu Pro Thr Met Cys Ser Val Asn Val Pro
        275                 280                 285

ttg tac aga acc acc act agt gtg cca ttc ggc gtt ggc acc gga gtt     912
Leu Tyr Arg Thr Thr Thr Ser Val Pro Phe Gly Val Gly Thr Gly Val
    290                 295                 300

ggt gcc tac tga                                                     924
Gly Ala Tyr
305
```

<210> 113
<211> 307
<212> PRT
<213> Triticum aestivum

<400> 113

Met Lys Thr Phe Leu Val Phe Ala Leu Leu Ala Val Ala Ala Thr Ser
1               5                   10                  15

Ala Ile Ala Gln Met Glu Thr Arg Cys Ile Pro Gly Leu Glu Arg Pro
            20                  25                  30

Trp Gln Gln Gln Pro Leu Pro Pro Gln Gln Thr Phe Pro Gln Gln Pro
        35                  40                  45

Leu Phe Ser Gln Gln Gln Gln Gln Gln Leu Phe Pro Gln Gln Pro Ser
    50                  55                  60

Phe Ser Gln Gln Gln Pro Pro Phe Trp Gln Gln Gln Pro Pro Phe Ser
65                  70                  75                  80

Gln Gln Gln Pro Ile Leu Pro Gln Gln Pro Pro Phe Ser Gln Gln Gln
                85                  90                  95

Gln Leu Val Leu Pro Gln Gln Pro Pro Phe Ser Gln Gln Gln Gln Pro
            100                 105                 110

Val Leu Pro Pro Gln Gln Ser Pro Phe Pro Gln Gln Gln Gln Gln His
        115                 120                 125

Gln Gln Leu Val Gln Gln Gln Ile Pro Val Val Gln Pro Ser Ile Leu
    130                 135                 140

Gln Gln Leu Asn Pro Cys Lys Val Phe Leu Gln Gln Gln Cys Ser Pro
145                 150                 155                 160

Val Ala Met Pro Gln Arg Leu Ala Arg Ser Gln Met Leu Gln Gln Ser
                165                 170                 175

Ser Cys His Val Met Gln Gln Gln Cys Cys Gln Gln Leu Pro Gln Ile
            180                 185                 190

Pro Gln Gln Ser Arg Tyr Glu Ala Ile Arg Ala Ile Ile Tyr Ser Ile
        195                 200                 205

Ile Leu Gln Glu Gln Gln Gln Val Gln Gly Ser Ile Gln Ser Gln Gln
    210                 215                 220

Gln Gln Pro Gln Gln Leu Gly Gln Cys Val Ser Gln Pro Gln Gln Gln
225                 230                 235                 240

Ser Gln Gln Gln Leu Gly Gln Gln Pro Gln Gln Gln Gln Leu Ala Gln
                245                 250                 255

Gly Thr Phe Leu Gln Pro His Gln Ile Ala Gln Leu Glu Val Met Thr
            260                 265                 270

Ser Ile Ala Leu Arg Ile Leu Pro Thr Met Cys Ser Val Asn Val Pro
        275                 280                 285

Leu Tyr Arg Thr Thr Thr Ser Val Pro Phe Gly Val Gly Thr Gly Val
    290                 295                 300

Gly Ala Tyr
305

<210> 114
<211> 8482
<212> DNA
<213> Künstliche Sequenz

<220>
<223> Beschreibung der künstlichen Sequenz: binary expression vector

<400> 114

```
ttccatggac atacaaatgg acgaacggat aaaccttttc acgccctttt aaatatccga 60
ttattctaat aaacgctctt ttctcttagg tttacccgcc aatatatcct gtcaaacact 120
gatagtttaa actgaaggcg ggaaacgaca atcagatcta gtaggaaaca gctatgacca 180
tgattacgcc aatcaccact ttgtacaaga aagctgggtc tagatgacgg acaatcagta 240
aattgaacgg agaatattat tcataaaaat acgatagtaa cgggtgatat attcattaga 300
atgaaccgaa accggcggta aggatctgag ctacacatgc tcaggttttt tacaacgtgc 360
acaacagaat tgaaagcaaa tatcatgcga tcataggcgt ctcgcatatc tcattaaagc 420
aggaggcctt ctagactgca ggcggccgcc caccgcggtg ggctggctat gaagaaatta 480
taatcgtgta aaacttagtg agtgtgtatg aatgaaagta ttgcaaaatc ctcattatat 540
agactacatg cataactagt tgcatgtaaa tttgtagttt tcttcattat tgcatcctcc 600
aagtggatgt catggtttta cacatggctt ccatgcaaat catttccaaa atatttttaa 660
actttccaca gggcatccat gcatgcacct caaaacttgt gtgtggtaac attgttgtct 720
tgaaaaatta ctaaaccttt tgtccacgtg acgttcatgc acctcaaatc ttgtgtggta 780
ccattattat cctcaagaat tattgaatgt ttggtgtata tgccatccat gcagcattgc 840
aacaattaaa tctccaaacc ttgtggtacc atattcactc actttaattc tcctatagta 900
gaaatattag caaatattta catttccagt tgattagtat atgtatttag aagacaaaaa 960
taatttagaa tcaattaatc aacttgcaaa ttgctaagtg ttggcaaacg ttagcataaa 1020
aggtgttata aatttagtac caaatataaa aatttatcgc aaatcaaata cataacacac 1080
atagtaaaac aaaaacaaat tacaagggtt tagacgttta gtggcaatgt gtaaatttgc 1140
tcgactgaat tggttccttt aagcctgctt ttttgtacaa acttgtgata attcactggc 1200
cgtcgtttta caacgactca ggatcctgtc aaacactgat agtttaaact gaaggcggga 1260
aacgacaatc tgatcatgag cggagaatta agggagtcac gttatgaccc ccgccgatga 1320
cgcgggacaa gccgttttac gtttggaact gacagaaccg caacgttgaa ggagccactc 1380
agccgcgggt ttctggagtt taatgagcta agcacatacg tcagaaacca ttattgcgcg 1440
ttcaaaagtc gcctaaggtc actatcagct agcaaatatt tcttgtcaaa aatgctccac 1500
tgacgttcca taaattcccc tcggtatcca attagagtct catattcact ctcaatccaa 1560
ataatctgca ccggatctgg atcgtttcgc atgattgaac aagatggatt gcacgcaggt 1620
tctccggccg cttgggtgga gaggctattc ggctatgact gggcacaaca gacaatcggc 1680
```

tgctctgatg ccgccgtgtt ccggctgtca gcgcaggggc gcccggttct ttttgtcaag 1740
accgacctgt ccggtgccct gaatgaactg caggacgagg cagcgcggct atcgtggctg 1800
gccacgacgg gcgttccttg cgcagctgtg ctcgacgttg tcactgaagc gggaagggac 1860
tggctgctat tgggcgaagt gccggggcag gatctcctgt catctcacct tgctcctgcc 1920
gagaaagtat ccatcatggc tgatgcaatg cggcggctgc atacgcttga tccggctacc 1980
tgcccattcg accaccaagc gaaacatcgc atcgagcgag cacgtactcg gatggaagcc 2040
ggtcttgtcg atcaggatga tctggacgaa gagcatcagg ggctcgcgcc agccgaactg 2100
ttcgccaggc tcaaggcgcg catgcccgac ggcgaggatc tcgtcgtgac ccatggcgat 2160
gcctgcttgc cgaatatcat ggtggaaaat ggccgctttt ctggattcat cgactgtggc 2220
cggctgggtg tggcggaccg ctatcaggac atagcgttgg ctacccgtga tattgctgaa 2280
gagcttggcg gcgaatgggc tgaccgcttc ctcgtgcttt acggtatcgc cgctcccgat 2340
tcgcagcgca tcgccttcta tcgccttctt gacgagttct tctgagcggg acccaagctc 2400
tagatcttgc tgcgttcgga tatttcgtg gagttcccgc cacagacccg gatgatcccc 2460
gatcgttcaa acatttggca ataaagtttc ttaagattga atcctgttgc cggtcttgcg 2520
atgattatca tataatttct gttgaattac gttaagcatg taataattaa catgtaatgc 2580
atgacgttat ttatgagatg ggtttttatg attagagtcc cgcaattata catttaatac 2640
gcgatagaaa acaaaatata gcgcgcaaac taggataaat tatcgcgcgc ggtgtcatct 2700
atgttactag atcgggcctc ctgtcaagct ctgcttggta ataattgtca ttagattgtt 2760
tttatgcata gatgcactcg aaatcagcca attttagaca agtatcaaac ggatgttaat 2820
tcagtacatt aaagacgtcc gcaatgtgtt attaagttgt ctaagcgtca atttgtttac 2880
accacaatat atcctgccac cagccagcca acagctcccc gaccggcagc tcggcacaaa 2940
atcaccacgc gttaccacca cgccggccgg ccgcatggtg ttgaccgtgt tcgccggcat 3000
tgccgagttc gagcgttccc taatcatcga ccgcacccgg agcgggcgcg aggccgccaa 3060
ggcccgaggc gtgaagtttg gcccccgccc taccctcacc ccggcacaga tcgcgcacgc 3120
ccgcgagctg atcgaccagg aaggccgcac cgtgaaagag gcggctgcac tgcttggcgt 3180
gcatcgctcg accctgtacc gcgcacttga gcgcagcgag gaagtgacgc ccaccgaggc 3240
caggcggcgc ggtgccttcc gtgaggacgc attgaccgag gccgacgccc tggcggccgc 3300
cgagaatgaa cgccaagagg aacaagcatg aaaccgcacc aggacggcca ggacgaaccg 3360
tttttcatta ccgaagagat cgaggcggag atgatcgcgg ccgggtacgt gttcgagccg 3420
cccgcgcacg tctcaaccgt gcggctgcat gaaatcctgg ccggtttgtc tgatgccaag 3480
ctggcggcct ggccggccag cttggccgct gaagaaaccg agcgccgccg tctaaaaagg 3540
tgatgtgtat ttgagtaaaa cagcttgcgt catgcggtcg ctgcgtatat gatgcgatga 3600
gtaaataaac aaatacgcaa ggggaacgca tgaaggttat cgctgtactt aaccagaaag 3660
gcgggtcagg caagacgacc atcgcaaccc atctagcccg cgccctgcaa ctcgccgggg 3720
ccgatgttct gttagtcgat tccgatcccc agggcagtgc ccgcgattgg gcggccgtgc 3780
gggaagatca accgctaacc gttgtcggca tcgaccgccc gacgattgac cgcgacgtga 3840
aggccatcgg ccggcgcgac ttcgtagtga tcgacggagc gccccaggcg gcggacttgg 3900
ctgtgtccgc gatcaaggca gccgacttcg tgctgattcc ggtgcagcca agcccttacg 3960
acatatgggc caccgccgac ctggtggagc tggttaagca gcgcattgag gtcacggatg 4020
gaaggctaca agcggccttt gtcgtgtcgc gggcgatcaa aggcacgcgc atcggcggtg 4080
aggttgccga ggcgctggcc gggtacgagc tgcccattct tgagtcccgt atcacgcagc 4140
gcgtgagcta cccaggcact gccgccgccg gcacaaccgt tcttgaatca gaacccgagg 4200
gcgacgctgc ccgcgaggtc caggcgctgg ccgctgaaat taaatcaaaa ctcatttgag 4260
ttaatgaggt aaagagaaaa tgagcaaaag cacaaacacg ctaagtgccg gccgtccgag 4320
cgcacgcagc agcaaggctg caacgttggc cagcctggca gacacgccag ccatgaagcg 4380
ggtcaacttt cagttgccgg cggaggatca caccaagctg aagatgtacg cggtacgcca 4440
aggcaagacc attaccgagc tgctatctga atacatcgcg cagctaccag agtaaatgag 4500
caaatgaata aatgagtaga tgaattttag cggctaaagg aggcggcatg gaaaatcaag 4560
aacaaccagg caccgacgcc gtggaatgcc ccatgtgtgg aggaacgggc ggttggccag 4620
gcgtaagcgg ctgggttgtc tgccggccct gcaatggcac tggaaccccc aagcccgagg 4680
aatcggcgtg agcggtcgca aaccatccgg cccggtacaa atcggcgcgg cgctgggtga 4740
tgacctggtg gagaagttga aggccgcgca ggccgcccag cggcaacgca tcgaggcaga 4800
agcacgcccc ggtgaatcgt ggcaagcggc cgctgatcga atccgcaaag aatcccggca 4860
accgccggca gccggtgcgc cgtcgattag gaagccgccc aagggcgacg agcaaccaga 4920
ttttttcgtt ccgatgctct atgacgtggg cacccgcgat agtcgcagca tcatggacgt 4980
ggccgttttc cgtctgtcga agcgtgaccg acgagctggc gaggtgatcc gctacgagct 5040
tccagacggg cacgtagagg tttccgcagg gccggccggc atggccagtg tgtgggatta 5100

```
cgacctggta ctgatggcgg tttcccatct aaccgaatcc atgaaccgat accgggaagg 5160
gaagggagac aagcccggcc gcgtgttccg tccacacgtt gcggacgtac tcaagttctg 5220
ccggcgagcc gatggcggaa agcagaaaga cgacctggta gaaacctgca ttcggttaaa 5280
caccacgcac gttgccatgc agcgtacgaa gaaggccaag aacggccgcc tggtgacggt 5340
atccgagggt gaagccttga ttagccgcta caagatcgta aagagcgaaa ccgggcggcc 5400
ggagtacatc gagatcgagc tagctgattg gatgtaccgc gagatcacag aaggcaagaa 5460
cccggacgtg ctgacggttc accccgatta ctttttgatc gatcccggca tcggccgttt 5520
tctctaccgc ctggcacgcc gcgccgcagg caaggcagaa gccagatggt tgttcaagac 5580
gatctacgaa cgcagtggca gcgccggaga gttcaagaag ttctgtttca ccgtgcgcaa 5640
gctgatcggg tcaaatgacc tgccggagta cgatttgaag gaggaggcgg ggcaggctgg 5700
cccgatccta gtcatgcgct accgcaacct gatcgagggc gaagcatccg ccggttccta 5760
atgtacggag cagatgctag ggcaaattgc cctagcaggg gaaaaaggtc gaaaaggtct 5820
ctttcctgtg gatagcacgt acattgggaa cccaaagccg tacattggga accggaaccc 5880
gtacattggg aacccaaagc cgtacattgg gaaccggtca cacatgtaag tgactgatat 5940
aaaagagaaa aaaggcgatt tttccgccta aaactcttta aaacttatta aaactcttaa 6000
aacccgcctg gcctgtgcat aactgtctgg ccagcgcaca gccgaagagc tgcaaaaagc 6060
gcctaccctt cggtcgctgc gctccctacg ccccgccgct tcgcgtcggc ctatcgcggc 6120
cgctggccgc tcaaaaatgg ctggcctacg gccaggcaat ctaccagggc gcggacaagc 6180
cgcgccgtcg ccactcgacc gccggcgccc acatcaaggc accctgcctc gcgcgtttcg 6240
gtgatgacgg tgaaaacctc tgacacatgc agctcccgga gacggtcaca gcttgtctgt 6300
aagcggatgc cgggagcaga caagcccgtc agggcgcgtc agcgggtgtt ggcgggtgtc 6360
ggggcgcagc catgacccag tcacgtagcg atagcggagt gtatactggc ttaactatgc 6420
ggcatcagag cagattgtac tgagagtgca ccatatgcgg tgtgaaatac cgcacagatg 6480
cgtaaggaga aaataccgca tcaggcgctc ttccgcttcc tcgctcactg actcgctgcg 6540
ctcggtcgtt cggctgcggc gagcggtatc agctcactca aaggcggtaa tacggttatc 6600
cacagaatca ggggataacg caggaaagaa catgtgagca aaaggccagc aaaaggccag 6660
gaaccgtaaa aaggccgcgt tgctggcgtt tttccatagg ctccgccccc ctgacgagca 6720
tcacaaaaat cgacgctcaa gtcagaggtg gcgaaacccg acaggactat aaagatacca 6780
ggcgtttccc cctggaagct ccctcgtgcg ctctcctgtt ccgaccctgc cgcttaccgg 6840
atacctgtcc gcctttctcc cttcgggaag cgtggcgctt tctcatagct cacgctgtag 6900
gtatctcagt tcggtgtagg tcgttcgctc caagctgggc tgtgtgcacg aaccccccgt 6960
tcagcccgac cgctgcgcct tatccggtaa ctatcgtctt gagtccaacc cggtaagaca 7020
cgacttatcg ccactggcag cagccactgg taacaggatt agcagagcga ggtatgtagg 7080
cggtgctaca gagttcttga agtggtggcc taactacggc tacactagaa ggacagtatt 7140
tggtatctgc gctctgctga agccagttac cttcggaaaa agagttggta gctcttgatc 7200
cggcaaacaa accaccgctg gtagcggtgg tttttttgtt tgcaagcagc agattacgcg 7260
cagaaaaaaa ggatctcaag aagatccttt gatcttttct acggggtctg acgctcagtg 7320
gaacgaaaac tcacgttaag ggattttggt catgcatgat atatctccca atttgtgtag 7380
ggcttattat gcacgcttaa aaataataaa agcagacttg acctgatagt ttggctgtga 7440
gcaattatgt gcttagtgca tctaacgctt gagttaagcc gcgccgcgaa gcggcgtcgg 7500
cttgaacgaa tttctagcta gacattattt gccgactacc ttggtgatct cgcctttcac 7560
gtagtggaca aattcttcca actgatctgc gcgcgaggcc aagcgatctt cttcttgtcc 7620
aagataagcc tgtctagctt caagtatgac gggctgatac tgggccggca ggcgctccat 7680
tgcccagtcg gcagcgacat ccttcggcgc gattttgccg gttactgcgc tgtaccaaat 7740
gcgggacaac gtaagcacta catttcgctc atcgccagcc cagtcgggcg gcgagttcca 7800
tagcgttaag gtttcattta gcgcctcaaa tagatcctgt tcaggaaccg gatcaaagag 7860
ttcctccgcc gctggaccta ccaaggcaac gctatgttct cttgcttttg tcagcaagat 7920
agccagatca atgtcgatcg tggctggctc gaagatacct gcaagaatgt cattgcgctg 7980
ccattctcca aattgcagtt cgcgcttagc tggataacgc cacggaatga tgtcgtcgtg 8040
cacaacaatg gtgacttcta cagcgcggag aatctcgctc tctccagggg aagccgaagt 8100
ttccaaaagg tcgttgatca aagctcgccg cgttgtttca tcaagcctta cggtcaccgt 8160
aaccagcaaa tcaatatcac tgtgtggctt caggccgcca tccactgcgg agccgtacaa 8220
atgtacggcc agcaacgtcg gttcgagatg gcgctcgatg acgccaacta cctctgatag 8280
ttgagtcgat acttcggcga tcaccgcttc ccccatgatg tttaactttg ttttagggcg 8340
actgccctgc tgcgtaacat cgttgctgct ccataacatc aaacatcgac ccacggcgta 8400
acgcgcttgc tgcttggatg cccgaggcat agactgtacc ccaaaaaaac agtcataaca 8460
agccatgaaa accgccactg cg                                          8482
```

<210> 115
<211> 575
<212> DNA
<213> Brassica napus

<220>

<221> misc_feature
<222> (1)..(6)
<223> restriction site

<220>
<221> misc_feature
<222> (570)..(575)
<223> restriction site

<220>
<221> misc_feature
<222> (7) .. (575)
<223> coding for homogentisate-1,2-dioxygenase (HDG)

<400> 115

```
gtcgacgggc cgatgggggc gaagggtctt gctgcaccaa gagattttct tgcaccaacg 60
gcatggtttg aggaagggct acggcctgac tacactattg ttcagaagtt tggcggtgaa 120
ctctttactg ctaaacaaga tttctctccg ttcaatgtgg ttgcctggca tggcaattac 180
gtgccttata agtatgacct gcacaagttc tgtccataca acactgtcct tgtagaccat 240
ggagatccat ctgtaaatac agttctgaca gcaccaacgg ataaacctgg tgtggccttg 300
cttgattttg tcatattccc tcctcgttgg ttggttgctg agcatacctt tcgacctcct 360
tactaccatc gtaactgcat gagtgaattt atgggcctaa tctatggtgc ttacgaggcc 420
aaagctgatg gatttctacc tggtggcgca agtcttcaca gttgtatgac acctcatggt 480
ccagatacaa ccacatacga ggcgacgatt gctcgtgtaa atgcaatggc tccttataag 540
ctcacaggca ccatggcctt catgtttgag gtacc                            575
```

<210> 116
<211> 1386
<212> DNA
<213> Arabidopsis thaliana

<220>
<221> CDS
<222> (1)..(1383)
<223> coding for homogentisate-1,2-dioxygenase (HDG)

<400> 116

```
atg gaa gag aag aag aag gag ctt gaa gag ttg aag tat caa tca ggt    48
Met Glu Glu Lys Lys Lys Glu Leu Glu Glu Leu Lys Tyr Gln Ser Gly
 1               5                  10                  15

ttt ggt aac cac ttc tca tcg gaa gca atc gcc gga gct tta ccg tta    96
Phe Gly Asn His Phe Ser Ser Glu Ala Ile Ala Gly Ala Leu Pro Leu
            20                  25                  30

gat cag aac agt cct ctt ctt tgt cct tac ggt ctt tac gcc gaa cag   144
Asp Gln Asn Ser Pro Leu Leu Cys Pro Tyr Gly Leu Tyr Ala Glu Gln
        35                  40                  45

atc tcc ggt act tct ttc act tct cct cgc aag ctc aat caa aga agt   192
Ile Ser Gly Thr Ser Phe Thr Ser Pro Arg Lys Leu Asn Gln Arg Ser
    50                  55                  60

tgg ttg tac cgg gtt aaa cca tcg gtt aca cat gaa ccg ttc aag cct   240
Trp Leu Tyr Arg Val Lys Pro Ser Val Thr His Glu Pro Phe Lys Pro
65                  70                  75                  80

cgt gta cca gct cat aag aag ctt gtg agt gag ttt gat gca tca aat   288
Arg Val Pro Ala His Lys Lys Leu Val Ser Glu Phe Asp Ala Ser Asn
                85                  90                  95
```

```
agt cgt acg aat ccg act cag ctt cgg tgg aga cct gag gat att cct     336
Ser Arg Thr Asn Pro Thr Gln Leu Arg Trp Arg Pro Glu Asp Ile Pro
            100                 105                 110

gat tcg gag att gat ttc gtt gat ggg tta ttt acc att tgt gga gct     384
Asp Ser Glu Ile Asp Phe Val Asp Gly Leu Phe Thr Ile Cys Gly Ala
        115                 120                 125

gga agc tcg ttt ctt cgc cat ggc ttc gct att cac atg tat gtg gct     432
Gly Ser Ser Phe Leu Arg His Gly Phe Ala Ile His Met Tyr Val Ala
    130                 135                 140

aac aca gga atg aaa gac tcc gca ttt tgc aac gct gat ggt gac ttc     480
Asn Thr Gly Met Lys Asp Ser Ala Phe Cys Asn Ala Asp Gly Asp Phe
145                 150                 155                 160

ttg tta gtt cct caa aca gga agg cta tgg att gaa act gag tgt gga     528
Leu Leu Val Pro Gln Thr Gly Arg Leu Trp Ile Glu Thr Glu Cys Gly
                165                 170                 175

agg ctt ttg gta act cct ggt gag att gct gtt ata cca caa ggt ttc     576
Arg Leu Leu Val Thr Pro Gly Glu Ile Ala Val Ile Pro Gln Gly Phe
            180                 185                 190

cgt ttc tcc ata gat tta ccg gat ggg aag tct cgt ggt tat gtt gct     624
Arg Phe Ser Ile Asp Leu Pro Asp Gly Lys Ser Arg Gly Tyr Val Ala
        195                 200                 205

gaa atc tat ggg gct cat ttt cag ctt cct gat ctt gga cca ata ggt     672
Glu Ile Tyr Gly Ala His Phe Gln Leu Pro Asp Leu Gly Pro Ile Gly
    210                 215                 220

gct aat ggt ctt gct gca tca aga gat ttt ctt gca cca aca gca tgg     720
Ala Asn Gly Leu Ala Ala Ser Arg Asp Phe Leu Ala Pro Thr Ala Trp
225                 230                 235                 240

ttt gag gat gga ttg cgg cct gaa tac aca att gtt cag aag ttt ggc     768
Phe Glu Asp Gly Leu Arg Pro Glu Tyr Thr Ile Val Gln Lys Phe Gly
                245                 250                 255

ggt gaa ctc ttt act gct aaa caa gat ttc tct cca ttc aat gtg gtt     816
Gly Glu Leu Phe Thr Ala Lys Gln Asp Phe Ser Pro Phe Asn Val Val
            260                 265                 270

gcc tgg cat ggc aat tac gtg cct tat aag tat gac ctg aag aag ttc     864
Ala Trp His Gly Asn Tyr Val Pro Tyr Lys Tyr Asp Leu Lys Lys Phe
        275                 280                 285

tgt cca tac aac act gtg ctt tta gat cat gga gat cca tct ata aat     912
Cys Pro Tyr Asn Thr Val Leu Leu Asp His Gly Asp Pro Ser Ile Asn
    290                 295                 300

aca gtc ctt aca gca cca act gat aaa cct ggt gtg gcc ttg ctt gat     960
Thr Val Leu Thr Ala Pro Thr Asp Lys Pro Gly Val Ala Leu Leu Asp
305                 310                 315                 320

ttt gtc ata ttt cct cct cga tgg ttg gtt gct gag cat act ttt cga    1008
Phe Val Ile Phe Pro Pro Arg Trp Leu Val Ala Glu His Thr Phe Arg
                325                 330                 335

cct cct tac tat cat cgt aac tgc atg agt gaa ttt atg ggc tta atc    1056
Pro Pro Tyr Tyr His Arg Asn Cys Met Ser Glu Phe Met Gly Leu Ile
            340                 345                 350

tac ggt gca tac gag gcg aaa gct gat gga ttt ctc cct ggc ggt gca    1104
Tyr Gly Ala Tyr Glu Ala Lys Ala Asp Gly Phe Leu Pro Gly Gly Ala
        355                 360                 365
```

```
agt ctt cat agc tgt atg aca cct cat ggt cca gat act acc acg tac      1152
Ser Leu His Ser Cys Met Thr Pro His Gly Pro Asp Thr Thr Thr Tyr
    370                 375                 380

gag gcg aca att gct cga gta aat gca atg gct cct tct aaa ctc aca      1200
Glu Ala Thr Ile Ala Arg Val Asn Ala Met Ala Pro Ser Lys Leu Thr
385                 390                 395                 400

ggt acg atg gct ttc atg ttc gaa tca gca ttg atc cct aga gtc tgt      1248
Gly Thr Met Ala Phe Met Phe Glu Ser Ala Leu Ile Pro Arg Val Cys
                405                 410                 415

cat tgg gct ctg gag tct cct ttc ctg gat cac gac tac tac cag tgt      1296
His Trp Ala Leu Glu Ser Pro Phe Leu Asp His Asp Tyr Tyr Gln Cys
            420                 425                 430

tgg att ggc ctc aag tct cat ttc tcg cgc ata agc ttg gac aag aca      1344
Trp Ile Gly Leu Lys Ser His Phe Ser Arg Ile Ser Leu Asp Lys Thr
        435                 440                 445

aat gtt gaa tca aca gag aaa gaa cca gga gct tcg gag taa              1386
Asn Val Glu Ser Thr Glu Lys Glu Pro Gly Ala Ser Glu
    450                 455                 460
```

<210> 117
<211> 461
<212> PRT
<213> Arabidopsis thaliana

<400> 117

```
Met Glu Glu Lys Lys Lys Glu Leu Glu Glu Leu Lys Tyr Gln Ser Gly
1               5                   10                  15

Phe Gly Asn His Phe Ser Ser Glu Ala Ile Ala Gly Ala Leu Pro Leu
            20                  25                  30

Asp Gln Asn Ser Pro Leu Leu Cys Pro Tyr Gly Leu Tyr Ala Glu Gln
        35                  40                  45

Ile Ser Gly Thr Ser Phe Thr Ser Pro Arg Lys Leu Asn Gln Arg Ser
    50                  55                  60

Trp Leu Tyr Arg Val Lys Pro Ser Val Thr His Glu Pro Phe Lys Pro
65                  70                  75                  80

Arg Val Pro Ala His Lys Lys Leu Val Ser Glu Phe Asp Ala Ser Asn
                85                  90                  95

Ser Arg Thr Asn Pro Thr Gln Leu Arg Trp Arg Pro Glu Asp Ile Pro
            100                 105                 110

Asp Ser Glu Ile Asp Phe Val Asp Gly Leu Phe Thr Ile Cys Gly Ala
        115                 120                 125

Gly Ser Ser Phe Leu Arg His Gly Phe Ala Ile His Met Tyr Val Ala
    130                 135                 140

Asn Thr Gly Met Lys Asp Ser Ala Phe Cys Asn Ala Asp Gly Asp Phe
145                 150                 155                 160

Leu Leu Val Pro Gln Thr Gly Arg Leu Trp Ile Glu Thr Glu Cys Gly
                165                 170                 175

Arg Leu Leu Val Thr Pro Gly Glu Ile Ala Val Ile Pro Gln Gly Phe
            180                 185                 190

Arg Phe Ser Ile Asp Leu Pro Asp Gly Lys Ser Arg Gly Tyr Val Ala
        195                 200                 205
```

Glu Ile Tyr Gly Ala His Phe Gln Leu Pro Asp Leu Gly Pro Ile Gly
210 215 220

Ala Asn Gly Leu Ala Ala Ser Arg Asp Phe Leu Ala Pro Thr Ala Trp
225 230 235 240

Phe Glu Asp Gly Leu Arg Pro Glu Tyr Thr Ile Val Gln Lys Phe Gly
245 250 255

Gly Glu Leu Phe Thr Ala Lys Gln Asp Phe Ser Pro Phe Asn Val Val
260 265 270

Ala Trp His Gly Asn Tyr Val Pro Tyr Lys Tyr Asp Leu Lys Lys Phe
275 280 285

Cys Pro Tyr Asn Thr Val Leu Leu Asp His Gly Asp Pro Ser Ile Asn
290 295 300

Thr Val Leu Thr Ala Pro Thr Asp Lys Pro Gly Val Ala Leu Leu Asp
305 310 315 320

Phe Val Ile Phe Pro Pro Arg Trp Leu Val Ala Glu His Thr Phe Arg
325 330 335

Pro Pro Tyr Tyr His Arg Asn Cys Met Ser Glu Phe Met Gly Leu Ile
340 345 350

Tyr Gly Ala Tyr Glu Ala Lys Ala Asp Gly Phe Leu Pro Gly Gly Ala
355 360 365

Ser Leu His Ser Cys Met Thr Pro His Gly Pro Asp Thr Thr Thr Tyr
370 375 380

Glu Ala Thr Ile Ala Arg Val Asn Ala Met Ala Pro Ser Lys Leu Thr
385 390 395 400

Gly Thr Met Ala Phe Met Phe Glu Ser Ala Leu Ile Pro Arg Val Cys
405 410 415

His Trp Ala Leu Glu Ser Pro Phe Leu Asp His Asp Tyr Tyr Gln Cys
420 425 430

Trp Ile Gly Leu Lys Ser His Phe Ser Arg Ile Ser Leu Asp Lys Thr
435 440 445

Asn Val Glu Ser Thr Glu Lys Glu Pro Gly Ala Ser Glu
450 455 460

<210> 118
<211> 815
<212> DNA
<213> Arabidopsis thaliana

<220>
<221> CDS
<222> (37)..(705)
<223> coding for maleylacetoacetate isomerase (MAAI)

<400> 118

```
gtaatctccg aagaagaaca aattccttgc tgaatc atg tct tat gtt acc gat    54
                                        Met Ser Tyr Val Thr Asp
                                         1               5

ttt tat cag gcg aag ttg aag ctc tac tct tac tgg aga agc tca tgt   102
Phe Tyr Gln Ala Lys Leu Lys Leu Tyr Ser Tyr Trp Arg Ser Ser Cys
            10                  15                  20

gct cat cgc gtc cgt atc gcc ctc act tta aaa ggg ctt gat tat gaa   150
Ala His Arg Val Arg Ile Ala Leu Thr Leu Lys Gly Leu Asp Tyr Glu
        25                  30                  35

tat ata ccg gtt aat ttg ctc aaa ggg gat caa tcc gat tca gat ttc   198
Tyr Ile Pro Val Asn Leu Leu Lys Gly Asp Gln Ser Asp Ser Asp Phe
    40                  45                  50

aag aag atc aat cca atg ggc act gta cca gcg ctt gtt gat ggt gat   246
Lys Lys Ile Asn Pro Met Gly Thr Val Pro Ala Leu Val Asp Gly Asp
55                  60                  65                  70

gtt gtg att aat gac tct ttc gca ata ata atg tac ctg gat gat aag   294
Val Val Ile Asn Asp Ser Phe Ala Ile Ile Met Tyr Leu Asp Asp Lys
                75                  80                  85

tat ccg gag cca ccg ctg tta cca agt gac tac cat aaa cgg gcg gta   342
Tyr Pro Glu Pro Pro Leu Leu Pro Ser Asp Tyr His Lys Arg Ala Val
            90                  95                  100

aat tac cag gcg acg agt att gtc atg tct ggt ata cag cct cat caa   390
Asn Tyr Gln Ala Thr Ser Ile Val Met Ser Gly Ile Gln Pro His Gln
        105                 110                 115

aat atg gct ctt ttt agg tat ctc gag gac aag ata aat gct gag gag   438
Asn Met Ala Leu Phe Arg Tyr Leu Glu Asp Lys Ile Asn Ala Glu Glu
    120                 125                 130

aaa act gct tgg att act aat gct atc aca aaa gga ttc aca gct ctc   486
Lys Thr Ala Trp Ile Thr Asn Ala Ile Thr Lys Gly Phe Thr Ala Leu
135                 140                 145                 150

gag aaa ctg ttg gtg agt tgc gct gga aaa tac gcg act ggt gat gaa   534
Glu Lys Leu Leu Val Ser Cys Ala Gly Lys Tyr Ala Thr Gly Asp Glu
                155                 160                 165

gtt tac ttg gct gat ctt ttc cta gca cca cag atc cac gca gca ttc   582
Val Tyr Leu Ala Asp Leu Phe Leu Ala Pro Gln Ile His Ala Ala Phe
            170                 175                 180

aac aga ttc cat att aac atg gaa cca ttc ccg act ctt gca agg ttt   630
Asn Arg Phe His Ile Asn Met Glu Pro Phe Pro Thr Leu Ala Arg Phe
        185                 190                 195

tac gag tca tac aac gaa ctg cct gca ttt caa aat gca gtc ccg gag   678
Tyr Glu Ser Tyr Asn Glu Leu Pro Ala Phe Gln Asn Ala Val Pro Glu
    200                 205                 210

aag caa cca gat act cct tcc acc atc tgattctgtg aaccgtaagc         725
Lys Gln Pro Asp Thr Pro Ser Thr Ile
215                 220

ttctctcagt ctcagctcaa taaaatctct taggaaacaa caacaacacc ttgaacttaa 785

atgtatcata tgaaccagtt tacaaataat                                 815
```

<210> 119
<211> 223
<212> PRT

<213> Arabidopsis thaliana

<400> 119

```
Met Ser Tyr Val Thr Asp Phe Tyr Gln Ala Lys Leu Lys Leu Tyr Ser
1               5                   10                  15

Tyr Trp Arg Ser Ser Cys Ala His Arg Val Arg Ile Ala Leu Thr Leu
            20                  25                  30

Lys Gly Leu Asp Tyr Glu Tyr Ile Pro Val Asn Leu Leu Lys Gly Asp
        35                  40                  45

Gln Ser Asp Ser Asp Phe Lys Lys Ile Asn Pro Met Gly Thr Val Pro
    50                  55                  60

Ala Leu Val Asp Gly Asp Val Val Ile Asn Asp Ser Phe Ala Ile Ile
65                  70                  75                  80

Met Tyr Leu Asp Asp Lys Tyr Pro Glu Pro Pro Leu Leu Pro Ser Asp
                85                  90                  95

Tyr His Lys Arg Ala Val Asn Tyr Gln Ala Thr Ser Ile Val Met Ser
            100                 105                 110

Gly Ile Gln Pro His Gln Asn Met Ala Leu Phe Arg Tyr Leu Glu Asp
        115                 120                 125

Lys Ile Asn Ala Glu Glu Lys Thr Ala Trp Ile Thr Asn Ala Ile Thr
    130                 135                 140

Lys Gly Phe Thr Ala Leu Glu Lys Leu Leu Val Ser Cys Ala Gly Lys
145                 150                 155                 160

Tyr Ala Thr Gly Asp Glu Val Tyr Leu Ala Asp Leu Phe Leu Ala Pro
                165                 170                 175

Gln Ile His Ala Ala Phe Asn Arg Phe His Ile Asn Met Glu Pro Phe
            180                 185                 190

Pro Thr Leu Ala Arg Phe Tyr Glu Ser Tyr Asn Glu Leu Pro Ala Phe
        195                 200                 205

Gln Asn Ala Val Pro Glu Lys Gln Pro Asp Thr Pro Ser Thr Ile
    210                 215                 220
```

<210> 120
<211> 1227
<212> DNA
<213> Arabidopsis thaliana

<220>
<221> CDS
<222> (1)..(1224)
<223> coding for fumarylacetoacetate hydrolase (FAAH)

<400> 120

```
atg gcg ttg ctg aag tct ttc atc gat gtt ggc tca gac tcg cac ttc    48
Met Ala Leu Leu Lys Ser Phe Ile Asp Val Gly Ser Asp Ser His Phe
 1               5                  10                  15

cct atc cag aat ctc cct tat ggt gtc ttc aaa ccg gaa tcg aac tca    96
Pro Ile Gln Asn Leu Pro Tyr Gly Val Phe Lys Pro Glu Ser Asn Ser
            20                  25                  30

act cct cgt cct gcc gtc gct atc ggc gat ttg gtt ctg gac ctc tcc    144
Thr Pro Arg Pro Ala Val Ala Ile Gly Asp Leu Val Leu Asp Leu Ser
        35                  40                  45

gct atc tct gaa gct ggg ctt ttc gat ggt ctg atc ctt aag gac gca    192
Ala Ile Ser Glu Ala Gly Leu Phe Asp Gly Leu Ile Leu Lys Asp Ala
    50                  55                  60

gat tgc ttt ctt cag cct aat ttg aat aag ttc ttg gcc atg gga cgg    240
Asp Cys Phe Leu Gln Pro Asn Leu Asn Lys Phe Leu Ala Met Gly Arg
65                  70                  75                  80
```

```
cct gcg tgg aag gaa gcg cgt tct acg ctg caa aga atc ttg tca ttt      288
Pro Ala Trp Lys Glu Ala Arg Ser Thr Leu Gln Arg Ile Leu Ser Phe
                85                  90                  95

ttg tta ttt ggc ttc aag gtt ttg gtt ttg gta tgt ttt cat gca gct      336
Leu Leu Phe Gly Phe Lys Val Leu Val Leu Val Cys Phe His Ala Ala
            100                 105                 110

aat gaa cct atc ttg cga gac aat gat gtt ttg agg aga aaa tca ttc      384
Asn Glu Pro Ile Leu Arg Asp Asn Asp Val Leu Arg Arg Lys Ser Phe
        115                 120                 125

cat cag atg agt aaa gtg gaa atg att gtt cct atg gtg att ggg gac      432
His Gln Met Ser Lys Val Glu Met Ile Val Pro Met Val Ile Gly Asp
    130                 135                 140

tat aca gac ttc ttt gca tct atg cat cac gcg aag aac tgc gga ctt      480
Tyr Thr Asp Phe Phe Ala Ser Met His His Ala Lys Asn Cys Gly Leu
145                 150                 155                 160

atg ttc cgt ggg cct gag aat gcg ata aac cca aat tgg ttt cgt ctt      528
Met Phe Arg Gly Pro Glu Asn Ala Ile Asn Pro Asn Trp Phe Arg Leu
                165                 170                 175

ccc att gca tat cat gga cgg gca tca tct att gtc atc tct ggg act      576
Pro Ile Ala Tyr His Gly Arg Ala Ser Ser Ile Val Ile Ser Gly Thr
            180                 185                 190

gac att att cga cca aga ggt cag ggc cat cca caa gga aac tct gaa      624
Asp Ile Ile Arg Pro Arg Gly Gln Gly His Pro Gln Gly Asn Ser Glu
        195                 200                 205

cca tat ttt gga cct tcg aag aaa ctt gat ttt gag ctt gag atg gct      672
Pro Tyr Phe Gly Pro Ser Lys Lys Leu Asp Phe Glu Leu Glu Met Ala
    210                 215                 220

gct gtg gtt ggt cca gga aat gaa ttg gga aag cct att gac gtg aat      720
Ala Val Val Gly Pro Gly Asn Glu Leu Gly Lys Pro Ile Asp Val Asn
225                 230                 235                 240

aat gca gcc gat cat ata ttt ggt cta tta ctg atg aat gac tgg agt      768
Asn Ala Ala Asp His Ile Phe Gly Leu Leu Leu Met Asn Asp Trp Ser
                245                 250                 255

gct agg gat att cag gcg tgg gag tat gta cct ctt ggt cct ttc ctg      816
Ala Arg Asp Ile Gln Ala Trp Glu Tyr Val Pro Leu Gly Pro Phe Leu
            260                 265                 270

ggg aag agt ttt ggg act act ata tcc cct tgg att gtt acc ttg gat      864
Gly Lys Ser Phe Gly Thr Thr Ile Ser Pro Trp Ile Val Thr Leu Asp
        275                 280                 285

gcg ctt gag cct ttt ggt tgt caa gct ccc aag cag gat cca cct cca      912
Ala Leu Glu Pro Phe Gly Cys Gln Ala Pro Lys Gln Asp Pro Pro Pro
    290                 295                 300

ttg cca tat ttg gct gag aaa gag tct gta aat tac gat atc tcc ttg      960
Leu Pro Tyr Leu Ala Glu Lys Glu Ser Val Asn Tyr Asp Ile Ser Leu
305                 310                 315                 320

gag cta gca cac cat acc gtt aac ggt tgc aat ttg agg cct ggt gat     1008
Glu Leu Ala His His Thr Val Asn Gly Cys Asn Leu Arg Pro Gly Asp
                325                 330                 335

ctc ctt ggc aca gga acc ata agc gga ccg gag cca gat tca tat ggg     1056
Leu Leu Gly Thr Gly Thr Ile Ser Gly Pro Glu Pro Asp Ser Tyr Gly
            340                 345                 350
```

```
tgc cta ctt gag ttg aca tgg aat gga cag aaa cct cta tca ctc aat   1104
Cys Leu Leu Glu Leu Thr Trp Asn Gly Gln Lys Pro Leu Ser Leu Asn
        355                 360                 365

gga aca act cag acg ttt ctc gaa gac gga gac caa gtc acc ttc tca   1152
Gly Thr Thr Gln Thr Phe Leu Glu Asp Gly Asp Gln Val Thr Phe Ser
    370                 375                 380

ggt gta tgc aag gga gat ggt tac aat gtt ggg ttt gga aca tgc aca   1200
Gly Val Cys Lys Gly Asp Gly Tyr Asn Val Gly Phe Gly Thr Cys Thr
385                 390                 395                 400

ggg aaa att gtt cct tca ccg cct tga                               1227
Gly Lys Ile Val Pro Ser Pro Pro
                405
```

<210> 121
<211> 408
<212> PRT
<213> Arabidopsis thaliana

<400> 121

```
Met Ala Leu Leu Lys Ser Phe Ile Asp Val Gly Ser Asp Ser His Phe
1               5                   10                  15

Pro Ile Gln Asn Leu Pro Tyr Gly Val Phe Lys Pro Glu Ser Asn Ser
            20                  25                  30

Thr Pro Arg Pro Ala Val Ala Ile Gly Asp Leu Val Leu Asp Leu Ser
        35                  40                  45

Ala Ile Ser Glu Ala Gly Leu Phe Asp Gly Leu Ile Leu Lys Asp Ala
    50                  55                  60

Asp Cys Phe Leu Gln Pro Asn Leu Asn Lys Phe Leu Ala Met Gly Arg
65                  70                  75                  80

Pro Ala Trp Lys Glu Ala Arg Ser Thr Leu Gln Arg Ile Leu Ser Phe
                85                  90                  95

Leu Leu Phe Gly Phe Lys Val Leu Val Leu Val Cys Phe His Ala Ala
            100                 105                 110

Asn Glu Pro Ile Leu Arg Asp Asn Asp Val Leu Arg Arg Lys Ser Phe
        115                 120                 125

His Gln Met Ser Lys Val Glu Met Ile Val Pro Met Val Ile Gly Asp
    130                 135                 140

Tyr Thr Asp Phe Phe Ala Ser Met His His Ala Lys Asn Cys Gly Leu
145                 150                 155                 160

Met Phe Arg Gly Pro Glu Asn Ala Ile Asn Pro Asn Trp Phe Arg Leu
                165                 170                 175

Pro Ile Ala Tyr His Gly Arg Ala Ser Ser Ile Val Ile Ser Gly Thr
            180                 185                 190

Asp Ile Ile Arg Pro Arg Gly Gln Gly His Pro Gln Gly Asn Ser Glu
        195                 200                 205

Pro Tyr Phe Gly Pro Ser Lys Lys Leu Asp Phe Glu Leu Glu Met Ala
    210                 215                 220

Ala Val Val Gly Pro Gly Asn Glu Leu Gly Lys Pro Ile Asp Val Asn
225                 230                 235                 240

Asn Ala Ala Asp His Ile Phe Gly Leu Leu Leu Met Asn Asp Trp Ser
                245                 250                 255
```

```
Ala Arg Asp Ile Gln Ala Trp Glu Tyr Val Pro Leu Gly Pro Phe Leu
            260                 265                 270

Gly Lys Ser Phe Gly Thr Thr Ile Ser Pro Trp Ile Val Thr Leu Asp
        275                 280                 285

Ala Leu Glu Pro Phe Gly Cys Gln Ala Pro Lys Gln Asp Pro Pro Pro
    290                 295                 300

Leu Pro Tyr Leu Ala Glu Lys Glu Ser Val Asn Tyr Asp Ile Ser Leu
305                 310                 315                 320

Glu Leu Ala His His Thr Val Asn Gly Cys Asn Leu Arg Pro Gly Asp
                325                 330                 335

Leu Leu Gly Thr Gly Thr Ile Ser Gly Pro Glu Pro Asp Ser Tyr Gly
            340                 345                 350

Cys Leu Leu Glu Leu Thr Trp Asn Gly Gln Lys Pro Leu Ser Leu Asn
        355                 360                 365

Gly Thr Thr Gln Thr Phe Leu Glu Asp Gly Asp Gln Val Thr Phe Ser
    370                 375                 380

Gly Val Cys Lys Gly Asp Gly Tyr Asn Val Gly Phe Gly Thr Cys Thr
385                 390                 395                 400

Gly Lys Ile Val Pro Ser Pro Pro
                405
```

<210> 122
<211> 11667
<212> DNA
<213> Künstliche Sequenz

<220>
<223> Beschreibung der künstlichen Sequenz: supression construct 2 p3300.1-Toc159-GFP-RNAi

<400> 122

```
aattcgtttc tccataataa tgtgtgagta gttcccagat aagggaatta gggttcctat 60
agggtttcgc tcatgtgttg agcatataag aaacccttag tatgtatttg tatttgtaaa 120
atacttctat caataaaatt tctaattcct aaaaccaaaa tccagtacta aaatccagat 180
cccccgaatt aattcggcgt taattcagca attcgtaatc atggtcatag ctgtttcctg 240
tgtgaaattg ttatccgctc acaattccac acaacatacg agccggaagc ataaagtgta 300
aagcctgggg tgcctaatga gtgagctaac tcacattaat tgcgttgcgc tcactgcccg 360
ctttccagtc gggaaacctg tcgtgccagc tgcattaatg aatcggccaa cgcgcgggga 420
gaggcggttt gcgtattggc tagagcagct tgccaacatg gtggagcacg acactctcgt 480
ctactccaag aatatcaaag atacagtctc agaagaccaa agggctattg agacttttca 540
acaaagggta atatcgggaa acctcctcgg attccattgc ccagctatct gtcacttcat 600
caaaaggaca gtagaaaagg aaggtggcac ctacaaatgc catcattgcg ataaaggaaa 660
ggctatcgtt caagatgcct ctgccgacag tggtcccaaa gatggacccc cacccacgag 720
gagcatcgtg gaaaaagaag acgttccaac cacgtcttca aagcaagtgg attgatgtga 780
taacatggtg gagcacgaca ctctcgtcta ctccaagaat atcaaagata cagtctcaga 840
agaccaaagg gctattgaga cttttcaaca aagggtaata tcgggaaacc tcctcggatt 900
ccattgccca gctatctgtc acttcatcaa aaggacagta gaaaaggaag gtggcaccta 960
caaatgccat cattgcgata aaggaaaggc tatcgttcaa gatgcctctg ccgacagtgg 1020
tcccaaagat ggacccccac ccacgaggag catcgtggaa aaagaagacg ttccaaccac 1080
gtcttcaaag caagtggatt gatgtgatat ctccactgac gtaagggatg acgcacaatc 1140
ccactatcct tcgcaagacc ttcctctata taaggaagtt catttcattt ggagaggaca 1200
cgctgaaatc accagtctct ctctacaaat ctatctctct cgagtctacc atgagcccag 1260
aacgacgccc ggccgacatc cgccgtgcca ccgaggcgga catgccggcg gtctgcacca 1320
tcgtcaacca ctacatcgag acaagcacgg tcaacttccg taccgagccg caggaaccgc 1380
```

aggagtggac ggacgacctc gtccgtctgc gggagcgcta tccctggctc gtcgccgagg 1440
tggacggcga ggtcgccggc atcgcctacg cgggcccctg gaaggcacgc aacgcctacg 1500
actggacggc cgagtcgacc gtgtacgtct ccccccgcca ccagcggacg ggactgggct 1560
ccacgctcta cacccacctg ctgaagtccc tggaggcaca gggcttcaag agcgtggtcg 1620
ctgtcatcgg gctgcccaac gacccgagcg tgcgcatgca cgaggcgctc ggatatgccc 1680
cccgcggcat gctgcgggcg gccggcttca agcacgggaa ctggcatgac gtgggtttct 1740
ggcagctgga cttcagcctg ccggtaccgc cccgtccggt cctgcccgtc accgagattt 1800
gactcgagtt tctccataat aatgtgtgag tagttcccag ataagggaat tagggttcct 1860
atagggtttc gctcatgtgt tgagcatata agaaaccctt agtatgtatt tgtatttgta 1920
aaatacttct atcaataaaa tttctaattc ctaaaaccaa aatccagtac taaaatccag 1980
atcccccgaa ttaattcggc gttaattcag tacattaaaa acgtccgcaa tgtgttatta 2040
agttgtctaa gcgtcaattt gtttacacca caatatatcc tgccaccagc cagccaacag 2100
ctccccgacc ggcagctcgg cacaaaatca ccactcgata caggcagccc atcagtccgg 2160
gacggcgtca gcgggagagc cgttgtaagg cggcagactt tgctcatgtt accgatgcta 2220
ttcggaagaa cggcaactaa gctgccgggt ttgaaacacg gatgatctcg cggagggtag 2280
catgttgatt gtaacgatga cagagcgttg ctgcctgtga tcaccgcggt ttcaaaatcg 2340
gctccgtcga tactatgtta tacgccaact ttgaaaacaa ctttgaaaaa gctgttttct 2400
ggtatttaag gttttagaat gcaaggaaca gtgaattgga gttcgtcttg ttataattag 2460
cttcttgggg tatctttaaa tactgtagaa aagaggaagg aaataataaa tggctaaaat 2520
gagaatatca ccggaattga aaaaactgat cgaaaaatac cgctgcgtaa aagatacgga 2580
aggaatgtct cctgctaagg tatataagct ggtgggagaa aatgaaaacc tatatttaaa 2640
aatgacggac agccggtata aagggaccac ctatgatgtg gaacgggaaa aggacatgat 2700
gctatggctg gaaggaaagc tgcctgttcc aaaggtcctg cactttgaac ggcatgatgg 2760
ctggagcaat ctgctcatga gtgaggccga tggcgtcctt tgctcggaag agtatgaaga 2820
tgaacaaagc cctgaaaaga ttatcgagct gtatgcggag tgcatcaggc tctttcactc 2880
catcgacata tcggattgtc cctatacgaa tagcttagac agccgcttag ccgaattgga 2940
ttacttactg aataacgatc tggccgatgt ggattgcgaa aactgggaag aagacactcc 3000
atttaaagat ccgcgcgagc tgtatgattt tttaaagacg gaaaagcccg aagaggaact 3060
tgtcttttcc cacggcgacc tgggagacag caacatcttt gtgaaagatg gcaaagtaag 3120
tggctttatt gatcttggga gaagcggcag ggcggacaag tggtatgaca ttgccttctg 3180
cgtccggtcg atcagggagg atatcgggga agaacagtat gtcgagctat tttttgactt 3240
actggggatc aagcctgatt gggagaaaat aaaatattat attttactgg atgaattgtt 3300
ttagtaccta gaatgcatga ccaaaatccc ttaacgtgag ttttcgttcc actgagcgtc 3360
agaccccgta gaaaagatca aaggatcttc ttgagatcct ttttttctgc gcgtaatctg 3420
ctgcttgcaa acaaaaaaac caccgctacc agcggtggtt tgtttgccgg atcaagagct 3480
accaactctt tttccgaagg taactggctt cagcagagcg cagataccaa atactgtcct 3540
tctagtgtag ccgtagttag gccaccactt caagaactct gtagcaccgc ctacatacct 3600
cgctctgcta atcctgttac cagtggctgc tgccagtggc gataagtcgt gtcttaccgg 3660
gttggactca agacgatagt taccggataa ggcgcagcgg tcgggctgaa cggggggttc 3720
gtgcacacag cccagcttgg agcgaacgac ctacaccgaa ctgagatacc tacagcgtga 3780
gctatgagaa agcgccacgc ttcccgaagg gagaaaggcg gacaggtatc cggtaagcgg 3840
cagggtcgga acaggagagc gcacgaggga gcttccaggg ggaaacgcct ggtatcttta 3900
tagtcctgtc gggtttcgcc acctctgact tgagcgtcga tttttgtgat gctcgtcagg 3960
ggggcggagc ctatggaaaa acgccagcaa cgcggccttt ttacggttcc tggccttttg 4020
ctggcctttt gctcacatgt tctttcctgc gttatcccct gattctgtgg ataaccgtat 4080
taccgccttt gagtgagctg ataccgctcg ccgcagccga acgaccgagc gcagcgagtc 4140
agtgagcgag gaagcggaag agcgcctgat gcggtatttt ctccttacgc atctgtgcgg 4200
tatttcacac cgcatatggt gcactctcag tacaatctgc tctgatgccg catagttaag 4260
ccagtataca ctccgctatc gctacgtgac tgggtcatgg ctgcgccccg acacccgcca 4320
acacccgctg acgcgccctg acgggcttgt ctgctcccgg catccgctta cagacaagct 4380
gtgaccgtct ccgggagctg catgtgtcag aggttttcac cgtcatcacc gaaacgcgcg 4440
aggcagggtg ccttgatgtg ggcgccggcg gtcgagtggc gacggcgcgg cttgtccgcg 4500
ccctggtaga ttgcctggcc gtaggccagc catttttgag cggccagcgg ccgcgatagg 4560
ccgacgcgaa gcggcggggc gtagggagcg cagcgaccga agggtaggcg ctttttgcag 4620
ctcttcggct gtgcgctggc cagacagtta tgcacaggcc aggcgggttt taagagtttt 4680
aataagtttt aaagagtttt aggcggaaaa atcgcctttt ttctctttta tatcagtcac 4740
ttacatgtgt gaccggttcc caatgtacgg ctttgggttc ccaatgtacg ggttccggtt 4800

cccaatgtac ggctttgggt tcccaatgta cgtgctatcc acaggaaaga gacctttttcg 4860
accttttttcc cctgctaggg caatttgccc tagcatctgc tccgtacatt aggaaccggc 4920
ggatgcttcg ccctcgatca ggttgcggta gcgcatgact aggatcgggc cagcctgccc 4980
cgcctcctcc ttcaaatcgt actccggcag gtcatttgac ccgatcagct tgcgcacggt 5040
gaaacagaac ttcttgaact ctccggcgct gccactgcgt tcgtagatcg tcttgaacaa 5100
ccatctggct tctgccttgc ctgcggcgcg gcgtgccagg cggtagagaa aacggccgat 5160
gccgggatcg atcaaaaagt aatcggggtg aaccgtcagc acgtccgggt tcttgccttc 5220
tgtgatctcg cggtacatcc aatcagctag ctcgatctcg atgtactccg gccgcccggt 5280
ttcgctcttt acgatcttgt agcggctaat caaggcttca ccctcggata ccgtcaccag 5340
gcggccgttc ttggccttct tcgtacgctg catggcaacg tgcgtggtgt ttaaccgaat 5400
gcaggtttct accaggtcgt ctttctgctt tccgccatcg gctcgccggc agaacttgag 5460
tacgtccgca acgtgtggac ggaacacgcg gccgggcttg tctcccttcc cttcccggta 5520
tcggttcatg gattcggtta gatgggaaac cgccatcagt accaggtcgt aatcccacac 5580
actggccatg ccggccggcc ctgcggaaac ctctacgtgc ccgtctggaa gctcgtagcg 5640
gatcacctcg ccagctcgtc ggtcacgctt cgacagacgg aaaacggcca cgtccatgat 5700
gctgcgacta tcgcgggtgc ccacgtcata gagcatcgga acgaaaaaat ctggttgctc 5760
gtcgcccttg ggcggcttcc taatcgacgg cgcaccggct gccggcggtt gccgggattc 5820
tttgcggatt cgatcagcgg ccgcttgcca cgattcaccg gggcgtgctt ctgcctcgat 5880
gcgttgccgc tgggcggcct gcgcggcctt caacttctcc atcaggtcat cacccagcgc 5940
cgcgcccgatt tgtaccgggc cggatggttt gcgaccgtca cgccgattcc tcgggcttgg 6000
gggttccagt gccattgcag ggccggcaga caacccagcc gcttacgcct ggccaaccgc 6060
ccgttcctcc acacatgggg cattccacgg cgtcggtgcc tggttgttct tgattttcca 6120
tgccgcctcc tttagccgct aaaattcatc tactcattta ttcatttgct catttactct 6180
ggtagctgcg cgatgtattc agatagcagc tcggtaatgg tcttgccttg gcgtaccgcg 6240
tacatcttca gcttggtgtg atcctccgcc ggcaactgaa agttgacccg cttcatggct 6300
ggcgtgtctg ccaggctggc caacgttgca gccttgctgc tgcgtgcgct cggacggccg 6360
gcacttagcg tgtttgtgct tttgctcatt ttctctttac ctcattaact caaatgagtt 6420
ttgatttaat ttcagcggcc agcgcctgga cctcgcgggc agcgtcgccc tcgggttctg 6480
attcaagaac ggttgtgccg gcggcggcag tgcctgggta gctcacgcgc tgcgtgatac 6540
gggactcaag aatgggcagc tcgtacccgg ccagcgcctc ggcaacctca ccgccgatgc 6600
gcgtgccttt gatcgcccgc gacacgacaa aggccgcttg tagccttcca tccgtgacct 6660
caatgcgctg cttaaccagc tccaccaggt cggcggtggc ccatatgtcg taagggcttg 6720
gctgcaccgg aatcagcacg aagtcggctg ccttgatcgc ggacacagcc aagtccgccg 6780
cctgggggcgc tccgtcgatc actacgaagt cgcgccggcc gatggccttc acgtcgcggt 6840
caatcgtcgg gcggtcgatg ccgacaacgg ttagcggttg atcttcccgc acggccgccc 6900
aatcgcgggc actgccctgg ggatcggaat cgactaacag aacatcggcc ccggcgagtt 6960
gcagggcgcg ggctagatgg gttgcgatgg tcgtcttgcc tgacccgcct ttctggttaa 7020
gtacagcgat aaccttcatg cgttcccctt gcgtatttgt ttatttactc atcgcatcat 7080
atacgcagcg accgcatgac gcaagctgtt ttactcaaat acacatcacc tttttagacg 7140
gcggcgctcg gtttcttcag cggccaagct ggccggccag gccgccagct tggcatcaga 7200
caaaccggcc aggatttcat gcagccgcac ggttgagacg tgcgcgggcg gctcgaacac 7260
gtacccggcc gcgatcatct ccgcctcgat ctcttcggta atgaaaaacg gttcgtcctg 7320
gccgtcctgg tgcggtttca tgcttgttcc tcttggcgtt cattctcggc ggccgccagg 7380
gcgtcggcct cggtcaatgc gtcctcacgg aaggcaccgc gccgcctggc ctcggtgggc 7440
gtcacttcct cgctgcgctc aagtgcgcgg tacagggtcg agcgatgcac gccaagcagt 7500
gcagccgcct ctttcacggt gcggccttcc tggtcgatca gctcgcgggc gtgcgcgatc 7560
tgtgccgggg tgagggtagg gcgggggcca aacttcacgc ctcgggcctt ggcggcctcg 7620
cgcccgctcc gggtgcggtc gatgattagg gaacgctcga actcggcaat gccggcgaac 7680
acggtcaaca ccatgcggcc ggccggcgtg gtggtgtcgg cccacggctc tgccaggcta 7740
cgcaggcccg cgccggcctc ctggatgcgc tcggcaatgt ccagtaggtc gcgggtgctg 7800
cgggccaggc ggtctagcct ggtcactgtc acaacgtcgc cagggcgtag gtggtcaagc 7860
atcctggcca gctccgggcg gtcgcgcctg gtgccggtga tcttctcgga aaacagcttg 7920
gtgcagccgg ccgcgtgcag ttcggcccgt tggttggtca agtcctggtc gtcggtgctg 7980
acgcgggcat agcccagcag gccagcggcg gcgctcttgt tcatggcgta atgtctccgg 8040
ttctagtcgc aagtattcta ctttatgcga ctaaaacacg cgacaagaaa acgccaggaa 8100
aagggcaggg cggcagcctg tcgcgtaact taggacttgt gcgacatgtc gttttcagaa 8160
gacggctgca ctgaacgtca gaagccgact gcactatagc agcggagggg ttggatcaaa 8220

gtactttgat cccgagggga accctgtggt tggcatgcac atacaaatgg acgaacggat 8280
aaacctttc acgccctttt aaatatccgt tattctaata aacgctcttt tctcttaggt 8340
ttacccgcca atatatcctg tcaaacactg atagtttaaa ctgaaggcgg gaaacgacaa 8400
tctgatccaa gctcaagctg ctctagcatt cgccattcag gctgcgcaac tgttgggaag 8460
ggcgatcggt gcgggcctct tcgctattac gccagctggc gaaaggggga tgtgctgcaa 8520
ggcgattaag ttgggtaacg ccagggtttt cccagtcacg acgttgtaaa acgacggcca 8580
gtgccaagct tttggctaga gcagcttgcc aacatggtgg agcacgacac tctcgtctac 8640
tccaagaata tcaaagatac agtctcagaa gaccaaaggg ctattgagac ttttcaacaa 8700
agggtaatat cgggaaacct cctcggattc cattgcccag ctatctgtca cttcatcaaa 8760
aggacagtag aaaaggaagg tggcacctac aaatgccatc attgcgataa aggaaaggct 8820
atcgttcaag atgcctctgc cgacagtggt cccaaagatg gacccccacc cacgaggagc 8880
atcgtggaaa aagaagacgt tccaaccacg tcttcaaagc aagtggattg atgtgataac 8940
atggtggagc acgacactct cgtctactcc aagaatatca aagatacagt ctcagaagac 9000
caaagggcta ttgagacttt tcaacaaagg gtaatatcgg gaaacctcct cggattccat 9060
tgcccagcta tctgtcactt catcaaaagg acagtagaaa aggaaggtgg cacctacaaa 9120
tgccatcatt gcgataaagg aaaggctatc gttcaagatg cctctgccga cagtggtccc 9180
aaagatggac ccccacccac gaggagcatc gtggaaaaag aagacgttcc aaccacgtct 9240
tcaaagcaag tggattgatg tgatatctcc actgacgtaa gggatgacgc acaatcccac 9300
tatccttcgc aagacccttcc tctatataag gaagttcatt tcatttggag aggacacgct 9360
gaaatcacca gtctctctct acaaatctat ctctccatgg catgttctgc aggtcgactc 9420
tagaggatcc ccgggtaccg agctcgaaga tcttcgacgt cggaattcat ggactcaaag 9480
tcggttactc cagaaccaac caaccccttc tacgcttctt cggggcaatc aggaaaaacc 9540
tatgcttctg ttgtcgccgc cgctgctgct gcagccgccg ataaggagga tggtggtgct 9600
gtgagtagtg ccaaggagtt ggattcctca tcggaggctg tgtctggtaa ttcggataag 9660
gttggagctg atgatttatc tgactccgag aaggagaagc cgaatttggt gggtgatggg 9720
aaggtttccg acgaggtgga tggttcttta aaggaggatt ctactactcc tgaggctact 9780
ccgaagcctg aggtggtttc tggtgagaca attggtgtag atgatgtttc atcgttatct 9840
ccgaagccgg aggctgtttc tgatggtgta ggggttgtgg aggagaataa gaaggttaag 9900
gaggacgtgg aggatattaa agacgatggt gagagtaaga ttgaaaatgg gagtgttgat 9960
gttgatgtga aacaggcttc cacagatggg gagagtgaga aagcttccaa cacttgtcac 10020
tactttctct tatggtgttc aatgcttttc aagataccca gatcatatga aacggcatga 10080
cttcttcaag agcgccatgc ctgagggata cgtgcaggag aggaccatct tcttcaagga 10140
cgacgggaac tacaagacac gtgctgaagt caagtttgag ggagacaccc tcgtcaacag 10200
gatcgagctt aagggaatcg atttcaagga ggacggaaac atcctcggcc acaagttgga 10260
atacaactac aactcccaca acgtatacat catggccgac aagcaaaaga acggcatcaa 10320
agccaacttc aagacccgcc acaacatcga agacggcggc gtgcaactcg ctgatcatta 10380
tcaacaaaat actccaattg gcgatggccc tgtcctttta ccagacaacc attacctgtc 10440
cacacaatct gccctttcga aagatcccac cgaaaagaga gaccacatgg tccttcttga 10500
gtttgtaaca gctgctggga ttacacatgg catggatgaa ctatacaaac atgatgagct 10560
ttaaggatcc ttaaagctca tcatgtttgt atagttcatc catgccatgt gtaatcccag 10620
cagctgttac aaactcaaga aggaccatgt ggtctctctt ttcggtggga tctttcgaaa 10680
gggcagattg tgtggacagg taatggttgt ctggtaaaag gacagggcca tcgccaattg 10740
gagtattttg ttgataatga tcagcgagtt gcacgccgcc gtcttcgatg ttgtggcggg 10800
tcttgaagtt ggctttgatg ccgttctttt gcttgtcggc catgatgtat acgttgtggg 10860
agttgtagtt gtattccaac ttgtggccga ggatgtttcc gtcctccttg aaatcgattc 10920
ccttaagctc gatcctgttg acgagggtgt ctccctcaaa cttgacttca gcacgtgtct 10980
tgtagttccc gtcgtccttg aagaagatgg tcctctcctg cacgtatccc tcaggcatgg 11040
cgctcttgaa gaagtcatgc cgtttcatat gatctgggta tcttgaaaag cattgaacac 11100
cataagagaa agtagtgaca agtgttggaa gctttctcac tctccccatc tgtggaagcc 11160
tgtttcacat caacatcaac actcccattt tcaatcttac tctcaccatc gtctttaata 11220
tcctccacgt cctccttaac cttcttattc tcctccacaa cccctacacc atcagaaaca 11280
gcctccggct tcggagataa cgatgaaaca tcatctacac caattgtctc accagaaacc 11340
acctcaggct tcggagtagc ctcaggagta gtagaatcct cctttaaaga accatccacc 11400
tcgtcggaaa ccttcccatc acccaccaaa ttcggcttct ccttctcgga gtcagataaa 11460
tcatcagctc caaccttatc cgaattacca gacacagcct ccgatgagga atccaactcc 11520
ttggcactac tcacagcacc accatcctcc ttatcggcgg ctgcagcagc agcggcggcg 11580

acaacagaag catagggtttt tcctgattgc cccgaagaag cgtagaaggg gttggttggt 11640
tctggagtaa ccgactttga gtccatg 11667

<210> 123
<211> 36
<212> DNA
<213> Künstliche Sequenz

<220>
<223> Beschreibung der künstlichen Sequenz: oligonucleotide primer

<400> 123
ctcgaggaat tcatggactc aaagtcggtt actcca 36

<210> 124
<211> 40
<212> DNA
<213> Künstliche Sequenz

<220>
<223> Beschreibung der künstlichen Sequenz: oligonucleotide primer

<400> 124
ggatccataa gcaagctttc tcactctccc catctgtgga 40

<210> 125
<211> 26
<212> DNA
<213> Künstliche Sequenz

<220>
<223> Beschreibung der künstlichen Sequenz: oligonucleotide primer

<400> 125
aagcttccaa cacttgtcac tacttt 26

<210> 126
<211> 26
<212> DNA
<213> Künstliche Sequenz

<220>
<223> Beschreibung der künstlichen Sequenz: oligonucleotide primer

<400> 126
ggatccttaa agctcatcat gtttgt 26

## Beschreibung

Die gezielte Inhibition der Genexpression definierter Gene ist eine der am meisten beforschten Technologie der Biotechnologie. Die Expression von antisense-RNA ist dabei der am häufigsten verwendet Ansatz und vielfach beschrieben (u.a. EP-A1 0 458 367; EP-A1 0 140 308; van der Krol AR et al. (1988) BioTechniques 6(10):658-676; de Lange P et al. (1995) Curr Top Microbiol Immunol 197:57-75). Antisense-RNA vermittelte Ansätze haben jedoch den Nachteil, dass stöchiometrische Mengen der antisense-RNA erforderlich sind, um eine wirksame Inhibition der Ziel-mRNA zu bewirken. Weitere Probleme stehen im Zusammenhang mit dem Einbringen der antisense-RNA in ausreichenden Mengen in die Zellen und mit der Labilität der antisense-RNA. Ansätze basierend auf antisense-RNA sind daher meist ineffizient.

Ein weiterer Ansatz zur Genregulation ist die "Co-Suppression" und meint die Verminderung der Expression eines endogenen Zielgens durch transgene Expression einer sense-RNA dieses Zielgens (EP-A1 0 465 572). Der Co-Suppression liegen vermutlich mehr als ein Mechanismus zugrunde. Nachteilig ist die mangelnde Verlässlichkeit und Reproduzierbarkeit des Verfahrens. In manchen Fällen erfolgt Suppression, während in anderen Fällen - bedingt durch die Expression der sense-RNA - die erwartete Überexpression erfolgt. Auch ist der erhaltene Phänotyp oft nicht stabil. Die Anwendung der Co-Suppression ist im wesentlichen auf Pflanzen beschränkt.

Verschiedene Abwandlungen der Verfahren basierend auf antisense-RNA oder Cosuppression sind bekannt. So beschreibt WO 93/23551 ein Verfahren zur Inhibition mehrerer Gene durch Expression einer chimären antisense-RNA oder sense-RNA. Das Verfahren kann die üblichen mit antisense-RNA oder sense-RNA verbundenen Probleme nicht lösen und bleibt ineffizient.

WO 98/36083 und WO 99/15682 beschreiben die Regulation der Genexpression mittels viraler Expressionssysteme ("virus induced gene silencing" VIGS).

WO 99/32619 und WO 99/53050 beschreiben Verfahren zur Inhibition einzelner Zielgene unter Verwendung einer RNA mit doppelsträngiger Struktur, wobei das Zielgen und die Region der RNA Duplex zumindest eine teilweise Identität aufweisen (siehe auch: Montgomery MK et al. (1998) Proc Natl Acad Sci USA 95:15502- 15507; Sharp PA (1999) Genes & Development 13(2):139-141; Fire A et al. (1998) Nature 391:806-11). Das Verfahren wird heute auch als "RNA-Interference" (RNAi) bezeichnet und hat in Mechanismus und Wirkung Ähnlichkeiten mit dem oben erwähnten VIGS Verfahren.

Die beschriebenen Verfahren, insbesondere das RNAi-Verfahren, lösen zwar einige Probleme im Zusammenhang mit der Verminderung einzelner Zielgene. Für andere Probleme, insbesondere für die parallele Suppression mehrerer Zielgene, konnte jedoch bislang keine befriedigende Lösung bereit gestellt werden. Zahlreiche Ansätze in der Biotechnologie erfordern nicht nur die Verminderung eines einzelnen Zielgens, sondern mehrerer Zielgene, wie beispielsweise verschiedener Gene eines oder verschiedener Stoffwechselwege oder ganzer Genfamilien. Bislang war dies nur mit erheblichen Arbeits- und Zeitaufwand zu realisieren. Die Ansätze erforderten oft die individuelle Regulation der einzelnen Zielgene durch sukzessive Transformation beispielsweise mit verschiedenen Expressionskonstrukten, die jeweils für eine antisense RNA eines Zielgens kodierten. Neben dem erheblichen Arbeits- und Zeitaufwand, besteht dabei der Nachteil, das für viele Systeme und Organismen nur eine beschränkte Anzahl von Selektionsmarkern, geeigneten Promotoren etc. zur Verfügung steht, was multiple Transformationen erheblich erschwert und beispielsweise die Deletion der Marker nach der Transformation und Selektion erfordert. Die mehrfache Verwendung eines Promotors hat oft unerwünschte Folgen, wie beispielsweise ein epigenetisches Gene-Silencing. Hierbei kommt es infolge der mehrfach verwendeten Kontrollsequenzen zu einer Inaktivierung derselben, vergleichbar der oben beschriebenen Cosuppression.

Es stellte sich also die Aufgabe, neue Verfahren bereit zu stellen, die eine effizient Verminderung der Expression mindestens zweier endogener Zielgene in einem pflanzlichen Organismus ermöglichen. Diese Aufgabe wird durch die vorliegende Erfindung gelöst.

Die Erfindung betrifft ein Verfahren zur Verminderung der Expression von mindestens zwei verschiedenen, endogenen Zielgenen in einem pflanzlichen Organismus durch Einbringen eines zumindest teilweise doppelsträngigen Ribonukleinsäuremoleküls in besagten pflanzlichen Organismus, wobei das doppelsträngige Ribonukleinsäuremolekül aus einem ganz oder teilweise selbstkomplementären RNA Strang besteht, welcher umfasst

a. mindestens zwei "sense"-Ribonukleotidsequenzen, wobei jeweils mindestens eine dieser "sense"-Ribonukleotidsequenzen im wesentlichen identisch ist zu mindestens einem Teil des "sense"-RNA-Transkriptes eines jeden der besagten endogenen Zielgene und

b. "antisense"-Ribonukleotidsequenzen, die zu besagten "sense"-Ribonukleotidsequenzen unter a) im wesentlichen komplementären sind,

wobei zunächst die "sense"-Ribonukleotidsequenzen a) aufeinander folgen, und dann eine Aneinanderreihung der im wesentlichen komplementären "antisense"-Ribonukleotidsequenzen b) folgt, und wobei der RNA-Strang eine einzelne Haarnadel ausbildet, welche nachfolgende Primärstruktur hat

$$5'\text{-}S(1)\text{-}S(2)\text{-}.....\text{-}S(n)\text{-}AS(n)\text{-}....\text{-}AS(2)\text{-}AS(1)\text{-}3'$$

in welcher S die "sense"-Ribonukleotidsequenzen, AS die "antisense"-Ribonukleotidsequenzen darstellen, und wobei die Anzahl der Einheiten n größer oder gleich zwei ist,
und wobei der pflanzliche Organismus zu einer Pflanzengattung gehört, ausgewählt aus Cucurbita, Rosa, Vitis, Juglans, Fragaria, Lotus, Medicago, Onobrychis, Trifolium, Trigonella, Vigna, Citrus, Linum, Geranium, Manihot, Daucus, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus.

Die vorliegende Erfindung löst die oben geschilderten Probleme und ermöglicht eine schnelle, besonders effiziente Methode zur Regulation der Expression verschiedener Zielgene. Insbesondere ergeben sich folgende Vorteile:

a) Transgene pflanzliche Organismen gemäß Anspruch 1, in denen mehr als ein Zielgen inhibiert wird, können in einer einzigen Transformation erzeugt werden.

b) Die Transkriptionsrate für jeden Ribonukleotidsequenz der dsRNA ist gleich. Dadurch werden multiple Phänotypen durch unterschiedliche Expressionshöhen verhindert, wie sie bei individueller Expression separater Ribonukleotidsequenzen - beispielsweise durch den unterschiedlichen Ort der Insertion in das Genom - oft entstehen. Dieser Vorteil gewährleistet eine gleichbleibend hohe Inhibition aller Zielgene und vermindert dramatisch die erforderlichen Selektionsschritte zu Generierung eines Organismus gemäß Anspruch 1, bei dem alle Zielgene effizient supprimiert werden.

c) Ein ökonomischer Umgang mit Kontrollelementen wie Promotoren und Selektionsmarkern wird ermöglicht. Zudem erübrigen sich Probleme, wie sie bei der mehrfachen Verwendung eines bestimmten Kontrollelementes, insbesondere eines Promoters, entstehen können ("epigenic gene silencing").

d) Eine Segregation der einzelnen Ribonukleotidsequenzen bei nachfolgenden Züchtungs- und Kreuzungsschritten, wie sie bei der Verwendung mehrerer Expressionskonstrukte zwangsläufig entsteht, wird verhindert. Dadurch wird die nachfolgende Züchtung stabiler Linien erheblich erleichtert und beschleunigt.

e) Pflanzliche Organismen gemäß Anspruch 1 mit komplexen beispielsweise polyploide Genomen, wie beispielsweise manche Pflanzen, sind einer effizienten Gensuppression zugänglich. Aufgrund der zahlreichen Kopien für einzelne Gene sind diese Organismen klassischen verfahren der Mutagenese und Selektion nicht zugänglich.

Überraschenderweise konnte bei dem erfindungsgemäßen Verfahren keine störende Interferenz zwischen den einzelnen Ribonukleotidsequenzabschnitte untereinander beobachtet werden.

"Endogenes Zielgen eines pflanzlichen Organismus" meint jede Nukleinsäuresequenz in einem pflanzlichen Organismus gemäß Anspruch 1 oder einem Teil, Organ, Gewebe, Samen etc. desselben, die zur Transkription befähigt ist. Dabei kann es sich um natürlicherweise vorkommende oder aber künstlich eingeführte Sequenzen (wie beispielsweise transgene Sequenzen) handeln, wobei natürlicherweise vorkommende Sequenzen bevorzugt sind. Natürlicherweise vorkommende Sequenzen sind bevorzugt und umfassen sowohl die eigenen Sequenzen des pflanzlichen Organismus als auch Gene von Pathogenen, die in dem pflanzlichen Organismus nach einem Befall durch ein Pathogen präsent sind. Das Zielgen kann in der chromosomalen DNA oder der DNA der Organellen (wie beispielsweise der Plastiden z.B. Chloroplasten etc.) lokalisiert sein oder aber sich extrachromosomal in der Zelle befinden. Die natürlicherweise vorkommenden, eigenen Sequenzen des pflanzlichen Organsimus umfassen bevorzugt Gene desselben, die stabil im Genom vorliegen, wobei das Genom die Gesamtheit der genetischen Information meint und sowohl die chromosomale als auch die plastidäre DNA umfasst. Bevorzugt ist das endogene Zielgen ein natürlicherweise in der chromosomalen DNA vorkommendes Gen. Bevorzugt sind Gene deren verminderte Expression zu einem veränderten Phänotyp führt.

"Verminderung" oder "vermindern" der Expression eines Zielgens ist im Zusammenhang weit auszulegen und umfasst die teilweise oder im wesentlichen vollständige, auf unterschiedliche zellbiologische Mechanismen beruhende Unterbindung oder Blockierung der Expression des Zielgens oder der von ihm abgeleiteten RNA, mRNA, rRNA, tRNA und/oder des dadurch kodierten Proteinproduktes in einer Zelle oder einem Organismus oder einem davon abgeleiteten Teil, Gewebe, Organ, Zelle oder Samen. Eine Verminderung im Sinne der Erfindung umfasst die mengenmässige Verringerung einer vom Zielgen exprimierten RNA, mRNA, rRNA, tRNA und/oder des dadurch kodierten Proteinproduktes bis hin zu einem im wesentlichen vollständigen Fehlen derselben. Dabei wird die Expression einer bestimmten RNA, mRNA, rRNA, tRNA und/oder des dadurch kodierten Proteinproduktes in einer Zelle oder einem Organismus im Vergleich zu der selben Zelle oder Organismus, die dem Verfahren nicht unterworfen wurden, bevorzugt um mehr als 50%, besonders bevorzugt um mehr als 80%, ganz besonders bevorzugt um mehr als 90%, am meisten bevorzugt mehr al 95% vermindert. Dabei kann die Verminderung durch dem Fachmann geläufigen Verfahren ermittelt werden. So kann die Verminderung der Proteinmenge beispielsweise durch immunologischen Nachweis des Proteins bestimmt werden. Weiterhin können biochemische Techniken wie Northern-Hybridisierung, "nuclease protection assay", Reverse Transkription (quantitative RT-PCR), ELISA ("enzyme linked immunosorbent assay"), Western-Blotting, Radioimmunoassay (RIA) oder andere Immunoassays sowie "fluorescence activated cell analysis" (FACS) eingesetzt werden. Je nach Art des verminderten Proteinproduktes kann auch dess Aktivität oder der Einfluss auf den Phänotyp des Organismus oder der Zelle ermittelt werden.

"Proteinmenge" meinte die Menge eines bestimmten Polypeptides in einem Organismus, einem Gewebe, einer Zelle oder einem Zellkompartiment.

"Verminderung" der Proteinmenge meint die mengenmäßige Verminderung der Menge eines bestimmten Polypeptides in einem Organismus, einem Gewebe, einer Zelle oder einem Zellkompartiment - beispielsweise durch das erfindungsgemäße Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter, Nährstoffzufuhr etc.). Der Verminderung beträgt dabei mindestens 10 %, bevorzugt mindestens 10% oder mindestens 20%, besonders bevorzugt um mindestens 40% oder 60%, ganz besonders bevorzugt um mindestens 70% oder 80%, am meisten bevorzugt um mindestens 90% oder 95%. Verfahren zur Bestimmung der Proteinmenge sind dem Fachmann bekannt. Beispielhaft seien zu nennen: Das Mikro-Biuret Verfahren (Goa J (1953) Scand J Clin Lab Invest 5:218-222), die Folin-Ciocalteu-Methode (Lowry OH et al. (1951) J Biol Chem 193:265-275) oder die Messung der Adsorption von CBB G-250 (Bradford MM (1976) Analyt Biochem 72:248-254).

"Verschieden" meint in Bezug auf zwei endogene Zielgene bevorzugt, dass die von den beiden endogenen Zielgenen transkribierte RNA oder mRNA nicht identisch ist. Bevorzugt ist die Homologie der von den beiden endogenen Zielgenen transkribierte RNA oder mRNA geringer als 90%, bevorzugt geringer als 80%, besonders bevorzugt geringer als 70%, ganz besonders bevorzugt geringer als 60%, am meisten bevorzugt geringer als 50% über jeweils die gesamte Länge der transkribierten RNA oder mRNA.

"Zumindest teilweise doppelsträngiges Ribonukleinsäuremolekül" (infolge dsRNA) meint Ribonukleinsäuremolekül, die ganz oder teilweise doppelsträngig sind. Bevorzugt ist die Ribonukleinsäuresequenz überwiegend vollständig doppelsträngig. "Überwiegend vollständig doppelsträngig" meint, dass zumindest 50%, bevorzugt 70%, besonders bevorzugt 80%, ganz besonders bevorzugt 90% der in dem Molekül vorhandenen Basen in Paarung mit einer anderen Base der dsRNA vorliegen oder - entsprechend der Sequenz der dsRNA und den Basenpaarregeln sowie gegebenenfalls einer RNA-Sekundärstrukturvoraussage mittels eines geeigneten Computeralgorithmus - zumindest theoretisch vorliegen können.

"Im wesentlichen identisch" meint, dass eine "sense"-Ribonukleotidsequenz der dsRNA auch Insertionen, Deletionen sowie einzelne Punktmutationen im Vergleich zu der Sequenz des "sense"-RNA-Transkriptes eines endogenen Zielgens aufweisen kann. Mutationen umfassen Substitutionen, Additionen, Deletionen, Inversion oder Insertionen eines oder mehrerer Basen einer Nukleinsäuresequenz. Bevorzugt beträgt die Homologie zwischen einer "sense"-Ribonukleotidsequenz einer dsRNA und mindestens einem Teil des "sense"-RNA-Transkript eines endogenen Zielgens mindestens 60 %, bevorzugt mindestens 70 %, ganz besonders bevorzugt mindestens 90 %, am meisten bevorzugt 95%. Die Sequenzen können auch identisch mit der korrespondierenden Sequenz des Zielgens sein. Eine 100%ige Sequenzidentität zwischen der "sense"-Ribonukleotidsequenz der dsRNA und mindestens einem Teil des "sense"-Stranges der Transkriptes eines endogenen Gens ist bevorzugt, wenn gleich nicht zwingend erforderlich, um eine effiziente Verminderung der Expression des endogenen Gens zu bewirken. Einzelne Mutationen werden toleriert. Das Verfahren ist demnach tolerant gegenüber Sequenzabweichungen, wie sie infolge genetischer Mutationen, Polymorphismen oder evolutionärer Divergenzen vorliegen können. So ist es beispielsweise auch möglich mit einer einzigen dsRNA, die ausgehend von einer bestimmten endogenen Gen generiert wurde, die Expression weiterer homologer endogener Gene des gleichen Organismus oder aber auch die Expression homologer endogener Gene in anderen verwandten Arten zu unterdrücken.

Unter Homologie wird das Maß der Übereinstimmung zwischen zwei Nukleotid-, Ribonukleotid- oder Proteinsequenzen verstanden, die bevorzugt durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:

Gap Weight: 50 Length Weight: 3

(fortgesetzt)

Average Match: 10 Average Mismatch:0

Dem Fachmann ist bewusst, dass wenn die Homologie zwischen DNA (z.B. Genen) und RNA bestimmt wird, Thymin (T) in der DNA Sequenz als äquivalent zu Uracil (U) in der RNA Sequenz betrachtet wird.

"Teil des "sense"-RNA-Transkriptes eines endogenen Zielgens" meint Fragmente einer RNA oder mRNA transkribiert von einem endogenen Zielgen. Dabei hat besagtes Teil bevorzugt eine Sequenzlänge von mindestens 10 Basen, bevorzugt mindestens 25 Basen, besonders bevorzugt mindestens 50 Basen, ganz besonders bevorzugt mindestens 100 Basen, am meisten bevorzugt mindestens 200 Basen oder mindestens 300 Basen. Umfasst ist auch die vollständige transkribierte RNA oder mRNA.

Alternativ, kann eine "im wesentlichen identische" dsRNA auch als Nukleinsäuresequenz definiert werden, die befähigt ist, mit einem Teil eines Transkriptes, bevorzugt der mRNA, eines endogenen Zielgenes zu hybridisieren (z.B. in 400 mM NaCl, 40 mM PIPES pH 6,4, 1 mM EDTA bei 50°C oder 70°C für 12 bis 16 h oder unter anderen Standardhybridisierungsbedingungen).

"Standardhybridisierungsbedingungen" ist breit zu verstehen und meint weniger stringente als auch - bevorzugt - stringente Hybridisierungsbedingungen. Solche Hybridisierungsbedingungen sind unter anderem bei Sambrook J, Fritsch EF, Maniatis T et al., in Molecular Cloning (A Laboratory Manual), 2. Auflage, Cold Spring Harbor Laboratory Press, 1989, Seiten 9.31-9.57) oder in Current Protocols in Molecular Biology, John Wiley &Sons, N.Y. (1989), 6.3.1-6.3.6. beschrieben.

Beispielhaft können die Bedingungen während des Waschschrittes ausgewählt sein aus dem Bereich von Bedingungen begrenzt von solchen mit geringer Stringenz (mit ungefähr 2X SSC bei 50°C) und - bevorzugt - solchen mit hoher Stringenz (mit ungefähr 0,2X SSC bei 50°C bevorzugt bei 65°C) (20X SSC: 0,3 M Natriumcitrat, 3 M NaCl, pH 7.0). Darüberhinaus kann die Temperatur während des Waschschrittes von niedrig stringenten Bedingungen bei Raumtemperatur, ungefähr 22°C, bis zu - bevorzugt - stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige beispielhafte Bedingungen für Hybridisierung und Waschschritt sind infolge gegeben:

(1) Hybridisierungbedingungen zum Beispiel aus nachfolgenden Bedingungen ausgewählt sein:

- a) 4X SSC bei 65°C,
- b) 6X SSC bei 45°C,
- c) 6X SSC, 100 $\mu$g/ml denaturierter, fragmentierte Fischsperma-DNA bei 68°C,
- f) 50% Formamid, 4X SSC bei 42°C,
- h) 2X oder 4X SSC bei 50°C (schwach stringente Bedingung),
- i) 30 bis 40 % Formamid, 2X oder 4X SSC bei 42°C (schwach stringente Bedingung).

(2) Waschschritte können zum Beispiel aus nachfolgenden Bedingungen ausgewählt sein:

- a) 0,015 M NaCl/0,0015 M Natriumcitrat/0,1% SDS bei 50°C.
- b) 0,1X SSC bei 65°C.
- c) 0,1X SSC, 0,5% SDS bei 68°C.
- d) 0,1X SSC, 0,5% SDS, 50% Formamid bei 42°C.
- e) 0,2X SSC, 0,1% SDS bei 42°C.
- f) 2X SSC bei 65°C (schwach stringente Bedingung).

"Im wesentlichen komplementär" meint, dass die "antisense"-Ribonukleotidsequenzen der dsRNA auch Insertionen, Deletionen sowie einzelne Punktmutationen im Vergleich zu dem Komplement der "sense"-Ribonukleotidsequenzen aufweisen kann. Bevorzugt beträgt die Homologie mindestens 80 %, bevorzugt mindestens 90 %, ganz besonders bevorzugt mindestens 95 %, am meisten bevorzugt 100% zwischen den "antisense"-Ribonukleotidsequenzen und dem Komplement der "sense"-Ribonukleotidsequenzen. Komplement meint dabei - in der dem Fachmann geläufigen Weise - den entsprechend den Basenpaarregeln abgeleiteten Gegenstrang.

Die doppelsträngige Struktur der dsRNA wird ausgehend von einem einzigen, ganz oder teilweise selbstkomplementären RNA-Strang (bei dem die oben erwähnten "sense"- und "antisense"-Ribonukleotidsequenzen der dsRNA alle kovalent miteinander verbunden sind) gebildet. Die Ausbildung der doppelsträngigen Struktur kann in vitro aber auch in vivo - beispielsweise in der eukaryotischen Zelle selber - erfolgen.

Die einzelnen "sense"-Ribonukleotidsequenzen können mit den korrespondierenden, im wesentlichen komplementären "antisense"-Ribonukleotidsequenzen eine doppelsträngige RNA-Struktur mittels Basenpaarung ausbilden und bilden eine Untereinheit der dsRNA.

Im Falle eines selbstkomplementären Stranges sollen zunächst die "sense"-Ribonukleotidsequenzen (S) der einzelnen Untereinheiten aneinander gefügt werden, wodrauf dann eine Aneinanderreihung der im wesntlichen komplementären "antisense"-Ribonukleotidsequenzen (AS) folgt. Die Anzahl der Einheiten n ist größer oder gleich zwei. Es entsteht eine Struktur mit einer einzelnen Haarnadel. Die Primärstruktur der dsRNA kann dabei schematisch beispielsweise wie folgt aussehen:

5'-S(1)-S(2)-.....-S(n)-AS(n)-....-AS(2)-AS(1)-3'

Die bevorzugte Sekundärstruktur ist in Fig. 2-A wiedergegeben.

Ist die dsRNA - bevorzugt - in der Lage eine Haarnadelstruktur auszubilden, so entspricht der Stamm der Haarnadel dem doppelsträngige Anteil der dsRNA, der durch Basenpaarung zwischen auf dem gleich RNA-Moleküle lokalisierten "sense"- und "antisense"-Ribonukleotidsequenz gebildet wird. Dabei werden "sense"- und "antisense"-Ribonukleotidsequenzen bevorzugt durch einen "Linker" verbunden. Der "Linker" ist bevorzugt ein Intron, das aus der dsRNA herausgespleißt werden kann. Selbstkomplementären dsRNA-Strukturen ausgehend von einem einzelnen RNA-Molekül sind bevorzugt, da sie lediglich die Expression eines Konstruktes erfordern und die komplementären RNA-Stränge stets in einem äquimolaren Verhältnis umfassen.

Bei der Verwendung eines Linkers (I) - bevorzugt eines Intron - sei die nachfolgende schematische Primärstruktur für die dsRNA beispielhaft genannt:

Dies ist eine bevorzugte Variante von a), bei der an der Stelle der Haarnadelschlaufe ein Linker (I) - bevorzugt ein Intron - insertiert wird:

5'-S(1)-S(2)-.....-S(n)-I-AS(n)-....-AS(2)-AS(1)-3'

Die bevorzugte Sekundärstruktur ist in Fig. 2-C wiedergegeben.

Die dsRNA Moleküle sind jedoch auch ohne den Linker funktionell. Dabei ist jedoch zu berücksichtigen, dass die letzten ca. 10 Nukleotide der terminalen Untereinheit S(n) in diesem Fall nicht mehr korrekt paaren. In diesem Fall ist die Länge für diese Untereinheit um 10 Nukleotide zu ergänzen. Der Linker ist bevorzugt ein Intron, besonders bevorzugt ein Intron in sense-Orientierung. Bevorzugt handelt es sich um ein Intron eines pflanzlichen Gens. Beispielhaft jedoch nicht einschränkend seien zu nennen: Das Intron 3 der Alkoholdehydrogenase 1 (Adh1) aus Mais (GenBank Acc.-No.: AF044293; GI: 2828164), das Intron 4 der beta-Conglycinin alpha Untereinheit aus Soja (GenBank Acc.-No.: AB051865); eines der Introns des rbcS-3A Gens für Ribulose-1.5-bisphosphatcarboxylase (RBC) kleine Untereinheit aus Erbse (GenBank Acc.-No.: X04333). Diese und weitere geeignete Introns sind dem Fachmann bekannt (McCullough AJ & Schuler MA (1997) Nuc Acids Res 25:1071-1077). Für die Anwendung in dem erfindungsgemäßen Verfahren wird das Intron bevorzugt in Kombination mit Spleißakzeptor- und Spleißdonorsequenzen eingesetzt, die ein späteres Herausspleißen aus der dsRNA ermöglichen. Diese Spleißsequenzen können die flankirenden Sequenzen des Intron selber sein, oder aber auch durch dentsprechende Sequenzen der überigfen dsRNA bereitgestellt werden.

Jede der einzelnen "sense"-Ribonukleotidsequenzen der dsRNA ist im wesentlichen identisch zu mindestens einem Teil des "sense"-RNA-Transkriptes eines endogenen Zielgens. Dabei sind jedoch nicht alle "sense"-Ribonukleotidsequenzen zu dem "sense"-RNA-Transkript eines einzigen endogenen Zielgens identisch, sondern die jeweils maximale Identität von mindestens zwei der "sense"-Ribonukleotidsequenzen besteht zu den "sense"-RNA-Transkripten von unterschiedlichen endogenen Zielgenen. Dabei beträgt die Homologie zwischen den Transkripten der beiden endogenen Zielgene unter 90%, bevorzugt unter 80%, besonders bevorzugt unter 70%, ganz besonders bevorzugt unter 60%, am meisten bevorzugt unter 50%.

Mindestens zwei der in der erfindungsgemäßen dsRNA umfassten einzelnen "sense"-Ribonukleotidsequenzen sind unterschiedlich. Unterschiedlich bedeutet zum einen, dass die Zielgene zu deren Transkripten sie die jeweils maximale Identität aufweisen, nicht identisch sind. Bevorzugt vermindert mindestens eine Untereinheit der dsRNA die Expression eines anderen Gens als mindstens eine andere Untereinheit. Unterschiedlich kann zum anderen auch heißen,dass die "sense"-Ribonukleotidsequenzen der Untereinheiten selber im wesentlichen nicht identisch sind und bevorzugt eine Homologie zu einander unter 60%, besonders bevorzugt unter 50% ganz besonders bevorzugt unter 40% aufweisen. Die dsRNA kann in einer weiteren Ausführungsform meherer Kopien einer Untereinheit enthalten. Weiterhin kann die dsRNA auch mehrere verschiedene Untereinheiten enthalten, die aber gegen das gleiche endogene Zielgens gerichtet sind und deren "sense"-Ribonukleotidsequenzen beispielsweise im wesentlichen identisch sind zu unterschiedlichen Teilen des "sense"-RNA-Transkriptes des besagten endogenen Zielgens.

Dabei kann jede der einzelnen "sense"-Ribonukleotidsequenzen auch zu dem Transkript mehrerer endogener Zielgene im wesentlichen identisch sein. Dies ist besonders dann der Fall, wenn die Zielgene über ähnliche Sequenzabschnitte verfügen, wie es beispielsweise bei Mitgliedern von Genfamilien (z.B. Speicherproteinen) der Fall ist. Dies ist eine besonders vorteilhafte Anwendungsform, da - bei entsprechender Wahl der Ribonukleotidsequenz einer Untereinheit - besagte Untereinheit die Expression von mehr als einem Zielgen vermindern kann.

Vorzugsweise wird die Sequenz der dsRNA so gewählt, dass die angestrebte dsRNA Struktur nach Ausbildung der Duplex - im Vergleich zu anderen möglichen Faltungsvarianten der Primärstruktur der dsRNA - die jeweils geringste freie Energie hat. Dies kann beispielsweise durch Vermeidung von Sequenzduplikationen etc. gewährleistet werden. Die spezifische Sekundärstruktur kann beispielsweise mit geeigneten Computerprogrammen vorausgesagt und optimiert werden (z.B. FOLDRNA; Zuker and Stiegler (1981) Nucleic Acids Res 9(1):133-48).

Jede Untereinheit der dsRNA hat in einer bevorzugten Ausführungsform eine Länge von mindestens 20 Basenpaaren, bevorzugt mindestens 50 Basenpaaren, besonders bevorzugt mindestens 100 Basenpaare, ganz besonders bevorzugt mindestens 250 Basenpaare.

In einer weiterhin bevorzugten Ausführungsform hat jede Einheit eine Länge eine ganzzahligen Vielfachen von 21 oder 22 Basenpaaren, also beispielsweise 21, 22, 42, 43, 44, 63, 64, 65, 66, 84, 85, 86, 87, 88, 105, 106, 107, 108, 109, 110, 126, 127, 128, 129, 131, 132, 147, 148, 149, 150, 151, 152, 153, 154, 168, 169, 170, 171, 172, 173, 174, 175, 176, 189, 190, 191, 192, 193, 194, 195, 196, 197, 198, 210, 211, 212, 213, 214, 215, 216, 217, 218, 219 oder 220 Basenpaare, bevorzugt 21, 22, 42, 44, 63, 66, 84, 88, 105, 110, 126, 132, 147, 154, 168, 176, 189, 198, 210 oder 220 Basenpaare, ganz besonders bevorzugt 21, 42, 63, 84, 105, 126, 147, 168, 189 oder 210 Basenpaare, am meisten bevorzugt 180 oder 210 Basenpaare.

Die "sense"- und/oder "antisense"-Ribonukleotidsequenzen der einzelnen Untereinheiten können direkt oder aber durch einen "Spacer" (SP; Abstandshalter) miteinander verbunden und/oder flankiert sein. Die einzelnen "Spacer" (SP) können dabei identisch oder aber auch unterschiedlich sein. Der "Spacer" genügt dabei bevorzugt den gleichen Längenanforderungen wie sie oben für die Länge der Untereinheiten selber gegeben sind. Der "Spacer" kann eine doppelstränge Struktur ausbilden, kann aber auch - beispielsweise in Form einer Blase - in ungepaarter Formation bestehen, d.h. die Basen in Strang und Gegenstrang müssen nicht zwingenderweise komplementär sein. Bevorzugte Ausführungsformen sind zum Beispiel durch nachfolgende Primärstrukturen beschrieben:

e) Dies ist eine bevorzugte Variante von c):

5’SP-S(1)-SP-S(2)-SP-..-S(n)-AS(n)-SP-..-AS(2)-SP-AS(1)-SP-3’

Die bevorzugte Sekundärstruktur ist in Fig. 3-A wiedergegeben.

Der "Spacer" kann weitere Funktionselemente umfassen. Beispielhaft jedoch nicht einschränkend sind zu nennen:

i) Sequenzen kodierend für eine von einem Ribozym als Substrat erkannten Erkennungssequenz (RE). Beispielsweise kann die dsRNA nachfolgende lineare Struktur vor der Faltung einnehmen:

5’-S(1)-(RE)-S(2)-...-S(n)-AS(n)-..-AS(2)-(RE)-AS(1)-3’

Die bevorzugte Sekundärstruktur ist in Fig. 3-B wiedergegeben. Das entsprechende Ribozym (R) kann separat exprimiert werden kann aber auch auf der dsRNA selber kodiert sein. Dabei ist die Sequenz kodierend für ein Ribozym bevorzugt so angeordnet, dass sie im gefalteten dsRNA Molekül einer Sequenz gegenüber liegt, die für dieses Ribozym als Substrat fungieren kann. Beispielsweise kann die dsRNA nachfolgende lineare Struktur vor der Faltung einnehmen:

5'-S(1)-(R)(RE)-S(2)-...-S(n)-AS(n)-..-AS(2)-(R)(RE)-AS(1)-3'

Die bevorzugte Sekundärstruktur ist in Fig. 3-C wiedergegeben. Durch die genannten Ausführungsformen werden nach Transkription die einzelnen Untereinheiten durch Wirkung des Ribozym voneinander getrennt. Diese Trennung ist vorteilhaft, jedoch nicht zwingend erforderlich. Entsprechend nutzbare Ribozyme und Erkennungssequenzen sind dem Fachmann bekannt.

Ribozyme meint katalytische RNA-Moleküle. Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "antisense"-RNAs diese enzymähnliche, RNA-spaltende Eigenschaft und steigert so deren Effizienz bei der Inaktivierung der Ziel-RNA. Die Herstellung und Verwendung entsprechender Ribozym-"antisense"-RNA-Moleküle ist beispielsweise beschrieben bei Haseloff et al. (1988) Nature 334: 585-591. Auf diese Art können Ribozyme (z.B. "Hammerhead"-Ribozyme; Haselhoff und Gerlach (1988) Nature 334:585-591) verwendet werden, um die eines bestimmte RNA katalytisch zu spalten. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250(3):329-338). Geeignete Zielsequenzen und Ribozyme können zum Beispiel wie bei "Steinecke P, Ribozymes, Methods in Cell Biology 50, Galbraith et al. eds, Academic Press, Inc. (1995), S.449-460" beschrieben, durch Sekundärstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu den Spacersequenzen aufweisen (siehe auch US 4,987,071 und US 5,116,742). Alternativ können solche Ribozyme auch über einen Selektionsprozess aus einer Bibliothek diverser Ribozyme identifiziert werden (Bartel D und Szostak JW (1993) Science 261:1411-1418).

ii) Sequenzen kodierend für Erkennungssequenzen für RNAasen Der "Spacer" kann Erkennungssequenzen für RNAsen, bevorzugt sequenzspezifische RNAsen wie beispielsweise RNAse III enthalten. RNAse III schneidet am Motiv 5'-AGNN-3, wenn vier dieser Motive in einer Schleife vorhanden sind (Nagel R & Ares M (2000) RNA 6:1142-1156). Die RNase kann eine pflanzeneigene RNAse sein, oder - wie beispiuelsweise für bakterielle RNase III Proteine - auch transgen exprimiert werden.

iii) Sequenzen kodierend für Intronspeißsignale (IS). Dabei sind die Spleißdonor und Spleißakzeptorsequenzen bevorzugt so lokalisiert, dass jeweils die Untereinheit als Intron herausgespleißt wird. Intronspleißsignale sind in Meritt et al.(1997) Plant Journal 12:937-943 oder in Egoavil et al. (1997) Plant Journal 12:971-980 beschrieben.

Die dsRNA bzw. ihre Vorläufermoleküle können auf verschiedene dem Fachmann geläufige Weise in einen Organismus oder eine Zelle eingebracht werden. "Einbringen" ist breit zu verstehen und umfasst im Rahmen der Erfindung alle Verfahren, die dazu geeignet eine dsRNA bzw. ihre Vorläufermoleküle, direkt oder indirekt, in einen Organismus oder eine Zelle, Kompartiment, Gewebe, Organ oder Samen desselben einzuführen oder dort zu generieren. Die Einbringung kann zu einer vorübergehenden (transienten) Präsenz einer dsRNA führen oder aber auch zu einer dauerhaften (stabilen). Umfasst sind Verfahren der direkten Transfektion oder Transformation der Zelle mit der als auch die Transformation oder Transfektion der Zelle mit Expressionskassetten, die befähigt sind, die der dsRNA zugrundeliegenden Ribonukleinsäuresequenzen in der Zelle zu exprimieren (infolge dsRNA-Expressionssystem). Die Expression der dsRNA kann transient oder - beispielsweise nach Integration in das Genom des Organismus - permanent erfolgen. Die Duplex-Bildung der dsRNA kann entweder außerhalb der Zelle oder innerhalb derselben initiiert werden.

Die dsRNA wird in einer Menge eingeführt, die zumindest eine Kopie pro Zelle ermöglicht. Höhere Mengen (z.B. mindestens 5, 10, 100, 500 oder 1000 Kopien pro Zelle) können ggf. eine effizienter Verminderung der Expression der Zielgene bewirken. Da dsRNA eine außerordentlich gute Mobilität innerhalb eines Organismus hat, ist es nicht zwingend erforderlich die dsRNA in jede Zelle des Organismus zu applizieren. Es ist ausreichend, die dsRNA in eine oder wenige Zellen einzubringen oder zu exprimieren, wobei die erfindungsgemäße Wirkung dann auch in anderen Zellen des gleichen Organismus erzielt werden kann.

Eine dsRNA - beispielsweise zur Verwendung in einer direkten Transformation oder Transfektion - kann kann *in vivo* oder *in* vitro, durch enzymatische, molekularbiologische oder chemisch-synthetische Verfahren synthetisiert werden. Dazu können eukaryotische, prokaryotische oder Bakteriophagen RNA Polymerasen (wie z.B. T3-, T7- oder SP6 RNA-Polymerase) verwendet werden. Entsprechende Verfahren zu in vitro Expression von RNA sind beschrieben (WO 97/32016; US 5,593,874; US 5,698,425, US 5,712,135, US 5,789,214, US 5,804,693). Eine chemisch oder enzymatisch in vitro synthetisierte dsRNA kann vor der Einführung in eine Zelle, Gewebe oder Organismus aus dem Reaktionsgemisch beispielsweise durch Extraktion, Präzipitation, Elektrophorese, Chromatographie oder Kombinationen dieser Verfahren ganz oder teilweise aufgereinigt werden. Die dsRNA kann direkt in die Zelle eingeführt werden (beispielsweise durch Partikelbeschuß oder Mikroinjektion) oder aber extrazellulär (z.B. in den interstitial Raum, das Gefäßsystem, das Verdauungssystem o.ä.) appliziert werden. Auch eine Applikation beispielsweise von dsRNA exprimierenden Organismen in Form von Nahrung ist denkbar. Es ist bekannt, dass dsRNA eine gute Zellgängigkeit und ausreichende Stabilität hat. Durch die hohe Wirksamkeit der dsRNA sind auch wenige Moleküle ausreichend, um eine gute Wirkung im Sinne der Erfindung zu erzielen.

Es können ferner Modifikationen sowohl des Zucker-Phosphat-Gerüstes als auch der Nukleoside in der dsRNA vorliegen. Beispielsweise können die Phosphodiesterbindungender der RNA dahingehend modifiziert sein, dass sie zumindest ein Stickstoff oder Schwefel-Heteroatom umfassen. Basen können dahingehend modifiziert werden, dass die Aktivität beispielsweise von Adenosindeaminase eingeschränkt wird. Die dsRNA kann enzymatisch oder ganz oder teilweise chemisch-synthetisch hergestellt werden.

Bevorzugt wird die dsRNA jedoch ausgehend von entsprechenden Expressionssystemen in der Zelle exprimiert. Ein weiterer Gegenstand der Erfindung betrifft besagte dsRNA-Expressionssysteme.

Wird die dsRNA als ein einzelner, selbstkomplementären RNA-Strang exprimiert, so umfasst das Expressionssystem eine Expressionskassette mit einer für den selbstkomplementären RNA-Strang kodierenden DNA Sequenz in funktioneller Verknüpfung mit einen Promotor, der geeignet ist, die Expression in der jeweiligen eukaryotischen Zelle zu gewährleisten. Optional kann die Expressionskassette weitere funktionelle Elemente wie beispielsweise Transkriptionsterminatoren und/oder Polyadenylierungssignale umfassen. Derartige Expressionskassetten sind ebenfalls Gegenstand der Erfindung.

Expressionskassette meint chimäre DNA-Moleküle in denen eine für das dsRNA-Molekül (bzw. für einen der Stränge desselben) kodierende Nukleinsäuresequenz mit mindestens einem genetischen Kontrollelement (beispielsweise einem Promotor, Enhancer, Silencer, Splice-Donor oder -Akzeptor, Polyadenylierungssignal) derart verknüpft ist, das die Transkription des dsRNA-Moleküls (bzw. eines der Stränge desselben) in dem Organismus gewährleistet ist. Entsprechend vorteilhafte Konstruktionen sind weiter unten beschrieb. Eine Polyadenylierung ist möglich, jedoch nicht erforderlich, ebenso müssen keine Elemente zur Initiierung einer Translation vorhanden sein.

Soll das Expressionskonstrukt in eine Pflanze eingeführt und die dsRNA in plantae erzeugt werden, so sind pflanzenspezifische genetische Kontrollelemente (beispielsweise pflanzenspezifische Promotoren) bevorzugt. Die dsRNA kann jedoch auch in anderen Organismen oder in vitro erzeugt und dann in die Pflanze eingebracht werden.

Unter einer funktionellen Verknüpfung versteht man zum Beispiel die sequentielle Anordnung eines Promotors mit der zu transkribierenden Nukleinsäuresequenz und ggf. weiterer regulativer Elemente wie zum Beispiel einem Terminator und/oder Polyadenylierungssignalen derart, dass jedes der regulativen Elemente seine Funktion bei der Transkription der Nukleinsäuresequenz, je nach Anordnung der Nukleinsäuresequenzen erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare. In einer bevorzugten Ausführungsform wird die zu transkribierende Nukleinsäuresequenz so hinter dem Promotor lokalisiert, das der Transkriptionsstart identisch ist mit dem gewünschten Beginn der dsRNA.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind.

Unter einer Expressionskassette sind aber auch solche Konstruktionen zu verstehen, bei denen zum Beispiel eine Nukleinsäuresequenz kodierend für eines dsRNA derart hinter einen endogenen Promotor platziert werden, dass der gleiche Effekt auftritt. Beide Ansätze führen zu Expressionskassetten im Sinne der Erfindung.

Prinzipiell können alle natürlichen Promotoren mit ihren Regulationssequenzen wie die oben genannten für das erfindungsgemässe Verfahren verwendet werden, solange sie die Expression in dem Zielorganismus gewährleisten.

Darüberhinaus können auch synthetische Promotoren vorteilhaft verwendet werden.

Es können weitere Promotoren funktionell mit der zu exprimierenden Nukleinsäuresequenz verknüpft sein, die eine Expression in weiteren Eukaryoten oder in Prokaryoten, wie zum Beispiel *E.coli* Bakterien ermöglichen.

Die in den Expressionskassetten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion der erfindungsgemässen Expressionskassette haben. Genetische Kontrollsequenzen modifizieren zum Beispiel die Transkription in prokaryotischen oder eukaryotischen Organismen. Vorzugsweise umfassen die erfindungsgemässen Expressionskassetten 5'-stromaufwärts von der jeweiligen transgen zu exprimierenden Nukleinsäuresequenz einen pflanzenspezifischen Promoter und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen ferner auch die 5'-untranslatierte Regionen, Introns oder nichtkodierende 3'-Region von Genen. Es ist gezeigt worden, dass diese eine signifikante Funktionen bei der Regulation der Genexpression spielen können. Kontrollsequenzen umfassen ferner Polyadenylierungssignale sowie Terminatorsequenzen.

Die Expressionskassette kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Methoden wie die cre/lox-Technologie erlauben eine gewebespezifische, unter Umständen induzierbare Entfernung der Expressionskassette aus dem Genom des Wirtsorganismus (Sauer B (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Bevorzugt kann die Expressionskassette, bestehend aus einer Verknüpfung von Promoter und zu transkribierender Nukleinsäuresequenz, integriert in einem Vektor vorliegen und durch zum Beispiel Transformation - nach einem der unten beschriebenen Verfahren - in den Organismus eingebracht werden. Die nachfolgende Expression kann transient sein oder aber auch - bevorzugt - stabil nach Insertion (beispielsweise unter Verwendung von Selektionsmarkern) der Expressionskassetten in das Genom erfolgen. Bevorzugt wird das dsRNA-Expressionssystem stabil in das Genom - beispielsweise die chromosomale DNA oder die DNA der Organellen (z.B. der Plastiden, Mitochondrien etc.) - einer Zelle integriert.

Die Einführung einer transgenen Expressionskassette in einen pflanzlichen Organismus oder Zellen, Geweben, Organe, Teile bzw. Samen desselben (bevorzugt in Pflanzen bzw. pflanzliche Zellen, Gewebe, Organe, Teile oder Samen) kann vorteilhaft unter Verwendung von Vektoren realisiert werden, in denen die transgenen Expressionskassetten enthalten sind. Vektoren können beispielhaft Plasmide, Cosmide, Phagen, Viren oder auch Agrobakterien sein. Die transgenen Expressionskassetten können in den Vektor (bevorzugt ein Plasmidvektor) über eine geeignete Restriktionsschnittstelle insertiert werden. Der entstandene Vektor wird zunächst in E.coli eingeführt. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu überprüfen. Bevorzugt sind solche Vektoren, die eine stabile Integration der Expressionskassette in das Wirtsgenom ermöglichen.

Die Herstellung eines transformierten pflanzlichen Organismus (bzw. einer transformierten Zelle oder Gewebes) erfordert, dass die entsprechende DNA (z.B. der Expressionsvektor) oder RNA in die entsprechende Wirtszelle eingebracht wird. Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73; Howell et al. (1980) Science 208:1265; Horsch et al.(1985) Science 227:1229-1231; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Eine transgener Organismus kann beispielsweise durch Einführung der entsprechenden Expressionssysteme in eine Zygote, Stammzelle, Protoplast oder eine andere geeignete von dem Organismus abgeleitete Zelle hergestellt werden.

Bevorzugte Pilze sind Aspergillus, Trichoderma, Ashbya, Neurospora, Fusarium, Beauveria oder weitere in Indian Chem Engr. Section B. Vol 37, No 1,2 (1995) auf Seite 15, Tabelle 6 beschriebene Pilze. Besonders bevorzugt ist der filamentöse Hemiascomycet Ashbya gossypii.

Bevorzugte Hefen sind Candida, Saccharomyces, Hansenula oder Pichia, besonders bevorzugt sind Saccharomyces cerevisiae oder Pichia pastoris (ATCC Accession No. 201178).

"Pflanzlicher Organismus" umfasst jeden Organismus gemäß Anspruch 1, der zur Photosynthese befähigt ist, sowie die von diesem abgeleitete Zellen, Gewebe, Teile oder Vermehrungsgut (wie Samen oder Früchte). Eingeschlossen sind reife Pflanze, Saatgut, Sprosse und Keimlinge, sowie davon abgeleitete Teile, Vermehrungsgut (zum Beispiel Knollen, Samen oder Früchte) und Kulturen, zum Beispiel Zell- oder Kalluskulturen. Reife Pflanzen meint Pflanzen zu jedem beliebigen Entwicklungsstadium jenseits des Keimlings. Keimling meint eine junge, unreife Pflanze in einem frühen Entwicklungsstadium.

"Pflanze" im Rahmen der Erfindung meint alle Gattungen und Arten höherer und niedrigerer Pflanzen des Pflanzenreiches, die nachfolgend aufgeführt sind. Eingeschlossen unter dem Begriff sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium. "Pflanze" umfasst solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Die in den Verfahren verwendeten pflanzlichen Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu beginn der Fermentation vorgelegt oder semikontinuierlich oder kontinuierlich nach gefüttert werden.

Nachfolgende Anwendung des erfindungsgemäßen Verfahrens seien beispielhaft jedoch nicht einschränkend zu nennen:

I. Pflanzenbiotechnologie

Bevorzugt wird das erfindungsgemäße Verfahren im Rahmen der Pflanzenbiotechnologie zur Erzeugung von Pflanzen mit vorteilhaften Eigenschaften eingesetzt. So kann Eignung der Pflanzen oder deren Samen als Nahrungs- oder Futtermittel verbessert werden, beispielsweise über eine Veränderung der Zusammensetzungen und/oder des Gehalt an Metaboliten, insbesondere Proteinen, Ölen, Vitaminen und/oder Stärke. Auch können Wachstumsrate, Ertrag oder die Resistenz gegen biotische oder abiotische Stressfaktoren erhöht werden. Nachfolgende Anwendungen im Bereich der Pflanzenbiotechnologie sind insbesondere vorteilhaft. Die angegebenen möglichen Zielgene sind beispielhaft jedoch nicht einschränkend zu verstehen:

1. Verbesserter Schutz gegen abiotische Stressfaktoren (Hitze, Kälte, Trockenheit, erhöhte Feuchtigkeit, Umweltgifte, UV-Strahlung). Bevorzugt werden Gene in iherer Expression vermindert, die am Stressreaktionen beteiligt sind.

2. Modifikation der Zusammensetzung und/oder des Gehaltes an Fettsäuren, Lipiden oder Ölen
Eine Veränderung des Fettsäuregehalten oder der Fettsäurezusammensetzung, vorzugsweise in einer Ölfrucht wie Raps oder Sonnenblume, kann beispielsweise erreicht werden durch Verminderung der Genexpression von Genen der Fettsäurebiosynthese vorzugsweise ausgewählt aus der Gruppe bestehend aus Genen kodierend für Acetyltransacylasen, Acyltransportproteinen ("acyl carrier protein"), Desaturasen wie Stearyldesaturasen oder mikrosomale $\Delta$12-Desaturasen insbesondere Fad2-1 Gene, Malonyltransacylase, $\beta$-Ketoacyl-ACP-synthetasen, 3-Keto-ACP-reduktasen, Enoyl-ACP-hydrasen, Thioesterasen wie Acyl-ACP-thioesterases, Enoyl-ACP-reduktasen. Verschiedene weitere vorteilhafte Ansätze zur Modifizierung der Lipidzusammensetzung sind beschrieben (Shure M et al. (1983) Cell 35:225-233; Preiss et al.(1987) Tailoring Genes for Crop Improvement (Bruening et al., eds.), Plenum Press, S.133-152; Gupta et al. (1988) Plant Mol Biol. 10:215-224; Olive et al. (1989) Plant Mol Biol 12:525-538; Bhattacharyya et al. (1990) Cell 60:155-122; Dunwell JM (2000) J Exp Botany 51Spec No:487-96; Brar DS et al. (1996) Biotech Genet Eng Rev 13:167-79; Kishore GM und Somerville CR (1993) Curr Opin Biotech 4(2):152-8). Bevorzugt sind insbesondere Fad2 Gene (z.B. beschrieben durch Genbank Acc.-Nr.: AF124360 (*Brassica carinata),* AF042841 *(Brassica rapa),* L26296 *(Arabidopsis thaliana),* A65102 (Corylus avellana)). Weitere vorteilhafte Gene und Verfahren zur Modifikation des Lipidgehaltes sind beispielsweise beschrieben in US 5,530,192 und WO 94/18337. Ein erhöhter Lipidgehalt kann auch erreicht werden durch Verminderung des Stärkegehalte bespielsweise infolge verminderter Expression von von Enzymen des Kohlenhydratstoffwechsels (z.B. ADP-Glucosepyrophosphorylasen).

3. Modifikation der Kohlenhydratzusammensetzung
Eine Modifikation der Kohlehydratzusammensetzung kann beispielsweise erreicht werden durch Verminderung der Genexpression von Genen des Kohlenhydratstoffwechsels oder der Kohlenhydratbiosynthese, beispielsweise der Biosynthese von Amylose, Pektinen, Cellulose oder Zellwandkohlenhydraten. Dadurch kann eine Vielzahl zellulärer Prozesse (Reifung, Halfestigkeit, Stärkezusammensetzung oder -gehalt etc.) in vorteilhafter Weise beeinflusst werden. Als Zielgene seien beispielhaft jedoch nicht einschränkend zu nennen Phosphorylasen, Stärkesynthetasen, Q-Enzyme, Sucrose-6-phosphatsynthetasen, Sucrose-6-phosphatphosphatasen, ADP-Glucosepyrophosphorylasen, Branching-Enzyme, Debranching-Enzyme sowie diverse Amylasen. Die entsprechenden Gene sind beschrieben (Dunwell JM (2000) J Exp Botany 51Spec No:487-96; Brar DS et al. (1996) Biotech Genet Eng Rev 13:167-79; Kishore GM und Somerville CR (1993) Curr Opin Biotech 4(2):152-8). Vorteilhafte Gene zur beeinflussung des Kohlenhydratstoffwechsels - insbesondere der Stärkebiosynthese - sind beschrieben in WO 92/11375, WO 92/11376, US 5, 365,016 und WO 95/07355.

5. Verminderung des Gehaltes von Speicherproteinen
Die Verminderung der Genexpression von Genen kodierend für Speicherproteine (infolge SP) hat zahlreiche Vorteile, wie beispielsweise Verminderung des allergenen Potentials oder Veränderung in der Zusammensetzung oder Menge anderer Metabolite. Speicherproteine sind u.a beschrieben in EP-A 0 591 530, WO 87/47731, WO 98/26064, EP-A 0 620 281; Kohno-Murase J et al. (1994) Plant Mol Biol 26(4): 1115-1124.
SP dienen zur Speicherung von Kohlenstoff, Stickstoff und Schwefel, die für das schnelle heterotrophe Wachstum bei Keimung von Samen oder Pollen benötigt werden. Sie haben meist keine enzymatische Aktivität. SP werden dabei nur im Embryo während der Samenentwicklung synthetisiert und akkumulieren dabei zum einen in Proteinspeichervakuolen (PSV) von unterschiedlich differentzierten Zellen im Embryo bzw. Endosperm.
"Speicherprotein" meint allgemein ein Protein, das mindestens eine der nachfolgenden wesentlichen Eigenschaften aufweist:

i) Speicherproteine werden im wesentlichen nur im Embryo während der Samenentwicklung exprimiert. "Im wesentlichen" bedeutet dabei, dass in dem besagten Stadium mindestens 50%, bevorzugt mindestens 70%, ganz besonders bevorzugt mindestens 90%, am meisten bevorzugt mindestens 95% der Gesamtexpression über die Lebensdauer einer Pflanze hinweg stattfindet.

ii) Speicherproteine werden während der Keimung des Samen wieder abgebaut. Dabei beträgt der Abbau während der Keimung mindestens 20%, bevorzugt mindestens 50%, ganz besonders bevorzugt mindestens 80%.

iii) Speicherproteine machen einen wesentlichen Anteil am Gesamtproteingehalt des nicht-keimenden Samens aus. Bevorzugt macht das Speicherprotein in dem nicht-keimenden Samen der Wildtyp-Pflanze mehr als 5 Gew.% des Gesamtproteins aus, besonders bevorzugt mindestens 10 Gew.%, ganz besonders bevorzugt mindestens 20 Gew.%, am meisten bevorzugt mindestens 30 Gew.-%.

Bevorzugt weisen Speicherproteine 2 oder alle der oben genannten wesentlichen Eigenschaften i), ii) oder iii) auf. Speicherproteine können in Untergruppen entsprechend weiterer charakteristischer Eigenschaften, wie beispielsweise ihrem Sedimentationskoeffizienten oder der Löslichkeit in verschiedenen Lösungen (Wasser, Salzlösung, Alkohol) aufgeteilt werden. Die Bestimmung des Sedimentationskoeffizienten kann in der dem Fachmann vertrauten Weise mittels Ultrazentrifugation durchgeführt werden (z.B. beschrieben bei Correia JJ (2000) Methods in Enzymology 321:81-100).
Insgesamt können vier grosse Genfamilien für Speicherproteine aufgrund ihrer Sequenzen zugeordnet werden: 2S-Albumine (Napin-ähnlich), 7S-Globuline (Phaseolin-ähnlich), 11S/12S-Globuline (Legumin-/Cruciferin-ähnlich) und die Zein-Prolamine.

2S Albumine sind weit verbreitet in Samen von Dikotyledonen, einschliesslich wichtiger kommerzieller Pflanzenfamilien wie Fabaceae (z.B. Sojabohne), Brassicaceae (z.B. Raps), Euphorbiaceae (z.B. Rizinus) oder Asteraceae (z.B. Sonnenblume). 2S Albumine sind kompakte globuläre Proteine mit konservierten Cysteinresten, die oft Heterodimere bilden.

7S-Globuline liegen in trimerer Form vor und enthalten keine Cysteinreste. Nach ihrer Synthese werden sie wie die 2S-Albumine in kleinere Fragmente gespalten und glykosyliert. Trotz Unterschiede in der Polypeptidgrösse sind die verschiedenen 7S-Globuline hoch konserviert und gehen vermutlich wie die 2S-Albumine auf einen gemeinsames Vorläuferprotein zurück. Die 7S-Globuline sind nur in geringen Mengen in Monokotyledonen vorhanden. In Dikotyledonen ist ihr Anteil immer kleiner verglichen mit den 11S/12S-Globulinen.

11S/12S-Globuline stellen neben den 2S-Albuminen die Hauptfraktion der Speicherproteine in Dikotyledonen. Die hohe Ähnlichkeit der verschiedenen 11S-Globuline aus verschiedenen Pflanzengattungen lassen wiederrum auf einen gemeinsames Vorläuferprotein in der Evolution schliessen.

Bevorzugt ist das Speicherprotein ausgewählt aus den Klassen der 2S-Albumine (Napin-ähnlich), 7S-Globuline (Phaseolin-ähnlich), 11S/12S-Globuline (Legumin-/Cruciferin-ähnlich) oder Zein-Prolamine.

Besonders bevorzugte 2S-Albumine umfassen

ii) 2S-Albumine aus Arten der Gattung Brassica, wie beispielsweise Brassica napus, Brassica nigra, Brassica juncea, Brassica oleracea oder Sinapis alba, ganz besonders bevorzugt die 2S-Albumine mit der SEQ ID NO: 32, 34, 36, 38, 40, 46 oder 48, am meisten bevorzugt die durch die Nukleinsäuren gemäß SEQ ID NO: 31, 33, 35, 37, 39, 45 oder 47 kodierten Proteine,

iii) 2S-Albumine aus Soja, ganz besonders bevorzugt die 2S-Albumine mit der SEQ ID NO: 42 oder 44, am meisten bevorzugt die durch die Nukleinsäuren gemäß SEQ ID NO: 41 oder 43 kodierten Proteine,

iv) 2S-Albumine aus Sonnenblume (Helianthus annus), ganz besonders bevorzugt die 2S-Albumine mit der SEQ ID NO: 50 oder 52, am meisten bevorzugt die durch die Nukleinsäuren gemäß SEQ ID NO: 49 oder 51 kodierten Proteine,

sowie die entsprechenden Homologen und funktionellen Äquivalente zu i) oder ii) oder iii) oder iv) aus identischen oder anderen Pflanzenarten, insbesondere Raps, Sonnenblume, Lein, Sesam, Färberdistel, Ölbaum, Soja oder verschiedene Nussarten. Funktionelle Äquivalente zeichnen sich bevorzugt neben den oben genannten wesentlichen Eigenschaften durch charakteristische Eigenschaften wie einen 2S-Sedimentationskoeffizienten und/oder durch eine Löslichkeit in Wasser aus.

Funktionelle Äquivalente der 2S-Albumine haben in einer weiteren bevorzugten Ausführungsform eine Homologie von mindestens 60%, bevorzugt mindestens 80%, ganz besonders bevorzugt mindestens 90%, am meisten bevorzugt mindestens 95% zu einer der Proteinsequenzen mit der SEQ ID NO: 2, 4, 6, 8, 32, 34, 36, 38, 40, 42, 44, 46, 48, 50 oder 52 wobei die Homologie sich bevorzugt über eine Länge von mindestens 30 Aminosäuren, bevorzugt mindestens 50 Aminosäuren besonders bevorzugt über 100 Aminosäuren, am meisten bevorzugt über die gesamte Länge der jeweiligen Proteine erstreckt, und weisen die gleichen wesentlichen Eigenschaften eines Speicherproteins und - bevorzugt- die charakteristischen Eigenschaften eines 2S-Speicherproteins auf.

Besonders bevorzugte 7S-Globuline umfassen solche aus Arabidopsis oder Soja, ganz besonders bevorzugt die Proteine mit der SEQ ID NO: 94 oder 96, am meisten bevorzugt die durch die Nukleinsäuren gemäß SEQ ID NO: 93 oder 95 kodierten Proteine. Funktionelle Äquivalente zeichnen sich bevorzugt neben den oben genannten wesentlichen Eigenschaften durch charakteristische Eigenschaften wie einen 7S-Sedimentationskoeffizienten und/oder durch eine Löslichkeit in Salzlösung aus. Als weitere charakteristische Eigenschaft können 7S-Globuline keine Cysteinreste enthalten.

Funktionelle Äquivalente der 7S-Globuline haben in einer weiteren bevorzugten Ausführungsform eine Homologie von mindestens 60%, bevorzugt mindestens 80%, ganz besonders bevorzugt mindestens 90%, am meisten bevorzugt mindestens 95% zu einer der Proteinsequenzen mit der SEQ ID NO: 94 oder 96 wobei die Homologie sich bevorzugt über eine Länge von mindestens 30 Aminosäuren, bevorzugt mindestens 50 Aminosäuren besonders bevorzugt über 100 Aminosäuren, am meisten bevorzugt über die gesamte Länge der jeweiligen Proteine erstreckt, und weisen die gleichen wesentlichen Eigenschaften eines Speicherproteins und - bevorzugt- die charakteristischen Eigenschaften eines 7S-Speicherproteins auf.

Besonders bevorzugte 11S/12S-Globuline umfassen bevorzugt 11S-Globuline aus Raps und Soja insbesondere

i) 11S-Globuline aus Raps mit der SEQ ID NO: 10, 12, 14, 16 oder 18, am meisten bevorzugt die durch die Nukleinsäuren gemäß SEQ ID NO: 9, 11, 13, 15 oder 17 kodierten Proteine,

ii) die 11S-Globuline aus Soja mit der SEQ ID NO: 20, 22, 24, 26 oder 28, am meisten bevorzugt die durch die Nukleinsäuren gemäß SEQ ID NO: 19, 21, 23, 25 oder 27 kodierten Proteine,

sowie die entsprechenden Homologen und funktionellen Äquivalente aus anderen Pflanzenarten, insbesondere Raps, Sonnenblume, Lein, Sesam, Färberdistel, Ölbaum, Soja oder verschiedene Nussarten, wie beispielsweise das Sonnenblume 11S Speicherprotein (SEQ ID NO: 30), insbesondere das durch die Nukleinsäuresequenz gemäß SEQ ID NO: 29 kodierte Protein. Funktionelle Äquivalente zeichnen sich bevorzugt neben den oben genannten wesentlichen Eigenschaften durch charakteristische Eigenschaften wie einen 11S- oder 12S-Sedimentationskoeffizienten und/oder durch eine Löslichkeit in Salzlösung (PBS; phosphatgepufferte Salzlösung) und/oder eine schlechte Löslichkeit in Wasser aus.

Funktionelle Äquivalente der 11S- oder 12S Albumine haben in einer weiteren bevorzugten Ausführungsform eine Homologie von mindestens 60%, bevorzugt mindestens 80%, ganz besonders bevorzugt mindestens 90%, am meisten bevorzugt mindestens 95% zu einer der Proteinsequenzen mit der SEQ ID NO: 10, 12, 14, 16, 18, 20, 22, 24, 26, 28, 30, 60, 62, 64, 66, 68 oder 70 wobei die Homologie sich bevorzugt über eine Länge von mindestens 30 Aminosäuren, bevorzugt mindestens 50 Aminosäuren besonders bevorzugt über 100 Aminosäuren, am meisten bevorzugt über die gesamte Länge der jeweiligen Proteine erstreckt, und weisen die gleichen wesentlichen Eigenschaften eines Speicherproteins und - bevorzugt- die charakteristischen Eigenschaften eines 11S- oder 12S-Speicherproteins auf.

Besonders bevorzugte Zein-Prolamine umfassen bevorzugt solche aus monokotyledonen Pflanzen, insbesondere Mais, Rais, Hafer, Gerste oder Weizen. Ganz besonders bevorzugt sind die Mais Zein-Prolamine beschrieben durch SEQ ID NO: 98, 100, 102 oder 104 - insbesondere die durch SEQ ID NO 97, 99, 101 oder 103 kodierten Protein -, das Reis Prolamin gemäß SEQ ID NO: 106 - insbesondere das durch SEQ ID NO 105 kodierte Protein -, das Hafer Prolamin gemäß SEQ ID NO: 108 - insbesondere das durch SEQ ID NO 107 kodierte Proteine-, das Gerste Prolamin gemäß SEQ ID NO: 110 und/oder 111 - insbesondere das durch SEQ ID NO 109 kodierte Protein - und das das Weizen Prolamin gemäß SEQ ID NO: 113 - insbesondere das durch SEQ ID NO 112 kodierte Protein. Funktionelle Äquivalente zeichnen sich bevorzugt durch eine Löslichkeit in 70%iger ethanolischer Lösung und eine schlechte Löslichkeit in Wasser oder Salzlösung aus.

Funktionelle Äquivalente der Zein-Prolamine haben in einer weiteren bevorzugten Ausführungsform eine Homologie von mindestens 60%, bevorzugt mindestens 80%, ganz besonders bevorzugt mindestens 90%, am meisten bevorzugt mindestens 95% zu einer der Proteinsequenzen mit der SEQ ID NO: 98, 100, 102, 104, 106, 108, 110, 111 oder 113 wobei die Homologie sich bevorzugt über eine Länge von mindestens 30 Aminosäuren, bevorzugt mindestens 50 Aminosäuren besonders bevorzugt über 100 Aminosäuren, am meisten bevorzugt über die gesamte Länge der jeweiligen Proteine erstreckt, und weisen die gleichen wesentlichen Eigenschaften eines Speicherproteins und - bevorzugt- die charakteristischen Eigenschaften eines Zein-Prolamine auf.

Funktionelle Äquivalente meint insbesondere natürliche oder künstliche Mutationen der obengenannten Speicherproteine sowie homologe Polypeptide aus anderen Pflanzen, die die gleichen wesentlichen und - bevorzugt - charakteristischen Eigenschaften aufweisen. Bevorzugt sind homologe Polypeptide aus oben beschriebenen bevorzugten Pflanzen. Die zu den im Rahmen dieser Erfindung offenbarten Speicherproteinen homologen Sequenzen aus anderen Pflanzen - beispielsweise solchen deren genomische Sequenz ganz oder teilweise bekannt ist, wie beispielsweise aus Arabidopsis thaliana, Brassica napus, Nicotiana tabacum oder Solanum tuberosum - durch Homologievergleiche aus Datenbanken auffinden. können z.B. durch Datenbanksuche oder Durchmustern von Gen-Banken - unter Verwendung der beispielhaft aufgeführten Speicherprotein-Sequenzen als Suchsequenz bzw. Sonde - leicht aufgefunden werden.

Mutationen umfassen Substitutionen, Additionen, Deletionen, Inversion oder Insertionen eines oder mehrerer Aminosäurereste.

Ein weiterer Gegenstand der Erfindung umfasst ein zumindest teilweise doppelsträngiges Ribonukleinsäuremolekül, wobei das doppelsträngige Ribonukleinsäuremolekül umfasst

i) einen "sense"-RNA-Strang umfassend mindestens zwei Ribonukleotidsequenzabschnitte, wobei jeweils mindestens einer dieser Ribonukleotidsequenzabschnitte im wesentlichen identisch ist zu mindestens einem Teil des "sense"-RNA-Transkriptes einer Speicherprotein-Nukleinsäuresequenz gemäß SEQ ID NO: 1, 3, 5, 7, 9, 11, 13, 15 , 17, 19, 21, 23, 25, 27, 29, 31, 33, 35, 37, 39, 41, 43, 45, 47, 49, 51, 59, 61, 63, 65, 67, 69, 71, 93, 95, 97, 99, 101, 103, 105, 107, 109 oder 112 oder eines funktionellen Äquivalentes derselben, wobei jedoch nicht alle Ribonukleotidsequenzabschnitte zu dem "sense"-RNA-Transkript eines einzigen einer Speicherprotein-Nukleinsäuresequenz identisch sind, und

ii) einen "antisense"-RNA-Strang, der zu dem RNA-sense-Strang unter i) im wesentlichen komplementären ist.

Bevorzugt haben zumindest zwei der Speicherprotein-Nukleinsäuresequenzen, zu deren "sense"-RNA-Transkript die besagten Ribonukleotidsequenzabschnitte im wesentlichen identisch sind, untereinander eine Homologie von unter 90%, bevorzugt unter 80%, ganz besonders bevorzugt unter 60% am meisten bevorzugt unter 50% über die gesamte Länge ihrer kodierenden Nukleotidsequenz.
In einer weiteren bevorzugten Ausführungsform enthält die dsRNA mehrere Sequenzabschnitte, die eine gleichzeitige Suppression mehrerer Speicherproteine, bevorzugt von Speicherproteinen aus verschiedenen Klassen - wie beispielsweise einem 2S-Albumin, 7S-Globuline, 11S/12S-Globulin oder die Zein-Prolamine - bewirken.
Am meisten bevorzugt sind doppelsträngige RNA Moleküle beschrieben durch die Ribonukleinsäuresequenz gemäß SEQ ID NO: 84, 86 oder 88. Diese werden bevorzugt kodiert durch Nukleotidsequenzen entsprechend SEQ ID NO: 83, 85 oder 87.

5. Erreichen einer Resistenz gegen pflanzliche Pathogene
Eine Resistenz gegen pflanzliche Pathogene wie Arachniden, Pilze, Insekten, Nematoden, Protozoen, Viren, Bakterien und Krankheiten kann erreicht werden durch Verminderung der Genexpression von Genen, die für das Wachstum, Überleben, bestimmte Entwicklungsstufen (beispielsweise Verpuppung) oder die Vermehrung eine bestimmten Pathogens essentiell sind. Eine entsprechende Verminderung kann eine vollständige Inhibition vorgenannter Schritte aber auch eine Verzögerung derselben bewirken. Dies können pflanzliche Gene sein, die dem Pathogen beispielsweise das Eindringen ermöglichen, können aber auch pathogen-eigene Gene sein. Bevorzugt ist die dsRNA gg. Gene des Pathogens gerichtet. Als anti-Pathogenes Agens kann dabei die dsRNA selber, jedoch auch die Expressionssysteme, Expressionskassetten oder transgenen Organismen wirken. Pflanzen können beispielsweise mit geeigneten Formulierungen vorgenannter Agentien behandelt, beispielsweise besprüht oder estäubt werden Die Pflanzen selber können jedoch in Form eines transgenen Organismus die Agentien beinhalten und diese - beispielsweise in Form eines Fraßgiftes - an die Pathogene weitergeben. Verschiedene essentielle Gene diverser Pathogene sind dem Fachmann bekannt (z.B. für Nematodenresistenz WO 93/10251, WO 94/17194).
Am meisten bevorzugt als Pathogen sind Pilzpathogene wie Phytophthora infestans, Fusarium nivale, Fusarium graminearum, Fusarium culmorum, Fusarium oxysporum, Blumeria graminis, Magnaporthe grisea, Sclerotinia sclerotium, Septoria nodorum, Septoria tritici, Alternaria brassicae, Phoma lingam, bakterielle Pathogene wie Corynebacterium sepedonicum, Erwinia carotovora, Erwinia amylovora, Streptomyces scabies, Pseudomonas syringae pv. tabaci, Pseudomonas syringae pv. phaseolicola, Pseudomonas syringae pv. tomato, Xanthomonas campestris pv. malvacearum und Xanthomonas campestris pv. oryzae, und Nematoden wie Globodera rostochiensis, G. pallida, Heterodera schachtii, Heterodera avenae, Ditylenchus dipsaci, Anguina tritici und Meloidogyne hapla.
Eine Virusresistenz kann beispielsweise durch Verminderung der Expression eines viralen Hüllproteins, einer viralen Replikase, einer viralen Protease etc. erreicht werden. Zahlreiche Pflanzenviren und entsprechende Zielgene sind dem Fachmann bekannt.

6. Verhinderung von Halmbruch
Eine verminderte Anfälligkeit gegen Halmbruch kann beispielsweise erreicht werden durch Verminderung der Genexpression von Genen des Kohlenhydratstoffwechsels (s.o.). Vorteilhafte Gene sind beschrieben (u.a. WO 97/13865) und umfassen gewebspezifische Polygalacturonasen oder Cellulasen.

7. Verzögerung der Fruchtreifung
Eine verzögerte Fruchtreifung kann beispielsweise erreicht werden durch Verminderung der Genexpression von Genen ausgewählt aus der Gruppe bestehend aus Polygalacturonasen, Pectinesterasen, β-(1-4)glucanasen (Cellulasen),β-Galactanasen (β-Galactosidasen), oder Gene der Ethylenbiosynthese wie 1-Aminocyclopropan-1-carboxylatsynthase, Gene der Carotinoidbiosynthese wie z.B. Gene derPrephytoen- oder Phytoenbiosynthese beispielsweise Phytoendesaturasen. Weitere vorteilhafte Gene sind bespielsweise in WO 91/16440, WO 91/05865, WO 91/16426, WO 92/17596, WO 93/07275 oder WO 92/04456.

8. Erzielen einer männlichen Sterilität ("male sterility"). Entsprechende Zielgene sind beschrieben u.a. in WO 94/29465, WO89/10396, WO 92/18625.

9. Verminderung unerwünschter oder toxischer Pflanzeninhaltsstoffe wie z.B. Glucosinolaten. Entsprechende Zielgene sind beschrieben (u.a. in WO 97/16559).

10. Verzögerung von Alterserscheinungen. Entsprechende Zielgene sind u.a. Cinnamoyl-CoA:NADPH-Reduktasen oder Cinnamoylalkoholdehydrogenasen. Weitere Zielgene sind beschrieben (u.a. in WO 95/07993).

11. Modifikation der Lignifikation und/oder des Ligningehaltes vor allem in Baumarten. Entsprechende Zielgene sind beschrieben u.a. in WO 93/05159, WO 93/05160.

12. Modifikation des Faseranteils in Nahrungsmitteln vorzugsweise in Samen durch Verminderung der Expression der Coffeinsäure-O-methyltransferase oder der Cinnamoylalkoholdehydrogenase.

13. Modifaktion der Faserqualität in Baumwolle. Entsprechende Zielgene sind beschrieben u.a. in US 5,597,718.

14. Verminderung der Stoßanfälligkeit von beispielsweise Kartoffeln durch Verminderung beispielsweise der Polyphenoloxidase (WO 94/03607) etc.

15. Steigerung der Vitamin E Biosynthese beispielsweise durch Verminderung der Expression von Genen aus dem Homogentisatabbauweg wie z.B. der Homogentisat-1,2-dioxygenase (HGD; EC-Nr.: 1.13.11.5), der Maleylacetoacetatisomerase (MAAI; EC-Nr.: 5.2.1.2.) oder der Fumarylacetoacetathydrolase (FAAH; EC-Nr.: 3.7.1.2).
Ein weiterer Gegenstand der Erfindung umfasst ein zumindest teilweise doppelsträngiges Ribonukleinsäuremolekül, wobei das doppelsträngige Ribonukleinsäuremolekül umfasst

i) einen "sense"-RNA-Strang umfassend mindestens zwei Ribonukleotidsequenzabschnitte, wobei jeweils mindestens einer dieser Ribonukleotidsequenzabschnitte im wesentlichen identisch ist zu mindestens einem Teil des "sense"-RNA-Transkriptes eines Gens aus dem Homogentisatabbauweg gemäß SEQ ID NO: 115, 116, 118 oder 120 oder eines funktionellen Äquivalentes derselben, wobei jedoch nicht alle Ribonukleotidsequenzabschnitte zu dem "sense"-RNA-Transkript eines einzigen einer Speicherprotein-Nukleinsäuresequenz identisch sind, und

ii) einen "antisense"-RNA-Strang, der zu dem RNA-sense-Strang unter i) im wesentlichen komplementären ist.

16. Verminderung des Nikotingehaltes beispielsweise in Tabak durch verminderte Expression beispielsweise der N-Methyl-putrescinoxidase und der Putrescin-N-methyltransferase.

17. Verminderung des Coffeingehaltes in der Kaffeebohne (Coffea arabica) durch durch Verminderung der Genexpression von Genen der Coffeinbiosynthese wie 7-Methylxanthine-3-methyltransferase.

18. Verminderung des Theophyllin-Gehaltes im Tee (Camellia sinensis) durch durch Verminderung der Genexpression von Genen der Theophyllin-Biosynthese wie beispielsweise 1-Methylxanthin-3-methyltransferase.

19. Erhöhung des Methioningehaltes durch Verminderung der Threoninbiosynthese, beispielsweise durch Verminderung derExpression der Threoninsynthase (Zeh M et al .(2001) Plant Physiol 127(3):792-802).

Weitere Beispiele für vorteilhafte Gene sind zum Beispiel genannt bei Dunwell JM, Transgenic approaches to crop improvement, J Exp Bot. 2000;51 Spec No; Seite 487-96.

Jede der oben genannten Anwendungen kann als solche isoliert angewendet werden. Natürlich können auch mehr als eine der oben genannten Ansätze gleichzeitig angewendet werden. Dabei wird bei allen Anwendungen die Expression von mindestens zwei unterschiedlichen Zielgenen, wie oben definiert, vermindert. Diese Zielgene können dabei aus einer einzigen für eine Anwendung bevorzugten Gruppe von Genen stammen oder aber auch unterschiedlichen Anwendungsgruppen zugeordnet sein.

Zur Anwendung der erfindungsgemäßen Verfahren stehen dem Fachmann geläufige Werkzeuge, wie Expressionsvektoren mit für Pflanzen geeigneten Promotoren, sowie Verfahren zur Transformation und Regeneration von Pflanzen zur Verfügung. Pflanzenspezifische Promotoren meint grundsätzlich jeden Promotor, der die Expression von Genen, insbesondere Fremdgenen, in Pflanzen oder Pflanzenteilen, -zellen, -geweben, -kulturen steuern kann. Dabei kann die Expression beispielsweise konstitutiv, induzierbar oder entwicklungsabhängig sein. Bevorzugt sind:

a) Konstitutive Promotoren

"Konstitutive" Promotoren meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten (Benfey et al.(1989) EMBO J 8:2195-2202). Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al. (1980) Cell 21:285-294; Odell et al. (1985) Nature 313:810-812; Shewmaker et al. (1985) Virology 140:281-288; Gardner et al. (1986) Plant Mol Biol 6:221- 228) oder der 19S CaMV Promotor (US 5,352,605; WO 84/02913; Benfey et al. (1989) EMBO J 8:2195-2202). Ein weiterer geeigneter konstitutiver Promotor ist der "Rubisco small subunit (SSU)"-Promotor (US 4,962,028), der LeguminB-Promotor (GenBank Acc.-Nr. X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), den Ubiquitin 1 Promotor (Christensen et al. (1992) Plant Mol Biol 18:675-689; Bruce et al. (1989) Proc Natl Acad Sci USA 86:9692-9696), den Smas Promotor, den Cinnamylalkoholdehydrogenase-Promotor (US 5,683,439), die Promotoren der vakuolärer ATPase Untereinheiten oder der Promotor eines prolinreichen Proteins aus Weizen (WO 91/13991), sowie weitere Promotoren von Genen, deren konstitutive Expression in Pflanzen dem Fachmann bekannt ist.

b) Gewebespezifische Promotoren

Bevorzugt sind ferner Promotoren mit Spezifitäten für die Antheren, Ovarien, Blüten, Blätter, Stengel, Wurzeln und Samen.

Samenspezifische Promotoren wie zum Beispiel der Promotor des Phaseolins (US 5,504,200; Bustos MM et al. (1989) Plant Cell 1(9):839-53), des 2S Albumingens (Joseffson LG et al. (1987) J Biol Chem 262:12196-12201), des Legumins (Shirsat A et al. (1989) Mol Gen Genet 215(2): 326-331), des USP (unknown seed protein; Bäumlein H et al. (1991) Mol Gen Genet 225(3):459-67), des Napin Gens (US 5,608,152; Stalberg K et al. (1996) L Planta 199:515-519), des Saccharosebindeproteins (WO 00/26388) oder der Legumin B4-Promotor (LeB4; Bäumlein H et al. (1991) Mol Gen Genet 225: 121-128; Baeumlein et al. (1992) Plant Journal 2(2):233-9; Fiedler U et al. (1995) Biotechnology (NY) 13(10):1090f), der Oleosin-Promoter aus Arabidopsis (WO 98/45461), der Bce4-Promoter aus Brassica (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2 oder lpt1-Gen (WO 95/15389, WO 95/23230) oder die Promotoren beschrieben in WO 99/16890 (Promotoren des Hordein-Gens, des Glutelin-Gens, des Oryzin-Gens, des Prolamin-Gens, des Gliadin-Gens, des Glutelin-Gens, des Zein-Gens, des Kasirin-Gens oder des Secalin-Gens). Weitere samenspezifische Promotoren sind beschrieben in WO89/03887.

Knollen-, Speicherwurzel- oder Wurzel-spezifische Promotoren wie beispielsweise der Patatin Promotor Klasse I (B33), der Promotor des Cathepsin D Inhibitors aus Kartoffel.

Blattspezifische Promotoren wie Promotor der cytosolischen FBPase aus Kartoffel (WO 97/05900), der SSU Promotor (small subunit) der Rubisco (Ribulose-1,5-bisphosphatcarboxylase) oder der ST-LSI Promotor aus Kartoffel (Stockhaus et al. (1989) EMBO J 8:2445-2451).

Blütenspezifische Promotoren wie beispielsweise der Phytoen Synthase Promotor (WO 92/16635) oder der Promotor des P-rr Gens (WO 98/22593).

- Antheren-spezifische Promotoren wie den 5126-Promotor (US 5,689,049, US 5,689,051), den glob-I Promotor und den $\gamma$-Zein Promotor.

c) Chemisch induzierbare Promotoren

Die Expressionskassetten können auch einen chemisch induzierbaren Promotor enthalten (Übersichtsartikel: Gatz et al. (1997) Annu Rev Plant Physiol Plant Mol Biol 48:89-108), durch den die Expression des exogenen Gens in der Pflanze zu einem bestimmten Zeitpunkt gesteuert werden kann. Derartige Promotoren, wie z.B. der PRP1 Promotor (Ward et al. (1993) Plant Mol Biol 22:361-366), durch Salicylsäure induzierbarer Promotor (WO 95/19443), ein durch Benzolsulfonamid-induzierbarer Promotor (EP 0 388 186), ein durch Tetrazyklin-induzierbarer Promotor (Gatz et al. (1992) Plant J 2:397-404), ein durch Abscisinsäure induzierbarer Promotor (EP 0 335 528) bzw. ein durch Ethanol- oder Cyclohexanon-induzierbarer Promotor (WO 93/21334) können ebenfalls verwendet werden.

d) Stress- oder Pathogen-induzierbare Promotoren

Ferner sind Promotoren bevorzugt, die durch biotischen oder abiotischen Stress induziert werden wie beispielsweise der pathogen-induzierbare Promotor des PRP1-Gens (Ward et al. (1993) Plant Mol Biol 22:361-366), der hitzeinduzierbare hsp70- oder hsp80-Promoter aus Tomate (US 5,187,267), der kälteinduzierare alpha-Amylase Promoter aus der Kartoffel (WO 96/12814), der licht-induzierbare PPDK Promotor oder der verwundungsinduzierte pinII-Promoter (EP375091).

Pathogen-induzierbare Promotoren umfassen die von Genen, die infolge eines Pathogenbefalls induziert werden wie beispielsweise Gene von PR-Proteinen, SAR-Proteinen, $\beta$-1,3-Glucanase, Chitinase usw. (beispielsweise Redolfi et al. (1983) Neth J Plant Pathol 89:245-254; Uknes, et al. (1992) The Plant Cell 4:645-656; Van Loon (1985) Plant Mol Viral 4:111-116; Marineau et al. (1987) Plant Mol Biol 9:335-342; Matton et al. (1987) Molecular Plant-Microbe Interactions 2:325-342; Somssich et al. (1986) Proc Natl Acad Sci USA 83:2427-2430; Somssich et al. (1988) Mol Gen Genetics 2:93-98; Chen et al. (1996) Plant J 10:955-966; Zhang and Sing (1994) Proc Natl Acad Sci USA 91:2507-2511; Warner, et al. (1993) Plant J 3:191-201; Siebertz et al. (1989) Plant Cell 1:961-968(1989).

Umfasst sind auch verwundungs-induzierbare Promotoren wie der des pinII Gens (Ryan (1990) Ann Rev Phytopath 28:425-449; Duan et al. (1996) Nat Biotech 14:494-498), des wun1 und wun2-Gens (US 5,428,148), des win1- und win2-Gens (Stanford et al. (1989) Mol Gen Genet 215:200-208), des Systemin (McGurl et al. (1992) Science 225:1570-1573), des WIP1-Gens (Rohmeier et al. (1993) Plant Mol Biol 22:783-792; Eckelkamp et al. (1993) FEBS Letters 323:73-76), des MPI-Gens (Corderok et al. (1994) The Plant J 6(2):141-150) und dergleichen.

e) Entwicklungsabhängige Promotoren

Weitere geeignete Promotoren sind beispielsweise fruchtreifung-spezifische Promotoren, wie beispielsweise der fruchtreifung-spezifische Promotor aus Tomate (WO 94/21794, EP 409 625). Entwicklungsabhängige Promotoren schließt zum Teil die Gewebespezifischen Promotoren ein, da die Ausbildung einzelner Gewebe naturgemäß entwicklungsabhängig erfolgt.

Besonders bevorzugt sind konstitutive sowie samenspezifische Promotoren.

Genetische Kontrollsequenzen umfassen auch weitere Promotoren, Promotorelemente oder Minimalpromotoren, die die expressionsteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26): 17131 -17135) und Hitzestress (Schoffl F et al., Molecular & General Genetics 217(2-3):246-53, 1989) beschrieben.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15:8693-8711) und dergleichen. Sie können ferner die Gewebsspezifität fördern (Rouster J et al. (1998) Plant J 15:435-440).

Als Kontrollsequenzen geeignete Polyadenylierungssignale sind pflanzliche Polyadenylierungssignale, vorzugsweise solche, die im wesentlichen T-DNA Polyadenylierungssignale aus *Agrobacterium tumefaciens,* insbesondere des Gens 3 der T-DNA (Octopin Synthase) des Ti-Plasmids pTiACHS entsprechen (Gielen et al. (1984) EMBO J 3:835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Eine Expressionskassetten und die von ihr abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemässen Expressionskassetten, Vektoren oder transgenen Organismen haben. Beispielhaft aber nicht einschränkend seien zu nennen:

a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat® (N-(phosphonomethyl)glycin) verleihen, das für das Glyphosat® degradierende Enzyme kodierende gox Gen (Glyphosatoxidoreduktase), das deh Gen (kodierend für eine Dehalogenase, die Dalapon inaktiviert), Sulfonylurea- und Imidazolinon inaktivierende Acetolactatsynthasen sowie bxn Gene, die für Bromoxynil degradierende Nitrilaseenzyme kodieren, das aasa-Gen, das eine Resistenz gegen das Antibiotikum Apectinomycin verleih, das Streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen Sulfonylharnstoff-Herbizide verleiht (z.B. mutierte ALS-Varianten mit z.B. der S4 und/oder Hra Mutation).

b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784), die Chloramphenicoltransferase, eine Luziferase (Ow et al. (1986) Science 234:856-859), das Aequorin-Gen (Prasher et al. (1985) Biochem Biophys Res Commun 126(3): 1259-1268), die β-Galactosidase, ganz besonders bevorzugt ist die β-Glucuronidase (Jefferson et al. (1987) EMBO J 6:3901-3907).

c) Replikationsursprünge, die eine Vermehrung der erfindungsgemässen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

d) Elemente, die für eine Agrobakterium vermittelte Pflanzentransformation erforderlich sind, wie zum Beispiel die rechte oder linke Begrenzung der T-DNA oder die vir-Region.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al. (1986) Plant Cell Reports 5:81-84).

Verschiedene Methoden und Vektoren zum Einschleusen von Genen in das Genom von Pflanzen sowie zur Regeneration von Pflanzen aus Pflanzengeweben oder Pflanzenzellen sind bekannt (Plant Molecular Biology and Biotechnology (CRC Press, Boca Raton, Florida), Kapitel 6/7, S. 71-119 (1993); White FF (1993) Vectors for Gene Transfer in Higher Plants; in: Transgenic Plants, Bd. 1, Engineering and Utilization, Hrsgb.: Kung und Wu R, Academic Press, 15-38; Jenes B et al. (1993) Techniques for Gene Transfer, in: Transgenic Plants, Bd. 1, Engineering and Utilization, Hrsgb.: Kung und R. Wu, Academic Press, S.128-143; Potrykus (1991) Annu Rev Plant Physiol Plant Molec Biol 42:205-225; Halford NG, Shewry PR (2000) Br Med Bull 56(1):62-73). Dazu zählen beispielhaft die oben erwähnten. Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, die Liposomen vermittelte Transformation (wie z.B. in US 4,536,475 beschrieben), biolistische Verfahren mit der Genkanone ("particle bombardment" Methode; Fromm ME et al. (1990) Bio/Technology. 8(9):833-9; Gordon-Kamm et al. (1990) The Plant Cell 2:603), die Elektroporation, die Inkubation trockener Embryonen in DNA-haltiger Lösung und die Mikroinjektion. Im Falle dieser "direkten" Transformationsmethoden sind keine besonderen Anforderungen an das verwendete Plasmid gestellt. Einfache Plasmide wie die der pUC-Reihe, pBR322, M13mp Reihe, pACYC184 etc. können verwendet werden. Sollen vollständige Pflanzen aus den transformierten Zellen regeneriert werden, so ist er erforderlich, das sich auf dem Plasmid ein zusätzliches selektionierbares Markergen befindet.

Neben diesen "direkten" Transformationstechniken kann eine Transformation auch durch bakterielle Infektion mittels Agrobacterium (z.B. EP 0 116 718), virale Infektion mittels viraler Vektoren (EP 0 067 553; US 4,407,956; WO 95/34668; WO 93/03161) oder mittels Pollen (EP 0 270 356; WO 85/01856; US 4,684,611) durchgeführt werden.

Die für die Agrombacterium-Transformation meist verwendeten Stämme Agrobacterium tumefaciens oder Agrobacterium rhizogenes eine auch durch bakterielle Infektion mittels enthalten ein Plasmid (Ti bzw. Ri Plasmid), das auf die Pflanze nach Agrobaterium-Infektion übertragen wird. Ein Teil dieses Plasmids, genannt T-DNA (transferred DNA), wird in das Genom der Pflanzenzelle integriert. Alternativ können durch Agrobakterium auch binäre vektoren (Mini-Ti-Plasmide) auf Pflanzen übertragen und in deren Genom integriert werden. Die Agrobacterium-vermittelte Transformation ist am besten für dicotyledone, diploide Pflanzenzellen geeignet, wohingegen die direkten Transformationstechniken sich für jeden Zelltyp eignen. Verfahren zur Agrobakterium vermittelten Transformation sind beispielsweise beschrieben bei Horsch RB et al. (1985) Science 225:1229f. Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden.

Für die Agrobacterium Tranformation werden bevorzugt binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten.

Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA; Bevan et al.(1984) Nucl Acids Res 12:8711), pBinAR, pPZP200 oder pPTV.

Die mit einem solchen Vektor transformierten Agrobakterien können dann in bekannter Weise zur Transformation von Pflanzen, insbesondere von Kulturpflanzen, wie z.B. von Raps, verwendet werden, indem beispielsweise verwundete Blätter oder Blattstücke in einer Agrobakterienlösung gebadet und anschliessend in geeigneten Medien kultiviert werden. Die Transformation von Pflanzen durch Agrobakterien ist beschrieben (White FF, Vectors for Gene Transfer in Higher Plants; in Transgenic Plants, Vol. 1, Engineering and Utilization, herausgegeben von S.D. Kung und R. Wu, Academic Press, 1993, S. 15 - 38; Jenes B et al.(1993) Techniques for Gene Transfer, in: Transgenic Plants, Vol. 1, Engineering and Utilization, herausgegeben von S.D. Kung und R. Wu, Academic Press, S.128-143; Potrykus (1991) Annu Rev Plant Physiol Plant Molec Biol 42:205- 225). Aus den transformierten Zellen der verwundeten Blätter bzw. Blattstücke können in bekannter Weise transgene Pflanzen regeneriert werden, die integriert die oben beschriebenen erfindungsgemässen Expressionssysteme enthalten.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind oben genannt und umfassen bevorzugt das bar Gen, dass Resistenz gegen das Herbizid Phosphinotricin verleiht (Rathore KS et al. (1993) Plant Mol Biol 21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten vorzugsweise kultiviert werden, um sicherzustellen, dass die genomische Integration stabil und vererblich ist.

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden. Entsprechende Verfahren sind beschriebe (Fennell et al. (1992) Plant Cell Rep. 11: 567-570; Stoeger et al (1995) Plant Cell Rep. 14:273-278; Jahne et al. (1994) Theor Appl Genet 89:525-533).

Die Wirksamkeit der Expression der transgen exprimierten Nukleinsäuren kann beispielsweise *in vitro* durch Sprossmeristemvermehrung unter Verwendung einer der oben beschriebenen Selektionsmethoden ermittelt werden. Zudem kann eine in Art und Höhe veränderte Expression eines Zielgens und die Auswirkung auf den Phänptyp der Pflanze an Testpflanzen in Gewächshausversuchen getestet werden.

III. Biotechnologische Anwendungen

Das erfindungsgemäße Verfahren läßt sich vorteilhaft in biotechnologischen Verfahren anwenden.

Als Selektionsmarker können prinipiell viele der auch für Pflanzen bevorzugten Selektionssysteme verwendet werden.

Die pflanzlichen Teile - wie zum Beispiel bei transgenen pflanzlichen Organismen Wurzeln, Blätter etc.- , und transgenes Vermehrungsgut wie Saaten oder Früchte, können zur Herstellung von Nahrungs- oder Futtermitteln, Pharmazeutika oder Feinchemikalien, wie beispielsweise Enzymen, Vitaminen, Aminosäuren, Zucker, Fettsäuren, natürliche und synthetische Geschmacks-, Aroma- und Farbstoffe verwendet werden. Besonders bevorzugt ist die Produktion von Triaclyglyceriden, Lipiden, Ölen, Fettsäuren, Stärke, Tocopherolen und Tocotrienolen sowie Carotinoiden. Von Menschen und Tieren verzehrbare erfindungsgemässe, genetisch veränderte Pflanzen können auch beispielsweise direkt oder nach an sich bekannter Aufbereitung als Nahrungsmittel oder Futtermittel verwendet werden.

Sequenzen

1. SEQ ID NO: 1

Nukleinsäuresequenz kodierend für A.thaliana Albumin 2S subunit 1 (GenBank Acc.-No.: M22032)

2. SEQ ID NO: 2
Proteinsequenz kodierend für A.thaliana Albumin 2S subunit 1

3. SEQ ID NO: 3
Nukleinsäuresequenz kodierend für A.thaliana Albumin 2S subunit 3 (GenBank Acc.-No.: M22035)

4. SEQ ID NO: 4
Proteinsequenz kodierend für A.thaliana Albumin 2S subunit 3

5. SEQ ID NO: 5
Nukleinsäuresequenz kodierend für A.thaliana Albumin 2S subunit 2 (GenBank Acc.-No.: M22034)

6. SEQ ID NO: 6
Proteinsequenz kodierend für A.thaliana Albumin 2S subunit 2

7. SEQ ID NO: 7
Nukleinsäuresequenz kodierend für A.thaliana Albumin 2S subunit 4 (GenBank Acc.-No.: M22033)

8. SEQ ID NO: 8
Proteinsequenz kodierend für A.thaliana Albumin 2S subunit 4

9. SEQ ID NO: 9 .
Nukleinsäuresequenz kodierend für B.napus Cruciferin Speicherprotein (GenBank Acc.-No.: X59294)

10. SEQ ID NO: 10
Proteinsequenz kodierend für B.napus Cruciferin Speicherprotein

11. SEQ ID NO: 11
Nukleinsäuresequenz kodierend für Brassica napus Cruciferin (GenBank Acc.-No.: X14555)

12. SEQ ID NO: 12
Proteinsequenz kodierend für Brassica napus Cruciferin

13. SEQ ID NO: 13
Nukleinsäuresequenz kodierend für B.napus BnC2 Cruciferin Speicherprotein (GenBank Acc.-No.: X59295)

14. SEQ ID NO: 14
Proteinsequenz kodierend für B.napus BnC2 Cruciferin Speicherprotein

15. SEQ ID NO: 15
partielle Nukleinsäuresequenz kodierend für B.napus Cruciferin cru4 subunit (GenBank Acc.-No.- X57848)

16. SEQ ID NO: 16
partielle Proteinsequenz kodierend für B.napus Cruciferin cru4 subunit

17. SEQ ID NO: 17
Nukleinsäuresequenz kodierend für B.napus cru1 Cruciferin subunit (GenBank Acc.-No.: X62120)

18. SEQ ID NO: 18
Proteinsequenz kodierend für B.napus cru1 Cruciferin subunit

19. SEQ ID NO: 19
Nukleinsäuresequenz kodierend für Glycinin A-1a-B-x subunit aus des Sojabohne (GenBank Acc.-No.: M36686)

20. SEQ ID NO : 20
Proteinsequenz kodierend für Glycinin A-1a-B-x subunit aus des Sojabohne

21. SEQ ID WO: 21
Nukleinsäuresequenz kodierend für Sojabohne Glycinin subunit G2 (GenBank Acc.-No..- X15122)

22. SEQ ID NO: 22
Proteinsequenz kodierend für Sojabohne Glycinin subunit G2

23. SEQ ID NO: 23
Nukleinsäuresequenz kodierend für Sojabohne A5A4B3 Glycinin subunits (GenBank Acc.-No.: X02626)

24. SEQ ID NO: 24
Proteinsequenz kodierend für Sojabohne A5A4B3 Glycinin sudunits

25. SEQ ID NO: 25 .
Nukleinsäuresequenz kodierend für Sojabohne (G.max) Glycinin Speicherprotein subunit A3-B4 (GenBank Acc.-No.: M10962)

26. SEQ ID NO: 26
Proteinsequenz kodierend für Sojabohne (G.max) Glycinin Speicherprotein subunit A3-B4

27. SEQ I NO: 27
Nukleinsäuresequenz kodierend für Sojabohne Glycinin subunit G3 (GenBank Acc.-Xo.: X15123)

28. SEQ ID NO: 28
Proteinsequenz kodierend für Sojabohne Glycinin subunit G3

29. SEQ ID NO: 29
Nukleinsäuresequenz kodierend für Sonnenblume 11S. Speicherprotein (G3-D1) (GenBank Acc.-No.: M28832)

30. SEQ ID NO: 30
Proteinsequenz kodierend für sonnenblume 11S Speicherprotein (G3-D1)

31. SEQ ID NO: 31
Nukleinsäuresequenz kodierend für Raps (B.napus) Napin (GenBank Acc.-No.: J02586)

32. SEQ ID NO: 32
Proteinsequenz kodierend für Raps (B.napus) Napin

33. SEQ ID NO: 33
Nukleinsäuresequeuz kodierend für Brassica juncea 2S Speicherprotein (GenBank Acc.-No.: X65040)

34. SEQ ID NO: 34
Proteinsequenz kodierend für Brassica juncea 2S Speicherprotein

35. SEQ ID NO: 35
Nukleinsäuresequenz kodierend für Brassica oleracea 2S Speicherprotein (GenBank Acc.-No.: Z65038)

36. SEQ ID NO: 36
Proteinsequenz kodierend für Brassica oleracea 2S Speicherprotein

37. SEQ ID NO: 37
Nukleinsäuresequenz kodierend für Brassica napus cv. Topas Napin (GenBank Acc.-No.: U04945)

38. SEQ ID NO: 38
Proteinsequenz kodierend für Brassica napus cv. Topas Napin

39. SEQ ID NO: 39
partielle Nukleinsäuresequenz kodierend für Sinapis alba sin1 Speicherprotein (GenBank Acc.-No.: X91799)

40. SEQ ID NO: 40
partielle Proteinsequenz kodierend für Sinapis alba sin1 Speicherprotein

41. SEQ ID NO: 41
Nukleinsäuresequenz kodierend für Sojabohne (Glycine max)napin-type2SAlbumin 1 (GenBankAcc.-No.: U71194)

42. SEQ ID NO: 42
Proteinsequenz kodierend für Sojabohne (Glycine max) napintype 2S Albumin 1

43. SEQ ID NO: 43
Nukleinsäuresequenz kodierend für Sojabohne (Glycine max) 2S Albumin (GenBank Acc.-No.: AF005030)

44. SEQ. ID NO: 44
Proteinsequenz kodierend für Sojabohne (Glycine max) 25 Albumin

45. SEQ ID NO: 45
Nukleinsäuresequenz kodierend für Brassica nigra 2S Speicherprotein (GenBank Acc.-No.: X65039)

46. SEQ ID NO: 46
Proteinsequenz kodierend für Brassica nigra 2S Speicherprotein

47. SEQ ID NO: 47
Nukleinsäuresequenz kodierend für Sinapis alba sin5 Speicherprotein (GenBank Acc.-No.: X91798)

48. SEQ ID NO: 48
Proteinsequenz kodierend für Sinapis alba sin5 Speicherprotein

49. SEQ ID NO: 49
Nukleinsäuresequenz kodierend für Sonnenblume HaG5 2 S Albumin (GenBank Acc.-No.: X06410)

50. SEQ ID NO: 50
proteinsequenz kodierend für Sonnenblume HaG5 2 S Albumin

51. SEQ ID NO: 51
partielle Nukleinsäuresequenz kodierend für Sonnenblume (Helianthus annuus) 2S Albumin (GenBank Acc.-No.: X76101)

52. SEQ ID NO: 52
partielle Proteinsequenz kodierend für Sonnenblume (Helianthus annuus) 2S Albumin

53. SEQ ID NO: 53
Nukleinsäuresequenz kodierend für dsRNA zur Suppression von Arabidopsis thaliana 12S Speicherprotein AtCru3 (Insert von Vektor pCR2.1-AtCRU3-RNAi)

54. SEQ ID NO: 54
Ribonukleinsäuresequenz kodierend für dsKNA zur Suppression von Arabidopsis thaliana 12S speicherprotein AtCru3

55. SEQ ID NO: 55
Nükleinsäuresequenz kodierend für dsRNA zur Suppression von Arabidopsis thaliana 12S Speicherprotein AtCral

56. SEQ ID NO: 56
Ribonukleinsäuresequenz kodierend für dsRNA zur Suppression von Arabidopsis thaliana 12S Speicherprotein AtCral

57. SEQ ID NO: 57
Nukleinsäuresequenz kodierend für dsRNA zur Suppression von Arabidopsis thaliana 2S Speicherprotein At2S2

58. SEQ ID NO: 58
Ribonukleinsäuresquenz kodierend für dsRNA zur Suppression von Arabidopsis thaliana 2S Speicherprotein At2S2

59. SEQ ID NO: 59
Nukleinsäuresequenz kodierend für Arabidopsis thaliana 12S Cruciferin Speicherprotein (ATCRU3 ; GenBank Acc.-No.: U66916)

60. SEQ ID NO: 60
Proteinsequenz kodierend für Arabidopsis thaliana 12S Cruciferin Speicherprotein (ATCRU3)

61. SEQ ID NO: 61
Nukleinsäuresequenz kodierend für A. thaliana 12S Speicherprotein (CRAI; GenBank Acc.-No.: M37247)

62. EQ ID NO: 62
Proteinsequenz kodierend für A.thaliana 12S Speicherprotein (CRA1)

63. SEQ ID NO: 63
Nukleinsäuresequenz kodierend für Arabidopsis thaliana 12S Speicherprotein AT5g44120/MLN1_4 (GenBank Acc.-No.: AY070730)

64. SEQ ID NO: 64
Proteinsequenz kodierend für Arabidopsis thaliana 12S Speicherprotein AT5g44120/MLN1_4

65. SEQ ID NO: 65
Nukleinsäuresequenz kodierend für Arabidopsis 12S Speicherprotein (CRB; GenBank Acc.-No.: X14313; M37248)

66. SEQ ID NO: 66
Proteinsequenz kodierend für Arabidopsis 12S Speicherproteine (CRB)

67. SEQ ID NO: 67
Nukleinsäuresequenz kodierend für Arabidopsis thaliana putatives 12S Speicherproteine (aus GenBank Acc.-No.: AC003027)

68. SEQ ID NO: 68
Proteinsequenz kodierend für Arabidopsis thaliana putatives Speicherproteine (Protein_id="AAD10679.1)

69. SEQ ID NO: 69
Nukleinsäuresequenz kodierend für Arabidopsis thaliana Cruciferin 12S Spwicherprotein (At1g03890) (GenBank Acc.-No.: AY065432)

70. SEQ ID NO: 70
Proteinsequenz kodierend für Arabidopsis thaliana Cruciferin 12S Speicherprotein (At1g03890)

71.SEQ ID NO: 71
Nukleinsäuresequenz kodierend für Arabidopsis thaliana Prohibitin 1 (Atphb1) (GenBank Acc.-No.: U66594)

72. SEQ ID NO: 72
Proteinsequenz kodierend für Arabidopsis thaliana Prohibitin 1 (Atphb1)

73. SEQ ID NO: 73 Oligonukleotidprimer OPN1

74. SEQ ID NO: 74 Oligonukleotidprimer OPN2

75. SEQ ID NO: 75 oligonukleotidprimer OPN3

76. SEQ ID NO: 76 Oligonukleotidprimer OPN4

77. SEQ ID NO: 77 Oligonukleotidprimer OPN5

78. SEQ ID NO: 78 Oligonukleotidprimer OPN6

79. SEQ ID NO: 79 Oligonukleotidprimer OPN7

80. SEQ ID NO: 80 Oligonukleotidprimer OPN8

81. SEQ ID NO: 81 Oligonukleotidprimer OPN9

82. SEQ ID NO: 82 Oligonukleotidprimer OPN10

83. SEQ ID NO: 83
Nukleinsäuresequenz kodierend für sRNAi4-dsRNA zur Suppression mehrerer Speicherproteine

84. SEQ ID NO: 84
Ribonukleinsäuresequenz kodierend für sRNAi4-dsRNA zur Suppression mehrerer Speicherproteine

85. SEQ ID NO: 85
Nukleinsäuresequenz kodierend für sRNAi8-dsRNA zur Suppression mehrerer Speicherproteine

86. SEQ ID NO: 86
Ribonukleinsäuresequenz kodierend für sRNAi8-dsRNA zur Suppression mehrerer Speicherproteine

87. SEQ ID NO: 87 Oligonukleotidprimer OPN11

88. SEQ ID NO: 88 Oligonukleotidprimer OP12

89. SEQ ID NO: 89 Oligonukleotidprimer OPN13

90. SEQ ID NO: 90 Oligonukleotidprimer OPN15

91. SEQ ID NO: 91 Oligonukleotidprimer OPN16

92. SEQ ID NO: 92 Oligonukleotidprimer OPN17

93. SEQ ID NO : 93
Nukleinsäuresequenz kodierend für Arabidopsis thaliana "globulin-like protein" (GenBank Acc.-No.- NM_119834)

94. SEQ ID NO: 94
Proteinsequenz kodierend für Arabidopsis thaliana "globulinlike protein" (Protein_id="NP_195388.1)

95. SEQ ID NO: 95
Nukleinsäuresequenz kodierend für Glycine max 7S Samenglobulin (GenBank Acc.-No.: U59425)

96. SEQ ID NO: 96
Proteinsequenz kodierend für für Glycine max 7S Samenglobulin

97. SEQ ID NO: 97
Nukleinsäuresequenz kodierend für Zea mays 19kD Zein (GenBank Acc.-No.; E01144)

98. SEQ ID NO: 98
Proteinsequenz kodierend für Zea mays 19kD Zein

99. SEQ ID NO:99
Nukleinsäuresequenz kodierend für zea mays 19kD alpha Zein B1 (GenBank Acc.-No.: AF371269)

100. SEQ ID NO : 100
Proteinsequenz kodierend für zea mays 19kD alpha Zein B1

101. SEQ ID NO: 101
Nukleinsäuresequenz kodierend für Zea mays 19kD alpha Zein B2 (GenBank Acc.-No.: AF371270)

102. SEQ ID NO: 102
Proteinsequenz kodierend für Zea mays 19kD alpha Zein B2

103. SEQ ID NO: 103
Nukleinsäuresequenz kodierend für Zea mays 22kD alpha-zein (GenBank Acc.-No.: X61085)

104.SEQ ID NO: 104
Proteinsequenz kodierend für Zea mays 22kD alpha-zein

105.SEQ ID NO: 105
Nukleinsäuresequenz kodierend für Oryza sativa Prolamin (GenBank Acc.-No.: AB016503)

106.SEQ ID NO: 106
Proteinsequenz kodierend für Oryza sativa Prolamin

107.SEQ ID NO: 107
Nukleinsäuresequenz kodierend für A. sativa Avenin (GenBank Acc.-No.: M38446)

108.SEQ ID NO: 108
Proteinsequenz kodierend für A. sativa Avenin

109.SEQ ID NO: 109
Nukleinsäuresequenz kodierend für Hordeum vulgare C-Hordein (GenBank Acc.-No.: M36941)

110. SEQ ID NO: 110
Proteinsequenz Teil 1 kodierend für Hordeum vulgare C-Hordein

111. SEQ ID NO: 111
Proteinsequenz Teil 2 kodierend für Hordeum vulgare C-Hordein

112. SEQ ID NO: 112
Nukleinsäuresequenz kodierend für Triticum aestivum LMW Glutenin-1D1 (GenBank Acc.-No.: X13306)

113. SEQ ID NO: 113
Proteinsequenz kodierend für Triticum aestivum LMW Glutenin-1D1

114. SEQ ID NO: 114
Binärer Expressionsvektor für Agrobakterium vermittelte Pflanzentransformation pSUN2-USP.

115.SEQ ID NO: 115
Partielle Nukleinsäuresequenz kodierend für Homogentisat-1,2-dioxygenase aus Brassica napus (HGD; EC-Nr.: 1.13.11.5)

116.SEQ ID NO: 116
Nukleinsäuresequenz kodierend für Homogentisat-1,2-dioxygenase aus Arabidopsis thaliana (HGD; EC-Nr.: 1.13.11.5)

117.SEQ ID NO: 117
Proteinsequenz kodierend für Homogentisat-1,2-dioxygenase aus Arabidopsis thaliana (HGD; EC-Nr.: 1.13.11.5)

118.SEQ ID NO: 118
Nukleinsäuresequenz kodierend für Maleylacetoacetatisomerase aus Arabidopsis thaliana (MAAI; EC NI.: 5.2.1.2.)

119. SEQ ID NO: 119
Proteinsequenz kodierend für Maleylacetoacetatisomerase aus Arabidopsis thaliana (MAAI ; EC-Nr.: 5.2.1.2.)

120.SEQ ID NO: 120
Nukleinsäuresequenz kodierend für Fumarylacetoacetathydrolase aus Arabidopsis thaliana (FAAH; EC Nr.: 3.7.1.2)

121. SEQ ID NO: 121
Proteinsequenz kodierend für Fumarylacetoacetathydrolase aus Arabidopsis thaliana (FAAH; EC-Nr.: 3.7.1.2)

122. SEQ ID NO: 122
Nukleinsäuresequenz kodierend für supressionskonstrukt 2 (p3300.1-Toc159-GFP-RNAi)

123. SEQ ID NO: 123 Oligonukleotidprimer OPN18

124. SEQ ID NO: 124 Oligonukleotidprimer OPN19

125. SEQ ID NO: 125 Oligonukleotidprimer OPN20

126. SEQ ID NO: 126 Oligonukleotidprimer OPN21

Abbildungen

1. Fig.1: Schematische Darstellung der Speicherprotein-Suppressionskonstrukte.
Insert aus Vektor pCR2.1-sRNAi4 (1) (vgl. Beispiel 2d) und pCR2.1-sRNAi8 (2) (vgl. Beispiele) kodierend für eine die AtCru3-, AtCRB und At253-Expression supprimierende dsRNA.
In den beiden Konstrukten sind die "sense"-Ribonukleotidsequenzen und die komplementären "antisense"-Ribonukleotidsequenzen (symbolisiert durch auf dem Kopf stehende Buchstaben) für die einzelnen zu supprimierenden Zielgene (AtCru3; AtCRB, At2S3) unterschiedlich angeordnet. Schraffierte Bereiche (I1, I2 etc.) stellen Intronsequenzen (Linker) dar.
2. Fig.2A-D: Symbolische Darstellung verschiedener dsRNAs in ihrer Sekundärstruktur.

| | |
|---|---|
| S1, S2, ... S (n) : | "sense"-Ribonukleotidsequenz |
| AS1,AS2,...AS(n): | "antisense"-Ribonukleotidsequenz |
| I: | Intronsequenz |

Die Anordnung der einzelnen "sense"-Ribonukleotidsequenzen und "antisense"-Ribonukleotidsequenzen kann so erfolgen, dass zunächst alle "sense"-Ribonukleotidsequenzen aneinander gereiht werden und so quasi einen "Sense"-Strang bilden, wodrauf dann alle "antisense"-Ribonukleotidsequenzen aneinander zu einem "antisense"-Strang zusammengefügt werden (A und C).
Alternativ kann die Anordnung der einzelnen "sense"-Ribonukleotidsequenzen und "antisense"-Ribonukleotidsequenzen so erfolgen, dass Paare von jeweils komplementären "sense"-Ribonukleotidsequenzen und "antisense"-Ribonukleotidsequenzen aneinander gefügt werden (B und D).
"sense"-Ribonukleotidsequenzen und "antisense"-Ribonukleotidsequenzen können direkt aneinandergefügt sein (A und B) oder aber durch weitere Sequenzen beispielsweise Introns (I) voneinander getrennt sein (C und D).
3. Fig.3A-C: Symbolische Darstellung verschiedener dsRNAs in ihrer Sekundärstruktur.

| | |
|---|---|
| S1, S2, ... S(n): | "sense"-Ribonukleotidsequenz |
| AS1,AS2,...AS(n): | "antisense"-Ribonukleotidsequenz |
| SP: | "SPACER" |
| RE: | Erkennungssequenz für Ribozym |
| R: | Ribozym. |

"sense"-Ribonukleotidsequenzen und "antisensen"-RibonukleotidSequenzen können durch weitere Sequenzen ("SPACER"; SP) voneinander getrennt sein (A). Der Spacer kann beispielsweise eines Erkennunssseguenz für ein Ribozym sein. Das korrespondierende Ribozym kann separat exprimiert werden (B) oder aber auch ebenfalls von dem Spacer kodiert sein (C).
4. Fig.4: Abbildung des Supressionskonstrukts mit den entsprechenden Restriktionsenzymschnittstellen:
5. Fig.5A: Identifikation einer Pflanze, die den Albino-Phänotyp zeigt (links). Der Phänotyp ist identisch zur ppi2 Mutante, die Toc159 nicht mehr exprimieren kann. Als Kontrolle pflanzen mit Wildtyp Phänotyp, die parallel gewachsen sind.
Fig. 5B: Fluoreszenz-Analyse der Pflanzen aus Fig.5A. Anregung der Fluoreszenz durch Licht im Wellenlängenbereich 470-490 nm. Es wurde dieselbe Vergrösserung gewählt wie in Fig.5A.

Beispiele

Allgemeine Methoden:

Alle Chemikalien, wenn nicht anders erwähnt, stammen von den Firmen Fluka (Buchs), Merck (Darmstadt), Roth (Karlsruhe), Serva (Heidelberg) und Sigma (Deisenhofen). Restriktionsenzyme, DNA-modifizierende Enzyme und Molekularbiologie-Kits wurden von den Firmen Amersham-Pharmacia (Freiburg), Biometra (Göttingen), Roche (Mannheim), New England Biolabs (Schwalbach), Novagen (Madison, Wisconsin, USA), Perkin-Elmer (Weiterstadt), Qiagen (Hilden), Stratagen (Amsterdam, Niederlande), Invitrogen (Karlsruhe) und Ambion (Cambridgeshire, United Kingdom). Die verwendeten Reagenzien wurden entsprechend der Angaben des Herstellers eingesetzt.

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma ABI nach der Methode von Sanger (Sanger et al. (1977) Proo Natl Acad Sci USA 74:5463-5467).

Beispiel 1: Allgemeine Verfahren

Die Pflanze Arabidopsis thaliana repräsentiert ein Mitglied der höheren Pflanzen (Samenpflanzen). Diese Pflanze ist eng verwandt mit anderen Pflanzenarten aus der Familie der Cruciferen wie z.B. Brassica napus, aber auch mit anderen Pflanzenfamilien der Dikotyledonen. Aufgrund des hohen Grades an Homologie ihrer DNA-Sequenzen bzw. Polypeptidsequenzen kann Arabidopsis thaliana als Modellpflanze für andere Pflanzenarten eingesetzt werden.

a) Anzucht von Arabidopsis Pflanzen

Die Pflanzen werden entweder auf Murashige-Skoog Medium mit 0,5 % Saccharose (Ogas et al. (1997) Science 277:91-94) oder auf Erde gezogen (Focks & Benning (1998) Plant Physiol 118:91-101). Um einheitliche Keimungs- und Blühzeiten zu erreichen, werden die Samen nach Ausplattieren bzw. Ausstreuen auf Erde zwei Tage bei 4° C stratifiziert. Nach der Blüte werden die Schoten markiert. Entsprechend der Markierungen werden dann Schoten mit einem Alter von 6 bis 20 Tagen nach der Blüte geerntet.

b) Isolierung von total RNA und poly-$A^+$ RNA aus Pflanzen

Für die Herstellung von Supressionskonstrukten wird RNA bzw. poly$A^+$ DNA isoliert. RNA wurde aus Schoten von Arabidopsis Pflanzen nach folgender Vorschrift isoliert: Schotenmaterial im Alter von 6 bis 20 Tage nach Blühte wurde geerntet und in flüssigem Stickstoff schockgefroren. Das Material wurde vor der weiteren Verwendung bei -80°C gelagert. 75 mg des Materiales wurde im gekühlten Mörser zu einem feinem Pulver gemahlen und mit 200 $\mu$L des Lysis-Puffers aus dem Ambion RNAqueos-Kit versetzt. Die Isolierung der totalen RNA wurde dann nach Herstellerangaben durchgeführt. Die RNA wurde mit. 50 $\mu$L Elutionspuffer (Ambion) eluiert und die Konzentration durch Absorption einer 1 zu 100 verdünnten Lösung am Photometer (Eppendorf) bei 260 nm bestimmt. 40 $\mu$g/ml. RNA entspricht dabei einer Absorption von 1. Die RNA-Lösungen wurden mit RNAse freiem Wasser auf eine Konzentration von 1 $\mu$g/$\mu$L eingestellt. Die Konzentrationen wurden durch Agarosegelelektrophorese überprüft. Zur Isolierung von polyA+ RNA wurde oligo (dT)-Zellulose von Amersham Pharmacia nach Herstellerangaben verwendet. RNA bzw. polyA+ RNA wurde bei -70°C gelagert.

c) Konstruktion der cDNA-Bank

Zur Konstruktion der cDNA-Bank aus Arabidopsis Schoten-RNA wurde die Erststrangsynthese unter Verwendung von Reverser Transkriptase aus Maus-Leukämie-Virus (Clontech) und Oligo-d(T)-Primern, die Zweitstrangsynthese durch Inkubation mit DNA-Polymerase I, Klenow-Enzym und RNAse H-Spaltung bei 12°C (2 Std.), 16°C (1 Std.) und 22°C (1 Std.) erzielt. Die Reaktion wurde durch Inkubation bei 65°C (10 min) gestoppt und anschließend auf Eis überführt. Doppelsträngige DNA--Moleküle wurde mit T4-DNA-Polymerase (Roche, Mannheim) bei 37°C (30 min) mit glatten Enden verstehen. Die Nukleotide wurden durch Phenol/Chloroform-Extraktion und Sephadex-G50-Zentrifugiersäulen entfernt. EcoRI/XhoI-Adapter (Pharmacia, Freiburg, Deutschland) wurden mittels T4-DNA-Ligase (Roche, 12°C, über Nacht) an die cDNA-Enden ligiert, mit XhoI nachgeschnitten und durch Inkubation mit Polynukleotidkinase (Roche, 37°C, 30 min) phosphoryliert. Dieses Gemisch wurde der Trennung auf einem Low-Melting-Agarose-Gel unterwerfen. DNA-Moleküle über 300 Basenpaaren wurden aus dem Gel eluiert, Phenol-extrahiert, auf Elutip-D-Säulen (Schleicher und Schüll, Dassel, Deutschland) konzentriert und an Vektorarme ligiert und in lambda-ZAPII-Phagen oder lambda-ZAP-Express-Phagen unter Verwendung des Gigapack Gold-Kits (Stratagene, Amsterdam, Niederlande) verpackt, wobei Material des Herstellers verwendet und seine Anweisungen befolgt wurden.

d) Isolierung von genomischer DNA aus Pflanzen wie *Arabidopsis thaliana* oder *Brassica napus* (CTAB-Methode)

Zur Isolierung genomischer DNA aus Pflanzen wie *Arabidopsis thaliana* oder Brassica *napus* werden ca. 0,25 g Blattmaterial junger Pflanzen im vegetativen Stadium in flüssigem Stickstoff zu feinem Pulver gemörsert. Das pulverisierte Pflanzenmaterial wird zusammen mit 1 ml 65°C-warmem CTAB I-Puffer (CTAB : Hexadecyltrimethylammoniumbromid, auch genannt Cetyltrimethylammoniunbromid; Sigma Kat.-Nr.: H6269) und 20 (μl β-Mercaptoethanol in einen vorgewärmten zweiten Mörser gegeben und nach vollständiger Homogenisierung wird der Extrakt in ein 2 ml Eppendorf-Gefäß überführt und für 1 h bei 65°C unter regelmäßiger, vorsichtiger Durchmischung inkubiert. Nach Abkühlung auf Raumtemperatur wird der Ansatz mit 1 ml Chloroform/Octanol (24:1, mit 1M Tris/HCl, pH 8,0 ausgeschüttelt) durch langsames. Invertieren extrahiert und zur Phasentrennung für 5 min bei 8,500 rpm (7,500 x g) und Raumtemperatur zentrifugiert. Anschließend wird die wässrige Phase erneut mit 1 ml Chloroform/Octanol extrahiert, zentrifugiert und durch Invertieren mit 1/10 Volumen auf 65°C vorgewärmten STAB II-Puffer sorgfältig gemischt. Anschließend wird der Ansatz durch vorsichtiges Schwenken mit 1 ml Chloroform/Octanol-Gemisch (siehe oben) versetzt und zur erneuten Phasentrennung für 5 min bei 8,500 rpm (7,500 x g) und Raumtemperatur zentrifugiert. Die wässrige untere Phase wird in ein frisches Eppendorf-Gefäß überführt und die obere organische Phase wird in einem frischen Eppendorf-Gefäß erneut für 15 min bei 8,500 rpm (7,500 x g) und Raumtemperatur zentrifugiert. Die hieraus resultierende wässrige Phase wird mit der wässrigen Phase des vorherigen Zentrifugationssohrittes vereinigt und der gesamte Ansatz mit exakt demselben Volumen vorgewärmten STAB III-Puffer versetzt. Es folgt eine Inkubation bei 65°C, bis die DNA in Flocken ausfällt. Dies kann bis zu 1 h dauern oder durch Inkubation bei 37°C über Nacht erfolgen. Das aus dem anschließenden Zentrifugationsschritt (5 min, 2000 rpm (500 x g), 4°C) resultierende Sediment wird mit 250 μl auf 65°C vorgewärmtem CTAB IV-Puffer versetzt und für mindestens 30 min bzw. bis zur vollständigem Auslösung des Sediments bei 65°C inkubiert. Anschließend wird die Lösung zur Fällung der DNA mit 2,5 Volumina eiskaltem Ethanol vermischt und für 1h bei -20°C inkubiert. Alternativ kann der Ansatz mit 0.6 Volumina Isopropanol vermischt und ohne weitere Inkubation sofort für 15 min bei 8,500 rpm (7,500 x g) und 4°C zentrifugiert werden. Die sedimentierte DNA wird durch Invertierten des zweimal mit je 1 ml 80%igem eiskaltem Ethanol gewaschen, nach jedem Waschschritt erneut zentrifugiert (5 min, 8,500 rpm (7,500 x g), 4°C) und anschließend für ca. 15 min luftgetrocknet. Abschließen wird die DNA in 100 μl TE mit 100 μg/ml RNase resuspendiert und für 30 min bei Raumtemperatur inkubiert.. Die DNA Lösung ist nach einer weiteren Inkubationsphase über Nacht bei 4°C homogen und kann für weiterführende Experimente verwendet werden.

Lösungen für CTAB:

Lösung I (für 200 ml):

100 mM Tris/HCl pH 8,0 (2,42 g)
1,4 M NaCl (16,36 g)
20 mM EDTA (8,0 ml von 0,5 M Stammlösung)
2 % (w/v) CTAB (4,0 g)

Jeweils vor der Verwendung werden frisch zugesetzt:
2 % ß-Mercaptoethanol (20 μl für 1 ml Lösung I).

Lösung II (für 200 ml) :

0,7 M NaCl (8.18 g)
10 %(w/v) CTAB (20 g)

Lösung III (für 200 ml) :

50 mM Tris/HCl pH 8,0 (1,21 g)

10 mM EDTA (4 ml 0, 5 M von 0, 5 M Stammlösung)
1 %(w/v) CTAB (2,0 g)

Lösung IV (High-salt TE) (für 200 ml):

10 mM Tris/ HCl pH 8,0 (0,242 g)
0,1 mM EDTA (40 µl 0.5 M Stammlösung)
1 M NaCl (11, 69 g)

Chloroform/Octanol (24:1) (für 200 ml):

192 ml Chloroform
8 ml Octanoyl
Die Mischung wird 2x mit 1 M TrisHCl pH 8,0 ausgeschüttelt und vor Licht geschützt gelagert.

Beispiel 2: Herstellung von Suppressionskonstrukten

Ausgehend von der genomischer Arabidopsis thaliana DNA oder cDNA wurden über PCR mittels der aufgeführten Oligonukleotide folgende Fragmente von Speicherproteinsequenzen amplifiziert. Dabei kam nachfolgendes PCR Protokoll zum Einsatz:
Zusammensetzung des PCR-Ansatzes (50 µL) :

5,00 µL Template cDNA oder genamische DNA (ca. 1 µg)
5,00 µL 10x Puffer (Advantage-Polymerase) + 25 mM $MgCl_2$
5,00 µL 2mM dNTP
1,25 µL je Primer (10 pmol/µL)
0,50 µL Advantage-Polymerase (Clontech)

PCA-Programn: Anfangsdenaturierung für 2 min bei 95°C, dann 35 Zyklen mit 45. sec 95°C, 45 sec 55°C und 2 min 72°C. Abschliessende Extension von 5 min bei 72°C.

a) Ausgangsvektor pCR2.1-AtCRU3-RNAi

Aus genomischerArabidopsis thaliana DNA wird mit nachfolgenden Oligonukleotid-Primerpaar ein Exonbereich mit dem vollständigen anschließenden Intron einschließlich der an das Intron anschließenden Spleiß-Akzeptorsequenz des 12S Speicherprotein AtCRU3 (Basenpaar 1947 bis 2603 der Sequenz mit der GenBank Acc.-No: U66916) amplifiziert:

ONP1 (SEQ ID NO: 134):
5'-ATAAGAATGCGGCCGCGTGTTCCATTTGGCCGGAAACAAC-3'

ONP2 (SEQ ID NO: 135):
5'-CCCGGATCCTTCTGTAACATTTGACAAAACATG-3'

Das PCR-Produkt wird in den pCR2.1-TOPO Vektor (Invitrogen) gemäss Herstellerangaben kloniert, resultierend in dem pCR2.1-1 vektor und die Sequenz überprüft.
Für die den antisense-Strang der dsRNA kodierende Sequenz wird aus Arabidopsis thaliana DNA lediglich das gleiche Exon wie oben (Basenpaar 1947 bis 2384) mit dem nachfolgenden Primerpaar amplifiziert:

ONP3 (SEQ ID NO: 136);
5'ATAAGAATGCGGCCGCGTGTTCCATTTCGCCGGAAACAAC-3'

ONP4 (SEQ ID NO: 137) :
5' ATAAGAATGCGGCCGCGGATCCACCCTGGAGAACCACGAGTG-3'

Das PCR-Produkt wird in den pCR2.1-TOPO Vektor (Invitrogen) gemäss Heretellerangaben kloniert, resultierend in dem pCR2.1-2 Vektor und die Sequenz überprüft.
0,5 µg von Vektor pCR2.1-1 werden mit dem Restriktionsenzym BamHI (New England Biolabs) für 2 Stunden nach Rerstellerangaben inkubiert und dann für 15 min mit alkalischer Phosphatase (New England Biolabs) dephosphoryliert. Der so präparierte Vektor (1 μL) wird dann mit dem aus Vektor pCR2.1-2 gewonnenen Fragment ligiert. Dazu werden 0,5 μg von Vektor pCR2.1-2 2 Stunden mit BamHI (New England Biolabs) verdaut und die DNA-Fragmente per Gelelektorphorese aufgetrennt. Das neben dem Vektor (3,9 kb) entstandene 489 bp grosse Stück wird aus dem Gel ausgeschnitten und mit dem "Gelpurification"-Kit (Qiagen) nach Herstellerangaben aufgereinigt und mit 50 μL Elutionspuffer eluiert. 10 μL des Eluats werden mit Vektor per2.1-1 (s.o.) über Nacht bei 16°C ligiert (T4 Ligase, New England Biolabs). Die Ligationsprodukte werden dann in TOP10 Zellen (Stratagene) nach Herstellerangaben transformiert und entsprechend selektioniert. Positive Klone werden mit dem Primerpaar ONP1 und ONP2 durch PCR verifiziert. Der. erhaltene Vektor wird pcR2.1-AtCRU3-RNAi genannt. Die für die dsRNA kodierdende Nukleinsäuresequenz ist durch SEQ ID NO: 105 beschrieben.

b) Ausgangsvektor pCR2.1-AtCRB-RNAi

Mit nachfolgendem Oligonukleotid-Primerpaar wird ein Exonbereich des 12S Speicherproteine AtCRB (SEQ ID NO: 117 bzw. 118; Basenpaar 601 bis 1874 der Sequenz mit der (GenBank Acc.-No: M37248) aus Arabidopsis thaliana cDNA amplifiziert:

ONP5 (SEQ ID NO 138):
5' ATAACAATGCGCCCGCGGATCCCTCAGGGTCTTTTCTTGCCCACT-3'

ONP6. (SEQ ID NO: 139):
5'-CCGCTCGAGTTTACGGATGGAGCCACGAAG-3'

Das PCR-Produkt wird in den pCR2.1-TOPO Vektor (Invitrogen) gemäss Herstellerangaben kloniert, resultierend in dem pCR2.1-3 Vektor und die Sequenz überprüft.

Für den als Linker fungierenden Bereich wird aus Arabidopsis thaliana genomischer DNA ein Intron mit den entsprechenden Spliceakzeptor und -donorsequenzen der flankierenden Exons (Basenpaar 1874 bis 2117 der Sequenz mit der GenBank Acc.-No: M37248) mit dem nachfolgenden Primerpaar amplifiziert:

ONP7 (SEQ ID NO: 140):
5'-CCGCTCGAGGTAAGCTCAACAAATCTTTAG-3'

ONP8 (SEQ ID NO: 141) :
5'-ACGCGTCGACGCGTTCTGCGTGCAAGATATT-3'

Das PCR-Produkt wird in den pCR2.1-TOPO Vektor (Invitrogen) gemäss Hersteller angaben kloniert, resultierend in dem pCR2.1-4 Vektor und die Sequenz überprüft.

Das Konstrukt für AtCRB wird in einer ähnlichen Strategie wie für AtCRU3 erläutert, erstellt. Vektor pCR2.1-3 wird mit mit XhoI (New England Biolabs) für 2 Stunden inkubiert und dephosphoryliert (alkalische Phosphatase, New England Biolabs). Vektor pCR2.1-4 wird ebenfalls mit XhoI in derselben Weise inkubiert und die Gelfragmente per Gelelektrophorese aufgetrennt. Die entsprechenden Fragmente werden in der unter AtCRU3 beschriebenen Art und Weise aufgereinigt und ligiert, resultierend nach Batterien-Transformation in dem Vektor pCR2.1-AtCRB Exon/Intron. Dieser Vektor wird für 2 Stunden mit XhaI (NEB), anschliessend für 15 min mit Klenow-Fragment (NEB), dann für 2 Stunden mit SalI inkubiert und zuletzt 15 min mit alkalischer Phosphatase (NEB) behandelt. Parallel wird der Vektor pCR2.1-3 mit BamHI (NEB), dann 15 min mit Klenow-Fragment und anschliessend 2 stunden mit XhoI (NEB) inkubiert. Das Exon-Fragment von ATCRB wird nach Gelelektorphorese isoliert, gereinigt und zur Ligation eingesetzt. Beide Fragmente wurden dann ligiert und der Vektor pCR2.1-AtCRB-RNAi resultierte. Der erhaltene Vektor wird pCR2.1-AtCRB-RNAi genannt. Die für die dsRNA kodierdende Nukleinsäuresequenz ist durch SEQ ID NO: 107 beschrieben.

c) Ausgangsvektor pCR2.1-At-2S3-RNAi.

Mit nachfolgendem Oligonukleotid-Primerpaar wird ein Exonbereich des 2S Speicherprotein At2S3 (SEQ ID NO: 3 bzw. 4; Basenpaar 212 bis 706 der Sequenz mit der GenBank Acc.-No: M22035) amplifiziert:

ONP9 (SEQ ID NO: 142):
5'-ATAAGAATGCGGCCGCGGRTCCATGGCTAACAAGCTCTTCCTCGTC-3'

ONP10 (SEQ ID NO: 143) :

5'-ATAAGAATGCGGCCGCGGATCCCTAGTAGTAAGGAGGAAAG-3'

Das PCR-Produkt wird in den pCR2.1-TOPO Vektor (Invitrogen) gemäss Herstellerangaben kloniert, resultierend in dem per2.1-5 Vektor und die Sequenz überprüft. Für den als Linker fungierenden Bereich wird das gleiche Intron wie unter b) mit den Primern OPN 7 und OPN 8 amplifiziert eingesetzt.

Das Konstrukt für At2S3 wird in einer ähnlichen Strategie wie für AtCRU3 erläutert, erstellt. Vektor per2.1-5 wird mit mit XhoI (New England Biolabs) für 2 Stunden inkubiert und dephosphoryliert (alkalische Phosphatase, New England Biolabs). Vektor pCR2.1-3 werden ebenfalls mit XhoI in derselben Weise inkubiert und die Gelfragmente per Gelelektrophorese aufgetrennt. Die entsprechenden Fragmente werden in der unter AtCRU3 beschriebenen Art und Weise aufgereinigt und ligiert, resultierend nach Bakterientransformation in dem Vektor pCR2.1-At2S3 Exon/Intron. Dieser Vektor wird für 2 Stunden mit SalI (NEB), anschliessend für 15 min mit Klenow-Fragment (NEB) inkubiert und zuletzt 15 min mit alkalischer Phosphatase (NEB) behandelt. Parallel wird der Vektor pCR2.1-5 mit BamHI (NEB) und dann 15 min mit Klenow-Fragment inkubiert. Das Exon-Fragment von At2S3 wird nach Gelelektorphorese isoliert, gereinigt und zur Ligation eingesetzt. Beide Fragmente werden dann ligiert und der Vektor pCR2.1-At2S3-RNAi resultierte. Die für die dsRNA kodierdende Nukleinsäuresequenz ist durch SEQ ID NO: 109 beschrieben.

d) Herstellung von Super-Supressionskonstrukt 1

Die Vektoren pCR2.1-AtCRU3-RNAi und pCR2.1-4 (siehe oben) werden mit den Restriktionsenzymen XhoI und SalI für 2 Stunden bei 37°C inkubiert, die DNA-Fragmente durch Agarose-Gelelektrophorese aufgetrennt und sowohl der Vektor als auch das PCR-Insert aus pCR2.1-4 aufgeschnitten und mit dem "Gelpurification"-Kit von Qiagen nach Herstellerangaben aufgereinigt und mit 50 μL Elutionspuffer eluiert. Vom Vektor wird 1 μL, vom PCR-Insert aus pCR2.1-4 8 μL der Eluate für die Ligation eingesetzt, resultierend in dem Konstrukt pCR2.1-sRNAi1. Dieser Vektor wird für 2 Stunden mit dem Restriktionsenzym XhoI und dann für 15 min mit Klenow-Fragment inkubiert.

Der Vektor pCR2.1-AtCRB-RNAi (siehe oben) wird mit dem Enzym EcoRI für 2 Stunden inkubiert und ebenfalls 15 min mit Klenow-Fragment behandelt. Beide Inkubationsansätze werden durch Gelelektrophorese aufgetrennt und jeweils der Vektor (pCR2.1-sRNAi1) bzw. das Insert (aus pGR2.1-AtCRB-RNAi) aus dem Agarosegel ausgeschnitten und die DNA-Fragmente wie oben beschrieben aufgereinigt. Für die Ligation werden 1 μL des Eluates vom Vektor und 8 μL des Eluates vom Insert eingesetzt und bei 4°C über Nacht inkubiert. Das resultierende Konstrukt wird mit pCR2.1-sRNAi2 bezeichnet. Der resultierende Vektor wird mit dem Enzym XbaI und anschliessend mit Klenow-Fragment inkubiert. Der Vektor pCR2.1-4 wird mit den Enzymen EcoRV und XbaI und anschliessend mit Klenow-Fragmente inkubiert. Nach Gelelektrophorese und -reinigung wird das Fragment aus pCR2.1-4 mit dem Vektor pCR2.1-sRNAi2 ligiert, resultierende in dem Konstrukt pCR2.1-sRNAi3. Der resultierende Vektor wird dann mit dem Enzym ApaI für 2 Stunden und dann mit Klenow-Fragment für 15 min inkubiert. Als Insert wird der Vektor pCR2.1-At2S3-RNAi mit dem Enzym EcoRI für 2 Stunden und dann mit Klenow-Fragment für 15 min inkubiert. Nach Gelelektrophorese und -reinigung werden die Eluate ligiert, resultierend in dem Vektor pCR2.1-sRNAi4. Aus diesem Vektor wird dann das sRNAi4-Fragment (SEQ ID NO: 144; vgl. Fig. 1(1)), kodierend für die super-supprimierende dsRNA, durch Inkubation mit HindIII und PvuI ausgeschnitten und in den binären Vektor pSUN-USP (SEQ ID NO: 179) ligiert. Das Konstrukt dient der gleichzeitige Suppression von Arabidopsis thaliana Speicherproteinen CRB (SEQ ID NO:4), CRU3 (SEQ ID NO: 112) und At2S3 (SEQ ID NO: 118).

Der verwendete Vektor pSUN-USP ist ein binärer Vektor zur Pflanzentransformation auf Basis von pBinAR (Höfgen und Willmitzer (1990) Plant Science 66: 221-230). Eine gewebespezifische Expression Im Samen läßt sich unter Verwendung des gewebespezifischen Promotors USP-Promotors erzielt.

e) Herstellung von Super-Supressionskonstrukt 2

Ausgehend von Arabidopsis thaliana cDNA wird ein Fragment aus dem Speicherproteine AtCRU3 (SEQ ID NO: 111, 112) mit dem nachfolgeden Oligonukleotid-Primerpaar unter den in Beispiel 2 angegebenen PCR-Bedingungen amplifiziert:

OPN 11: 5'-AAAACGCCTGTGTTCCATTTGGCCGGAAACAAC-3' (SEQ ID NO: 148)
OPN 12: 5'-AAAGATATCACCCTGGAGAACGCCACGAGTG-3' (SEQ ID NO: 149).

Das erhaltene Fragment wird in den Vektor pCR2.1-TOPO Vektor (Invitrogen) gemäss Herstellerangaben kloniert, resultierend in den pCR2.1-6 und die Sequenzen überprüft.

Ausgehend von Arabidopsis thaliana cDNA wird ein Fargment aus dem Speicherprotein At2S3 (SEQ ID NO: 3, 4) mit dem nachfolgenden Oligonukleotid-Primerpaar unter den in Beispiel 2 angegebenen PCR-Bedingungen, amplifiziert:

OPN 13: 5'-AAAAGGCCTATGGCTAACAAGCTCTTCCTCGTC-3' (SEQ ID NO: 150)
OPN 14: 5'-AAAGATATCCTAGTAGTAAGGAGGGAAGAAAG-3' (SEQ ID NO: 151).

Das erhaltene Fragment wird in den Vektor pCR2.1-TOPO Vektor (Invitrogen) gemäss Herstellerangaben kloniert, resultierend in den pCR2.1-7 und die Sequenzen überprüft.

Aus den pCR2.1-3, pCR2.1-4 (siehe Beispiel 2) und pCR2.1-6 und pCR2.1-7 werden dann die Konstrukte folgendermassen zusammen ligiert : Der Vektor per2.1-3 wird 2 Stunden mit EcoRV inkubiert und anschliessend 15 min mit alkalischer Phosphatase dephosphoryliert. Der Vektor pCR2.1-6 wird mit den Enzymen Stul und EcoRV für 2 Stunden inkubiert und das PCR-Insert über Gelelektrophorese und -reinigung isoliert. Vektor pCR2.1-3 und Insert aus pCR2.1-6 werden dann .über Nacht bei 4°C ligiert, resultierend in dem Konstrukt pCR2.1-sRNAi5. Dieser Vektor wird dann mit EcoRV inkubiert und dephosphoryliert und mit dem Stul/ EcoRV inkubierten und gelaufgereinigten Fragment aus pCR2.1-7 ligiert, resultierend in dem Konstrukt pCR2.1-sRNAi6. Dieser Vektor wird dann mit Xhol inkubiert und dephosphoryliert. Der Vektor pcR2.1-4 wird mit Sall und Xholl inkubiert und das Insert aus pCR2.1-4 mit dem vorbereiteten Vektor pCR2.1-sRNAi6 ligiert, resultierend in dem Konstrukt pCR2.1-sRNAi7. Ausgehend von PCR2.1-sRNAi7 wird eine PCR mit den nachfolgenden Primerpaar unter den in Beispiel 2 gegebenen Bedingungen durchgeführt:

OPN 15: 5' CCGCTCGCTCAGGGTCTTTTCTTGCCACT (SEQ ID NO: 152)
OPN 16: 5'-CCGGTCGACCTAGTAGTAAGGAGGGAAGAAAG (SEQ ID NO: 153).

Das resultierende PCR-Produkt wird mit den Enzymen Xhol und Sall inkubiert. Das Fragmente wird dann in den Vektor pCR2.1-sRNAi7 (inkubiert mit xhol) ligiert, resultierend in dem Konstrukt pCR2.1-sRNAi8. Aus diesem Vektor wird dann das sRNAi8-Fragment (SEQ ID NO: 146 ; vgl. Fig. 1(2)), kodierend für die super-supprimierende dsRNA, durch Inkubation mit HindIII und Xbal ausgeschnitten und in den binären Vektor pSUN-USP (SEQ ID NO:: 179) ligiert. Das Konstrukt dient der gleichzeitigen Suppression von Arabidopsis thaliana Speicherproteinen CRB (SEQ ID NO:4), CRU3. (SEQ ID NO: 112) und At2S3 (SEQ ID NO : 118).

Beispiel 3: Transformation von Agrobaoterium

Die Agrobacterium-vermittelte Pflanzentransformation kann zum Beispiel unter Verwendung der Agrobacterium tumefaciens-Stämme GV3101 (pMP90) (Koncz und Schell (1986) Mol Gen Genet 204: 383-396) oder LBA4404 (Clontech.) durchgeführt werden. Die Transformation kann durch Standard-Transformationstechniken durchgeführt werden (Deblaere et al.(1984) Nucl Acids Res 13:4777-4788).

Beispiel 4: Pflanzentransformation

Die Agrobacterium-vermittelte pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molekular Biology and Biotechnology, Boca Raton: CRC Press, 1993, 360 S., ISBN 0-8493-5164-2).

Die Transformation mittels Agrobacterium von Arabisopsis thaliana wird durch die Methode nach Bechthold et al., 1993 (C.R. Acad. Sci. Ser. III Sci. Vie., 316, 1194-1199) durchgeführt. Beispielsweise kann Raps mittels Kotyledonen- oder Hypokotyltransformation transformiert werden (Moloney et al., Plant Cell Report 8 (1989) 238-242; De Block et al., Plant Physiol. 91 (1989) 694-701). Die Verwendung von Antibiotika für die Agrobacterium- und pflanzenselektion hängt von dem für die Transformtion verwendeten binären Vektor und Agrobacterium-Stamm ab. Die Rapsselektion wird gewöhnlich unter Verwendung von Kanamycin als selektierbarem Pflanzenmarker durchgeführt.

Der Agrobacterium-vermittelte Gentransfer in Lein (Linum usitatissimum) läßt sich unter Verwendung von beispielsweise einer von Mlynarova et al. (1994) Plant Cell Report 13:282-285 beschriebenen Technik durchführen.

Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuß, Polyethylenglycol-vermittelter DNA-Aufnahme oder über die Siliziumcarbonatfaser-Technik ist beispielsweise beschrieben von Freeling und walbot "The maize handbook" (1993) ISBN 3-540-97826-7, Springer Verlag New York).

Beispiel 5: Untersuchung der Expression eines rekombinanten Genproduktes in einem transformierten Organismus

Die Aktivität eines rekombinanten Genproduktes im transformierten Wirtsorganismus wurde auf der Transkriptions- und/oder der Translationsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnen Beispielteil), wobei ein Primer, der so gestaltet ist, daß er an das Gen von Interesse bindet, mit einer nachweisbaren Markierung (gewöhnlich radioaktiv oder chemilumineszent) markiert wird, so daß, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R. , et al. (1992) Mol. Microbiol. 6:317-326 beschriebenen, präpariert werden.

Northern-Hybridisierung:

Für die RNA-Hybridisierung wurden 20 $\mu$g Gesamt-RNA oder 1 $\mu$g poly(A)$^+$-RNA mittels Gelelektrophorese in Agarosegelen mit einer Stärke von 1,25 %, unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68°C unter Verwendung von Hybridisierungspuffer (10 % Dextransulfat Gew./Vol., 1 M NaCl, 1.% SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-$^{32}$P-dCTP (Amersham Pharmaria, Braunschweig, Deutschland). Die Hybridisierung wurde nach Zugabe der markierten DNA-Sonde im gleichen Puffer bei 68°C über Nacht durchgeführt. Die Waschschritte wurden zweimal für 15 min unter Verwendung von 2 X SSC und zweimal für 30 min unter Verwendung von 1 X SSC, 1 % SDS, bei 68°C durchgeführt. Die Exposition der verschlossene Filter wurde bei -70°C für einen Zeitraum von 1 bis 14 T durchgeführt.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (198B) Current Protocols in Molecular Biology, Wiley: New York). Bei diesem Verfahren werden die zellulären Gesamt-Proteine extrahiert, mittels Gelelektrophorese aufgetrennt, auf eine Matrix, wie Nitrocellulose, übertragen und mit einer Sonde, wie einem Antikörper, der spezifische an das gewünschte Protein bindet, inkubiert. Diese Sonde ist gewöhnlich mit einer chemilumineszenten oder kolorimetrischen Markierung versehen, die sich leicht nachweisen läßt. Das Vorliegen und die Menge der beobachtreten Markierung zeigt das Vorliegen und die Menge des gewünschten, in der Zelle vorliegenden mutierten Proteins an.

Beispiel 6: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie,' Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial chemistry, Bd. A2, S. 89-90 und S. 443-613, VCH: weinheim (1985); Fallon, A., et al.. (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons ; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press. Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssige Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion, in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Für die Öl-Analyse der mit den Supressionskonstrukten transformierten Arabidopsis Pflanzen wird folgendes Protokoll angewendet: Die Extraktion der Lipide aus Samen wird nach der Methode von Bligh & Dyer (1959) Can J Biochem Physiol 37:911 durchgeführt. Dazu werden 5 mg Arabidopsis Samen in 1,2 ml Qiagen-Microtubes (Qiagen, Hilden) auf einer Sartorius (Göttingen) Mikrowaage abgewogen. Das Samenmaterial wird mit 500 uL Chloroform/Methanol (2:1; enthält Mono-C17-glycerin von Sigma als internen Standard) in der Rätschmühle MM300 der Firma Retsch (Haan) homogenisiert und 20 min bei RT inkubiert. Nach Zugabe von 500 uL 50 mM Kaliumphosphatpuffer pH 7,5 erfolgt die Phasentrennung. Von der organischen Phase werden 50 μL abgenommen, mit 1500 uL Chloroform verdünnt und 5 μL auf die Kapillaren Chromarods SIII der Firma latroscan (SKS, Bechenheim) aufgetragen. Nach Auftrag der Proben werden diese für 15 min in einer Dünnschichtkammer, die gesättigt ist mit 6:2:2 Chloroform: Methanol: Toluol in einem ersten Schritt aufgetrennt. Nach Ablauf der Zeit werden die Kapillaren 4 min bei Raumtemperatur getrocknet und dann für 22 min in eine Dünnschichtkammer, die gesättigt ist mit 7:3 n-Hexan:Dieethylether gestellt. Nach einem weiteren Trocknungsschritt für 4 min bei Raumtemperatur werden die Proben in einem latroscan MK-5 (SKS, Bechenheim) entsprechend Fraser & Taggart, 1988 J. Chromatogr. 439:404 analysiert. Folgende Parameter wurden für die Messungen eingestellt: Slice width 50 msec, Treshold 20 mV, Noise 30, Skim ratio 0. Die Quantifizierung der Daten erfolgte anhang des internen Standards Mono-C17-glycerin (Sigma) sowie einer erstellten Eichkurve mit Tri-C17-glycerin (Sigma) mittels des Programms ChromStar (SKS, Beichenheim).

Für die quantitative Bestimmung der ölgehalte werden Samen von jeweils 10 Pflanzen derselben unabhängigen transgenen Linie analysiert. Insgesamt wurde der Ölgehalt von 30 transgene Linien der T1 Generation, 10 transgene Linien mit je 10 Pflanzen der T2 Generation und 5 transgene Linien mit je 10 Pflanzen der T3 Linien bestimmt. Dabei zeigen die transgenen Pflanzen einen signifikant höheren Ölgehalt als entsprechend gleichbehaudelte Kontrollpflanzen.

Beispiel 7:

Zum Nachweis der Funktionalität der multiplen RNAi Konstrukte wurden Gene ausgewählt, deren Supression einen deutlichen phänologischen Effekt hervorrufen. Ein solches Gen ist zum Beispiel Toc159. Dieses Gen ist essentiell für die Entwicklung und Funktionalität von Chloroplasten in Arabidopsis (Bauer et al. Nature, 403, 203-207). Ein Ausschalten dieses Gens führt zu chlorophylldefizienten Pflanzen, deren Blatt-Erscheinungsbild dann hell-grün bis weiss ist. Dieser Albino-Phänotyp ist sehr leicht zu unterscheiden von normalen Pflanzen.

Als weiteres visuelles Reprotergen wurde GFP, das grün-fluoreszierende Protein aus der Qualle Aequorea victoria eingesetzt. Dieses Reportergen ist ein häufig verwendetes Reportergen in Pflanzen (siehe z.B. Stewart, Plant Cell Rep 2001 20(5):376-82). Ausgehend von Arabidopsis thaliana cDNA oder vom Plasmid pEGFP (BD Clontech, Heidelberg, Genbank-Eintrag U476561) wurde über PCR mittels der aufgeführten Oligonukleotid erzeugt. Dabei wurde folgendes Protokoll eingesetzt:

Zusammensetzung des PCR-Ansatzes (50 μL):

5,00 µL Template cDNA oder genomische DNA (ca. 1 µg)
5, 00 µL 10x Puffer (Advantage-Polymerase) + 25 mM $MgCl_2$
5,00 µL 2mM dNTP
1,25 µL je Primer (10 pmol/µL)
0,50 µL Advantage-Polymerase (Clontech)

PCR-Programm: Anfangsdenaturierung für 2 min bei 95°C, dann 35 Zyklen mit 45 sec 95°C, 45 sec 55°C und 2 min 72°C. Abschliessende Extension von 5 min bei 72°C.

a) Ausgangsvektor pGEM-Toc159 : Ausgehend von Arabidopsis cDNA wurde mit nachfolgendem Oligonucleotid-Primerpaar ein Fragment aus Toc159 (Genbank Acc.-No. T14P8.24) amplifiziert:

ONP18 (SEQ ID NO: 123):
5'-CTCGAGGAATTCATGGACTCAAAGTCGGTTACTCCA

ONP19 (SEQ ID NO : 124):
5'-GGATCCATAAGCAAGCTTTCTCACTCTCCCCATCTGTGGA

Das PCR Produkt wurde in den Vektor pGEM-T easy von Promega (Mannheim) gemäss Herstellerangaben kloniert, resultierend in dem pGEM-Toc159 Vektor und die Sequenz überprüft.

b) Ausgangsvektor pGEM-GFP: Ausgehend von dem Plasmid pEGFP (BD Clontech, Heidelberg, GenbankAcc.-No.: U476561) wurde mit nachfolgendem Oligonukleotid-Primerpaar ein Fragment aus GFP amplifiziert:

ONP20 (SEQ ID NO: 125) : 5'-AAGCTTCCAACACTTGTCACTACTTT
ONP21 (SEQ ID NO: 126) : 5'-GGATCCTTAAAGCTCATGTTTGT

Das PCR Produkt wurde in den Vektor pGEM-T easy von Promega (Mannheim) gemäss Herstellerangaben kloniert, resultierend in dem pGEM-GFP Vektor und die Sequenz überprüft.

c) Herstellung des Konstruktes pGEM-159-GFP Der Vektor pGEM-GFP wurde mit den Restriktionsenzymen HindIII und BamHI für 2 Stunden inkubiert. Parallel wurde der Vektor pGEM-Toc159 mit den gleichen Restriktionsenzymen inkubiert, ansehliessend dann zusätzlich für 15 min mit alkalischer Phosphatase behandelt. Die alkalische Phosphatase wurde anschliessend durch Erhitzen auf 95 oC für 10 min inaktiviert. Die entstandenen DNA-Fragmente aus beiden Ansätzen wurden über Agarose-Gelelektrophorese aufgetrennt. Das 558 bp Fragment aus pGEM-GFP sowie das 3471 bp Fragment von pGEM-Toc159 wurden aus dem Gel ausgeschnitten und mit dem "Gelpurification"-Kit (Qiagen) nach Herstellerangaben aufgereinigt. Beide Fragmente wurden für 2 h bei 16°C ligiert (T4 Ligase, New England Biolabs) und anschliessend nach Herstellerangaben in E. coli DH5α Zellen (Stratagen) transformiert. Positive Klone wurden durch PCR mit dem Primerpaar OPN1 und OPN4 identifiziert und anschliessend verifiziert durch Sequenzierung. Der erhaltene Vektor wurde mit pGEM-159-GFP bezeichnet.

d) Herstellung des Supressionskonstruktes 1: Der Vektor pGEM-159-GFP wurde einerseits mit den Restriktionsenzymen XhoI und BamHI, ein weiterer Ansatz mit BamHI und SalI inkubiert. Der zweite Ansatz mit BamHI/ SalI wurde anschliessend für weitere 15 min mit alkalischer Phosphatase inkubiert. Die DMA-Fragmente aus beiden Ansätzen wurden über Agarose-Gelelektrophorese aufgetrennt und folgende Fragmente ausgeschnitten: Ansatz BamHI-XhoI das 1091 bp Fragment; Ansatz BamHI-SalI das 4029 bp Fragment. Beide Fragmente wurden nach Aufreinigung aus dem Agarose-Gel (siehe oben) für 2 h bei 16°C mit T4 Ligase inkubiert und anschliessend in E. coli DH5α Zellen (Stratagen) transformiert. Positive Klone wurden durch PCR mit dem Primerpaar OPN1 identifiziert und anschliessend verifiziert durch Sequenzierung. Der erhaltene Vektor wurde als Supressionskonstrukt 1 bezeichnet.

e) Herstellung des Supressionskonstruktes 2: Das Supressionskonstrukt 1 und der Vektor p3300.1 (Andreas Hilbrunner, Dissertation ETH Zürich, 2003) wurden für 2h Stunden mit dem Restriktionsenzym EcoRI inkubiert. Anschliessend wurde der Vektor p3300.1 15 min mit alkalischer Phosphatase behandelt. Beide Ansätze wurden gemischt und für 2 h bei 16°C mit T4 Ligase inkubiert. Der Ligationsansatz wurde dann in E. coli DH5α Zellen (Stratagen) transformiert. Das entstandene Supressionskonstrukt 2 wurde dann für die Agrobacterium- und Pflanzentransformation eingesetzt. Die Nukleinsäuresequenz kodierend für Supressionskonstrukt 2 (p3300.1-Toc159-GFP-RNAi) ist unter SEQ ID NO: 122 wiedergegeben. Die Transformtion von Agrobakterien und Pflanzen wurde wie in Beispiel 3 bzw. 4 beschrieben durchgeführt. Zum Nachweis der Funktionalität des Supressionskonstraktes 2 wurde dieses durch die nach Bechtold et al., 1993 (C.R. Acad. Sci. Ser. III Sci. Vie., 316, 1194-1199) beschriebene Blüten-Transformationsmethode in Arabidopsis transformiert. Aus Ausgangsmaterial wurden Arabidopsis Pflanzen der Varietät Columbia-0 verwendet, die bereits die T-DNA des binären Vektors pBIN-35S-GFP enthielten.

Durch Anregung durch ultraviolettes Licht im Wellenlängenbereich 470-490 nm die grüne Fluoreszenz von GFP in diesen Pflanzen angeregt werden und damit die Expression des eingebrachten Transgens überprüft werden. Dazu wurden Keimlinge 1 Woche nach Keimung oder Blattstücke bei älteren Pflanzen mit dem Fluoreszenzmikroskop MZFLIII von Leica analysiert. Zur Anregung von GFP wurden folgende Parameter eingestellt: Quecksilberlampe HBO 100W/DC, Filter GFP3, Bildbearbeitung Leica-Software. Speziell die Verwendung eines Filters (GFP3), der oberhalb einer Wellenlänge von 525 nm nicht mehr durchlässig ist, ermöglicht die GFP-Analyse von grünen Blattmaterial. Ohne diesen Filter könnte die starke Autofluoreszenz des Blattfarbstoffes Chlorophyll nicht ausgeschlossen werden. Die zur Transformation verwendete Arabidopsis Linie zeigte eine starke GFP Expression nach mikroskopischer Analyse.

Transformierte Samen wurden direkt auf Erde ausgelegt und angezogen. Nach einer Woche wurde nach Keimlingen gesucht, die keinen oder einen reduzierten Anteil des Blattfarbstoffes Chlorophyll enthielten. Solche Pflanzen waren leicht an ihrer hellgrünen oder weisen Erscheinungsbild zu erkennen. Diese Pflanzen wurden dann weiter durch Fluoreszenz-Mikroskopie untersucht und mit entsprechend parallel gewachsenen grünen Pflanzen verglichen. Fig. 5A zeigt beispielhaft ein solche identifizierte Pflanze, die sich deutlich in der Farbe der Blätter von parallel gewachsenen Pflanzen unterscheidet. Dabei ist der Albino-Phänotyp (weisse Blätter) auf die Wirkung des Toc159-Supressionskonstrukts zurückzuführen. Die nicht transformierten Nachkommen der mit Agrobacerium-Suspension behandelten Pflanzen, zeigen den Albino-Phänotyp nicht. Der auftretende Albino-Phänotyp ist damit ein spezifischer Effekt des eingebrachten Supressionskonstruktes.

Die Fluoreszenz-miroskopische Untersuchung der Albino-Pflanzen zeigte dann (Fig. 5B), dass keine GFP-Signale in solchen Pflanzen gefunden werden konnte. Im Vergleich dazu zeigten die parallel gewachsenen grünen Pflanzen deutliche GFP Signale. Die Abwesenheit des GFP-Signals in allen identifizierten Albino-Pflanzen demonstriert die Funktionalität des Supressionskonstruktes, denn nur die mit dem Supressionkonstrukt transformierten Pflanzen zeigten keine GPP-signale mehr. Es konnte keine Segregation der beiden angestrebten Phänotypen beobachtet werden. Damit konnte gezeigt werden, das durch Verwendung von nur einem Kontrollelemente (Promotor) zwei funktionell völlig unterschiedliche Gene, die ihrerseits durch unterschiedliche Kontrollelemente in ihrer Expression reguliert werden, ausgeschaltet werden konnten.

## Patentansprüche

**1.** Verfahren zur Verminderung der Expression von mindestens zwei verschiedenen, endogenen Zielgenen in einem pflanzlichen Organismus durch Einbringen eines zumindest teilweise doppelsträngigen Ribonukleinsäuremoleküls in besagten pflanzlichen Organismus, wobei das doppelsträngige Ribonukleinsäuremolekül aus einem ganz oder teilweise selbstkomplementären RNA Strang besteht, welcher umfasst

a. mindestens zwei "sense"-Ribonukleotidsequenzen, wobei jeweils mindestens eine dieser "sense"-Ribonukleotidsequenzen im wesentlichen identisch ist zu mindestens einem Teil des "sense"-RNA-Transkriptes eines jeden der besagten endogenen Zielgene und
b. "antisense"-Ribonukleotidsequenzen, die zu besagten "sense"-Ribonukleotidsequenzen unter a) im wesentlichen komplementären sind,

wobei zunächst die "sense"-Ribonukleotidsequenzen a) aufeinander folgen, und dann eine Aneinanderreihung der im wesentlichen komplementären "antisense"-Ribonukleotidsequenzen b) folgt, und wobei der RNA-Strang eine einzelne Haarnadel ausbildet, welche nachfolgende Primärstruktur hat

5'-S(1 )-S(2)-..... -S(n)-AS(n)-.... -AS(2)-AS(1 )-3'

in welcher S die "sense"-Ribonukleotidsequenzen, AS die "antisense"-Ribonukleotidsequenzen darstellen, und wobei die Anzahl der Einheiten n größer oder gleich zwei ist,
und wobei der pflanzliche Organismus zu einer Pflanzengattung gehört, ausgewählt aus Cucurbita, Rosa, Vitis, Juglans, Fragaria, Lotus, Medicago, Onobrychis, Trifolium, Trigonella, Vigna, Citrus, Linum, Geranium, Manihot, Daucus, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus.

**2.** Verfahren nach Anspruch 1, wobei die transkribierten RNAs von mindestens zwei der in ihrer Expression verminderten Zielgene untereinander eine Homologie von unter 90% haben.

**3.** Verfahren nach Anspruch 1 oder 2, **dadurch gekennzeichnet, dass** mindestens zwei der endogenen Zielgene ausgewählt sind aus jeweils unterschiedlichen Klassen von Speicherprotein ausgewählt aus den Speicherprotein-Klassen der 2S-Albumine, 7S-Globuline, 11S/12S-Globuline oder Zein-Prolamine.

**4.** Verfahren nach einem der Ansprüche 1 bis 3, wobei mindestens eine "sense"-Ribonukleotidsequenz im wesentlichen identisch ist zu mindestens einem Teil des "sense"-RNA-Transkriptes

a) einer Speicherprotein-Nukleinsäuresequenz gemäß SEQ ID NO: 1, 3, 5, 7, 9, 11, 13, 15, 17, 19, 21, 23, 25, 27, 29, 31, 33, 35, 37, 39, 41, 43, 45, 47, 49, 51, 59, 61, 63, 65, 67, 69, 71, 93, 95, 97, 99, 101, 103, 105, 107, 109 oder 112, oder
b) eines Gens aus dem Homogentisatabbauweg gemäß SEQ ID NO: 115, 116, 118 oder 120, oder
c) einem Gen ausgewählt aus der Gruppe bestehend aus Acetyltransacylasen, Acyltransportproteinen, Fettsäuredesaturasen, Malonyltransacylasen, b-Ketoacyl-ACP-synthetasen, 3-Keto-ACP-reduktasen, Enoyl-ACP-hydrasen, Thioesterasen, Enoyl-ACP-reduktasen, ADP-Glucosepyrophosphorylasen, Phosphorylasen, Stärkesynthetasen, Q-Enzymen, Sucrose-6-phosphatsynthetasen, Sucrose-6-phosphatphosphatasen, ADP-Glucosepyrophosphorylasen, Branching-Enzymen, Debranching-Enzymen, Amylasen, Chalconsynthasen, Chalconisomerasen, Phenylalaninammonialyasen, Dehydrokaempferol(flavone)hydroxylasen, Dihydroflavonolreduktasen, Dihydroflavanol-2-hydroxylasen, Flavonoid-3' - hydroxylasen, Flavonoid-5' -hydroxylasen, Flavonoidglycosyltransferasen, Flavonoidmethyltransferasen, Flavonoidacyltransferasen, Polygalacturonasen, Cellulasen, Pectinesterasen, b-(1-4)Glucanasen, b-Galactanasen, 1-Aminocyclopropan-1-carboxylatsynthasen, Phytoendesaturasen, Cinnamoyl-CoA:NADPH-Reduktasen, Cinnamoylalkoholdehydrogenasen, Coffeinsäure-Omethyltransferasenn Cinnamoylalkoholdehydrogenasen, Polyphenoloxidasen, Homogentisat-1,2-dioxygenasen, Maleylacetoacetatisomerasen, Fumarylacetoacetathydrolasen, N-Methylputrescinoxidasen, Putrescin-N-methyltransferasen, 7-Methylxanthine-3-methyltransferasen, 1-Methylxanthin-3-methyltransferasen und Threoninsynthasen.

## Claims

**1.** A method for reducing the expression of at least two different endogenous target genes in a plant organism by introducing, into said plant organism, an at least partially double-stranded ribonucleic acid molecule, the double-stranded ribonucleic acid molecule consisting of a fully or partially selfcomplementary RNA strand, which comprises

a. at least two "sense" ribonucleotide sequences, where in each case at least one of these "sense" ribonucleotide sequences is essentially identical to at least one part of the "sense" RNA transcript of each of said endogenous target genes and
b. "antisense" ribonucleotide sequences which are essentially complementary to said "sense" ribonucleotide sequences of a),

where first the "sense" ribonucleotide sequences a) follow one another, followed by a sequential arrangement of the essentially complementary "antisense" ribonucleotide sequences b), and wherein the RNA strand forms a single hairpin which has the following primary structure

5'-S(1)-S(2)-.....-S(n)-AS(n)-....-AS(2)-AS(1)-3'

in which S means the "sense" ribonucleotide sequences, AS the "antisense" ribonucleotide sequences and where the number of the units n is greater than or equal to two,
and where the plant organism belongs to a plant genus selected from Cucurbita, Rosa, Vitis, Juglans, Fragaria, Lotus, Medicago, Onobrychis, Trifolium, Trigonella, Vigna, Citrus, Linum, Geranium, Manihot, Daucus, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea and Populus.

**2.** The method according to claim 1, wherein the transcribed RNAs of at least two target genes whose expression is reduced have less than 90% homology with one another.

**3.** The method according to claim 1 or 2, wherein at least two of the endogenous target genes are selected from in each case different classes of storage protein selected among the storage protein classes of the 2S albumins, 7S globulins, 11S/12S globulins or zein prolamins.

**4.** The method according to any of claims 1 to 3, wherein at least one "sense" ribonucleotide sequence is essentially identical to at least a part of the "sense" RNA transcript

a) of a storage protein nucleic acid sequence as shown in SEQ ID NO: 1, 3, 5, 7, 9, 11, 13, 15 , 17, 19, 21, 23, 25, 27, 29, 31, 33, 35, 37, 39, 41, 43, 45, 47, 49, 51, 59, 61, 63, 65, 67, 69, 71, 93, 95, 97, 99, 101, 103, 105, 107, 109 or 112, or
b) of a gene of the homgentisate catabolic pathway as shown in SEQ ID NO: 115, 116, 118 or 120, or
c) a gene selected from the group consisting of acetyl transacylases, acyl transport proteins, fatty acid desaturases, malonyl transacylases, β-ketoacyl-ACP synthetases, 3-keto-ACP reductases, enoyl-ACP hydrases, thioesterases, enoyl-ACP reductases, ADP-glucose pyrophosphorylases, phosphorylases, starch synthetases, Q-enzymes, sucrose-6-phosphate synthetases, sucrose-6-phosphate phosphatases, ADP-glucose pyrophosphorylases, branching enzymes, debranching enzymes, amylases, chalcone synthases, chalcone isomerases, phenylalanine ammonialyases, dehydrokempferol(flavone) hydroxylases, dihydroflavonol reductases, dihydroflavanol 2-hydroxylases, flavonoid 3'-hydroxylases, flavonoid 5'-hydroxylases, flavonoid glycosyltransferases, flavonoid methyltransferases, flavonoid acyltransferases, polygalacturonases, cellulases, pectin esterases, β-(1-4)glucanases, β-galactanases, 1-aminocyclopropane-1-carboxylate synthases, phytoene desaturases, cinnamoyl-CoA:NADPH-reductases, cinnamoyl alcohol dehydrogenases, caffeic acid O-methyltransferases, cinnamoyl alcohol dehydrogenases, polyphenol oxidases, homogentisate 1,2-dioxygenases, maleylacetoacetate isomerases, fumaryl-acetoacetate hydrolases, N-methylputrescin oxidases, putrescin N-methyltransferases, 7-methylxanthin 3-methyltransferases, 1-methylxanthin 3-methyltransferases and threonin synthases.

## Revendications

**1.** Procédé pour diminuer l'expression d'au moins deux gènes cibles endogènes différents dans un organisme végétal, par introduction d'une molécule d'acide ribonucléique au moins partiellement double brin dans ledit organisme végétal, la molécule d'acide ribonucléique double brin étant constituée d'un brin d'ARN en totalité ou en partie autocomplémentaire, qui comprend

a) au moins deux séquences ribonucléotidiques "sens", au moins l'une de ces séquences ribonucléotidiques "sens" étant pour l'essentiel identique à une partie du transcrit d'ARN "sens" de l'un desdits gènes cibles endogènes, et

b) des séquences ribonucléotidiques "antisens", qui pour l'essentiel sont complémentaires desdites séquences ribonucléotidiques "sens" de a),

procédé dans lequel tout d'abord les séquences ribonucléotidiques "sens" a) se suivent, puis vient une succession des séquences ribonucléotidiques "antisens" pour l'essentiel complémentaires b), le brin d'ARN formant une épingle à cheveux unique, qui a la structure primaire suivante :

5'-S(1)-S(2).....-S(n)-AS(n)-.....-AS(2)-AS(1)-3'

dans laquelle S représente les séquences ribonucléotidiques "sens", AS représente les séquences ribonucléotidiques "antisens", le nombre des unités n étant supérieur ou égal à deux,
et dans lequel l'organisme végétal appartient à un genre de plante choisi parmi Cucurbita, Rosa, Vitis, Juglans, Fragaria, Lotus, Medicago, Onobrychis, Trifolium, Trigonella, Vigna, Citrus, Linum, Geranium, Manihot, Daucus, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea et Populus.

**2.** Procédé selon la revendication 1, dans lequel les ARN transcrits d'au moins deux des gènes cibles dont l'expression a été diminuée présentent entre eux une homologie inférieure à 90 %.

**3.** Procédé selon la revendication 1 ou 2, **caractérisé en ce qu'**au moins deux des gènes cibles endogènes sont choisis parmi différentes classes de protéines de réserve choisies parmi les classes de protéines de réserve des 2S-albumines, des 7S-globulines, des 11S/12S-globulines ou les prolamines de type zéine.

**4.** Procédé selon l'une des revendications 1 à 3, dans lequel au moins une séquence ribonucléotidique "sens" est pour l'essentiel identique à au moins une partie du transcrit d'ARN "sens"

a) d'une séquence d'acides nucléiques de protéines de réserve selon SEQ ID N°1, 3, 5, 7, 9, 11, 13, 15, 17, 19, 21, 23, 25, 27, 29, 31, 33, 35, 37, 39, 41, 43, 45, 47, 49, 51, 59, 61, 63, 65, 67, 69, 71, 93, 95, 97, 99, 101, 103, 105, 107, 109 ou 112, ou
b) d'un gène de la voie de dégradation des homogentisates selon SEQ ID N°115, 116, 118, ou 120, ou
c) d'un gène choisi dans le groupe consistant en les acétyltransacylases, les protéines de transport des groupements acyle, les (acide gras)-désaturases, les malonyltransacylases, les b-cétoacyl-ACP-synthétases, les 3-céto-ACP-réductases, les énoyl-ACP-hydrases, les thioestérases, les énoyl-ACP-réductases, les ADP-glucosepyrophosphorylases, les phosphorylases, les amidon-synthétases, les enzymes Q, les saccharose-6-phosphate-synthétases, les saccharose-6-phosphate-phosphatases, les ADP-glucose-pyrophosphorylases, les enzymes de branchement, les enzymes de débranchement, les amylases, les chalconesynthases, les chalcone isomérases, les phénylalanine-ammonialyases, les déhydrocamphérol-(flavone)hydroxylases, les dihydroflavonol-réductases, les dihydroflavanol-2-hydroxylases, les flavonoïde-3'-hydroxylases, les flavonoïde-5'-hydroxylases, les flavonoïde-glycosyltransférases, les flavonoïde-méthyltransférases, les flavonoïde-acyltransférases, les polygalacturonases, les cellulases, les pectine-estérases, les b-(1-4)glucanases, les b-galactanases, les 1-amino-cyclopropane-1-carboxylate-synthases, les phytoène-désaturases, les cinnamoyl-CoA:NaDPH-réductases, les cinnamoylalcool-déhydrogénases, les (acide caféique)-O-méthyltransférases, les (alcool cinnamoylique)-déhydrogénases, les polyphénol-oxydases, les homogentisate-1,2-dioxygénases, les maléylacétoacétate-isomérases, les fumarylacéto-acétate-hydrolases, les N-méthyl-putrescine-oxydases, les putrescine-N-méthyltransférases, les 7-méthylxanthine-3-méthyltransférases, les 1-méthylxanthine-3-méthyltransférases et les thréonine-synthases.

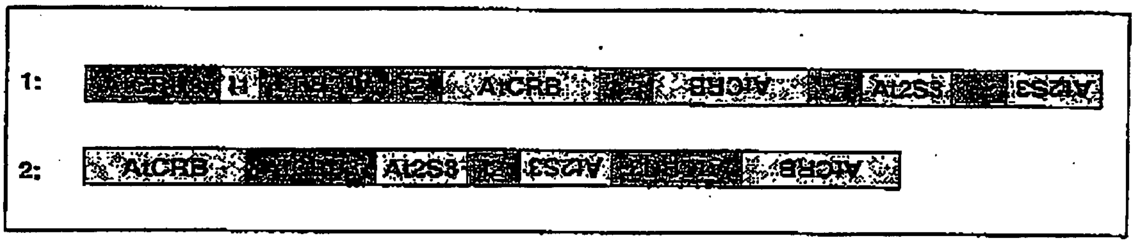

Fig. 1

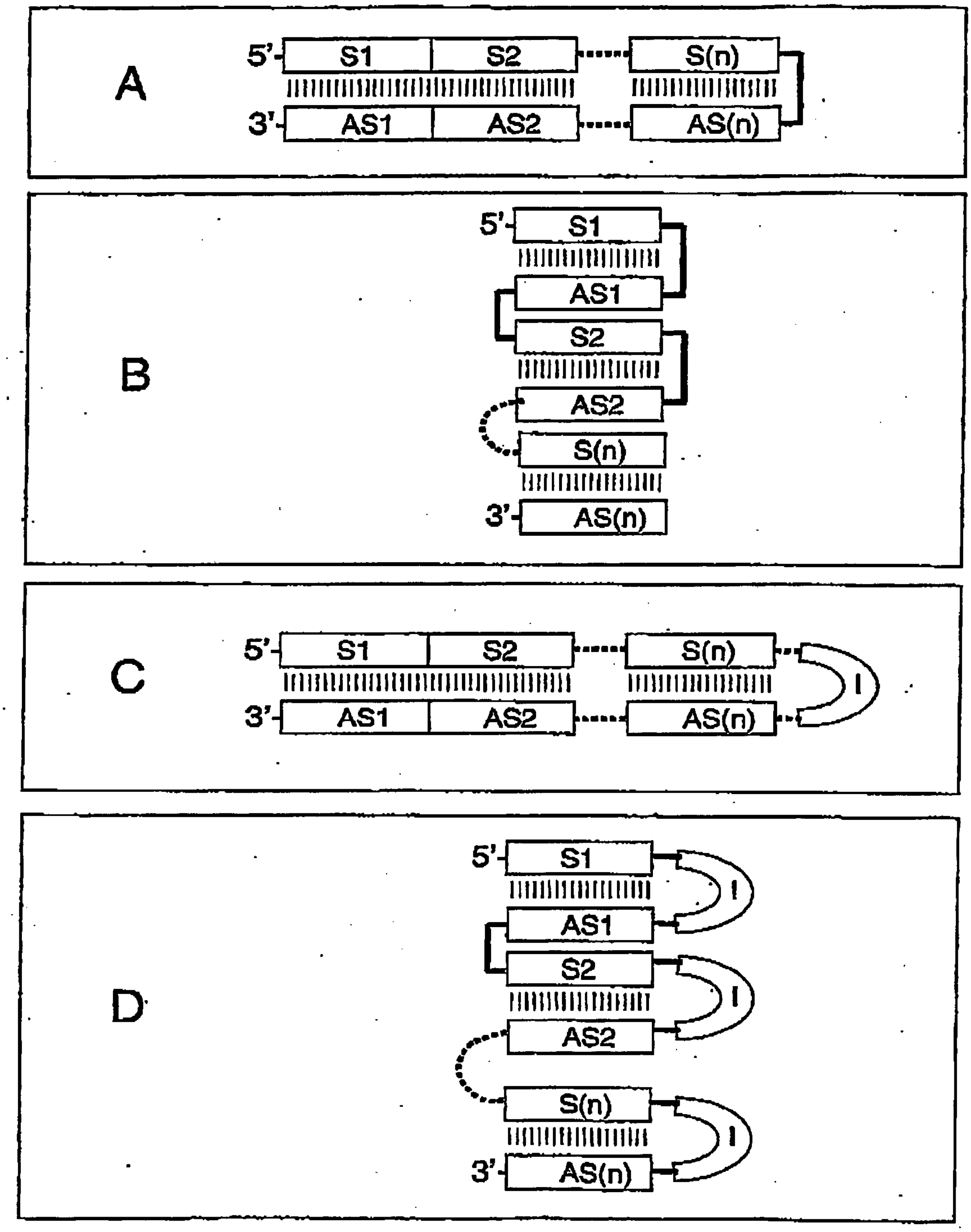
A
5'
S1
S2
S(n)
3'
AS1
AS2
AS(n)
B
5'
S1
AS1
S2
AS2
S(n)
3'
AS(n)
C
5'
S1
S2
S(n)
3'
AS1
AS2
AS(n)
I
D
5'
S1
I
AS1
S2
I
AS2
S(n)
I
3'
AS(n)

Fig.2

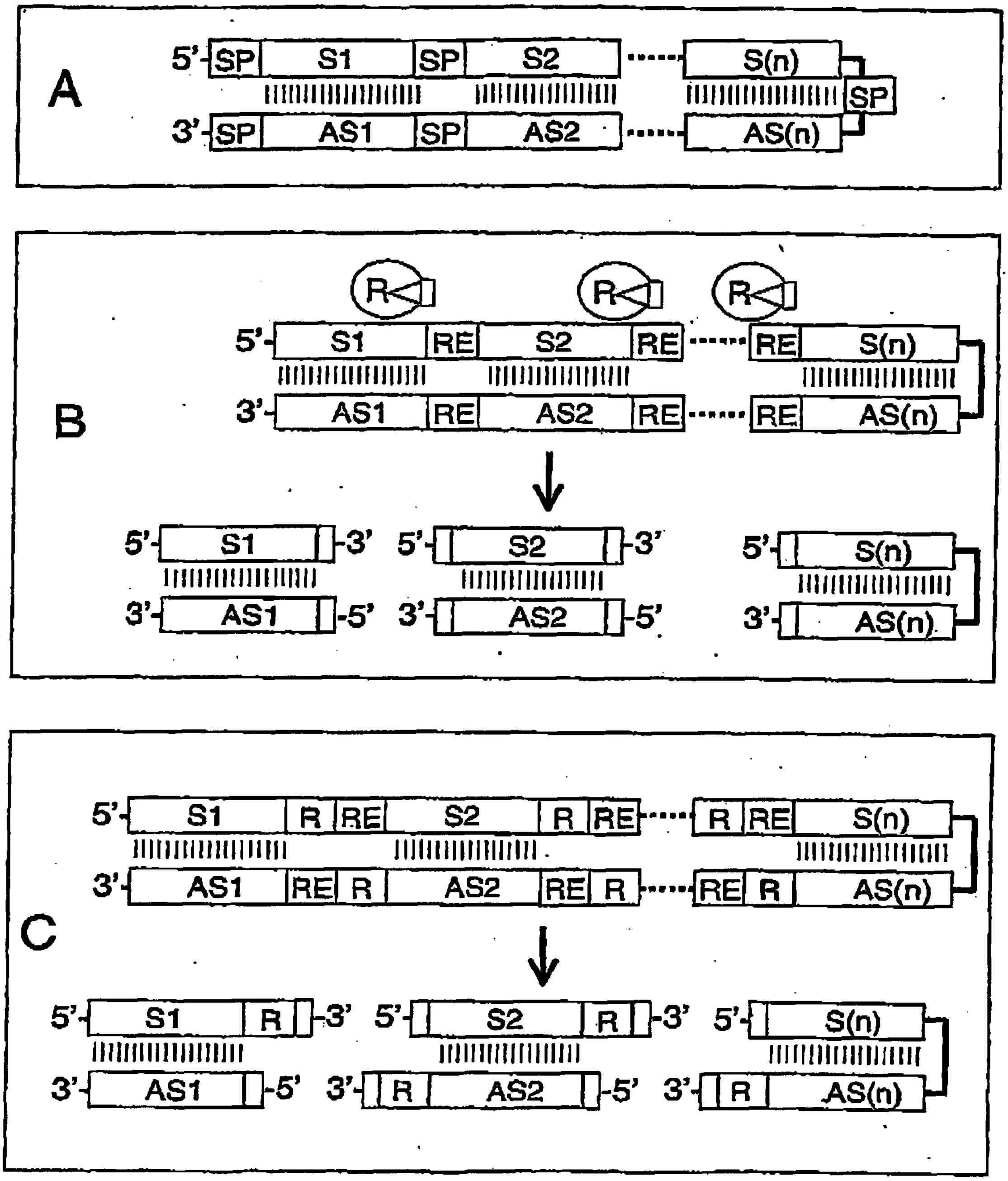
A
5'
SP
S1
SP
S2
S(n)
SP
3'
SP
AS1
SP
AS2
AS(n)
B
R
5'
S1
RE
S2
RE
RE
S(n)
3'
AS1
RE
AS2
RE
RE
AS(n)
5'
S1
3'
5'
S2
3'
5'
S(n)
3'
AS1
5'
3'
AS2
5'
3'
AS(n)
C
5'
S1
R
RE
S2
R
RE
R
RE
S(n)
3'
AS1
RE
R
AS2
RE
R
RE
R
AS(n)
5'
S1
R
3'
5'
S2
R
3'
5'
S(n)
3'
AS1
5'
3'
R
AS2
5'
3'
R
AS(n)

Fig.3

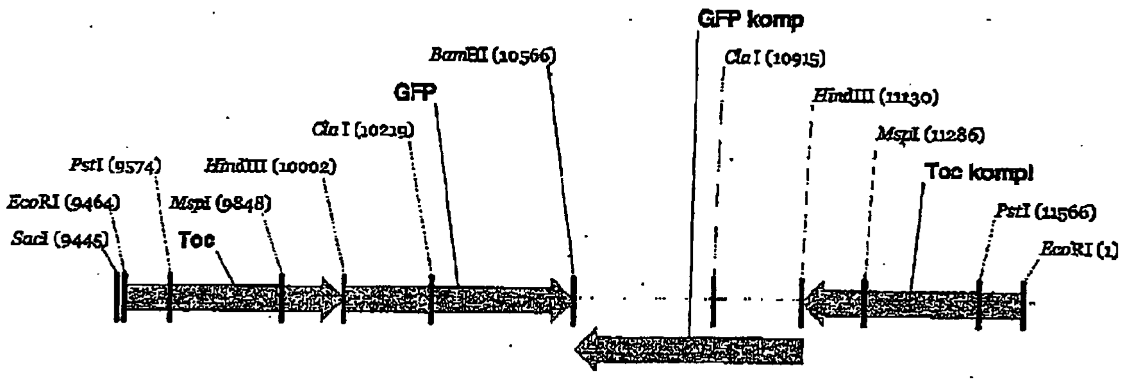
GFP komp
BamHI (10566)
ClaI (10915)
HindIII (11130)
GFP
ClaI (10219)
MspI (11286)
PstI (9574)
HindIII (10002)
Toc kompl
EcoRI (9464)
MspI (9848)
PstI (11566)
SacI (9445)
Toc
EcoRI (1)

Fig.4

A

B

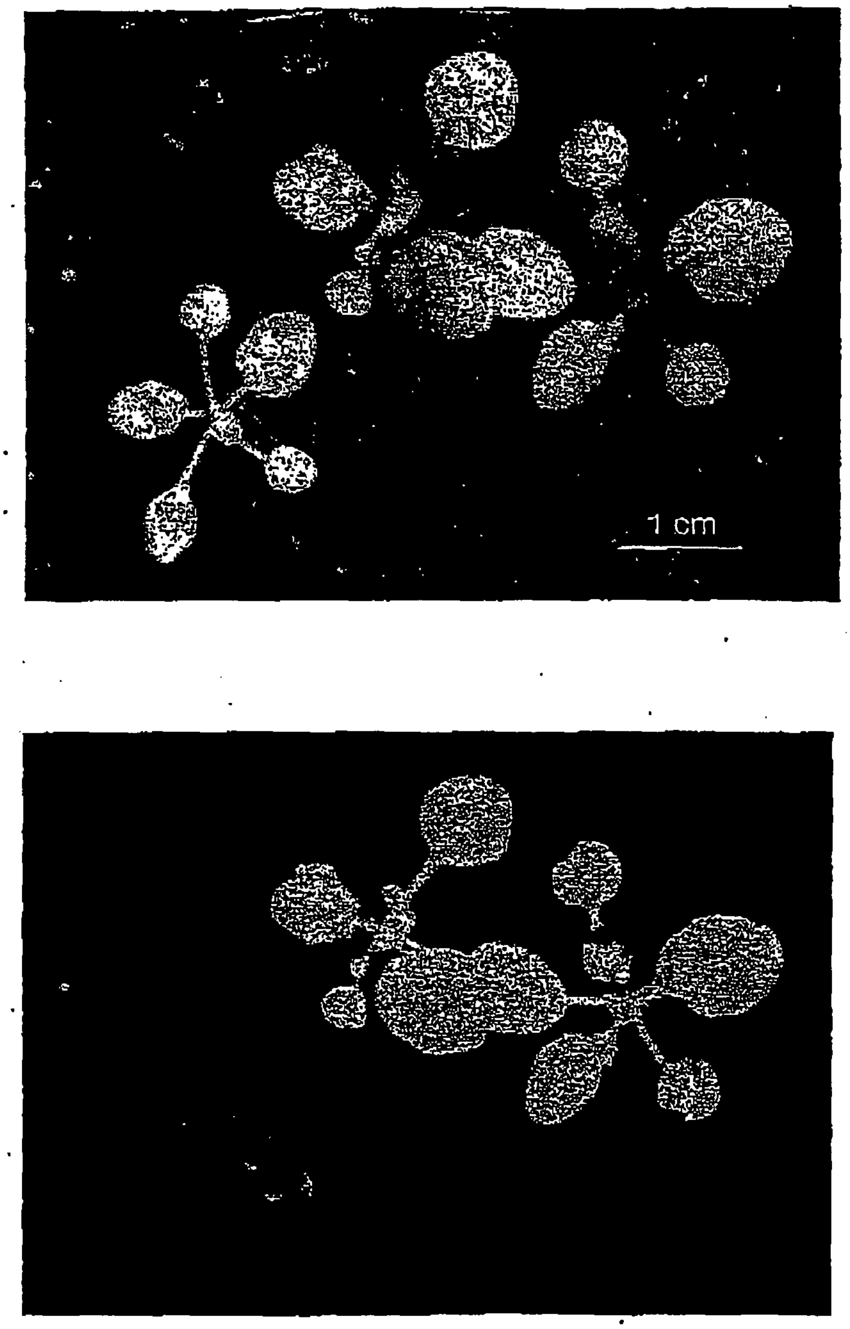


Fig.5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0458367 A1
- EP 0140308 A1
- EP 0465572 A1
- WO 9323551 A
- WO 9836083 A
- WO 9915682 A
- WO 9932619 A
- WO 9953050 A
- EP 0291533 A
- EP 0321201 A
- EP 0360257 A
- US 4987071 A
- US 5116742 A
- WO 9732016 A
- US 5593874 A
- US 5698425 A
- US 5712135 A
- US 5789214 A
- US 5804693 A
- US 5530192 A
- WO 9418337 A
- WO 9211375 A
- WO 9211376 A
- US 5365016 A
- WO 9507355 A
- EP 0591530 A
- WO 8747731 A
- WO 9826064 A
- EP 0620281 A
- WO 9310251 A
- WO 9417194 A
- WO 9713865 A
- WO 9116440 A
- WO 9105865 A
- WO 9116426 A
- WO 9217596 A
- WO 9307275 A
- WO 9204456 A
- WO 9429465 A
- WO 8910396 A
- WO 9218625 A
- WO 9716559 A
- WO 9507993 A
- WO 9305159 A
- WO 9305160 A
- US 5597718 A
- WO 9403607 A
- US 5352605 A
- WO 8402913 A
- US 4962028 A
- US 5683439 A
- WO 9113991 A
- US 5504200 A
- US 5608152 A
- WO 0026388 A
- WO 9845461 A
- WO 9113980 A
- WO 9515389 A
- WO 9523230 A
- WO 9916890 A
- WO 8903887 A
- WO 9705900 A
- WO 9216635 A
- WO 9822593 A
- US 5689049 A
- US 5689051 A
- WO 9519443 A
- EP 0388186 A
- EP 0335528 A
- WO 9321334 A
- US 5187267 A
- WO 9612814 A
- EP 375091 A
- US 5428148 A
- WO 9421794 A
- EP 409625 A
- WO 9845456 A
- US 4536475 A
- EP 0116718 A
- EP 0067553 A
- US 4407956 A
- WO 9534668 A
- WO 9303161 A
- EP 0270356 A
- WO 8501856 A
- US 4684611 A
- EP 120516 A
- EP 00424047 A
- EP 00397687 A
- US 5376543 A
- US 5169770 A

**In der Beschreibung aufgeführte Nicht-Patentliteratur**


- **VAN DER KROL AR et al.** *BioTechniques,* 1988, vol. 6 (10), 658-676
- **DE LANGE P et al.** *Curr Top Microbiol Immunol,* 1995, vol. 197, 57-75
- **MONTGOMERY MK et al.** *Proc Natl Acad Sci USA,* 1998, vol. 95, 15502-15507
- **SHARP PA.** *Genes & Development,* 1999, vol. 13 (2), 139-141
- **FIRE A et al.** *Nature,* 1998, vol. 391, 806-11
- **GOA J.** *Scand J Clin Lab Invest,* 1953, vol. 5, 218-222
- **LOWRY OH et al.** *J Biol Chem,* 1951, vol. 193, 265-275
- **BRADFORD MM.** *Analyt Biochem,* 1976, vol. 72, 248-254
- **ALTSCHUL et al.** *Nucleic Acids Res.,* 1997, vol. 25, 3389ff
- **SAMBROOK J ; FRITSCH EF ; MANIATIS T et al.** Molecular Cloning (A Laboratory Manual). Cold Spring Harbor Laboratory Press, 1989, 9.31-9.57
- Current Protocols in Molecular Biology. John Wiley &Sons, 1989, 6.3.1-6.3.6
- **MCCULLOUGH AJ ; SCHULER MA.** *Nuc Acids Res,* 1997, vol. 25, 1071-1077
- **ZUKER ; STIEGLER.** *Nucleic Acids Res,* 1981, vol. 9 (1), 133-48
- **TANNER NK.** *FEMS Microbiol Rev,* 1999, vol. 23 (3), 257-275
- **HASELOFF et al.** *Nature,* 1988, vol. 334, 585-591
- **HASELHOFF ; GERLACH.** *Nature,* 1988, vol. 334, 585-591
- **STEINECKE P et al.** *EMBO J,* 1992, vol. 11 (4), 1525-1530
- **DE FEYTER R et al.** *Mol Gen Genet.,* 1996, vol. 250 (3), 329-338
- **STEINECKE P ; RIBOZYMES et al.** Methods in Cell Biology. Academic Press, Inc, 1995, vol. 50, 449-460
- **BAYLEY CC et al.** *Plant Mol Biol.,* 1992, vol. 18 (2), 353-361
- **LLOYD AM ; DAVIS RW et al.** *Mol Gen Genet.,* 1994, vol. 242 (6), 653-657
- **BARTEL D ; SZOSTAK JW.** *Science,* 1993, vol. 261, 1411-1418
- **NAGEL R ; ARES M.** *RNA,* 2000, vol. 6, 1142-1156
- **MERITT et al.** *Plant Journal,* 1997, vol. 12, 937-943
- **EGOAVIL et al.** *Plant Journal,* 1997, vol. 12, 971-980
- **MANIATIS T ; FRITSCH EF ; SAMBROOK J.** Molecular Cloning: A Laboratory Manual. Cold Spring Harbor Laboratory, 1989
- **SILHAVY TJ ; BERMAN ML ; ENQUIST LW.** Experiments with Gene Fusions. Cold Spring Harbor Laboratory, 1984
- **AUSUBEL FM et al.** Current Protocols in Molecular Biology. Greene Publishing Assoc. and Wiley Interscience, 1987
- **GELVIN et al.** Plant Molecular Biology Manual. 1990
- **SAUER B.** *Methods.,* 1998, vol. 14 (4), 381-92
- **KEOWN et al.** *Methods in Enzymology,* 1990, vol. 185, 527-537
- **BILANG et al.** *Gene,* 1991, vol. 100, 247-250
- **SCHEID et al.** *Mol Gen Genet,* 1991, vol. 228, 104-112
- **GUERCHE et al.** *Plant Science,* 1987, vol. 52, 111-116
- **NEUHAUSE et al.** *Theor Appl Genet,* 1987, vol. 75, 30-36
- **KLEIN et al.** *Nature,* 1987, vol. 327, 70-73
- **HOWELL et al.** *Science,* 1980, vol. 208, 1265
- **HORSCH et al.** *Science,* 1985, vol. 227, 1229-1231
- **DEBLOCK et al.** *Plant Physiology,* 1989, vol. 91, 694-701
- Methods for Plant Molecular Biology. Academic Press Inc, 1988
- Methods in Plant Molecular Biology. Academic Press Inc, 1989
- *Indian Chem Engr. Section B.,* 1995, vol. 37 (1,2), 15
- **SHURE M et al.** *Cell,* 1983, vol. 35, 225-233
- **PREISS et al.** Tailoring Genes for Crop Improvement. Plenum Press, 1987, 133-152
- **GUPTA et al.** *Plant Mol Biol.,* 1988, vol. 10, 215-224
- **OLIVE et al.** *Plant Mol Biol,* 1989, vol. 12, 525-538
- **BHATTACHARYYA et al.** *Cell,* 1990, vol. 60, 155-122
- **DUNWELL JM.** *J Exp Botany,* 2000, vol. 51 (487-96
- **BRAR DS et al.** *Biotech Genet Eng Rev,* 1996, vol. 13, 167-79
- **KISHORE GM ; SOMERVILLE CR.** *Curr Opin Biotech,* 1993, vol. 4 (2), 152-8
- **KOHNO-MURASE J et al.** *Plant Mol Biol,* 1994, vol. 26 (4), 1115-1124
- **CORREIA JJ.** *Methods in Enzymology,* 2000, vol. 321, 81-100
- **ZEH M et al.** *Plant Physiol,* 2001, vol. 127 (3), 792-802
- **DUNWELL JM.** Transgenic approaches to crop improvement. *J Exp Bot.,* 2000, vol. 51, 487-96
- **BENFEY et al.** *EMBO J,* 1989, vol. 8, 2195-2202
- **FRANCK et al.** *Cell,* 1980, vol. 21, 285-294

- **ODELL et al.** *Nature,* 1985, vol. 313, 810-812
- **SHEWMAKER et al.** *Virology,* 1985, vol. 140, 281-288
- **GARDNER et al.** *Plant Mol Biol,* 1986, vol. 6, 221-228
- **HOLTORF S et al.** *Plant Mol Biol,* 1995, vol. 29, 637-649
- **CHRISTENSEN et al.** *Plant Mol Biol,* 1992, vol. 18, 675-689
- **BRUCE et al.** *Proc Natl Acad Sci USA,* 1989, vol. 86, 9692-9696
- **BUSTOS MM et al.** *Plant Cell,* 1989, vol. 1 (9), 839-53
- **JOSEFFSON LG et al.** *J Biol Chem,* 1987, vol. 262, 12196-12201
- **SHIRSAT A et al.** *Mol Gen Genet,* 1989, vol. 215 (2), 326-331
- **BÄUMLEIN H et al.** *Mol Gen Genet,* 1991, vol. 225 (3), 459-67
- **STALBERG K et al.** *L Planta,* 1996, vol. 199, 515-519
- **BÄUMLEIN H et al.** *Mol Gen Genet,* 1991, vol. 225, 121-128
- **BAEUMLEIN et al.** *Plant Journal,* 1992, vol. 2 (2), 233-9
- **FIEDLER U et al.** *Biotechnology,* 1995, vol. 13 (10), 1090f
- **STOCKHAUS et al.** *EMBO J,* 1989, vol. 8, 2445-2451
- **GATZ et al.** *Annu Rev Plant Physiol Plant Mol Biol,* 1997, vol. 48, 89-108
- **WARD et al.** *Plant Mol Biol,* 1993, vol. 22, 361-366
- **GATZ et al.** *Plant J,* 1992, vol. 2, 397-404
- **REDOLFI et al.** *Neth J Plant Pathol,* 1983, vol. 89, 245-254
- **UKNES et al.** *The Plant Cell,* 1992, vol. 4, 645-656
- **VAN LOON.** *Plant Mol Viral,* 1985, vol. 4, 111-116
- **MARINEAU et al.** *Plant Mol Biol,* 1987, vol. 9, 335-342
- **MATTON et al.** *Molecular Plant-Microbe Interactions,* 1987, vol. 2, 325-342
- **SOMSSICH et al.** *Proc Natl Acad Sci USA,* 1986, vol. 83, 2427-2430
- **SOMSSICH et al.** *Mol Gen Genetics,* 1988, vol. 2, 93-98
- **CHEN et al.** *Plant J,* 1996, vol. 10, 955-966
- **ZHANG ; SING.** *Proc Natl Acad Sci USA,* 1994, vol. 91, 2507-2511
- **WARNER et al.** *Plant J,* 1993, vol. 3, 191-201
- **SIEBERTZ et al.** *Plant Cell,* 1989, vol. 1, 961-968
- **RYAN.** *Ann Rev Phytopath,* 1990, vol. 28, 425-449
- **DUAN et al.** *Nat Biotech,* 1996, vol. 14, 494-498
- **STANFORD et al.** *Mol Gen Genet,* 1989, vol. 215, 200-208
- **MCGURL et al.** *Science,* 1992, vol. 225, 1570-1573
- **ROHMEIER et al.** *Plant Mol Biol,* 1993, vol. 22, 783-792
- **ECKELKAMP et al.** *FEBS Letters,* 1993, vol. 323, 73-76
- **CORDEROK et al.** *The Plant J,* 1994, vol. 6 (2), 141-150
- **LAM E ; CHUA NH.** *J Biol Chem,* 1991, vol. 266 (26), 17131-17135
- **SCHOFFL F et al.** *Molecular & General Genetics,* 1989, vol. 217 (2-3), 246-53
- The Maize Handbook. Springer, 1994
- **GALLIE et al.** *Nucl Acids Res,* 1987, vol. 15, 8693-8711
- **ROUSTER J et al.** *Plant J,* 1998, vol. 15, 435-440
- **GIELEN et al.** *EMBO J,* 1984, vol. 3, 835 ff
- **SCHENBORN E ; GROSKREUTZ D.** *Mol Biotechnol.,* 1999, vol. 13 (1), 29-44
- **SHEEN et al.** *Plant Journal,* 1995, vol. 8 (5), 777-784
- **OW et al.** *Science,* 1986, vol. 234, 856-859
- **PRASHER et al.** *Biochem Biophys Res Commun,* 1985, vol. 126 (3), 1259-1268
- **JEFFERSON et al.** *EMBO J,* 1987, vol. 6, 3901-3907
- **SAMBROOK et al.** Molecular Cloning. A Laboratory Manual. Cold Spring Harbor Laboratory Press, 1989
- **MCCORMICK et al.** *Plant Cell Reports,* 1986, vol. 5, 81-84
- Plant Molecular Biology and Biotechnology. CRC Press, 1993, 71-119
- Engineering and Utilization. **WHITE FF.** Vectors for Gene Transfer in Higher Plants; in: Transgenic Plants. Academic Press, 1993, vol. 1, 15-38
- Engineering and Utilization. **JENES B et al.** Techniques for Gene Transfer, in: Transgenic Plants. Academic Press, 1993, vol. 1, 128-143
- **POTRYKUS.** *Annu Rev Plant Physiol Plant Molec Biol,* 1991, vol. 42, 205-225
- **HALFORD NG ; SHEWRY PR.** *Br Med Bull,* 2000, vol. 56 (1), 62-73
- **FROMM ME et al.** *Bio/Technology,* 1990, vol. 8 (9), 833-9
- **GORDON-KAMM et al.** *The Plant Cell,* 1990, vol. 2, 603
- **HORSCH RB et al.** *Science,* 1985, vol. 225, 1229f
- **HOLSTERS et al.** *Mol Gen Genet,* 1978, vol. 163, 181-187
- **HOEKEMA.** The Binary Plant Vector System. Offsetdrukkerij Kanters B.V,
- **AN et al.** *EMBO J,* 1985, vol. 4, 277-287

- **BEVAN et al.** *Nucl Acids Res,* 1984, vol. 12, 8711
- Engineering and Utilization. **WHITE FF.** Vectors for Gene Transfer in Higher Plants; in Transgenic Plants. Academic Press, 1993, vol. 1, 15-38
- **POTRYKUS.** *Annu Rev Plant Physiol Plant Molec Biol,* 1991, vol. 42, 205-225
- **RATHORE KS et al.** *Plant Mol Biol,* 1993, vol. 21 (5), 871-884
- **FENNELL et al.** *Plant Cell Rep.,* 1992, vol. 11, 567-570
- **STOEGER et al.** *Plant Cell Rep.,* 1995, vol. 14, 273-278
- **JAHNE et al.** *Theor Appl Genet,* 1994, vol. 89, 525-533
- **VOET ; VOET.** Phospho-amiditmethode. Wiley Press, 896-897
- **SANGER et al.** *Proo Natl Acad Sci USA,* 1977, vol. 74, 5463-5467
- **OGAS et al.** *Science,* 1997, vol. 277, 91-94
- **FOCKS ; BENNING.** *Plant Physiol,* 1998, vol. 118, 91-101
- **HÖFGEN ; WILLMITZER.** *Plant Science,* 1990, vol. 66, 221-230
- **KONCZ ; SCHELL.** *Mol Gen Genet,* 1986, vol. 204, 383-396
- **DEBLAERE et al.** *Nucl Acids Res,* 1984, vol. 13, 4777-4788
- **GELVIN, STANTON B. ; SCHILPEROORT, ROBERT A.** Plant Molecular Biology Manual. Kluwer Academic Publ, 1995
- **GLICK, BERNARD R. ; THOMPSON, JOHN E.** Methods in Plant Molekular Biology and Biotechnology. CRC Press, 1993, 360
- **BECHTHOLD et al.** *C.R. Acad. Sci. Ser. III Sci. Vie.,* 1993, vol. 316, 1194-1199
- **MOLONEY et al.** *Plant Cell Report,* 1989, vol. 8, 238-242
- **DE BLOCK et al.** *Plant Physiol.,* 1989, vol. 91, 694-701
- **MLYNAROVA et al.** *Plant Cell Report,* 1994, vol. 13, 282-285
- **FREELING ; WALBOT.** The maize handbook. Springer, 1993
- **AUSUBEL et al.** Current Protocols in Molecular Biology. Wiley, 1988
- **BORMANN, E.R. et al.** *Mol. Microbiol.,* 1992, vol. 6, 317-326
- **AMASINO.** *Anal. Biochem.,* 1986, vol. 152, 304
- **AUSUBEL et al.** Current Protocols in Molecular Biology. Wiley
- **ULLMAN.** Encyclopedia of Industrial chemistry. VCH, 1985, vol. A2, 89-90, 443-613
- **FALLON, A. et al.** Applications of HPLC in Biochemistry. *Laboratory Techniques in Biochemistry and Molecular Biology,* 1987, vol. 17
- Product recovery and purification. **REHM et al.** Biotechnology. VCH, 1993, vol. 3, 469-714
- **BELTER, P.A. et al.** Bioseparations: downstream processing for Biotechnology. John Wiley and Sons, 1988
- **KENNEDY, J.F. ; CABRAL, J.M.S.** Recovery processes for biological Materials. John Wiley and Sons, 1992
- Biochemical Separations. **SHAEIWITZ, J.A. ; HENRY, J.D.** Ullmann's Encyclopedia of Industrial Chemistry. VCH, 1988, vol. B3, 1-27
- **DECHOW, F.J.** Separation and purification techniques in biotechnology. Noyes Publications, 1989
- **CAHOON et al.** *Proc. Natl. Acad. Sci. USA,* 1999, vol. 96 (22), 12935-12940
- **BROWSE et al.** *Analytic Biochemistry,* 1986, vol. 152, 141-145
- **CHRISTIE, WILLIAM W.** Advances in Lipid Methodology. Oily Press
- **CHRISTIE, WILLIAM W.** Gas Chromatography and Lipids. A Practical Guide - Ayr. Oily Press, 1989
- Repr. Oily Press Lipid Library, 1992, vol. IX, 307
- Progress in Lipid Research. Pergamon Press, 1952, vol. 1
- PROGRESS IN LIPID RESEARCH. 1977, vol. 16
- Applied Microbial Physiology. A Practical Approach. IRL Press, 103-129, 131-163, 165-192
- Literaturstellen darin. **CHRISTIE.** Advances on Lipid Methodology, Vierte. Oily Press, 1997, 119-169
- Gaschromatographie-Massenspektrometrie-Verfahren. *Lipide,* 1998, vol. 33, 343-353
- **BLIGH ; DYER.** *Can J Biochem Physiol,* 1959, vol. 37, 911
- **FRASER ; TAGGART.** *J. Chromatogr.,* 1988, vol. 439, 404
- **BAUER et al.** *Nature,* vol. 403, 203-207
- **STEWART.** *Plant Cell Rep,* 2001, vol. 20 (5), 376-82
- **ANDREAS HILBRUNNER.** *Dissertation ETH Zürich,* 2003
- **BECHTOLD et al.** *C.R. Acad. Sci. Ser. III Sci. Vie.,* 1993, vol. 316, 1194-1199